(19) Europäisches Patentamt
European Patent Office
Office européen des brevets

(11) EP 2 047 861 B1

(12) EUROPEAN PATENT SPECIFICATION

(45) Date of publication and mention
of the grant of the patent:
**31.07.2019 Bulletin 2019/31**

(51) Int Cl.:
**A61K 39/21** (2006.01)     **C07K 14/16** (2006.01)
**C12N 15/62** (2006.01)     **C12N 15/867** (2006.01)

(21) Application number: **07291251.2**

(22) Date of filing: **12.10.2007**

(54) **Lentiviral gene transfer vectors suitable for iterative administration and their medicinal applications**

Für wiederholte Verabreichung geeignete lentivirale Gentransfervektoren und deren medizinische Anwendung

Vecteurs de transfert de gène de lentivirus convenant à une administration répétée et leurs applications médicinales

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**15.04.2009 Bulletin 2009/16**

(73) Proprietors:
• **INSTITUT PASTEUR**
**75724 Paris Cedex 15 (FR)**
• **CENTRE NATIONAL DE**
**LA RECHERCHE SCIENTIFIQUE -CNRS-**
**75794 Paris Cedex 16 (FR)**

(72) Inventors:
• **Charneau, Pierre**
**75005 Paris (FR)**
• **Beignon, Anne-Sophie**
**75012 Paris (FR)**
• **Courbeyrette, Karine**
**91300 Massy (FR)**

(74) Representative: **Desaix, Anne et al**
**Ernest Gutmann - Yves Plasseraud S.A.S.**
**66, rue de la Chaussée d'Antin**
**75009 Paris (FR)**

(56) References cited:
**WO-A-2004/110485     WO-A-2007/054792**
**WO-A-2007/071997**

• BALIGA ET AL: "Vaccination of Mice with Replication-Defective Human Immunodeficiency Virus Induces Cellular and Humoral Immunity and Protects against Vaccinia Virus-gag Challenge" MOLECULAR THERAPY, ACADEMIC PRESS, SAN DIEGO, CA,, US, vol. 14, no. 3, 12 August 2006 (2006-08-12), pages 432-441, XP005844837 ISSN: 1525-0016
• IGLESIAS MARIA CANDELA ET AL: "Lentiviral vectors encoding HIV-1 polyepitopes induce broad CTL responses invivo" MOLECULAR THERAPY, ACADEMIC PRESS, SAN DIEGO, CA,, US, vol. 15, no. 6, June 2007 (2007-06), pages 1203-1210, XP008087284 ISSN: 1525-0016
• DELENDA C: "LENTIVIRAL VECTORS: OPTIMIZATION OF PACKAGING, TRANSDUCTION AND GENE EXPRESSION" JOURNAL OF GENE MEDICINE, WILEY,, US, vol. 6, no. SUPPL 1, February 2004 (2004-02), pages S125-S138, XP008051607 ISSN: 1099-498X
• PICHLMAIR ANDREAS ET AL: "Tubulovesicular structures within vesicular stomatitis virus G protein-pseudotyped lentiviral vector preparations carry DNA and stimulate antiviral responses via toll-like receptor 9" JOURNAL OF VIROLOGY, vol. 81, no. 2, January 2007 (2007-01), pages 539-547, XP002473033 ISSN: 0022-538X
• KEIL W ET AL: "EPITOPE MAPPING BY DELETION MUTANTS AND CHIMERAS OF TWO VESICULAR STOMATITIS VIRUS GLYCOPROTEIN GENES EXPRESSED BY A VACCINIA VIRUS VECTOR" VIROLOGY, vol. 170, no. 2, 1989, pages 392-407, XP002473034 ISSN: 0042-6822

EP 2 047 861 B1

- ROLLMAN ET AL: "The rationale behind a vaccine based on multiple HIV antigens" MICROBES AND INFECTION, ELSEVIER, PARIS, FR, vol. 7, no. 14, November 2005 (2005-11), pages 1414-1423, XP005171715 ISSN: 1286-4579
- BUFFA VIVIANA ET AL: "Evaluation of a self-inactivating lentiviral vector expressing simian immunodeficiency virus gag for induction of specific immune responses in vitro and in vivo." VIRAL IMMUNOLOGY WINTER 2006, vol. 19, no. 4, January 2006 (2006-01), pages 690-701, XP002482736 ISSN: 0882-8245
- CHEN ET AL: "Induction of primary anti-HIV CD4 and CD8 T cell responses by dendritic cells transduced with self-inactivating lentiviral vectors" CELLULAR IMMUNOLOGY, ACADEMIC PRESS, SAN DIEGO, CA, US, vol. 243, no. 1, 22 February 2007 (2007-02-22), pages 10-18, XP005899486 ISSN: 0008-8749
- DATABASE Geneseq [Online] 16 June 2005 (2005-06-16), "Human codon-optmized HIV B Gag-Pol-Env-Nef fusion protein." XP002482737 retrieved from EBI accession no. GSP:ADY99929 Database accession no. ADY99929 -& WO 2005/028634 A (UNIV EMORY [US]; FEINBERG MARK M D [US]; GARBER DAVID [US]) 31 March 2005 (2005-03-31)
- DATABASE Geneseq [Online] 29 January 2004 (2004-01-29), "gag-IApol fusion protein." XP002482738 retrieved from EBI accession no. GSP:ADE71160 Database accession no. ADE71160 & WO 02/22080 A (MERCK & CO INC [US]; EMINI EMILIO A [US]; YOUIL RIMA [US]; BETT ANDREW) 21 March 2002 (2002-03-21)
- DATABASE Geneseq [Online] 16 May 2003 (2003-05-16), "Human immunodeficiency virus (HIV) TRN-CTL-dgag fusion protein." XP002482739 retrieved from EBI accession no. GSP:AAE34803 Database accession no. AAE34803 -& MALM MARIA ET AL: "CROSS-CLADE PROTECTION INDUCED BY HUMAN IMMUNODEFICIENCY VIRUS-1 DNA IMMUNOGENS EXPRESSING CONSENSUS SEQUENCES OF MULTIPLE GENES AND EPITOPES FROM SUBTYPES A, B, C, AND FGH" VIRAL IMMUNOLOGY, MARY ANN LIEBERT, INC., NEW YORK, US, vol. 18, no. 4, 1 January 2005 (2005-01-01), pages 678-688, XP009081289 ISSN: 0882-8245 & WO 02/090558 A (FIT BIOTECH OYJ PLC [FI]; KROHN KAI [FI]; BLAZEVIC VESNA [FI]; TAEHTIN) 14 November 2002 (2002-11-14)
- DATABASE Geneseq [Online] 19 April 2007 (2007-04-19), "HIV-1 isolate CN54-derived gp41 Gag-Pol-Nef fusion protein, SEQ:18." XP002482740 retrieved from EBI accession no. GSP:AER57327 Database accession no. AER57327 & WO 2007/012691 A (CONSEJO SUPERIOR INVESTIGACION [ES]; HEENEY JONATHAN [NL]; MOOIJ PETRA) 1 February 2007 (2007-02-01)
- DATABASE Geneseq [Online] 27 August 2003 (2003-08-27), "HIV-1 GrttnC protein construct." XP002482741 retrieved from EBI accession no. GSP:AAE37597 Database accession no. AAE37597 -& BURGERS W A ET AL: "Design and preclinical evaluation of a multigene human immunodeficiency virus type 1 subtype C DNA vaccine for clinical trial" JOURNAL OF GENERAL VIROLOGY, SOCIETY FOR GENERAL MICROBIOLOGY, SPENCERS WOOD, GB, vol. 87, no. 2, 1 January 2006 (2006-01-01), pages 399-410, XP002423823 ISSN: 0022-1317 & WO 03/037919 A (SOUTH AFRICAN MEDICAL RES COUN [ZA]; UNIV CAPE TOWN [ZA]; WILLIAMSON C) 8 May 2003 (2003-05-08)

**Description**

The invention relates to the design of gene transfer vectors as defined in the claims suitable for iterative administration in a host, and to their medicinal application.

The invention especially relates to the use of such gene transfer vectors for multiple *in vivo* administration into a host in need thereof. The field of application of the present application concerns in particular animal treatment or treatment of human being.

The disclosure thus provides means suitable for use of the lentiviral vectors in iterative administration, either for prevention or for treatment of a disease in a mammalian host, especially in human beings. A particular application of these vectors is to elicit an immune response to prevent or to treat a pathogenic state, including virus infections, and preferably to elicit a protective, long-lasting immune response.

Viruses, in particular RNA-viruses, and especially lentiviruses have been used in the past to design gene transfer vectors especially due to the ability of lentiviruses to achieve mitosis-independent nuclear import that enables them to replicate efficiently in non dividing target cells. Accordingly, lentivirus based vectors have been explored for various applications including prophylactic or therapeutic vaccination or with a view to use these vectors as tools for gene therapy.

When testing lentiviral vectors *in vivo,* it has however been observed that the number of *in vivo* injections is limited by the humoral response of the host elicited against the envelope protein used for pseudotyping the vector particles.

The response which is elicited in the host against the envelope of the pseudotyped vector particles is accordingly a drawback for the efficient use of such vectors, when *in vivo* multiple administrations are required.

The present invention proposes means that are intended to remedy, at least in part, to the drawbacks due to the immune response against the envelope of the pseudotyped vector particles, when administrated several times to a host in the context of prophylaxy or treatment.

The invention thus relates to different structures of lentiviral vectors as defined in the claims, and especially to their association in a combination of compounds (also designated as a kit of compounds), for use in a host in need thereof, in conditions allowing iterative administration of said lentiviral vectors.

Accordingly, the invention takes advantage of the sequential use of different lentiviral vectors to deliver a transgene in a host.

The lentiviral vectors according to the invention and especially their combination, is in particular suitable for use in the field of medicinal treatment where especially an immune response, including a cellular immune response, elicited by endogenously expressed antigen is beneficial or necessary; accordingly, the invention provides tools for the design of vaccination protocols for use in hosts in need of preventive or curative treatment against retroviruses, or more generally against a pathogenic state, including to perform gene therapy *in vivo.* It is in particular suitable for any medicinal treatment which requires *in vivo* multiple injections of the vectors.

The inventors have in particular provided evidence that the lentiviral vectors as defined herein, especially when used in a combination, are appropriate to elicit a cellular immune response in a non-human primate model, which may be protective in the context of viral challenge, when the lentiviral vectors express an antigen of said virus.

The inventors have especially shown that a cellular protective immune response has been obtained in a non-human primate model in the context of viral challenge with Simian Immunodeficiency Virus.

In view of these results, the inventors have designed tools which would be suitable to elicit an efficient and preferably protective immune response when administered to a host, especially in situations of prevention or treatment of viral infections and in particular in human hosts, to provide an immune response against Human Immunodeficiency Virus.

Accordingly, the combination of lentiviral vectors of the invention, provides especially an efficient prime-boost system for use for iterative administration, enabling successively priming and boosting the immune response in a host, especially after injections in a host in need thereof. "Iterative" means that the active principle, i.e., the heterologous polynucleotide contained in the lentiviral vector of the invention is administered twice or more, especially three times, to the host, as a result of the administration of lentiviral vectors disclosed herein.

The disclosure accordingly discloses a combination of compounds comprising at least:

(i) lentiviral vector particle (also designated lentiviral vectors), pseudotyped with a first determined heterologous viral envelope protein or viral envelope proteins;
(ii) lentiviral vector particle (also designated as "lentiviral vectors"), pseudotyped with a second determined heterologous viral envelope protein or viral envelope proteins different from said first determined envelope protein or envelope proteins;

wherein said lentiviral vector particles of (i) and (ii) encode a heterologous determined polynucleotide which is a recombinant polynucleotide (or transgene) encoding one or several polypeptides and;

wherein said first and second viral envelope protein(s) do not sero-neutralize with each other and are suitable for *in vivo* transduction of mammalian cells.

The invention relates to a lentiviral vector comprising a recombinant polynucleotide encoding one or several polypeptides comprising at least one antigen for use in treatment in a prime-boost regimen by eliciting an immune response in a host against said polypeptides , wherein said antigen is a chimeric HIV-1 derived antigen which is a fusion protein comprising or consisting in the combination of the GAG derived antigen having a sequence chosen among the following:

MASVLSGGELDRWEKIRLRPGGKKKYKLKHIVWASRELERFAVNPGLLETSEGCRQ
ILGQLQPSLQTGSEELRSLYNTVATLYCVHQRIEVKDTKEALDKIEEEQNKSKKKAQ
QAAADTNHSSQVSQNYPIVQNLQGQMVHQAISPRTLNAWVKVVEEKAFSPEVIPMF
SALSEGATPQDLNTMLNTVGGHQAAMQMLKETINEEAAEWDRLHPVHAGPIAPGQ

MREPRGSDIAGTTSTLQEQIGWMTNNPPIPVGEIYKRWIILGLNKIVRMYSPTSILDIR
QGPKEPFRDYVDRFYKTLRAEQASQEVKNWMTETLLVQNANPDCKTILKALGPAAT
LEEMMTACQGVGGPGHKARVLAEAMSQVTNSATIMMQRGNFRNQRKTVKCFNCG
KEGHIAKNCRAPRKKGCWKCGKEGHQMKDCTERQANFLGKIWPSHKGRPGNFLQ
SRPEPTAPPEESFRFGEETTTPSQKQEPIDKELYPLASLRSLFGND, or

MGARASVLSGGELDRWEKIRLRPGGKKKYKLKHIVWASRELERFAVNPGLLETSEG
CRQILGQLQPSLQTGSEELRSLYNTVATLYCVHQRIEIKDTKEALDKIEEEQNKSKKK
AQQAAADTGHSSQVSQNYPIVQNIQGQMVHQAISPRTLNAWVKVVEEKAFSPEVIP
MFSALSEGATPQDLNTMLNTVGGHQAAMQMLKETINEEAAEWDRVHPVHAGPIAP
GQMREPRGSDIAGTTSTLQEQIGWMTNNPPIPVGEIYKRWIILGLNKIVRMYSPTSIL
DIRQGPKEPFRDYVDRFYKTLRAEQASQEVKNWMTETLLVQNANPDCKTILKALGP
AATLEEMMTACQGVGGPGHKARVLAEAMSQVTNSATIMMQRGNFRNQRKIVKCFN
CGKEGHIARNCRAPRKKGCWKCGKEGHQMKDCTERQANFLGKIWPSYKGRPGNF
LQSRPEPTAPPFLQSRPEPTAPPEESFRSGVETTTPSQKQEPIDKELYPLTSLRSLF
GNDPSSQ

with an antigen derived from NEF, POL, TAT or REV of a HIV-1 virus strain or with a combination of such antigens.

In the invention, the heterologous determined polynucleotide encodes one or several polypeptides comprising at least one antigen derived from a GAG antigen of HIV1. The antigen derived from GAG is defined in the present application and illustrated in the examples. It encompasses in particular fragments of GAG.

The disclosed heterologous determined polynucleotide which is a recombinant polynucleotide (or transgene) encoding one or several polypeptides may encode a non biologically active POL antigen of an Immunodeficiency Virus.

The encoded antigen derived from GAG may be a non biological functional GAG antigen and may not comprise such a biologically functional GAG; in other words the antigen is a biologically non functional GAG.

The lentiviral vectors defined herein are pseudotyped lentiviral vectors consisting of vector particles (accordingly also designated as "lentiviral vector particles") bearing envelope protein or envelope proteins (of a particular polyprotein envelope), wherein said envelope protein(s) originate from a virus which is different from the particular lentivirus which provides the vector genome of the lentiviral vector. Accordingly, said envelope protein or envelope proteins, are so-called "heterologous viral envelope protein or viral envelope proteins". In the following pages, reference will also be made to "envelope protein(s)" to encompass any type of envelope protein or envelope proteins suitable to perform the invention.

The lentiviral vectors defined herein are replacement vectors, meaning that the sequences of the original lentivirus encoding the lentiviral proteins are essentially deleted from the genome of the vector or, when present, are modified, and especially prevent expression of biologically active POL antigen and optionally of further structural and/or accessory and/or regulatory proteins of the lentivirus.

The "vector genome" of the vector particles also comprises the polynucleotide or transgene of interest. In a particular aspect of the diclosure, said transgene is also devoid of a polynucleotide encoding biologically active POL proteins. As a consequence, the vector genome does not enable to recover biologically active POL antigens. A biologically active POL antigen comprises the viral enzymes protease (RT), reverse tanscriptase (RT and RNase H) and integrase (IN) produced by cleavage of the GAG-POL polyprotein. The POL antigen is not biologically active, when the biological activity of at least one of these enzymes is not enabled. The biological activity of these enzymes is described with thses enzymes in Fields (Virology - Vol 2 Chapter 60, pages 1889-1893 Edition 1996).

In a particular aspect of the disclosure, the polynucleotide in the vector genome is devoid of the functional pol gene, and especially does not contain a complete pol gene.

The vector genome as defined herein contains, apart from the heterologous polynucleotide of therapeutic interest placed under control of proper regulatory sequences, the sequences of the lentiviral genome which are non-coding regions of said genome, and are necessary to provide recognition signals for DNA or RNA synthesis and processing. These sequences are cis-acting sequences.

Unless otherwise stated, or unless technically not relevant, the characteristics disclosed in the present application with respect any of to the various features, embodiments or examples of the structure or use of the lentiviral vectors, especially regarding their envelope protein(s), or the heterologous polynucleotide, may be combined according to any possible combinations.

The expression "combination of compounds" or "kit of compounds" means that the lentiviral vectors constituting active ingredients of the kits or combinations, are provided as separate compounds in said kit or combination, and are intended for separate administration to a host, especially separate administration in time. Accordingly the invention enables to perform a prime-boost administration in a host in need thereof, where the first administration step elicits an immune, especially cellular, immune response and the later administration step(s) boost(s) the immune reaction.

The compounds of the kit thus are provided separately to the host in need thereof, especially to a mammalian host, in particular a human patient.

Accordingly, said lentiviral vectors can be provided in separate packages or can be presented in a common package for a separate use thereof.

Therefore, the notice included in the packages and comprising the directions for use, may indicate that said lentiviral vector particles which are pseudotyped with distinct envelope protein or envelope proteins are for separate administration in time, especially for priming and subsequently boosting an immune reaction in a host.

In accordance with the disclosure, it is provided lentiviral vector particles which are pseudotyped with a first determined heterologous viral envelope protein, or viral envelope proteins, and lentiviral viral vector particles which are pseudotyped with a second determined heterologous viral envelope protein or viral envelope proteins. Accordingly, said first and second heterologous viral envelope protein(s) are different and in particular are originating from different virus strains. Thus, the lentiviral vector particles of the kit of compounds of the disclosure are distinct, at least due to the particular envelope protein(s) used for pseudotyping the vector particles.

In a particular aspect of the disclosure, the combination of compounds comprises a third or a further type of lentiviral vector particles wherein the envelope protein(s) of the third lentiviral vector is different from said first and second envelope protein(s) and especially originates from a different virus strain.

Apart from their pseudotyping envelope protein(s), the lentiviral vectors of the invention may be identical and especially may have identical vector genomes.

Alternatively, their vector genomes may be different, provided they carry the same heterologous determined polynucleotide (also designated as transgene), especially the same polynucleotide having a therapeutic interest.

In another aspect of the disclosure, the vector genomes of the lentiviral vectors are different by having a different polynucleotide, provided said different polynucleotides encode polypeptides having common antigenic determinants, or common epitopes. Hence the different polynucleotides may be variants from each other.

As specified above, the expression "vector genome" refers to the nucleic acid which constitutes the genome of the lentiviral vector particles. Accordingly the expression relates to any appropriate nucleic acid, i.e., DNA or RNA, either double or single stranded, including in the form containing the DNA flap as a triplex sequence. The nature of the nucleic acid (DNA, RNA) and its organization depend upon the stage of the cycle of the particles, and includes the vector plasmid - used for cotransfection of cells with the encapsidation plasmid and the envelope plasmid - for expression of the particles, or the RNA genome of the particles when formed, or the various forms (including the genomic mRNA transcript, linear unintegrated DNA retrotranscript, or unintegrated one or two LTR DNA circular forms or integrated proviruses) (see in Fields Virology) of nucleic acid of this genome in the transduced cells of the host to whom particles are administered, including the vector pre-integration complex.

As a result of administration of particles to the host, the heterologous polynucleotide allows endogeneous expression of the polypeptides that it encodes in the cells of the host that are transduced by the lentiviral vectors.

Said first and second viral and if any said third and possibly further, envelope protein(s), are selected for their capacity not to sero-neutralize with each other (i.e., not to cross-react). Accordingly, each of said first and second viral and if any said third or further, envelope protein(s), used for pseudotyping the vector particles in the combination, does not react with and especially is not recognized by antibodies directed against the other of said first and second and if any said third or further, envelope protein(s). Accordingly, each of said first and second and if any said third or further, viral envelope protein(s), when administered within a lentiviral vector, does not elicit the production of antibodies, that recognize the other viral envelope protein(s) where such production of said anti-envelope antibodies (so-called antivector immunity) would result in a failure to elicit an immune response against the product expressed from the polynucleotide.

In the disclosed kit of compounds, said first and second viral and if any said third or further, envelope protein(s) originate from human viruses, either DNA or RNA viruses.

In he disclosed kit of compounds, said first and second and if any said third or further, envelope protein(s) originate from viruses of the same virus family.

In accordance with an aspect of the disclosure, said first and second envelope viral protein(s) originate from different strain types of the same virus, or from non cross-reactive serotypes of the same virus.

In another aspect of the disclosure of said kit of compounds, said first and second and if any said third or further, envelope protein(s) originate from viruses of different genus.

In another aspect of the disclosure of said kit of compounds, said first and second and if any said third or further, envelope protein(s) originate from the same genus or from the same serotype but from different strain types, or from non cross-reactive serotypes of the virus.

The disclosure relates to a kit of compounds, wherein said first and second and if any said third or further, viral envelope protein or viral envelope proteins originate from *Rhabdoviridae* (including Rabies), especially from a *Vesiculovirus*, including Vesicular Stomatitis Virus (VSV) from *Paramyxoviridae*, especially from Measles Virus (MV) Respiratory Syncytia Virus (RSV), or from non-human retroviruses or from Orthomyxoviridae such as Influenza virus.

The above-cited viruses are RNA-viruses, capable of infecting mammalian hosts, especially human hosts. Some of them, such as viruses of the order of *Mononegavirales*, and especially viruses of the family of *Rabdoviridae* in particular of the genus of *Vesiculoviruses* in particular VSV have been proposed to provide envelope protein(s), also designated as surface proteins, to pseudotype viral vectors, especially lentiviral vector particles.

The glycoprotein of the vesicular stomatisis virus (VSV-G) is a transmembrane protein that functions as the surface coat of the wild type viral particles. It is also a common coat protein for engineered lentiviral vectors. Presently, nine virus species are definitively classified in the VSV gender, and nineteen rhabdoviruses are provisionally classified in this gender (see hereafter), all showing various degrees of cross-neutralisation. When sequenced, the protein G genes indicate sequence similarities. The VSV-G protein presents a N-terminal ectodomain, a transmembrane region and a C-terminal cytoplasmic tail. It is exported to the cell surface *via* the transGolgi network (endoplasmic reticulum and Golgi apparatus).

The VSV strains include several serotypes that may provide envelope protein(s) for the preparation of the lentiviral vectors: The VSV-G glycoprotein may especially be chosen among species classified in the vesiculovirus genus: Carajas virus (CJSV), Chandipura virus (CHPV), Cocal virus (COCV), Isfahan virus (ISFV), Maraba virus (MARAV), Piry virus (PIRYV), Vesicular stomatitis Alagoas virus (VSAV), Vesicular stomatitis Indiana virus (VSIV) and Vesicular stomatitis New Jersey virus (VSNJV) and/or stains provisionally classified in the vesiculovirus genus as Grass carp rhabdovirus, BeAn 157575 virus (BeAn 157575), Boteke virus (BTKV), Calchaqui virus (CQIV), Eel virus American (EVA), Gray Lodge virus (GLOV), Jurona virus (JURV), Klamath virus (KLAV), Kwatta virus (KWAV), La Joya virus (LJV), Malpais Spring virus (MSPV), Mount Elgon bat virus (MEBV), Perinet virus (PERV), Pike fry rhabdovirus (PFRV), Porton virus (PORV), Radi virus (RADIV), Spring viremia of carp virus (SVCV), Tupaia virus (TUPV), Ulcerative disease rhabdovirus (UDRV) and Yug Bogdanovac virus (YBV).

Vesicular stomatitis Indiana virus (VSIV) and Vesicular stomatitis New Jersey virus (VSNJV) are preferred strains to pseudotype the lentiviral vectors, or to design recombinant envelope protein(s) to pseudotype the lentiviral vectors.

The VSV strains of Indiana and New Jersey serotypes are particularly interesting to be used in the lentiviral vectors. Their VSV-G proteins are disclosed in Genebank, where several strains are presented. For VSV-G New Jersey strain reference is especially made to the sequence having accession number V01214.

Among VSV, Chandipura virus (CHPV), Cocal virus (COCV), Perinet virus (PERV), Piry virus (PIRYV), SVCV or Isfahan virus may be good candidates to design a third envelope protein or third envelope proteins, or further envelope protein(s).

According to another embodiment, the viral envelope protein(s) originate from other RNA-viruses, for example non-human retroviruses, such as murine retroviruses or from Influenza viruses.

Other examples of envelope protein(s) suitable for lentiviral pseudotyping are given later in the description, especially with a reference to their target cells in a host.

According to an aspect of the disclosure, the kit of compounds makes use of first and second and if any said third or further, viral envelope protein(s), that originate from *Rhabdoviridae*, in particular VSV or from *Paramyxoviridae* wherein the first and second and if any said third or further, envelope protein(s) originate from viruses of different genus, or originate from different virus strains in the same serogroup, especially in the vesicular stomatitis serogroup or alternatively originate from different serotypes of the same genus.

In an aspect of the disclosure, protein(s) or glycoprotein(s), suitable for use in the design of pseudotyped lentiviral vectors of the kit of compounds are especially produced as monomeric or multimeric protein(s).

In an aspect of the disclosure, said first and second and if any said third or further, viral envelope protein(s) are capable of uptake by antigen presenting cells and especially by dendritic cells by mean of fusion and/or of endocytosis.

Antigen Presenting Cells (APC) and especially Dentritic cells (DC) are proper target cells for pseudotyped lentiviral vectors which are used as vaccine compositions, either for a prophylactic or a therapeutic purpose.

The envelope protein(s) used to pseudotype the lentiviral vector particles may thus be selected with respect to the target cells in a host.

Polynucleotide encoding VSV envelope protein(s) (VSV-G) also targets splenocytes, in particular Antigen Presenting Cells (APC) or Dendritic Cells (DC), or liver cells including hepatocytes or non parenchymal cells.

Other target cells may be activated or proliferating cardiomyocytes.

Polynucleotides encoding envelope protein(s) suitable to target determined cells and to be used for pseudotyping the lentiviral vector are illustrated hereafter: polynucleotides encoding envelope protein(s) of VSV (VSV-G), LCMV (Lymphocytic choriomeningitis Virus), or RRV (Ross River Virus) may be used to prepare vectors suitable to target liver cells (Park 2003) (Kang et al, 2002).

Envelope protein(s) of Ebola or Marburg viruses may be used to target apical surface airway epithelium (Kobinger et al, 2001).

Envelope protein(s) of viruses of the Rhabdoviridae family (including Rabies or Rabies-related viruses like Mokola virus) or of the VSV family may provide neurotropic lentiviral vectors.

Envelope glycoprotein(s) of an Arenavirus such as Lymphocytic Choriomeningitis Virus (LCMV) may be used to transduce fibroblasts, epithelial cells, hematopoietic cells, neuroblastoma and glioma cell lines.

Alphaviruses envelope protein(s) such as the protein(s) of RRV or SFV (Semliki Forest Virus) may target Antigen Presenting Cells (APC), neurons or muscle cells.

Other envelope protein(s) may be used to pseudotype the lentiviral vector, such as HA protein (influenza hemaglutinin), RD114 protein, envelope protein(s) of Togaviridae, of Orthomyxoviridae (such as Influenza virus), Coronaviridae, Flaviridae, Filoviridae.

The envelope protein(s), also designated sometimes as surface protein in particular viruses, are said to "originate" from a different organism, and especially from different RNA virus strains, meaning that in said protein(s), essential features of the corresponding protein(s) expressed in a determined RNA virus are maintained. Said essential features, relate to the structure or to the function of the protein and are those which enable especially the obtained protein(s) to be expressed at the surface of the vector particles for pseudotyping said vectors. The envelope proteins are then capable of being recognized and internalized in the target cells of the hosts when present on the vector particles.

In an aspect of the disclousre, protein(s) or glycoprotein(s), suitable for use in the design of pseudotyped lentiviral vectors of the kit of compounds are used as multimeric proteins, such as VSV-G protein which is trimeric.

The envelope protein(s) are expressed from a polynucleotide containing the coding sequence for said protein(s), which polynucleotide is inserted in a plasmid (envelope expression plasmid or pseudotyping env plasmid) used for the preparation of the lentiviral vector of the invention. The polynucleotide encoding the envelope protein(s) is under the control of regulatory sequences for the transcription and/or expression of the coding sequence (including optionally a polynucleotide such as WPRE sequence from Invitrogen).

The disclosure relates to a nucleic acid construct which comprises an internal promoter suitable for the use in mammalian, especially in human, cells, *in vivo* and the nucleic acid encoding the envelope protein under the control of said promoter. The disclosure also concerns a plasmid containing this construct. Examples of suitable promoters encompass the promoters of the following genes: EF1$\alpha$, human PGK, PPI (preproinsulin), thiodextrin, HLA DR invariant chain (P33), HLA DR alpha chain, Ferritin L chain or Ferritin H chain, Beta 2 microglobulin, Chymosin beta 4, Chymosin beta 10, or Cystatin Ribosomal Protein L41.

The nucleotide sequence used for the expression of the envelope protein(s) required for pseudotyping the lentiviral vector particles may also be modified with respect to the nucleic acid encoding the native envelope protein(s) used as reference. The modification may be carried out to improve the codons usage (codon optimization) in the cells for the preparation of the vector particles and/or in the transduced cells of the host It may be modified to express a protein different from the native protein(s), especially one which has an improved pseudotyping capacity, an improved capacity in the level of production, or an improved capacity with respect to prevention of sero-neutralization with other envelope protein(s) used in the kit of compounds.

Such a modification of the envelope protein(s) may affect and especially improve their level of production in a cell host or their ability to pseudotype the vector particles possibly by improving the density of envelope protein(s) associated with pseudovirions. Said modification may derive from a mutation in the amino acid sequence of said protein(s), for instance by addition, deletion or substitution of one or several nucleotides or nucleotidic fragments or may relate to post translational modifications and in particular to the glycosylation status of said envelope protein(s).

The envelope protein(s) used to pseudotype the lentiviral vectors of the invention are indeed especially glycoproteins.

It has already been shown that pseudotyping viral vectors with Vesicular Stomatitis Virus glycoprotein (VSV-G) enables the transduction of a large range of cell types from different species. This VSV-G glycoprotein, in addition to its broad tropism, has an interesting stability when used for vector pseudotyping. Therefore, VSV-G have been used as a standard for evaluating the efficiency of other pseudotypes (Cronin J. et al, 2005). Accordingly, VSV-G is an appropriate candidate for pseudotyping the lentiviral vectors of the invention.

The disclosure relates to a kit of compounds as defined in the present application, wherein both said first and second and if any, said third or further viral envelope proteins are transmembrane glycosylated (G) proteins of a VSV virus, said G proteins having different VSV type-specificity in the lentiviral vectors of the kit.

In particular, said first G protein originates from a VSV-Indiana serotype and said second G protein originates from a VSV-New-Jersey serotype, or *vice-versa.*

It has been shown and reported in the following examples that having recourse in a kit, to pseudotyped viral particles wherein the envelope protein(s), are G proteins of respectively the VSV-Indiana serotype and the VSV-New Jersey serotype enables to prime and boost an immunological reaction when the lentiviral vectors pseudotyped with either of said G proteins are successively used to elicit a reaction in a host to whom they are administered. In such a case, it has been shown that no humoral response or a low humoral response is produced against the first envelope protein(s) used which could harm the response elicited in the host against the expression product of the polynucleotide, when said lentiviral vector peudotyped with a second, distinct, envelope protein(s) is administered. This is enabled by the fact that said distinct envelope protein(s) do not cross-neutralize or do not significantly cross-react with each other and accordingly does not give rise to an antivector immune response.

The disclosure concerns a G protein originating from a VSV which is modified with respect to its native form, and/or is encoded by a nucleic acid molecule which is modified with respect to the natural one, in order to improve pseudotyping. It may be as a result of improvement of envelope protein(s) uptake by the lentiviral particles which allows improvement of transduction of the lentiviral particles by the cells of the host to whom they are administered.

A disclosed kit of compounds comprises lentiviral vectors wherein one or two or more of them is (are) pseudotyped with recombinant envelope protein(s) comprising domains or fragments originating from different envelope protein(s) of different viruses, especially of different genus of different species of VSV.

In an aspect of the disclosure, at least one the first, second and if any third or further envelope protein(s) is (are) recombinant envelope protein(s) comprising the export determinant of the VSV-G of Indiana strain.

The export determinant of the VSV-G of the Indiana strain is a polypeptide encoded by the cytoplasmic fragment of the open reading frame of the envelope.

The export determinant of the VSV-G of the Indiana strain is a polypeptide comprising or having amino acid sequence YTDIE in the cytoplasmic tail (Nishimua N. et al. 2002).

Said recombinant envelope protein(s) may comprise the cytoplasmic tail of the VSV-G of an Indiana strain which is the intracellular portion of VSV-G delimited by a hydrophobic transmembrane domain.

A particular kit of compounds comprises lentiviral vectors wherein one or two or more of them is (are) pseudotyped with recombinant envelope protein(s) comprising the cytoplasmic domain of the indiana VSV and the ectodomain of a strain of a different VSV serotype. The transmembrane domain may also be the one of the Indiana VSV-G.

A particular kit of compounds comprises lentiviral vectors wherein one or both of them is (are) pseudotyped with recombinant envelope protein(s) comprising the transmembrane domain and the cytoplasmic domain of the indiana VSV and the ectodomain of the New-Jersey VSV.

Appropriate other modifications encompass mutations, especially point mutations, that improve pseudotyping. Such mutations for the VSV-G proteins may be carried out in the transmembrane domain by substituting or deleting one or several amino acid residues. Other examples of appropriate mutations are disclosed in Fredericksen B.L. et al (1995) or Nishimura N. et al (2003).

When reference is made to "fragments" in the present description, it refers to polynucleotides or polypeptides having respectively a nucleotide sequence or an amino acid sequence of at least or longer than 6 nucleotides, respectively of at least or longer than 2 amino acid residues.

It is also especially possible to modify the glycosylation status of the VSV-G, in order to improve transduction efficiency of the lentiviral vector pseudotyped with these VSV-G proteins, when administered to a host.

VSV-G proteins from various strains of VSV are disclosed in the figures and their sequences can also be derived from databases, especially from GenBank.

Considering the glycoproteins of the New-Jersey and Indiana strains of VSV, it has been proposed that glycosylation at two asparagine residues (N180 and N336) favour the efficient pseudotyping of lentiviral vectors. This particular feature may be applied in the preparation of the lentiviral vectors.

The disclosure describes the following constructs encoding VSV-G derived envelope proteins, and their use in the preparation of the combination of lentiviral vector particles of. The disclosure also describes the envelope proteins encoded by said constructs:

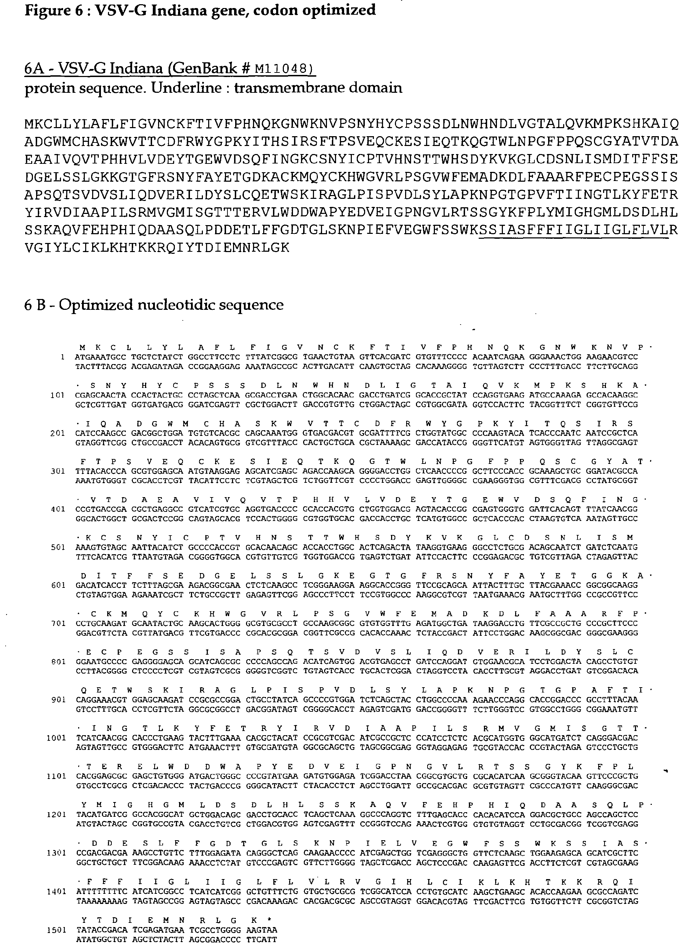

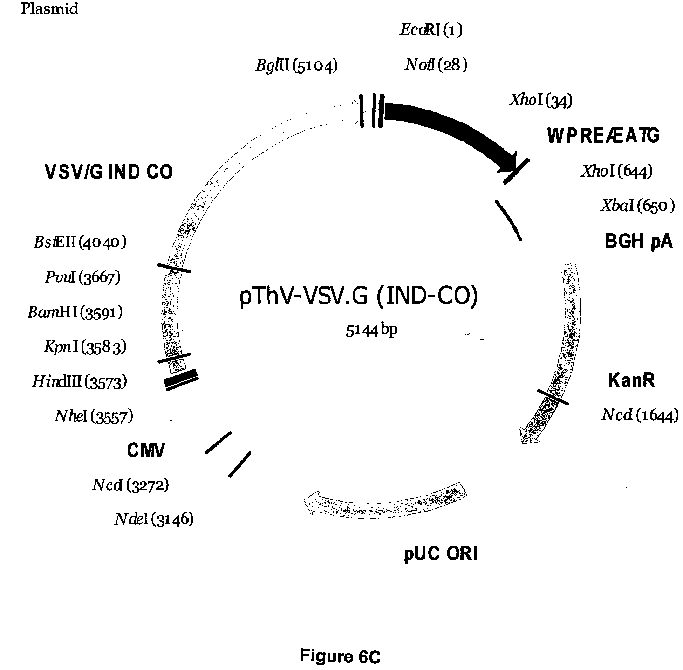

A VSV-G Indiana gene codon optimized is disclosed in figure 6 and is part of the invention. The invention also relates to encapsidation plasmids containing an envelope gene for VSV-G Indiana. A particular encapsidation plasmid is pThV-VSV.G (IND-CO) deposited at the CNCM (Paris, France) on October 10 2007, under number I-3842. Other constructs may be derived from this particular plasmid, especially by substituting the promoter for a promoter among those listed in the present application.

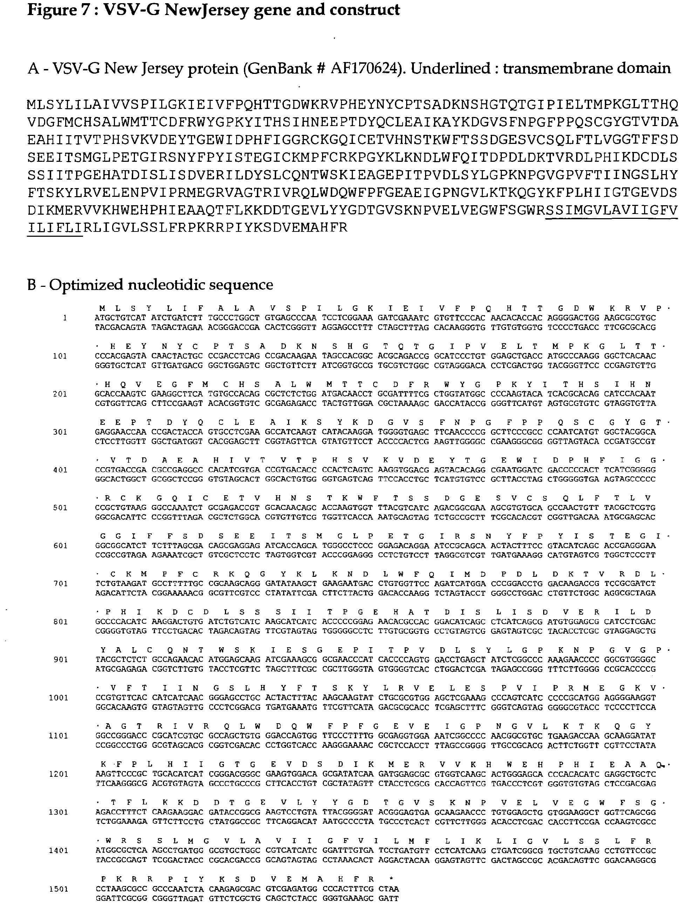

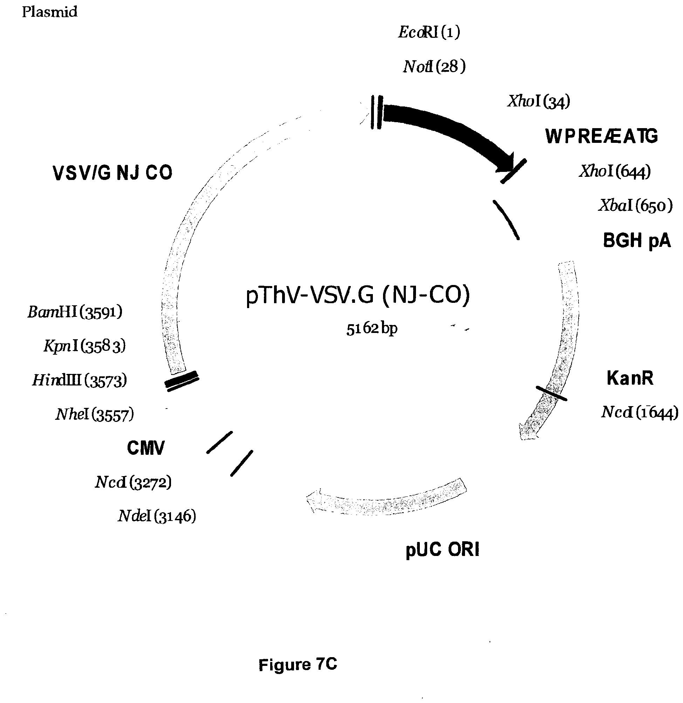

A VSV-G New-Jersey gene codon optimized is disclosed in figure 7 and is part of the invention. The disclosure also relates to encapsidation plasmids containing an envelope gene for VSV-G New jersey. A particular encapsidation plasmid is pThV-VSV.G (NJ-CO) deposited at the CNCM (Paris, France) on October 10, 2007, under number I-3843. Other constructs may be derived from this particular plasmid, especially by substituting the promoter for a promoter among those listed in the present application.

Other envelope genes having codon optimized sequences are illustrated in figures 6 to 12 and 14 to 19 and especially encompass VSV-G Chandipura gene and its expression product, VSV-G Cocal gene and its expression product, VSV-G Piry gene and its expression product, VSV-G Isfahan gene and its expression product, VSV-G Spring viremia carp virus gene and its expression product.

The disclosure describes fusion envelope proteins, especially fusion proteins involving several different fragments of VSV-G proteins of different viruses and to the nucleic acid constructs encoding such proteins. A particular fusion envelope is the fusion between the ectodomain of the New-Jersey envelope protein and the transmembrane domain and cytoplasmic domain of the Indiana envelope protein as illustrated in the figures.

Another fusion envelope protein comprises the ectodomain of one VSV-G protein selected among VSV-G Chandipura, VSV-G Cocal, VSV-G Pyri, VSV-G Isfahan, or VSV-G SVCV and the transmembrane and cytoplasmic domains of VSV-G Indiana. The disclosure also relates to a nucleic acid molecule encoding said fusion protein illustrated in the figures, and especially a codon optimized nucleic acid encoding the fusion protein also described in the figures.

The disclosure also concerns the expression vectors, especially the plasmids containing the nucleic acid constructs encoding the fusion proteins.

Basic, essential features characterizing the vector genome used in the construction of the pseudotyped lentiviral vector particles of the invention have been described hereabove. Additional features for the preparation of suitable vector genome (also designated as transfer vector) are disclosed hereafter, including in the examples and in the drawings.

In an aspect of the disclosure the pseudotyped lentiviral vectors are human lentivirus based vectors. Accordingly their genome is derived from a human lentivirus, especially from the HIV lentivirus. In particular, the pseudotyped lentiviral vector is an HIV-based vector, such as an HIV-1, or HIV-2 based vector.

In another aspect of the disclosure, the pseudotyped lentiviral vectors are primate or feline lentivirus based vectors.

As stated above, when considering it apart from the transgene that it finally contains, the vector genome is a replacement vector in which the nucleic acid between the 2 long terminal repeats (LTRs) in the original lentivirus genome have been restricted to cis-acting sequences for DNA or RNA synthesis and processing, or at least are deleted or mutated for essential nucleic acid segments that would enable the expression of lentiviral structure proteins including biological functional GAG polyprotein and possibly POL and ENV proteins. Accordingly, the vector genome is defective for the expression of biologically functional GAG, and advantageously for biologically functional POL and ENV proteins. The 5' LTR and 3' LTR sequences of the lentivirus are used in the vector genome, but the 3'-LTR at least is modified with respect to the 3'LTR of the original lentivirus at least in the U3 region. The 5'LTR may also be modified, especially in its promoter region.

In a particular embodiment the vector genome is accordingly devoid of the coding sequences for vif-, vpr-, vpu- and nef-accessory genes (for HIV-1 lentiviral vectors), or of their complete or functional genes.

In a preferred embodiment, the vector genome of the lentiviral vector particles comprises, as an inserted cis-acting fragment, at least one polynucleotide consisting in the DNA flap or containing such DNA flap. In a particular embodiment, the DNA flap is inserted upstream of the polynucleotide of interest, advantageously but not necessarily to be located in an approximate central position in the vector genome. A DNA flap may be obtained from a retrovirus, especially from a lentivirus, in particular a human lentivirus, or from a retrovirus-like organism such as retrotransposon. It may be alternatively obtained from the CAEV (Caprine Arthritis Encephalitis Virus) virus, the EIAV (Equine Infectious Anaemia Virus) virus, the VISNA virus, the SIV (Simian Immunodeficiency Virus) virus or the FIV (Feline Immunodeficiency Virus) virus. The DNA flap may be either prepared synthetically (chemical synthesis) or by amplification of the DNA providing the DNA Flap from the appropriate source as defined above such as by Polymerase chain reaction (PCR). In a more preferred embodiment, the DNA flap is obtained from an HIV retrovirus, for example HIV-1 or HIV-2 virus including any isolate of these two types.

The DNA flap (defined in Zennou V. et al 2000, Cell vol 101, 173-185 or in WO 99/55892 and WO 01/27304), is a structure which is central in the genome of some lentiviruses especially in HIV, where it gives rise to a 3-stranded DNA structure normally synthesized during especially HIV reverse transcription and which acts as a cis-determinant of HIV genome nuclear import. The DNA flap enables a central strand displacement event controlled in *cis* by the central polypurine tract (cPPT) and the central termination sequence (CTS) during reverse transcription. When inserted in lentiviral-derived vectors, the polynucleotide enabling the DNA flap to be produced during reverse-transcription, stimulates gene transfer efficiency and complements the level of nuclear import to wild-type levels (Zenou et al, Cell, 2000).

Sequences of DNA flaps have been disclosed in the prior art, especially in the above cited patent applications. These sequences are also disclosed in the attached figures as SEQ ID NO 1 to SEQ ID NO 7. They are preferably inserted as fragment possibly with additional flanking sequences in the vector genome in a position which is near the center of said vector genome. Alternatively they may be inserted immediately upstream from the promoter controlling the expression of the polynucleotide of the invention. Said fragments comprising the DNA flap, inserted in the vector genome may have a sequence of about 80 to about 200 bp, depending on its origin and preparation.

In HIV-1, the DNA flap is a stable 99-nucleotide-long plus strand overlap. When used in the genome vector of the lentiviral vector of the invention, it may be inserted as a longer sequence, especially when it is prepared as a PCR fragment. A particular appropriate polynucleotide comprising the structure providing the DNA flap is a 178-base pair polymerase chain reaction (PCR) fragment encompassing the cPPT and CTS regions of the HIV-1 DNA (Zennou et al 2000).

This PCR fragment may especially be derived from infection DNA clone of HIV-1 LAI especially pLAI3 of HIV1, as a fragment corresponding to the sequence from nucleotide 4793 to 4971. If appropriate, restriction sites are added to one or both extremities of the obtained fragment, for cloning. For example, Nar I restriction sites may be added to the 5' extremities of primers used to perform the PCR reaction.

It is specified that the DNA flap used in the genome vector and the polynucleotides of the encapsidation plasmid encoding the GAG and POL polyproteins should originate from the same lentivirus or from the same retrovirus-like organism.

Preferably, the other cis-activating sequences of the genome vector also originate from the same lentivirus or retrovirus-like organism, as the one providing the DNA flap.

The vector genome may further comprise one or several unique restriction site(s) for cloning the polynucleotide of interest.

The pseudotyped lentiviral vector is a replication-incompetent lentiviral vector as a result of the fact that *gag* and *pol* functional genes are exclusively provided in *trans* and therefore not present on the vector genome. In such a case, when the lentiviral vector has been administered to the host, it is not capable of replicating in the host cells. Accordingly, it provides the polynucleotide of therapeutic interest into the host cells for expression but does not form further lentiviral vector particles. This replication-incompetent of the lentiviral vector status is achieved especially when the lentiviral *gag, pol, env* genes are not provided in the vector genome. Other sequences involved in lentiviral replication may also be mutated in the vector genome, in order to achieve this status.

In an aspect of the disclosure, in said vector genome, the 3' LTR sequence of the lentiviral vector genome is devoid of at least the activator (enhancer) and possibly the promoter of the U3 region. In another particular embodiment, the 3' LTR region is devoid of the U3 region (delta U3). In this respect, reference is made to WO 01/27300 and WO 01/27304.

In an aspect of the disclosure, in the vector genome, the U3 region of the LTR 5' is replaced by a non lentiviral U3 or by a promoter suitable to drive tat-independent primary transcription.

The vector genome also comprises the psi (ψ) packaging signal. The packaging signal is derived from the N-terminal fragment of the *gag* ORF. In a particular embodiment, its sequence could be modified by frameshift mutation(s) in order to prevent any interference of a possible transcription/translation of gag peptide, with that of the transgene.

The vector genome may optionally also comprise elements selected among a splice donor site (SD), a splice acceptor site (SA) and/or a Rev-responsive element (RRE).

According to an aspect of the disclosure, the vector plasmid (or genome vector) comprises the following cis-acting sequences for a transgenic expression cassette:

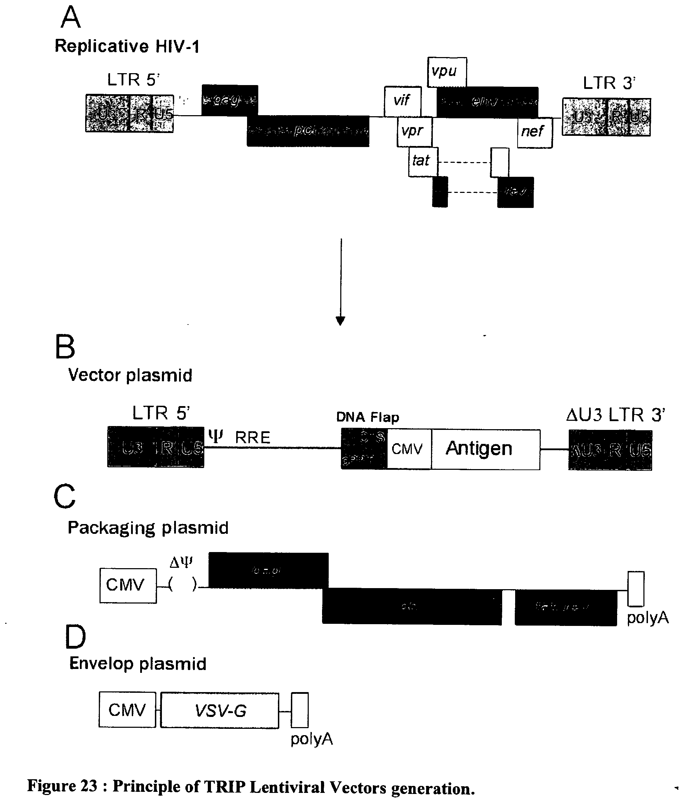

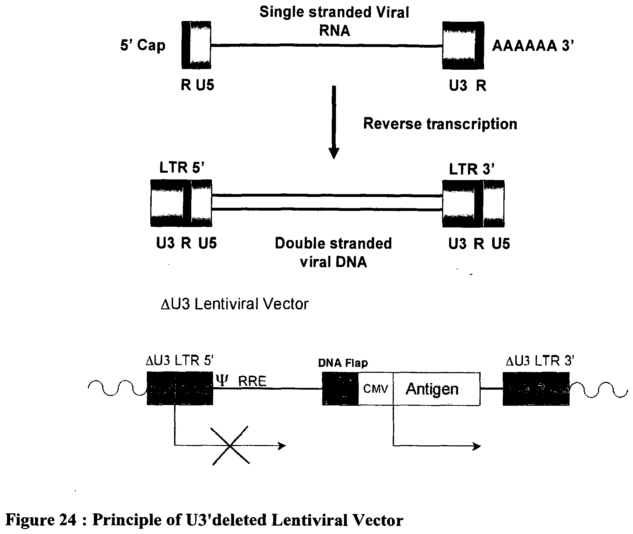

1. The LTR sequence (Long-Terminal Repeat), required for reverse transcription, viral DNA integration and transcription. The 3' LTR has been deleted in the U3 region, without perturbing the functions necessary for gene transfer, for two major reasons: first, to avoid trans-activation of a host gene, once the DNA is integrated in the genome and secondly to allow self-inactivation of the viral cis-sequences after retrotranscription. Thus, in target cells only sequences from the internal promotor will be transcribed (transgene) (Figures 23 and 24).

2. The ψ region, necessary for viral RNA encapsidation.

3. The RRE sequence (REV Responsive Element) allowing export of viral messenger RNA from the nucleus to the cytosol after binding of the *Rev* protein.

4. The DNA flap sequence (cPPT/CTS, normally contained in *Pol*) to facilitate nuclear import.

5. Optionally, the WPRE cis-active sequence (Woodchuck hepatitis B virus Post-Responsive Element) also added to optimize stability of mRNA (Zufferey et al., 1999). WPRE is not translated.

In order to obtain lentiviral vectors, the vector genome (as a vector plasmid) must be encapsidated in particles or pseudo-particles. Accordingly, lentiviral proteins, except the envelope proteins, have to be provided in trans to the vector genome in the producing system, especially in producing cells, together with the vector genome, having recourse to at least one encapsidation plasmid carrying the *gag* and *pol* lentiviral genes or integrative -incompetent *pol* gene, and preferably lacking the coding sequences for *Vif-, Vpr, Vpu-* and *Nef*-accessory genes (for HIV-1 lentiviral vectors).

A further plasmid is used, which carries a polynucleotide encoding the envelope protein(s) selected for pseudotyping each lentiviral vector.

In an aspect of the disclosure, the packaging plasmid encodes only the lentiviral proteins essential for viral particle synthesis. Accessory genes whose presence in the plasmid could raise safety concerns are accordingly removed. Viral proteins brought in *trans* are respectively as illustrated for HIV-1:

1. *Gag* proteins for building of the matrix (MA, with apparent Molecular Weight p17), the capsid (CA, p24) and nucleocapsid (NC, p6).

2. *Pol* encoded enzymes: integrase, protease and reverse transcriptase.

3. *Tat* and *Rev* coding regulatory proteins, *Tat* is necessary for the initiation of LTR-mediated transcription; it may be omitted if the U3 region of 5'LTR is substituted for a promoter driving tat-independent transcription.

In order to avoid any packaging of the mRNA generated from the genes contained in the packaging plasmid in the viral particles, the ψ region is removed from the packaging plasmid. A heterologous promoter is inserted in the plasmid to avoid recombination issues and a poly-A tail is added 3' from the sequences encoding the proteins.

The envelope plasmid encodes the envelope protein(s) for pseudotyping which are disclosed herein, under the control of an internal promoter.

All the described plasmids for the preparation of the lentiviral vector particles of the invention may be codon optimized (CO) in the segment encoding proteins. Codon optimization according to the invention is preferably performed to improve translation of the coding sequences contained in the plasmids, in mammalian cells, especially human cells. According to the invention, codon optimization is especially suited to directly or indirectly improve the preparation of the vector particles or to improve their uptake by the cells of the host to whom they are administered, or to improve the efficiency of the transfer of the polynucleotide of interest (transgene) in the genome of the transduced cells of the host. Methods for optimizing codons are well known in the art and codon optimization is especially performed using available programs to that effect. Codon optimization is illustrated for the coding sequences contained in the described pTRIP plasmids and pThV plasmids of the invention illustrated in the figures.

In an aspect of the disclosure, the pseudotyped lentiviral vector is also or alternatively integrative-incompetent. In such a case, the vector genome and thus the heterologous polynucleotide of therapeutic interest do not integrate into the genome of the transduced cells or in the cells of the host to whom it has been administered. Integration-incompetent lentiviral vectors are obtained by modifying the *pol* gene encoding the Integrase, contained in the encapsidation plasmid. Such integration-incompetent lentiviral vectors have been described in patent application WO 2006/010834. Accordingly the integrase capacity of the protein is altered whereas the correct expression from the encapsidation plasmid of the GAG, PRO and POL proteins and/or the formation of the capsid and hence of the vector particles, as well as other steps of the viral cycle, preceding or subsequent to the integration step, such as the reverse transcription, the nuclear import, stay intact. An integrase is said defective when the integration that it should enable is altered in a way that an integration step takes place less than 1 over 1000, preferably less than 1 over 10000, when compared to a lentiviral vector containing a corresponding wild-type integrase.

In an aspect of the disclosure, the defective integrase results from a mutation of class 1, preferably amino acid substitutions (one-amino acid substitution) or short deletions fulfilling the requirements of the preceding paragraph. The mutation is carried out within the pol gene. These vectors may carry a defective integrase with the mutation D64V in the catalytic domain of the enzyme, which specifically blocks the DNA cleaving and joining reactions of the integration step. The D64V mutation decreases integration of pseudotyped HIV-1 up to 1/10,000 of wild type, but keep their ability to transduce non dividing cells, allowing efficient transgene expression.

Other mutations in the *pol* gene which are suitable to affect the integrase capacity of the integrase of HIV-1 are the following: H12N, H12C, H16C, H16V, S81 R, D41A, K42A, H51A, Q53C, D55V, D64E, D64V, E69A, K71A, E85A, E87A, D116N, D116I, D116A, N120G, N120I, N120E, E152G, E152A, D-35-E, K156E, K156A, E157A, K159E, K159A, K160A, R166A, D167A, E170A, H171A, K173A, K186Q, K186T, K188T, E198A, R199C, R199T, R199A, D202A, K211A, Q214L, Q216L, Q221 L, W235F, W235E, K236S, K236A, K246A, G247W, D253A, R262A, R263A and K264H.

In an aspect of the disclosure, mutation in the pol gene is performed at either of the following positions D64, D116 or E152, or at several of these positions which are in the catalytic site of the protein. Any substitution at these positions is suitable, including those described above.

In an aspect of the disclosure, when the lentiviral vector is integration-incompetent, the lentiviral genome further comprises an origin of replication (ori), whose sequence is dependent on the nature of cells where the lentiviral genome has to be expressed. Said origin of replication may be from eukaryotic origin, preferably of mammalian origin, most preferably of human origin. It may alternatively be of viral origin, especially coming from DNA circular episomic viruses, such as SV40 or RPS. It is an advantage to have an origin or replication inserted in the lentiviral genome of the lentiviral vector. Indeed, since the lentiviral genome does not integrate into the cell host genome (because of the defective integrase), the lentiviral genome is lost in cells undergoing frequent cell divisions; this is particularly the case in immune cells, such as B or T cells. The presence of an origin of replication ensures that at least one lentiviral genome is present in each cell, even after cell division, maximazing the efficiency of the immune response.

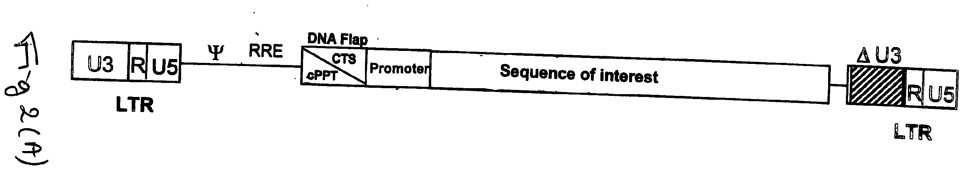

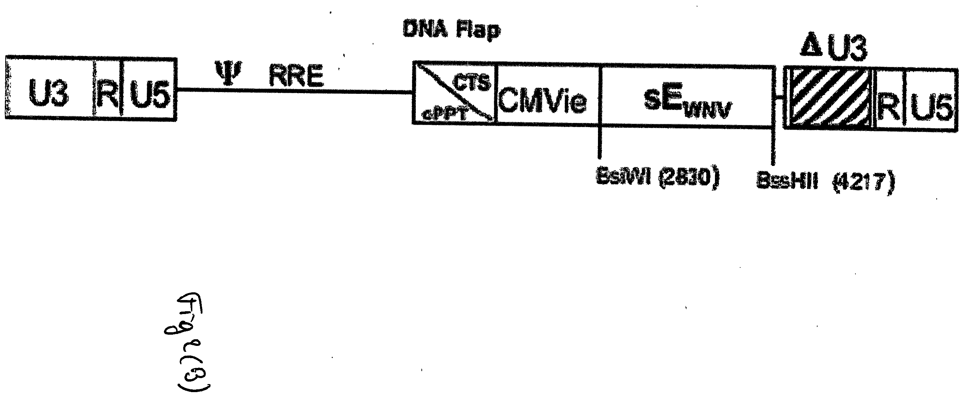

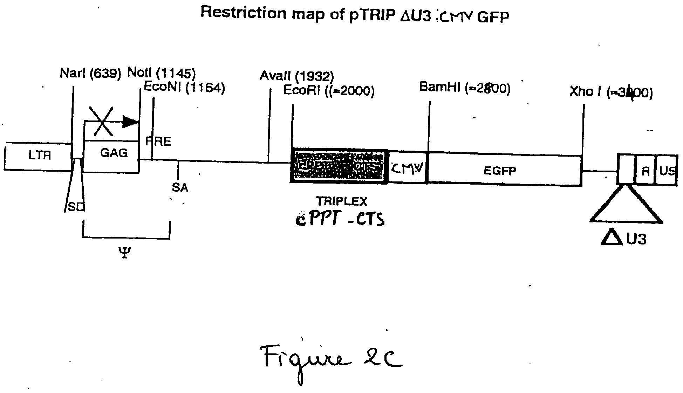

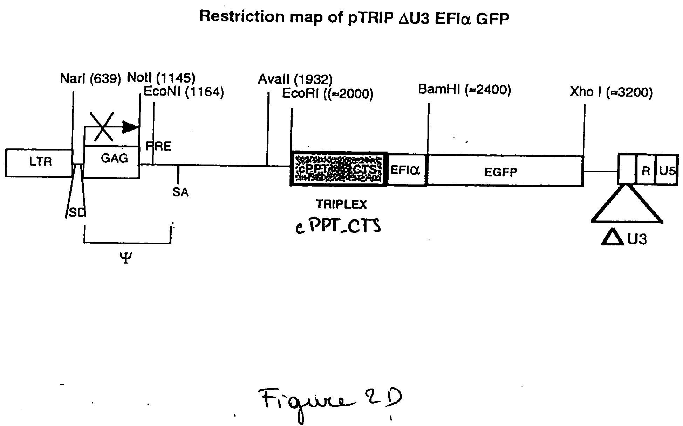

In an aspect of the disclosure, the lentiviral vector genome is a HIV-based genome and has the sequence features represented on figures 2 or 23 to 25, wherein said sequence of interest is selected for its therapeutic interest and the internal promoter enabling its expression (represented in the figures by a CMV promoter) is advantageously selected to be suitable for administration in human.

The internal promoter contained in the transgene or expression cassette of the vector genome may be selected from the promoters of the following genes: EF1$\alpha$, human PGK, PPI (preproinsulin), thiodextrin, HLA DR invariant chain (P33), HLA DR alpha chain, Ferritin L chain or Ferritin H chain, Beta 2 microglobulin, Chymosin beta 4, Chimosin beta 10, or Cystatin Ribosomal Protein L41.

The lentiviral vector genome of said lentiviral vectors of the invention may especially be derived from HIV-1 plasmid pTRIP$\Delta$U3.CMV-GFP deposited at the CNCM (Paris, France) on October 11, 1999 under number I-2330. The structure and restriction sites of the various sequences contained in the plasmid are shown on Figure 2C. The sequence of pTRIP$\Delta$U3.CMV-GFP is provided on Figure 6.

In an aspect of the disclosure, the lentiviral vector genome may be derived from HIV-1 plasmid pTRIP[delta]U3EF1[alpha]-GFP deposited at the CNCM on October 11, 1999 under number I-2328. A description of the constituting sequences of the plasmid is depicted in Figure 2D, with the restriction sites of the various sequences.

When the vector genome is derived from these particular plasmids, a sequence of a heterologous polynucleotide as disclosed in the present application is inserted therein, in addition or in replacement of the GFP coding fragment. The GFP coding sequence may also be substituted by a different marker. The CMV promoter may also be substituted by another promoter, especially one of the promoters disclosed above.

Other lentiviral vector genomes are those contained in the deposited material listed hereafter or are derived from these deposited plasmids, especially by substituting the transgene either for a different polynucleotide of interest and/or for a different internal promoter. The WPRE sequence also contained in the particular deposited pTRIP vectors may also be deleted.

The disclosure thus concerns the lentiviral vector genome provided by plasmid pTRIPDeltaU3-CMV-SIV-GAGco-WPRE deposited at the CNCM (Paris, France) on October 10, 2007 under Number I-3841. The composition of the plasmid is disclosed in the figures and its sequence is provided. This plasmid expresses the GAG protein of SIV as a non-myristilated protein. The ORF of the transgene has been codon optimized for the expression in human cells.

The disclosure also concerns the lentiviral vector genome provided by plasmid pTRIPDelta U3-CMV-SIV-GAG-WPRE deposited at the CNCM (Paris, France) on October 10, 2007 under Number I3840. The composition of the plasmid is disclosed in the figures and its sequence is provided. This plasmid expresses the GAG protein of SIV as a non-myristilated protein. The ORF of the transgene is not codon optimized.

Vector particles may be produced after transfection of appropriate cells, such as 293 T cells, by said plasmids, or by other processes. In the cells used for the expression of the lentiviral particles, all or some of the plasmids may be used to stably express their coding polynucleotides, or to transiently or semi-stably express their coding polynucleotides.

The concentration of particles produced can be determined by measuring the P24 (capsid protein for HIV-1) content of cell supernatants.

The lentiviral vector of the invention, once administered into the host, infects cells of the host, possibly specific cells, depending on the envelope proteins it was pseudotyped with. The infection leads to the release of the lentiviral genome into the cytoplasm of the host cell where the retrotranscription takes place. Once under a triplex form (via the DNA flap), the lentiviral genome is imported into the nucleus, where the polynucleotide of interest is expressed via the cellular machinery. When non-dividing cells are transduced (such as DC), the expression may be stable. When dividing cells are transduced, such as B cells, the expression is temporary in absence of origin of replication in the lentiviral genome, because of nucleic acid dilution and cell division. The expression may be longer by providing an origin of replication ensuring a proper diffusion of the lentiviral genome into daughter cells after cell division. The stability and/or expression may also be increased by insertion of MAR (Matrix Associated Region) or SAR (Scaffold Associated Region) elements.

If the lentiviral genome is non integrative, it does not integrate into the host cell genome. Nevertheless, the at least one polypeptide encoded by the transgene is sufficiently expressed and longer enough to be processed, associated with MHC molecules and finally directed towards the cell surface. Depending on the nature of the polynucleotide of interest, the at least one polypeptide epitope associated with the MHC molecule triggers a humoral or a cellular immune response. The preparation of integrative-incompetent lentiviral vector, has been disclosed herein: the encapsidation plasmid used to transcomplement the vector genome is mutated in the region of the integrase protein, in such a way that said integrase is not expressed or is not functionally expressed in the lentiviral vector when said vector is produced as pseudotyped particles in a cell host, after said lentiviral vector has been administered to a patient.

With a view to use the lentiviral vector particles and especially the kit of compounds in the design of medicinal treatment protocols, the lentiviral vectors of the invention carry in their vector genome, a heterologous polynucleotide (or transgene) having a therapeutic interest. By the expression "heterologous polynucleotide", it is meant that the vector genome comprises, irrespective from the ci-acting sequences in the vector genome that originate from the lentivirus genome and which are necessary or useful for the vector activity, at least one polynucleotide which is not necessary or which is not useful for the vector activity but which is suitable to obtain a biological effect, especially a medicinal effect when it is expressed in a host especially a human host. In a preferred embodiment, the polynucleotide of interest encodes a polypeptide and is preferably included in an expression cassette.

The heterologous polynucleotide encodes one polypeptide or several polypeptides which is (are) suitable for eliciting an immune response in a host, said immune response being a cellular immune response and possibly a humoral response. The encoded polypeptide(s) comprise(s) one or several epitopes or consist(s) in epitope(s) of an antigen. In a particular embodiment, it may be a polyepitope. It (they) may be processed in the cells of the host for presentation by the APC, especially the DC, of the host to give rise to an immune response, or it (they) may directly elicit an immune response. Accordingly, the polynucleotide of interest comprises or consists of sequences of B epitope(s) and/or T epitope(s) of one or several antigens, including association of both categories of epitopes, possibly giving rise to a chimeric (i., e., non natural) polypeptide.

In an aspect of the disclosure, the heterologous polynucleotide encodes an antigen or several antigens or fragments thereof including epitopes (B and/or T epitopes) of a pathogenic organism such as a virus, bacteria or parasite, or of a pathogenic agent or compound. It may encode an antigen of the pathogenic organism or recombinant antigens, to the extent that it does not enable expression of the pathogenic organism when the lentiviral vector is administered.

The heterologous polynucleotide may be expressed as endogenous antigen in the cells of the host especially after transfer of said polynucleotide in the genome of the host cells and processed in said cells for presentation in association with MHC molecules.

The polynucleotide of interest may be chosen so that the immune response elicited with the vector, possibly after presentation by APC, may especially encompass an elicitation of T lymphocytes response, including T helper or CTL cells (cytotoxic). A $CD8^+T$ cell response, against the processed expression product of said polynucleotide, in a host is especially of interest.

A $CD4^+T$ cell response may also be expressed.

The polynucleotide of interest may be chosen so that the lentivirus vector of the invention may also or alternatively be used to elicit a humoral immune response, especially a neutralizing humoral immune response, against the expression product of said polynucleotide, in a host.

the disclosure describes lentiviral vector particles intended for prevention or treatment of non lentiviral infections, the heterologous polynucleotide having a biological or a therapeutic interest is of a different origin than the polynucleotide constituting the vector genome. Especially, it is originating from a different organism than the lentivirus providing the sequences of the vector genome.

In an aspect of the disclosure, where prevention or treatment of a lentiviral infection is sought, the heterologous polynucleotide may be originating from the same family or the same serotype of lentivirus providing the vector, especially when the lentiviral vector particles are HIV-based lentiviral vectors.

The heterologous polynucleotide encodes an antigen derived from a lentiviral protein or an antigenic fragment thereof or a combination of such antigens. In such a case, said lentiviral protein antigen derived thereof or antigenic fragment thereof is used in conditions which prevent formation of native or replicative-competent lentiviral particles.

In an aspect of the disclosure, it is used in conditions which also prevent the formation of lentivirus pseudo particles such as GAG or GAG-POL pseudo particles. These antigens may be derived from the same lentivirus, especially HIV, in particular HIV-1, as the one used for the design of the lentiviral vector.

Accordingly, the polynucleotide can be a coding sequence of one or several a HIV polypeptide(s) or polyepitopes, especially HIV-1 polypeptides or polyepitopes, suitable to elicit a cellular, especially a cytotoxic T-lymphocyte (CTL) response, and possibly T helper response in a host.

In a preferred embodiment of the invention, the lentiviral vectors comprise in their genome, a recombinant polynucleotide encoding one or several polypeptides comprising at least one antigen derived from a GAG antigen from HIV1 having the sequence as defined in the claims.

GAG polyprotein encompasses the Matrix protein (MA), the Capsid protein (CA), and the Nucleocapsid protein (NP).

It is disclosed GAG derived antigens that encompass polypeptides derived from each of theses proteins, including fragments thereof or mutated (by deletion, substitution or addition) versions thereof. It also encompasses combinations of such polypeptides derived from each of these proteins.

It is disclosed, an antigen derived from GAG of an immunodeficiency virus having the amino acid sequence of the natural GAG antigens, especially of the GAG polyprotein or the Matrix protein or the Capsid protein or the nucleocapsid protein, or is a fragment of such polyprotein or of such protein, or is a GAG antigen which is modified with respect to the natural GAG antigen, especially by mutation, including by deletion, substitution or addition of one or several amino acid residues in the amino acid sequence, or which is modified by post translational modifications. The modified GAG antigen is selected to be either biologically functional or biologically non-functional.

the disclosure describes the recombinant polynucleotide encoding several polypeptides comprising at least one antigen derived from a GAG polyprotein of an Immunodeficiency Virus encoding a polypeptide which is a biologically non-functional GAG polypeptide (including an antigenic fragment of GAG) of SIV especially SIV$_{MAC}$, or of FIV, or of HIV in particular HIV-1 or HIV-2, and which is not capable of forming biologically functional capsids proteins within cells transduced with the lentiviral vectors, and especially does not induce secretion of capsid proteins from these cells that would enable formation of GAG pseudo particles or GAG-POL pseudo-particles.

In an aspect of the disclosure, the polynucleotide including the nucleic acid encoding the antigen derived from GAG does not enable the expression of POL biologically active polypeptides (polyprotein also designated as precursor) and thus does not comprise the *pol* native genes or an equivalent functional gene.

In a particular embodiment, the recombinant polynucleotide encoding one or several polypeptides comprising at least one antigen derived from a GAG antigen of an Immunodeficiency Virus also encodes a polypeptide derived from a NEF, TAT or REV antigens of HIV-1 and/or optionally from a POL antigen of HIV-1 or a combination thereof. These polypeptides are especially antigenic fragments of said antigens.

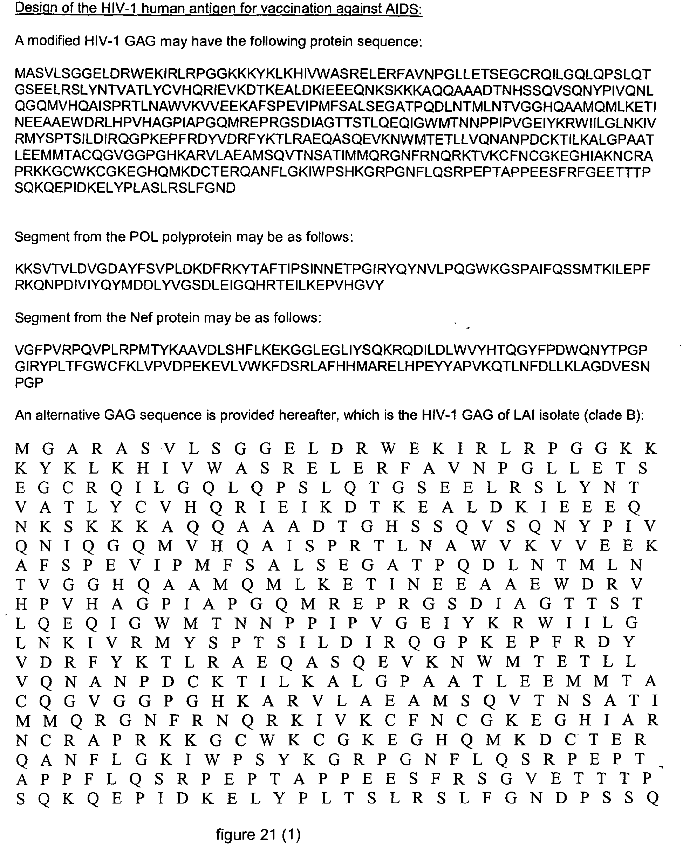

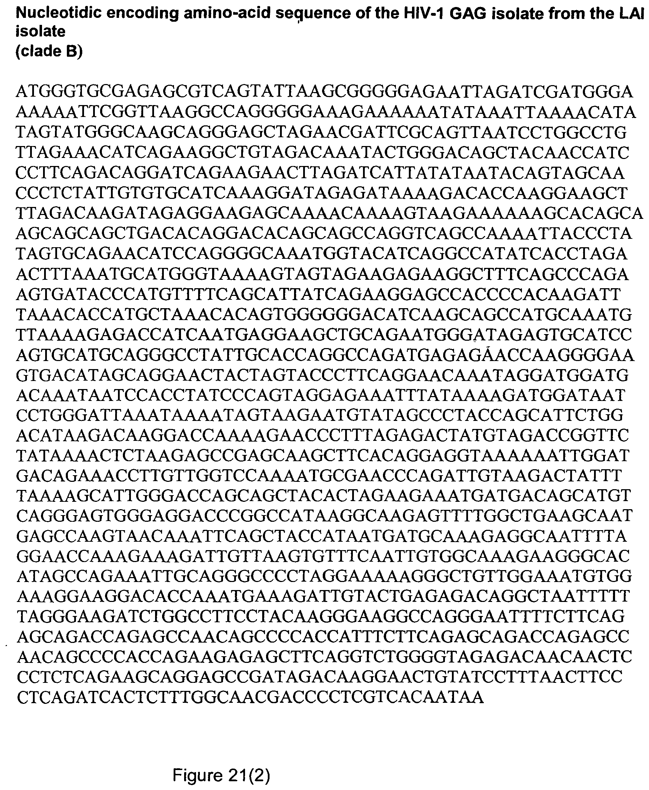

Examples of recombinant polynucleotide encoding an antigen derived from GAG (of HIV-1) and further nucleotide fragments encoding other antigens of HIV-1 in a fusion protein, is one which encodes a GAG protein as illustrated in figure 21 and a POL fragment or/and a NEF fragment or a fusion of such POL and NEF fragments also described on figure 21. These fragments may be fused 5' and/or 3' of the GAG antigen, may be contiguous to each other and/or to the GAG antigen or may be separated by a peptide such as the 2A peptide from picornavirus. Such construct is illustrated in the figures. The sequence of the 2A peptide is the following: APVKQTLNFDLLKLAGDVESNPGP. A particular organization of the structure of the fusion protein is one of the following: 5' GAG POL NEF 3', or 5' POL NEF GAG 3' or 5' POL GAG NEF 3', or 5' NEF GAG POL 3' or 5' NEF POL GAG 3' or 5' GAG NEF POL 3'.

According to the invention, the antigens derived from GAG and/or NEF and/or POL antigens are derived from HIV-1.

In a particular embodiment, the polypeptide derived from the GAG antigen is a GAGΔmyr protein which is not myristylated contrary to native GAG.

Non myristylated HIV-1 GAG may be obtained by mutating the coding sequence of GAG at codon 2 to change Gly residue [GGA] to Ala residue [GCA], or by deletion of said codon 2.

Other GAG derived antigen are antigens formed of fragments of at least one of the Matrix, Capsid and Nucleocapsid proteins of GAG, especially are formed of a fusion of fragments of each of said proteins.

It is observed that the encoded derived antigen may be derived from GAG antigen of HIV-1, especially of HIV-1 subtype B or from HIV-1 group O (figure 21) and be used in a combination of compounds to elicit an immune response against various HIV groups, including different HIV-1 subtypes, HIV-1 and possibly HIV-2.

The invention also relates to a lentiviral vector as defined herein which comprises in its genome, a recombinant polynucleotide which has a human codon optimized sequence encoding an antigen derived from a GAG polyprotein of a Human Immunodeficiency Virus (HIV), or encoding a fusion antigen including an antigen derived from GAG and from at least an antigenic fragment of NEF, TAT, REV or POL as disclosed herein.

A chimeric HIV-1 derived antigen of the invention is, in a particular embodiment, a fusion protein comprising or consisting in the combination of the GAG derived antigen having the sequence of figure 21, with an antigen derived from NEF, POL, TAT or REV of a HIV-1 virus strain or with a combination of such antigens.

A particular fusion protein as disclosed above is one wherein POL derived antigen comprises or has the amino acid sequence of figure 21.

A particular fusion protein as disclosed above is one wherein the NEF derived antigen comprises or has the amino acid sequence of figure 21.

The antigens encoded by the polynucleotide of the vector genome, and especially the GAG derived antigen, may be of natural, synthetic or recombinant origin and accordingly expressed by any conventional methods.

The disclosure also relates to nucleotidic constructs encoding such fusion antigen, including in their codon optimized version for expression in mammalian, especially in human cells.

According to an aspect of the disclosure, the recombinant polynucleotide encodes an antigen derived from the GAG polyprotein of HIV-1 consensus B strain.

In another aspect of the disclosure, the recombinant polynucleotide encodes an antigen derived from a GAG polyprotein and a cluster of epitopes of NEF antigen of HIV and optionally a cluster of epitopes of POL polyprotein of HIV.

The disclosure relates to nucleic acid molecules encoding the antigen disclosed herein. It relates in particular to the nucleic acid molecules inserted in plasmids deposited at the CNCM and especially the plasmids pTRIPDelta U3-CMV-SIV-GAG-WPRE or pTRIPDelta U3-CMV-SIV-GAG co-WPRE, deposited at the CNCM or the plasmids pThV-VSV-G(IND-co) or pThV-VSV-G(NJ-co) deposited at the CNCM, or to sequences hybridizing in stringent conditions with these nucleic acid molecules and especially having the same length or being shorter. Particular acid nucleics encode at least a GAG antigen or a fragment thereof and especially encodes a HIV-1 or HIV-2 GAG antigen or a fragment thereof.

The specificity of the cellular response is measured when comparing the response obtained with the lentivirus vector particles expressing a heterologous polynucleotide encoding an antigen of HIV or an antigen derived therefrom with the response obtained with particles not expressing said antigen. It is observed that the administration of the particles capable of expressing said HIV antigen or HIV-derived antigen elicit a T cell immune response which is not elicited with the particles not expressing the antigen.

This is illustrated in the examples with particles expressing an SIV derived antigen.

The response is advantageously protective which means that it enables to achieve a decrease in the viral load or to control the viral load measured in the plasma of the host infected with an Immunodeficiency Virus, who has received at least a prime and one or several boosting administrations of the compounds of the combination of compounds for a prophylactic or therapeutic use against infection by an immunodeficiency virus, especially by a HIV in a human host or by a $SIV_{MAC}$ in a non-human primate host.

In other words, when used for prophylactic or therapeutic treatment of an infection by an Immunodeficiency Virus, especially an HIV, the administered combination of compounds allows elimination of the virus from the body, or control of the viral load, for a long lasting period of time (over six months) and preferably enables protection against AIDS disease *in vivo.*

The ability of the combination of compounds to provide tools to elicit a protective specific cellular immune response in a human host, is derived from the experimental results which have been obtained in a macaque/SIVmac non-human primate model in conditions which essentially resemble those observed in the human/HIV-1 situation.

Accordingly, the disclosure relates to the use of a combination of compounds for the preparation of a medicinal product for sequential administration to a mammalian host, to elicit a protective specific cellular immune response against an Immunodeficiency Virus, especially HIV.

Particular lentiviral vectors have been designed according to the invention, to elicit a specific cellular immune response which is shown to be protective in the context of a virus challenge. Although for obvious reason, this demonstration has not yet been carried out in human being, the disclosed results on the non-human primate are highly in favour of similar expectation in human.

The particular lentiviral vectors obtained provide specific interesting candidates for therapeutic vaccination or for prophylactic vaccination against AIDS.

In another aspect of the disclosure, polynucleotides encoding B epitopes and/or T epitopes originating from a pathogenic organism are polynucleotides encoding the envelope E-glycoprotein ($E_{WNV}$) of the West Nile Virus (WNV) or its soluble form or encoding epitopes thereof. Insertion of the coding sequence of the soluble E glycoprotein of WNV ($sE_{WNV}$) may be achieved following the disclosure in (5), using $sE_{WNV}$ as described in (12).

According to another particular aspect of the disclosure, the heterologous polynucleotide encodes a polypeptide which is a tumor associated antigen (TAA) or a fragment thereof.

Non-limiting known examples of TAA are especially :

- mutated peptides found in melanoma such as β-catetin, MART-2, or leukaemia such as brc-abl,
- tissue specific proteins such as gp100, MART-1, tyrosinase, found in melanoma, or PSA, PAP, PSM, PSMA found in prostate cancer,
- cancer-testis antigen such as MAGE,
- Molecules related to tumorigenesis such as Survivin, hTERT, found in various cancers,
- Mucins like MUC-1 found in breast, ovarian or pancreas cancer,
- viral proteins including those of HPV (Human Papilloma Virus), especially HPV16 or HPV18, including the HPV16-E7 antigen (found expressed in cervical cancer), EBV (Epstein-Barr virus) causing lymphoma including EBV-EBNA protein (in lymphoma), , HBV (Hepatitis B Virus), HCV (Hepatitis C Virus), HHV (Human Herpes Virus) such as HHV8 or HTLV (Human T Leukemia Virus) such as HTLV-1, such HTLV-1 tax protein (in Acute T Leukemia).

More generally, these polynucleotides disclosed may be derived from the peptide sequences disclosed in the peptide database entitled Cancer Immunity. The polynucleotides may especially be selected among shared tumor-specific antigens, differenciation antigens, antigens overexpressed in tumors or tumor antigens resulting from mutations.

The disclosure also describes polynucleotide of interest that encodes human antigens.

In another aspect of the disclosure, the polynucleotide of interest may encode a polypeptide whose expression or functional expression is harmed in the host affected with the considered pathology. In a particular embodiment, the lentiviral vectors of the invention are used to deliver the polynucleotide to target cells in the host to seek for genetic correction in a medicinal treatment of gene therapy, for example of genetic diseases that result in serum protein deficiencies, or for genetic vaccination strategies against cancer or infectious, viral or autoimmune diseases. In another embodiment, other pathologies such as diabetes may be treated with the kit of compounds of the invention.

The heterologous polynucleotide is inserted in the vector genome, under the control of regulatory sequences for transcription and expression, including a promoter and possibly an enhancer. In a particular embodiment, the regulatory sequences are not of lentiviral origin. Suitable promoters encompass CMV, also referred to as CMVie promoter, or EF1α promoter, CGA promoter, CD11c promoter and house keeping gene promoters such as PGK promoter, ubiquitin promoter, actin promoter, histone promoter, alpha-tubulin promoter, beta-tubulin promoter, superoxide dismutase 1 (SOD-1) promoter, dihydrofolate reductase (DHFR) promoter, hypoxanthine phosphorybosyltransferase (HPRT) promoter, adenosine deaminase promoter, thymidylate synthetase promoter, dihydrofolate reductase P1 promoter, glucose-6-phosphate dehydrogenase promoter or nucleolin promoter.

The disclosed kit of compounds is especially suited for use in a medicinal treatment, wherein said lentiviral vector pseudotyped with said first viral envelope protein(s) is administered separately in time from said lentiviral vector pseudotyped with said second viral envelope protein(s), and if appropriate said prime and first boost are followed by one or several boosting step(s), later in time.

Accordingly, the disclosed kit of compounds is especially suited for iterative administration of active principles, especially in a prime-boost(s) type reaction, possibly encompassing several boosting steps.

In particular, the compounds of the disclosed kit are such that said lentiviral vectors pseudotyped either with said first viral envelope protein(s) or with said second viral envelope proteins are respectively used for priming an immunogenic reaction or alternatively for boosting said immunogenic reaction in a host in need thereof. The immune reaction may be further boosted by using a lentiviral vector having a third envelope protein(s) as described herein, and optionally additional boosting steps with further envelope proteins which do not sero-neutralize with the one of the other lentiviral vectors.

In an aspect of the disclosure, the lentiviral vector pseudotyped with the VSV-G of the Indiana strain is administered first, in order to prime the immunological reaction, and the lentiviral vector pseudotyped with the VSV-G of the New Jersey strain or with the recombinant or modified VSV-G as disclosed herein is administered in second instance, to boost the immunological reaction.

In another aspect of the disclosure, the lentiviral vector pseudotyped with the VSV-G of the New Jersey strain or with the recombinant or modified VSV-G as disclosed herein is administered first, in order to prime the immunological reaction, and the lentiviral vector pseudotyped with the VSV-G of the Indiana strain is administered in second instance, to boost the immunological reaction.

The disclosure describes an embodiment corresponding to an administration protocol with one round of administration of both compounds of the kit may be sufficient to elicit a strong response.

To possibly improve the intensity or the spectrum or the duration of the response, further administration steps may be performed. In particular, a lentiviral vector pseudotyped with an envelope chosen among VSV-G of Chandipura, Cocal, Perinet, Piry, SVCV or Isfahan viruses or a recombinant envelope comprising a domain of one of these envelopes, as described herein, may be used.

The disclosed kit of compounds is suitable for use in prophylactic treatment or therapeutic, including curative, treatment against a viral disease or against an infectious or tumoral disease, wherein said lentiviral vector comprises a polynucleotide encoding one or several viral antigens or fragments thereof suitable to elicit an immune response.

In addition to being suitable to prepare a combination of compounds for the therapeutic treatment of mammalian hosts infected with an Immunodeficiency Virus, in particular a human host infected with a HIV or a non-human primate host infected with a $SIV_{MAC}$ or an animal infected with FIV, the lentiviral vectors disclosed herein also provide tools for the design of a combination of compounds for a prophylactic use against infection by an immunodeficiency virus, especially by a HIV in a human host or by a $SIV_{MAC}$ in a non-human primate host or by FIV in an animal.

The combination of compounds disclosed herein may especially be used for the therapeutic treatment of human hosts infected with a HIV-1 or HIV-2.

The combination of compounds disclosed herein may especially be used for the prophylactic treatment of human hosts against infection by a HIV-1 or HIV-2.

The data provided in the experimental section hereafter provide indeed strong evidence of the relevancy of the designed lentiviral vector for transposition to medicinal applications in human. The level of protection achieved on the non-human primate model depicted in the examples is stronger than results reported in the literature with other vaccine candidates and it is noteworthy that it was obtained in the context of virus challenge with a particular high dose of infectious SIVmac virus.

From the experimental data obtained, it is even observed that the combination of compounds for the elicitation of a protective specific cellular immune response against an immunodeficiency virus may be prepared without adding an adjuvant of the immune response.

The skilled person may however decide to include in the combination of compounds, in association to all or part of the lentiviral vectors or/and as a further separate compound, an immunomodulating agent. For example, a cytokine such as 1112 may be included in the combination.

The disclosure provides a combination of compounds wherein said lentiviral vectors are formulated in compositions suitable for injection to a host, especially for sub-cutaneous injection.

The lentiviral vectors of the invention are thus in particular for use in an administration regimen involving injection to the host and encompassing priming the immune response and subsequently boosting the immune response in a mammalian host, wherein said (i) lentiviral vector pseudotyped with said first viral envelope protein(s) is administered separately in time from said (ii) lentiviral vector pseudotyped with said second viral envelope protein(s), and if any from said (iii) lentiviral vectors pseudotyped with said third viral envelope protein(s), each of said lentiviral vectors (i) and (ii) and if any (iii) being administered either for priming or for boosting the immune response.

The choice of the administration regimen may be adapted by the skilled person in view of the intensity and spectrum of the response obtained with selected doses used and number of boosting steps carried out.

In an aspect the disclosure, concerns a combination of compounds for sequential administration to a human host, to elicit a protective specific cellular immune response against a HIV and the administration regimen encompasses administering the same dose of lentiviral vector for prime and boost steps.

According to another aspect of the disclosure, the kit of compounds is suitable for use in gene therapy *in vivo*. Examples of diseases that may be treated with the compounds of the kit of the invention for *in vivo* gene therapy are neurodegenerative diseases such as Parkinson disease, Amyotrophic lateral sclerosis (ALS), Spinal Muscular Atrophy (SMA) which are motor neurone diseases. Another example of disease that can be treated with the kits of compounds of the invention is the spinal cord injury.

The disclosed kit of compounds is also suitable for the treatment of cancer, wherein iterative administration of the lentiviral vector may be necessary.

The disclosure also describes an immunogenic composition compring a lentiviral particle as defined in the present application, suitable for inhibiting *in vivo* a HIV-1 or HIV-2 infection or a SIV or a FIV infection in a mammalian host.

The disclosure also relates to a method of treatment of a host or patient in need thereof, which comprises the successive administration to the host of:

(i) a lentiviral vector, pseudotyped with a first determined heterologous viral envelope protein or viral envelope proteins;
followed by,
(ii) a lentiviral vector, pseudotyped with a second determined heterologous viral envelope protein or viral envelope proteins different
from said first determined envelope protein or envelope proteins; wherein said lentiviral vector of (i) and (ii) encodes a heterologous polynucleotide having a therapeutic interest.

In a particular aspect of the disclosure, a third step of administration to the host of a lentiviral vector pseudotyped with a third envelope protein(s) as disclosed herein is carried out.

According to a particular aspect of the disclosure, additional administration steps are performed in order to boost the immune reaction further.

The time left between the two first administration steps may be in the range of 3 to 12 weeks or more depending on the response to the prime. The time left between the first boost and the last boosting step may be in the range of a few weeks, especially more than 12 weeks, for example 6 months, and even may be one or even several years.

A treatment or a medicinal treatment according to the invention aims at improving the clinical condition of a patient, especially a human being, in need thereof, who has been diagnosed as infected (even at a stage of primo-infection) by a pathogen or as suffering from a pathological state, or this treatment aims at the elimination of the causative agent or organism of the disease, or at lowering said agent or organism. In a situation of viral infection, the treatment may result in a significant decrease of the viral load in the plasma of the host and possibly in a plasma viral load which is less than what can be detected when measured or, at lowering the size or the development of the tumor if any.

Medicinal treatment includes, when referring to a patient diagnosed with a pathological state, improving the clinical status of said patient and in a preferred embodiment, restoring to health.

It also encompasses a prophylactic treatment of a host in need thereof, especially vaccination to prevent the occurrence of a pathological state in a host.

Although it seems not to be necessary in the case of administering lentiviral vectors expressing SIV or HIV antigens, it may be decided, in other applications to further include in the combination of compounds, adjuvant and/or vehicle when used for systemic or local administration, or it may be devoid of such components.

In any cases suitable excipients for the formulation of the medicinal compositions may be added.

The compositions quoted above can be injected in a host via different routes: subcutaneous (s.c.), intradermal (i.d.), intramuscular (i.m.) or intravenous (i.v.) injection, oral administration and mucosal administration, especially intranasal administration or inhalation. The quantity to be administered (dosage) depends on the subject to be treated, including considering the condition of the patient, the state of the individual's immune system, the route of administration and the size of the host. Suitable dosages range expressed with respect to the content in equivalent p24 antigen of vector particles (for HIV-1 lentiviral vectors) and can be determined.

Other examples and features of the invention will become apparent in the examples and figures.

Figure 1: Various examples of DNA flap sequences derived from different viruses.

Figure 2: (A) vector genome construct organization for the purpose of the invention, based on a typical HIV-1 genome sequence; (B) Schematic representation of the TRIP/sEwnv vector ; (C) restriction map of pTRIP ΔU3: CMV GFP; (D) restriction map of pTRIP ΔU3' EFIα GFP.

The following abbreviations are used: U3, R and U5 represent the domains of the LTR; ΔU3: deletion of. the U3 domain: RRE: Rev-responsive element; ψ: encapsidation signal; cPPT and CTS represent the DNA flap; CMVie: cytomegalovirus immediate early promoter.

Details on the construct and especially on the DNA flap and on its insertion in a HIV-1 based genome are available in (Zennou et al 2000).

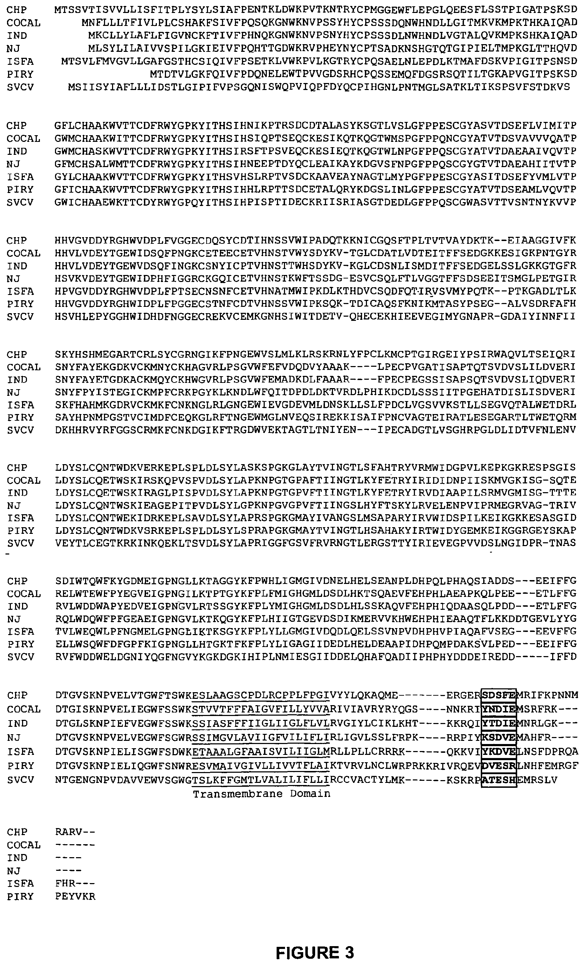

Figure 3: VSV-G protein sequences from various serotypes known in the Vesiculovirus genus for VSV species : Indiana (NCBI Accession Number J02428), Chandipura (J04350), Piry (D26175), New Jersey, Cocal (AF045556), Isfahan (AJ810084) and Spring viremia of carp virus (SVCV)(AY527273). The Indiana protein and New Jersey protein are those used in the examples.

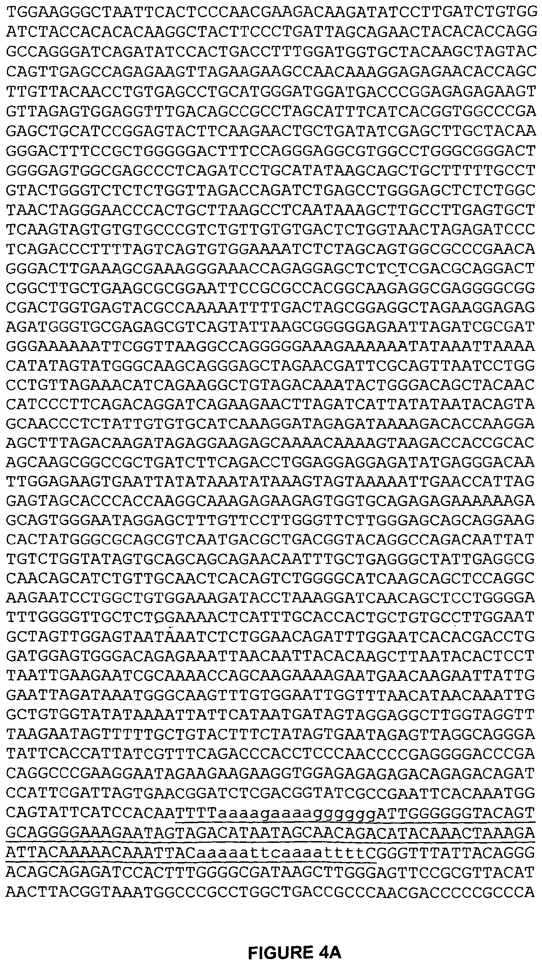

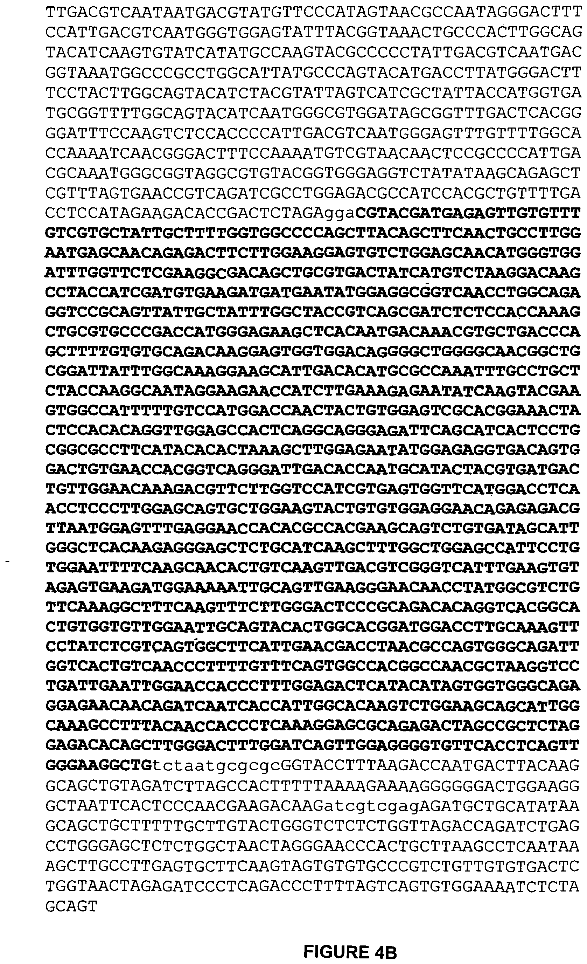

Figure 4: Nucleotide sequence of the TRIPsEwnv vector. The cPPT/CTS region is underlined. In this region, cPPT and CTS domains appear in lowercase. The sEwnv sequence, represented in bold, is a BsiWi-BssHII DNA insert. This vector has been deposited at the CNCM (Paris, France), under number I-3076, on August 27, 2003.

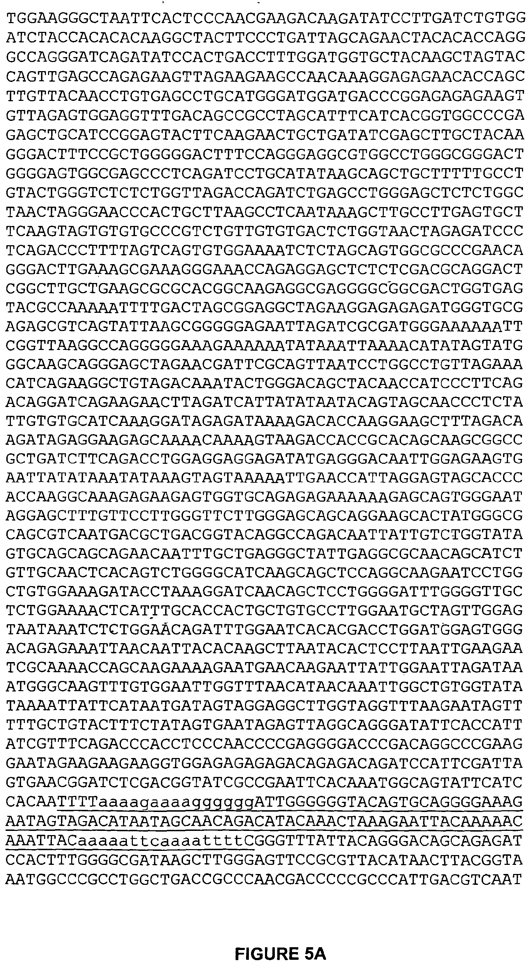

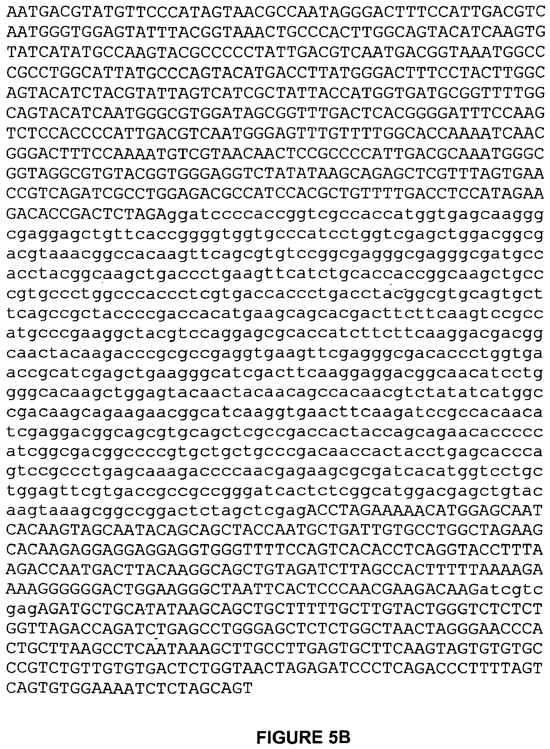

Figure 5: Nucleotide sequence of the TRIP GFP vector. The cPPT/CTS region is underlined. In this region, cPPT and CTS domains appear in lowercase. The GFP sequence is located between nucleotides 2816 to 3573. This vector has been deposited at the CNCM, under number I-2330, on October 11, 1999 (pTRIP [deltaU3] CMV GFP).

Figures 6-12 : VSV-G protein sequence and coding codon optimized nucleic acid for various strains of VSV. An envelope plasmid comprising each codon optimized sequence is described. The plasmid is derived from pThV plasmid and comprises

-    A CMV promoter that may be substituted by another promoter;
-    A codon optimized polynucleotide encoding VSV-G;
-    A WPRE (ΔATG) sequence which is optional ;
-    A polyA sequence
-    A kanR (kanamycine resistance gene) that may be substituted or deleted
-    An origin of replication (pUC ORI)

The VSV-G envelope represented are respectively:

Figure 6: Indiana VSV-G. This envelope has been inserted into plasmid pThV-VSV-G (IND-CO) deposited under I-3842.

Figure 7: New Jersey VSV-G. This envelope has been inserted into plasmid pThV-VSV-G (NJ-CO) deposited under I-3843. The deposited plasmids are in E coli cells.

Their suitable growth medium is LB Kanamycin 10μg/ml and the incubation temperature is 37°C. For storage they may be suspended in fluid with 50% LB and 50% Glycerol.

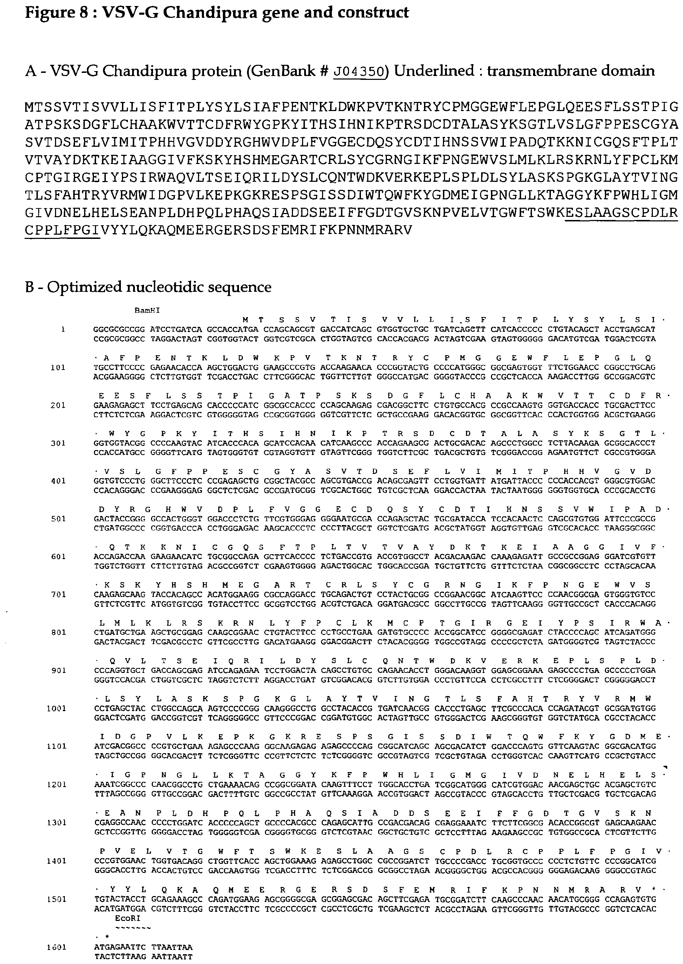

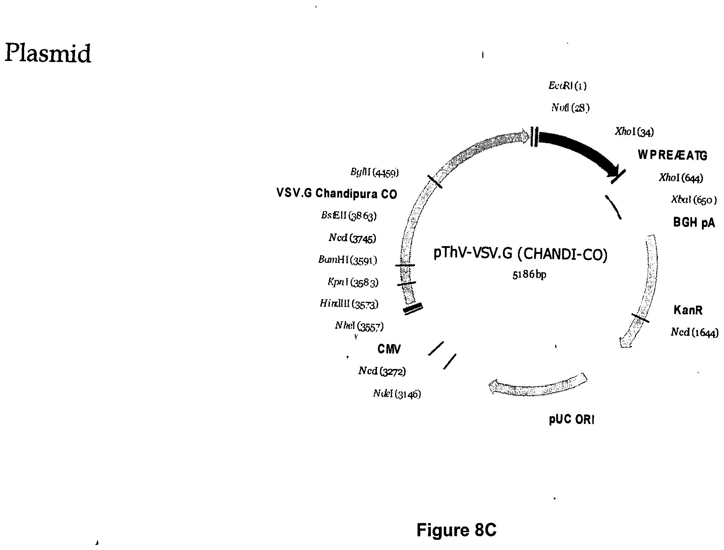

Figure 8: Chandipura VSV-G

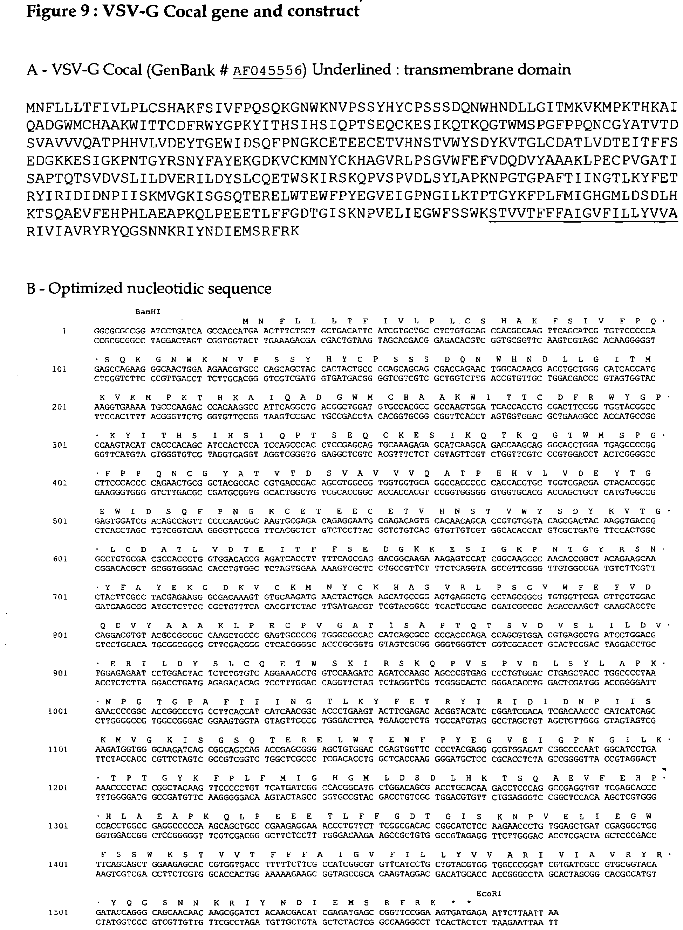

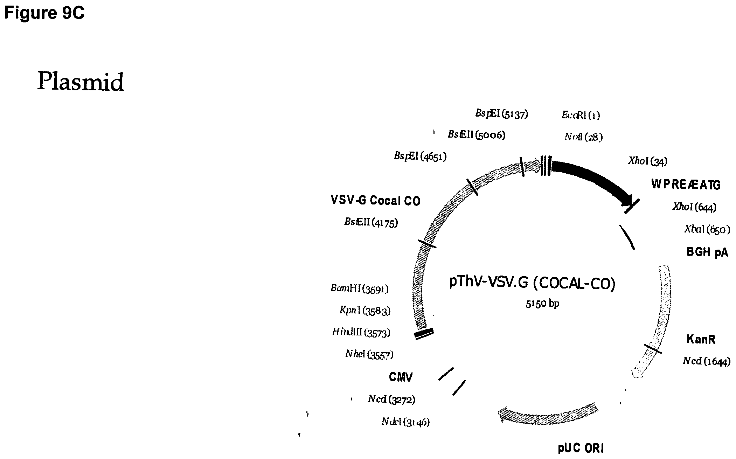

Figure 9: Cocal VSV-G

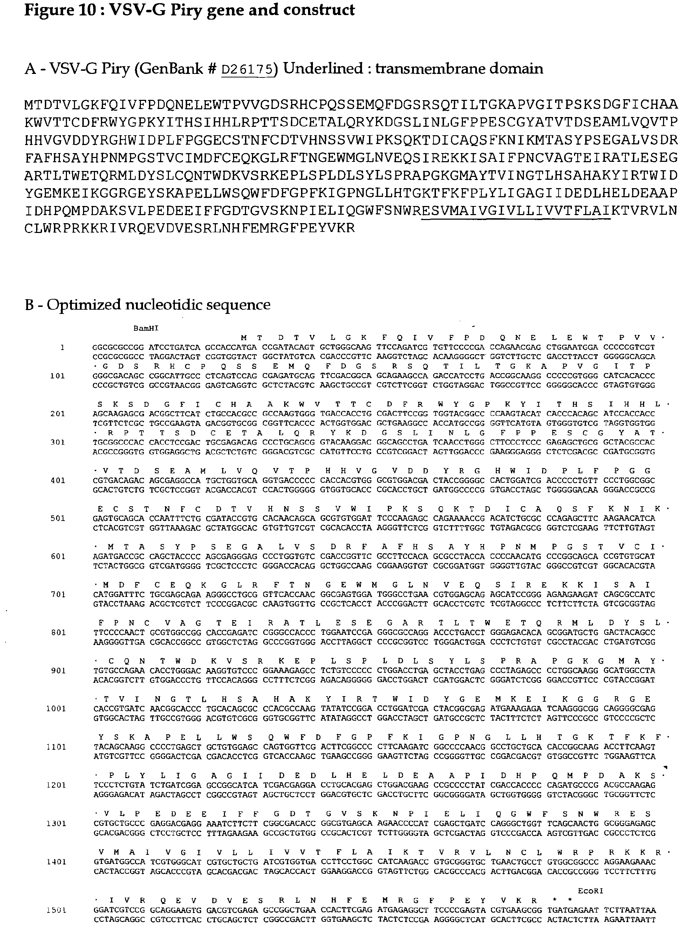

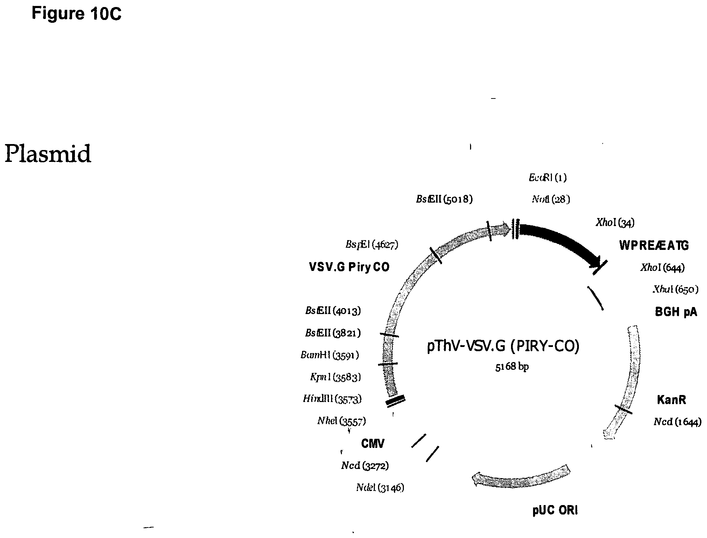

Figure 10: Piry VSV-G

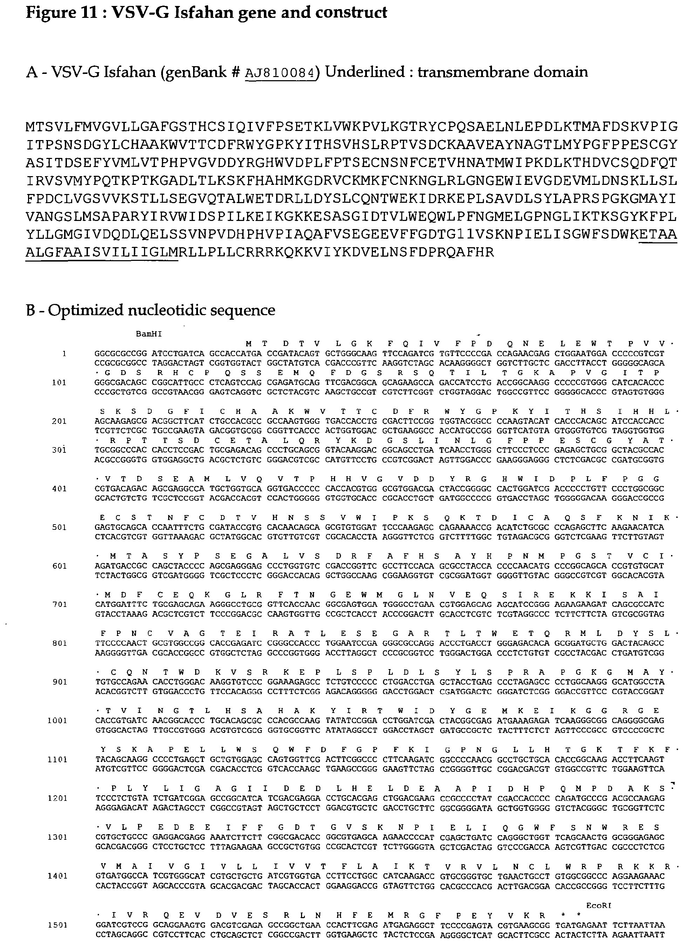

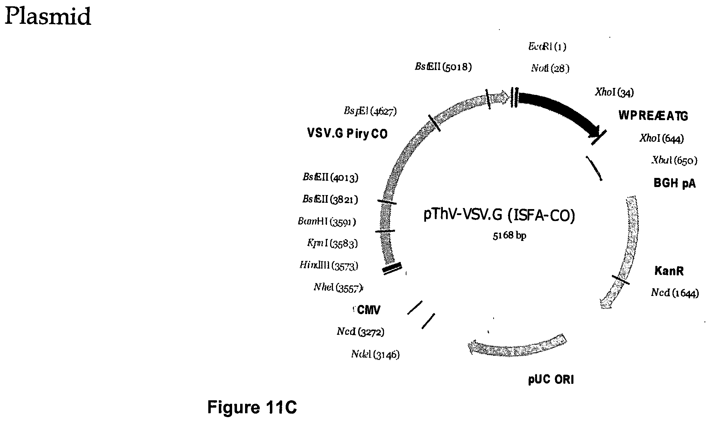

Figure 11: Isfahan VSV-G

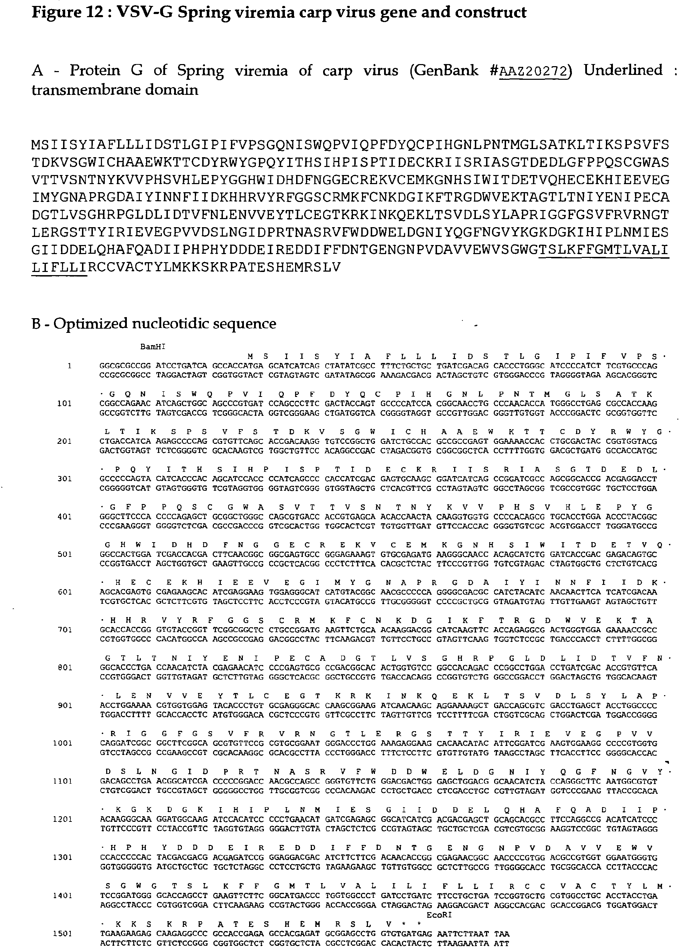

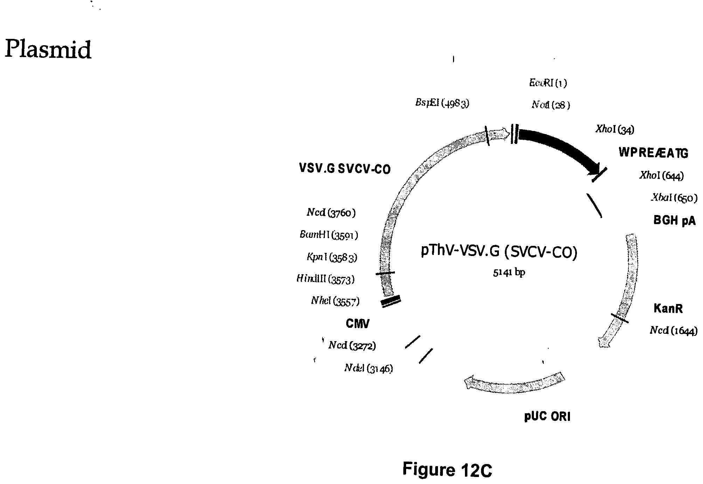

Figure 12: SVCV-VSV-G

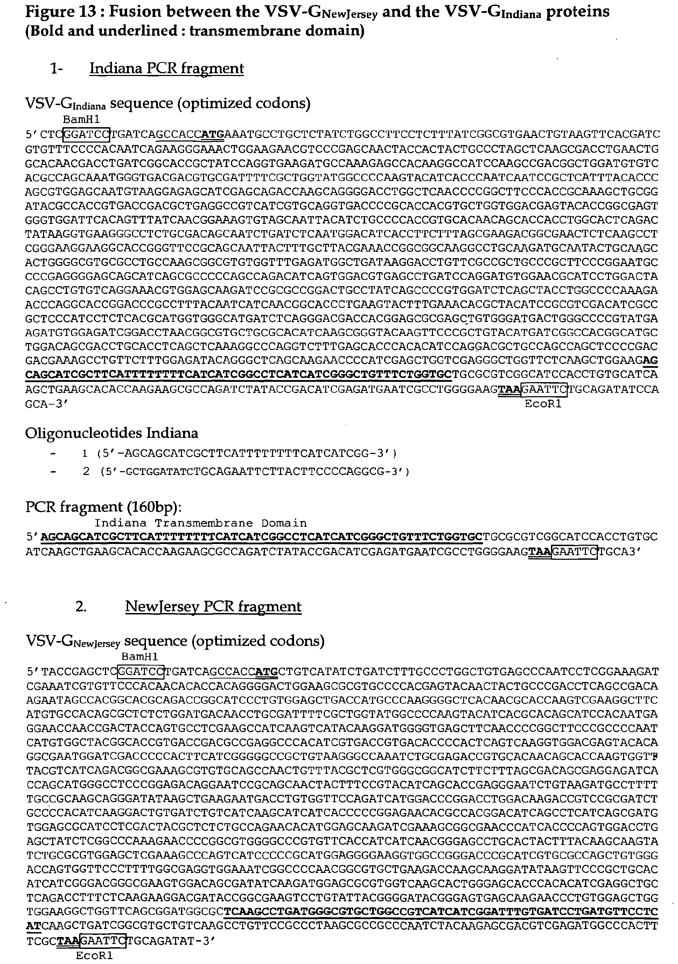

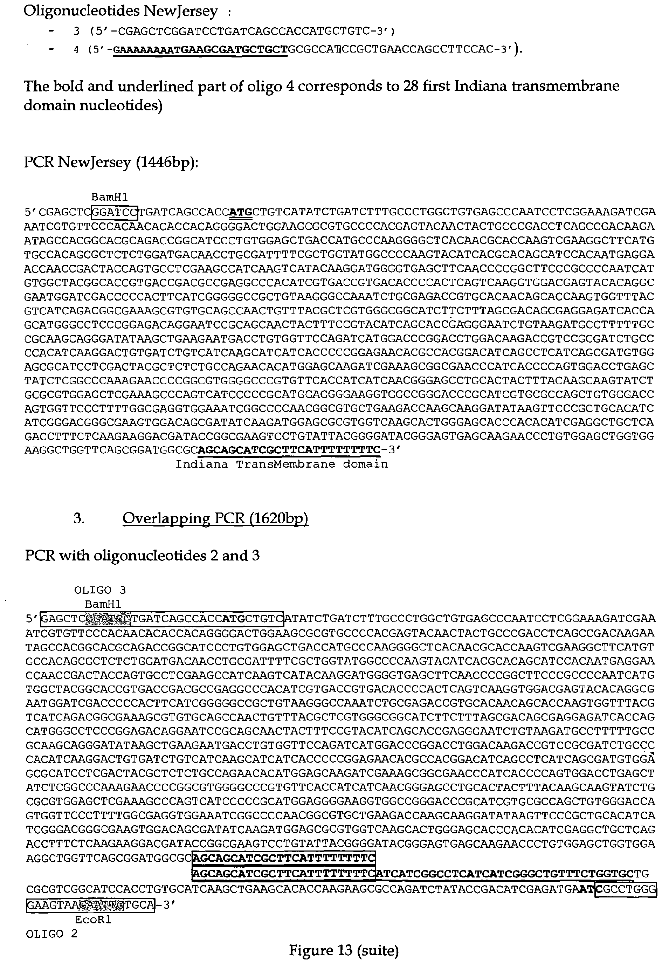

Figure 13 represents a fusion gene between the VSV-G New Jersey and the VSV-G Indiana genes. The transmembrane domain is in bold and is underlined.

The PCR strategy for the preparation of the fusion gene is disclosed. The oligonucelotides used as primers are described.

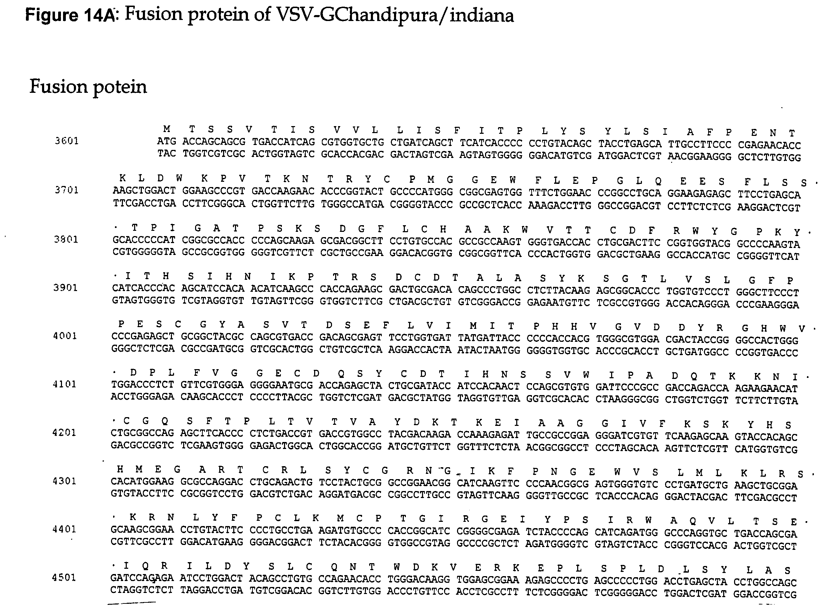

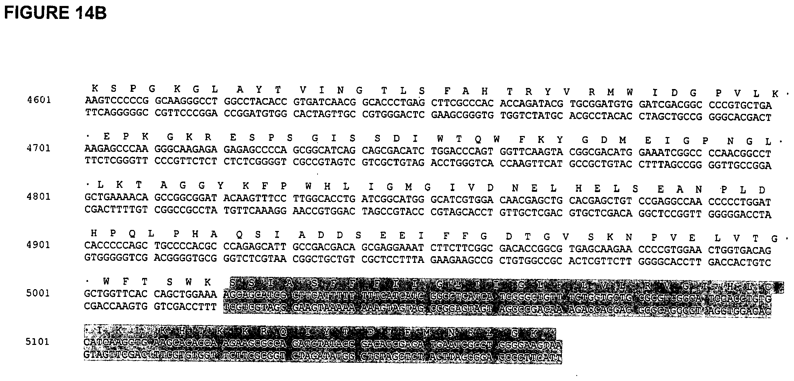

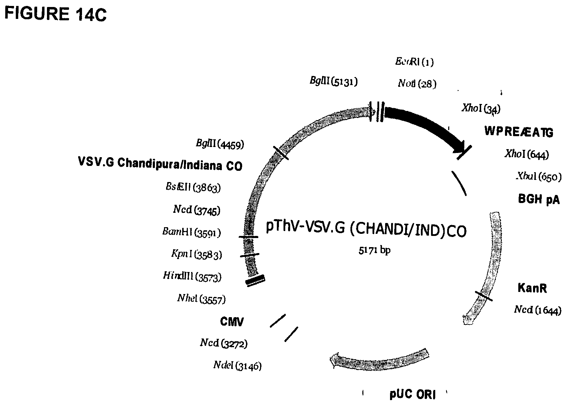

Figures 14 to 19 disclose fusion proteins obtained by recombining different domains of various VSV-G proteins. For each protein, the codon optimized (for expression in human cells) nucleic acid is provided, together with a plasmid comprising said nucleic acid.

Figure 14: fusion of VSV-G Chandipura / Indiana

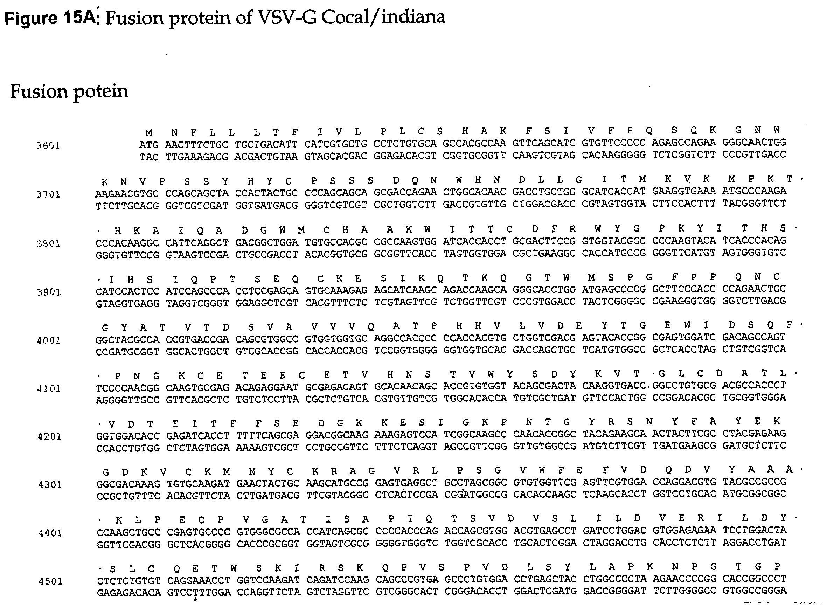

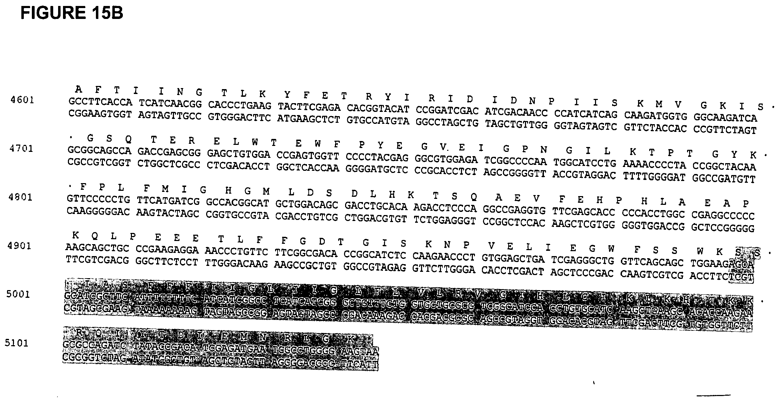

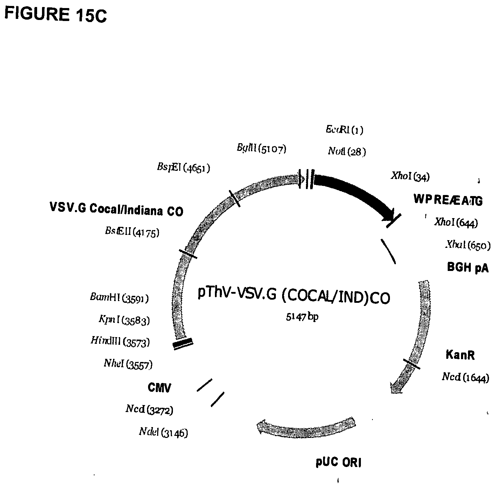

Figure 15: fusion of VSV-G local / Indiana

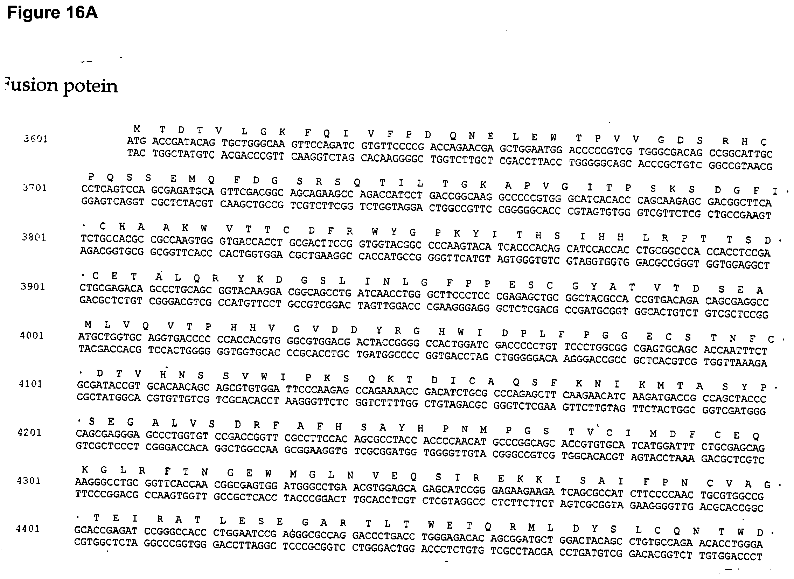

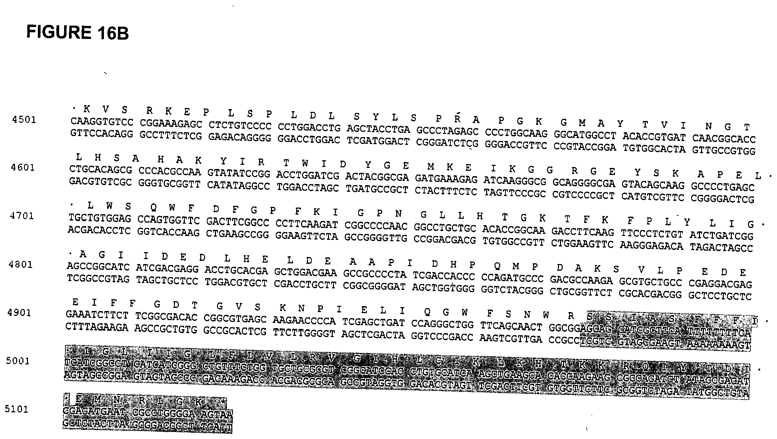

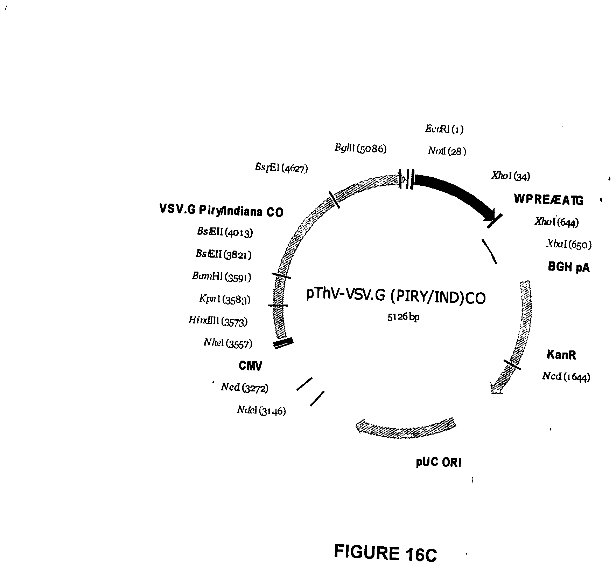

Figure 16: fusion of VSV-G Piry / Indiana

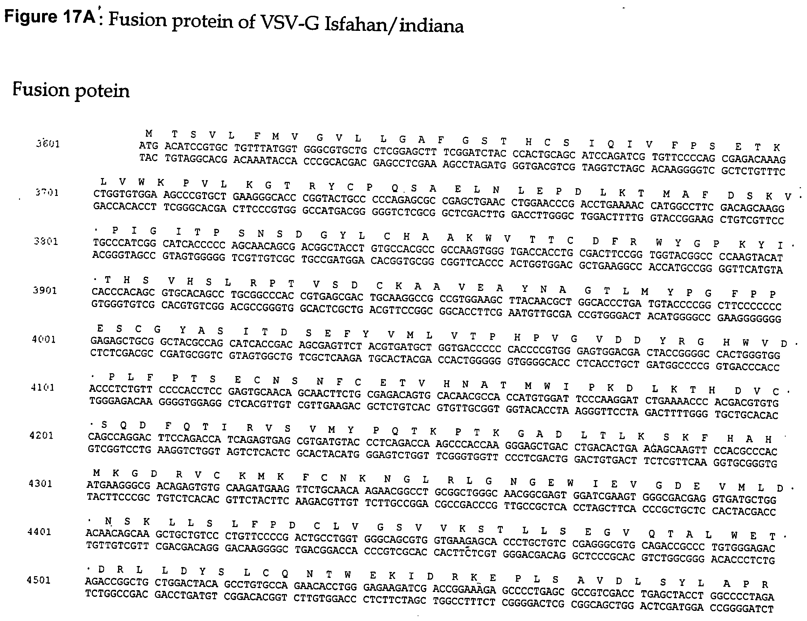

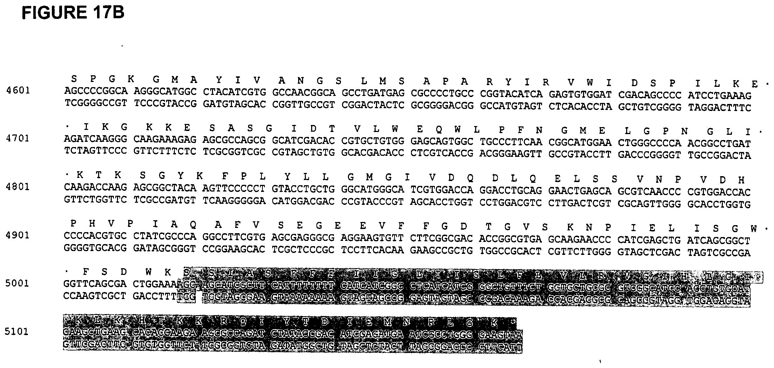

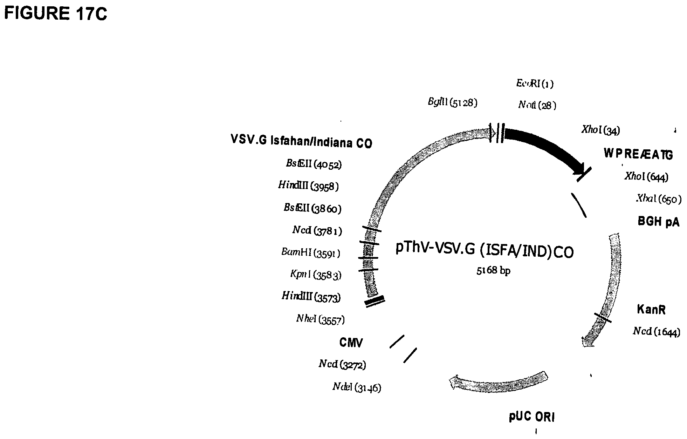

Figure 17: fusion of VSV-G Isfahan / Indiana

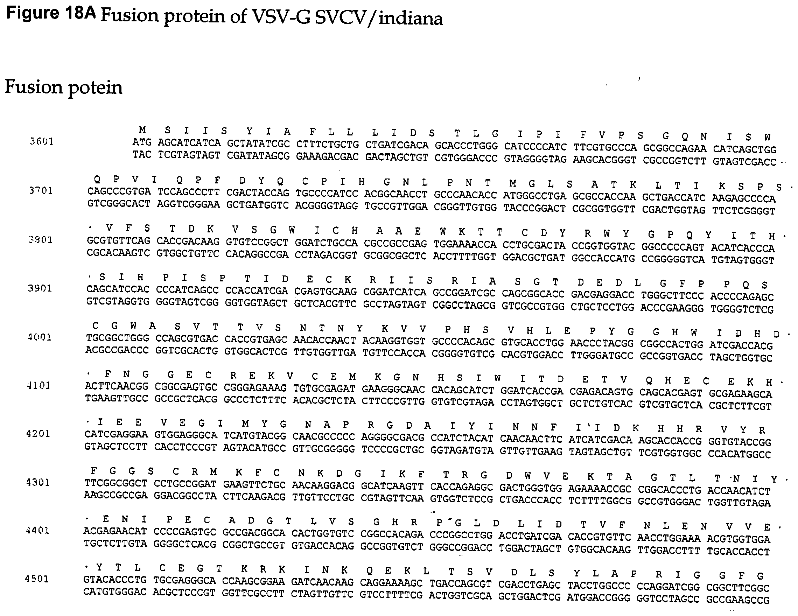

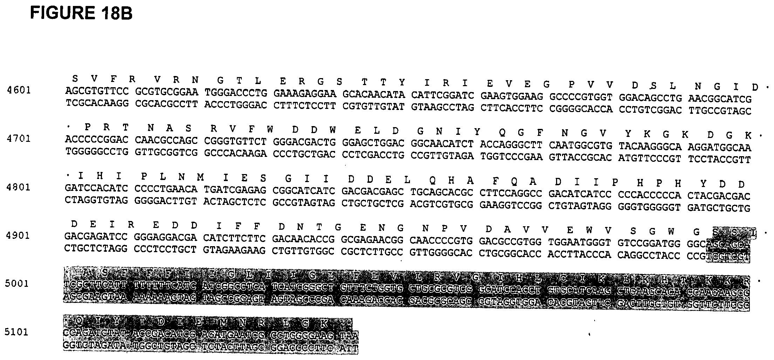

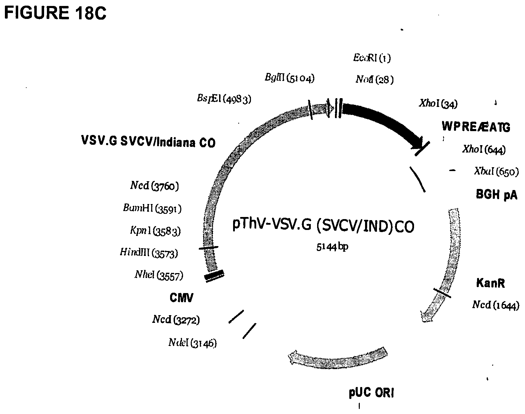

Figure 18: fusion of VSV-G SVCV / Indiana

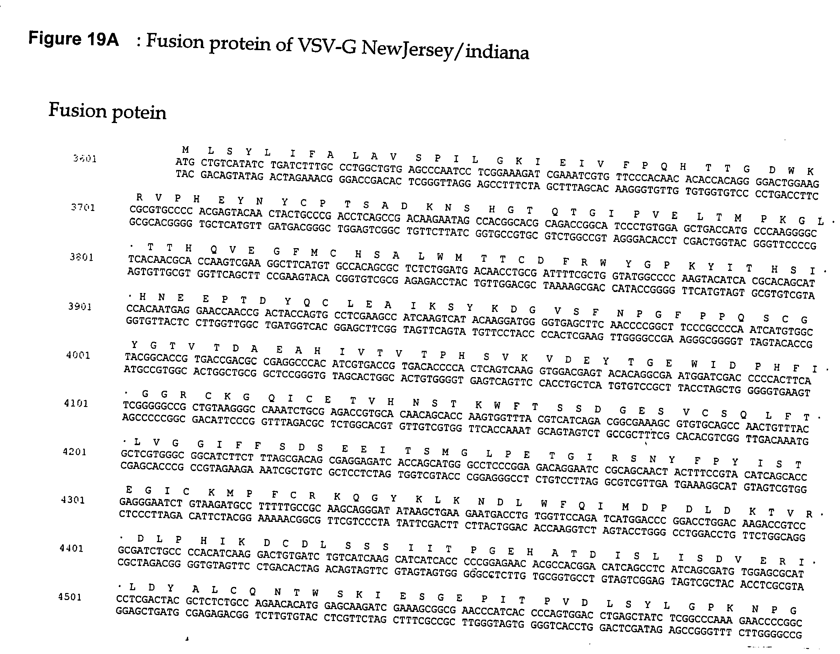

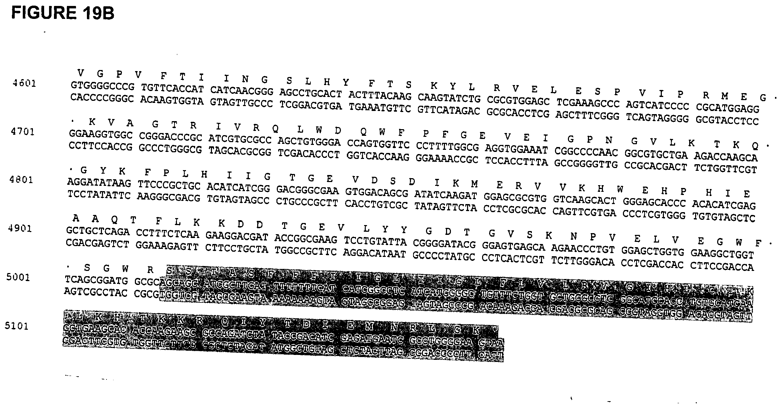

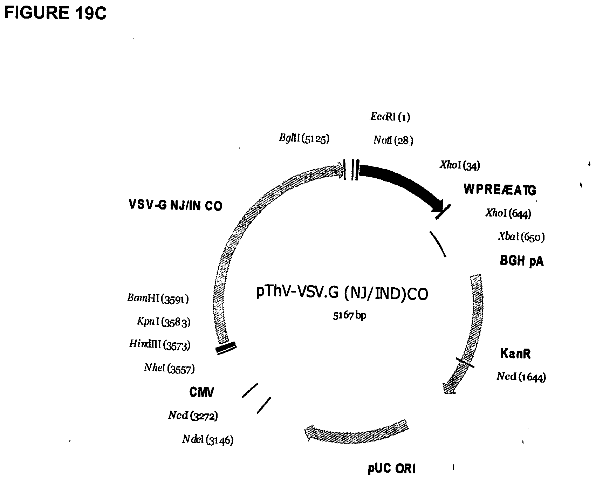

Figure 19: fusion of VSV-G New Jersey / Indiana.

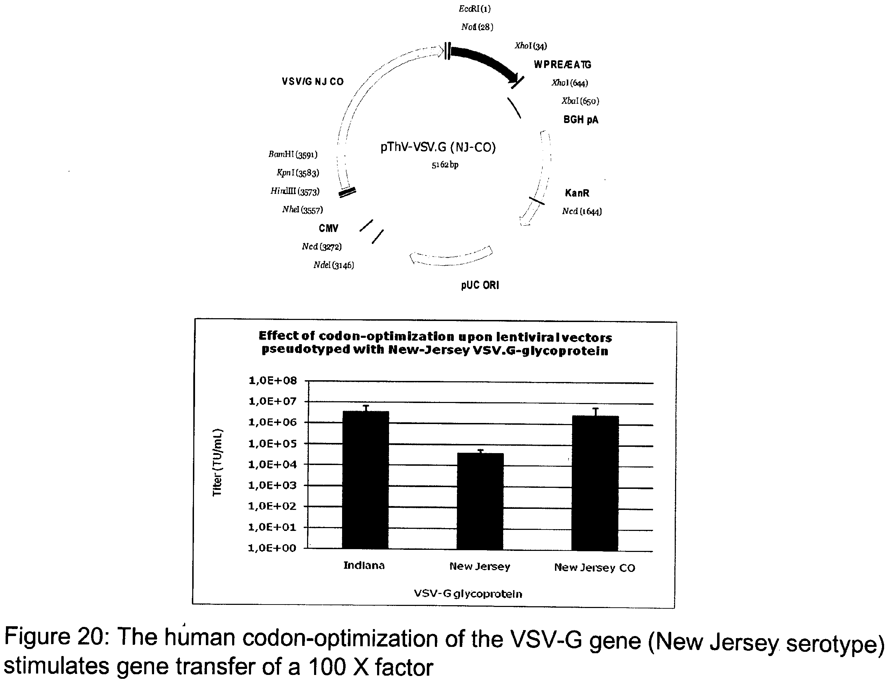

Figure 20: shows the effect of codon-optimization upon lentiviral vectors pseudotyped with New-Jersey VSV-G-glycoprotein. The human codon-optimization of the VSV-G gene (NJ serotype) stimulates gene transfer of a 100 x factor.

Figure 21: illustrates sequences of antigens of interest for the invention. The nucleic acids encoding these antigens, especially in a codon-optimized version for human cells may be inserted in the heterologous polynucleotide of the vector genome. The illustrated antigens are:

- A native GAG antigen of HIV-1 sub type B,
- a modified HIV-1 GAG, which is a delta Myr-GAG antigen prohibiting myristilation, and derived from the consensus sequence of the B subtype;
- an antigen derived from HIV-1 POL, which is a fragment of POL polyprotein;
- an antigen derived from HIV-1 NEF, which is a fragment of NEF protein.

These antigens may be used in combination in a fusion protein. The POL and/or NEF fragments may be inserted 5' or 3' of the GAG derived antigen.

They may be contiguous to each other and inserted 5' or 3' from the GAG derived antigen.

They may be separated and inserted, one in 5', the other in 3' from the GAG derived antigen.

The POL, NEF and GAG derived antigens may be separated or not by a peptide, especially one enabling auto-cleavage. A suitable separating peptide is a 2A peptide from picornavirus having sequence: APVKQTLNFDLLKLAGDVESNPGP.

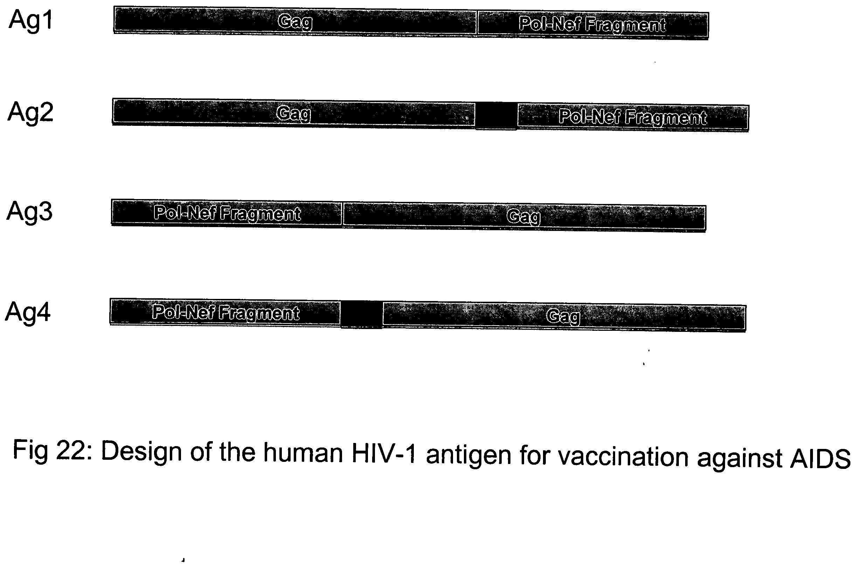

Figure 22 illustrates various antigen constructs according to figure 21, for the design of human HIV-1 antigen for vaccination against AIDS.

Figures 23 to 27: Principle of TRIP Lentiviral Vectors generation and application for the preparation of Lentiviral vector particles expressing an antigen derived from SIVmac239 GAG polyprotein. The same principle would apply for other antigens. The figures describe especially the following features:

Figure 23: Principle of TRIP Lentiviral Vectors generation.
HIV-1 genome (A) is split into a vector plasmid (B), containing the cis-acting sequences (LTR, encapsidation signal, RRE, DNA Flap) and the gene of interest (antigen for vaccination) under the control of an heterologous promoter (CMV) or another promoter, a packaging plasmid (C) containing genes gag, pol, tat and rev, necessary for encapsidation (during vector particle production) and for the early step of viral replication cycle (in transduced cells) and an envelop plasmid (D), containing an Indiana serotype of the glycoprotein G from the VSV. Packaging plasmid and envelop plasmid have heterologous transcriptional regulation elements from CMV and are deleted in encapsidation sequence, in cPPT, and CTS.

Figure 24: Principle of U3' deleted Lentiviral Vector
During reverse transcription of viral single stranded RNA, there is a duplication of U3' and U5' sequences which allow then forming the 5'LTR and 3'LTR in the double stranded viral DNA. Transcription of viral DNA begins in the cell from the LTR 5'. If the U3' region is deleted in vector plasmid (ΔU3), viral RNA is also ΔU3, consequently, after reverse transcription, viral DNA misses the U3 sequence in the 5'LTR, no transcription can begin from the viral LTR promoter. As a consequence, transcription is mediated only via the internal promoter of the transgene.

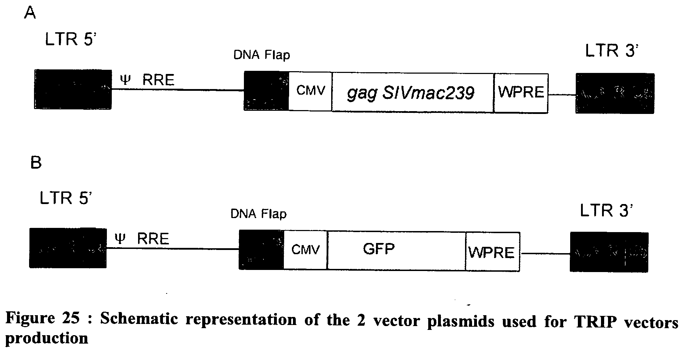

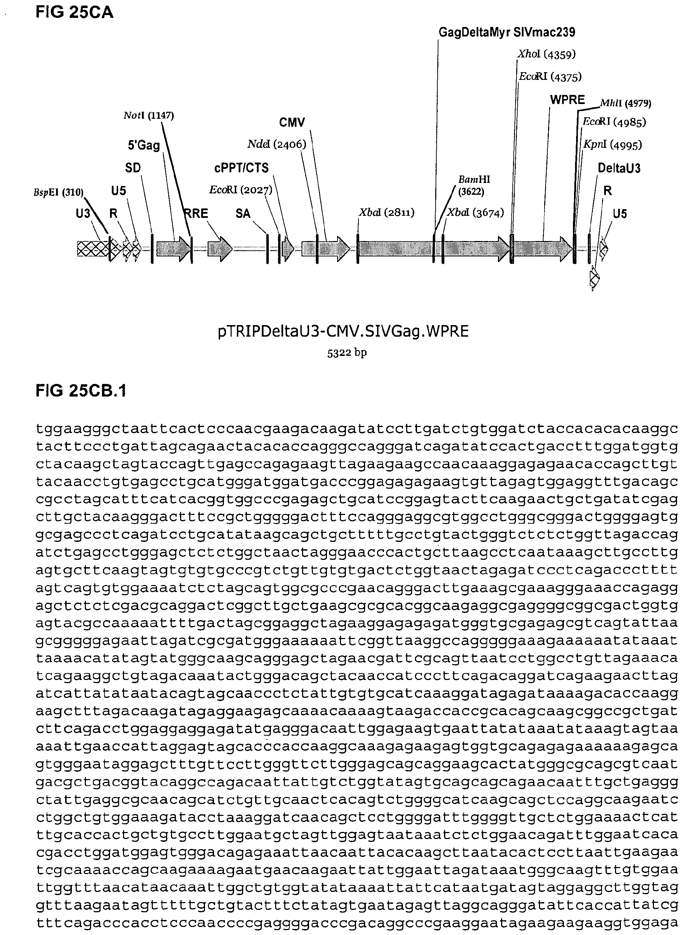

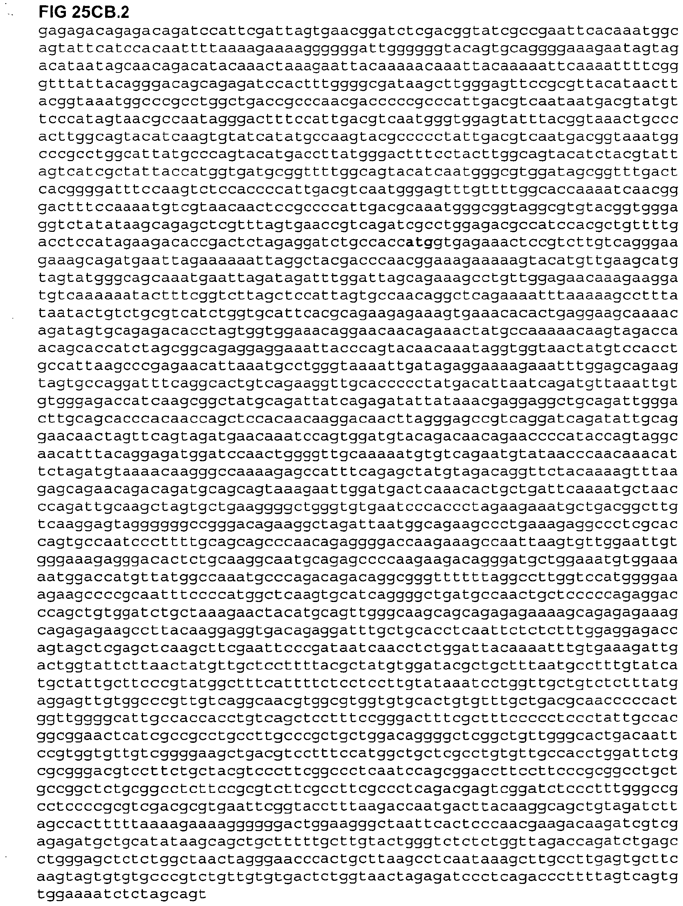

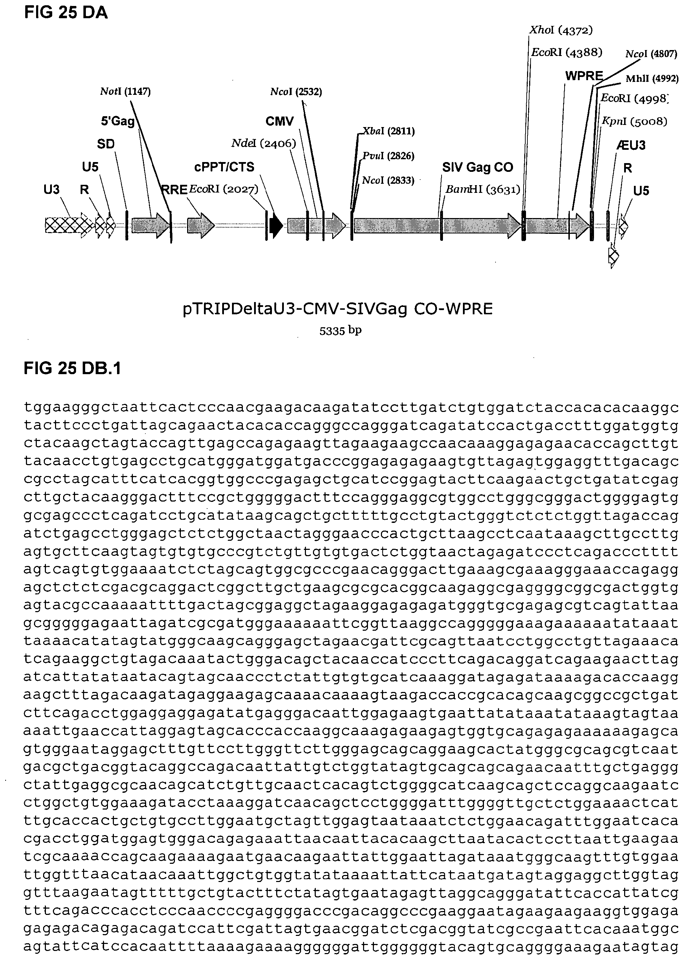

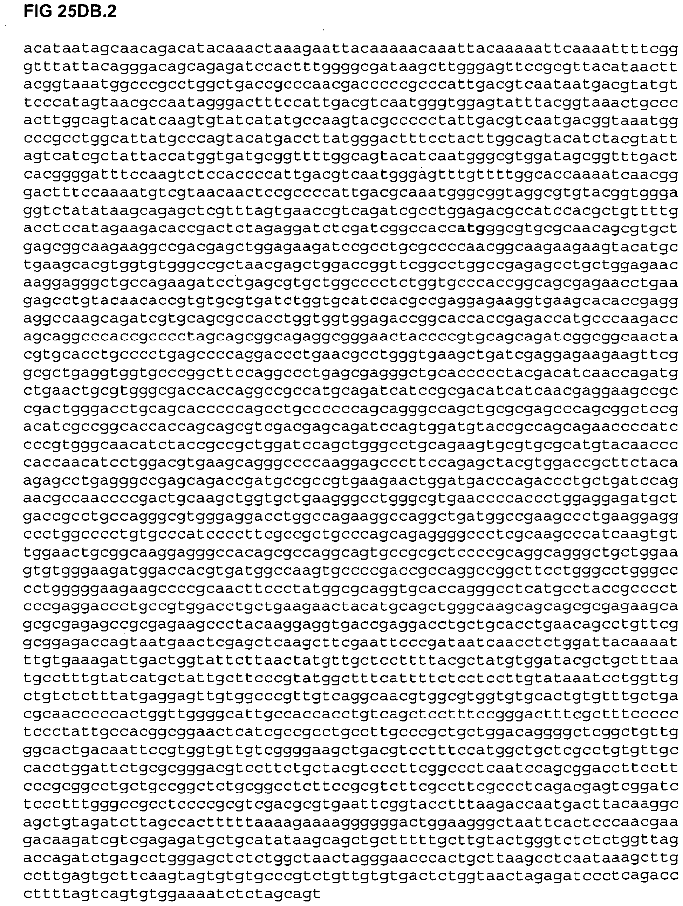

Figure 25: Schematic representation of the 2 vector plasmids used for TRIP vectors production

**A: TRIP-SIVmac239 Gag**.This vector plasmid contains the sequence encoding the antigen, SIVmac239 gag, deleted in the myristilation sequence. This allows to work only in L1, P1 bio-safety level because it abrogates protein secretion in transfected cells and in transduced cells.

**B: TRIP-GFP.**This vector plasmid contains the irrelevant antigen Green Fluorecent Protein (GFP).

Both vector plasmid contain upstream the CMV promoter for antigen expression and downstream the WPRE sequence to improve antigen expression. They also contain the viral sequences necessary for vector particle formation and early steps of viral replication.in transduced cells: Long Terminal Repeat (LTR), DNA Flap (cPPt, CTS), RRE, encapsidation signal ψ.

**C. pTRIP DeltaU3-CMV-SIVGag-WPRE** restriction map of the vector genome and its nucleic acid sequence. The vector construct has been deposited at the CNCM under I-3840.

**D. pTRIP DeltaU3-CMV-SIVGag co-WPRE** restriction map of the vector genome and its nucleic acid sequence. The vector construct has been deposited at the CNCM under I-3841;

The plasmids of the deposits are introduced in E coli cells. The culture medium of the cell is LB Ampi 100μg/ml and the incubation is at 37°C..Storage isin suspending fluid with 50% LB 50% Glycerol.

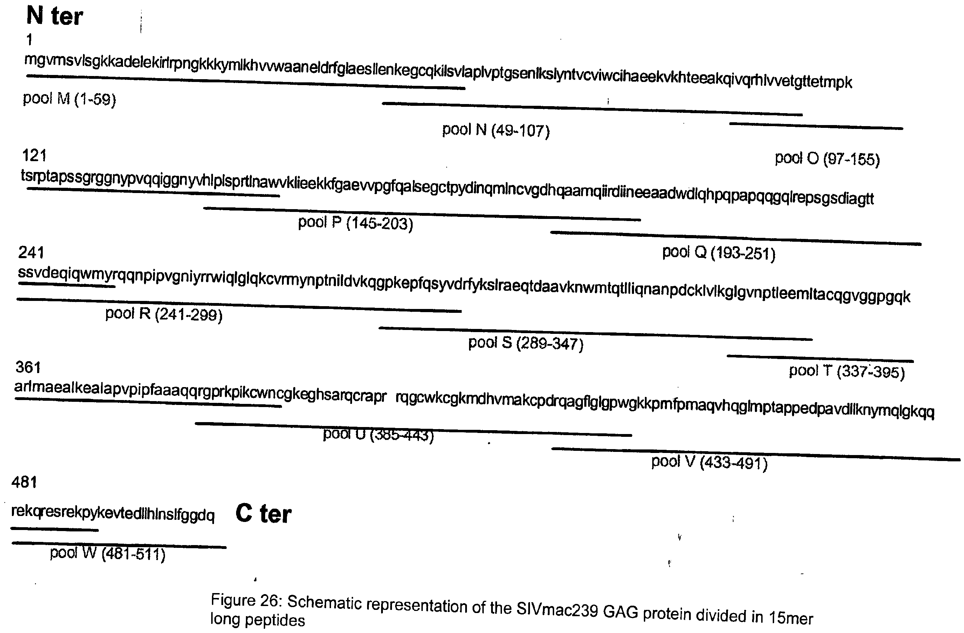

Figure 26: Schematic representation of the SIVmac239 GAG protein divided in 15mer long peptides

The SIV mac239 GAG protein is 511 Amino Acid long. This protein was divided into 125 peptides. These Peptides are 15 amino acids in length; there is 11 amino acids overlap between sequential peptides.

Peptides are dispatched into 11 pools named from letter M to W, containing 5 to 12 peptides.

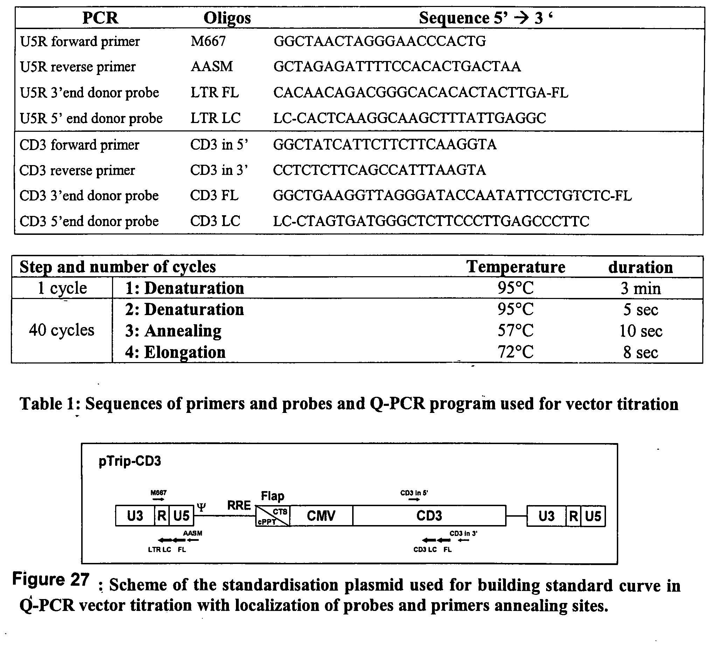

Figure 27: Scheme of the standardisation plasmid used for building standard curve in Q-PCR vector titration with localization of probes and primers annealing sites.

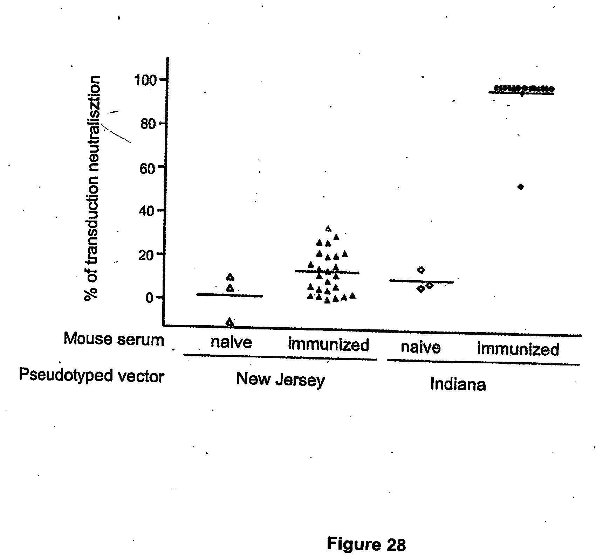

Figure 28: *in vitro* neutralization of transduction of cells with a lentiviral vector pseudotyped with the Indiana VSV-G or with the New Jersey VSV-G, wherein the cells are from a naïve mice or from a mice previously immunized with a lentiviral vector pseudotyped with the Indiana VSV-G.

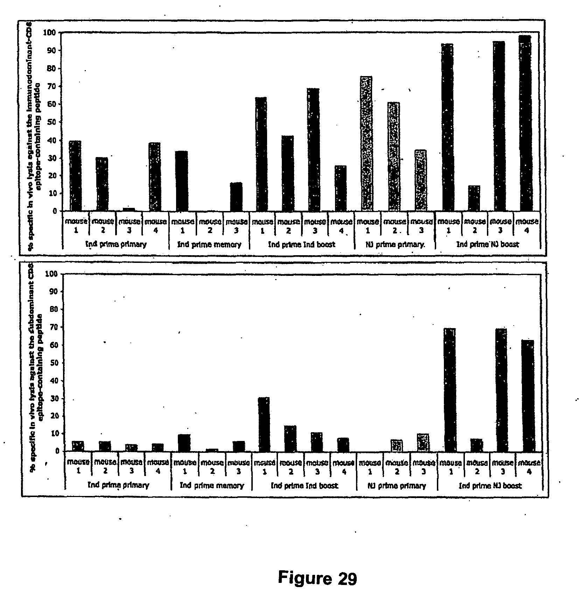

Figure 29: *in vivo* specific lysis against an immunodominant -CD8 epitope containing peptide (A) or against a subdominant CD8 epitope containing peptide (B). Prime or Prime-Boost reactions were performed on individual mice, either with lentiviral vectors having the same VSV-G envelope or with lentiviral vectors having different VSV-G envelopes in the prime and boost reactions.

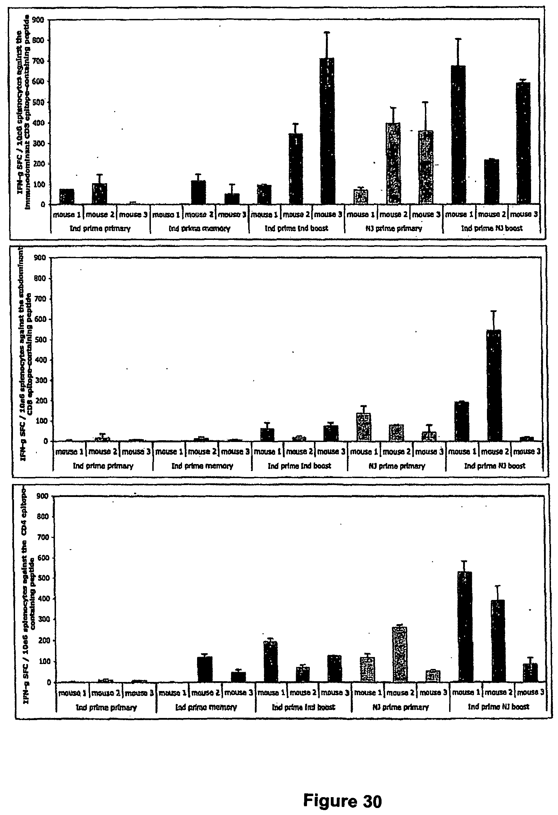

Figure 30: IFN-gamma Elispot test for determining the lysis against an immunodominant -CD8 epitope containing peptide (A) or against a subdominant CD8 epitope containing peptide (B) or against a CD4 containing peptide (C). Prime or Prime-Boost reactions were performed on individual mice, either with lentiviral vectors having the same VSV-G envelope or with lentiviral vectors having different VSV-G envelopes in the prime and boost reactions.

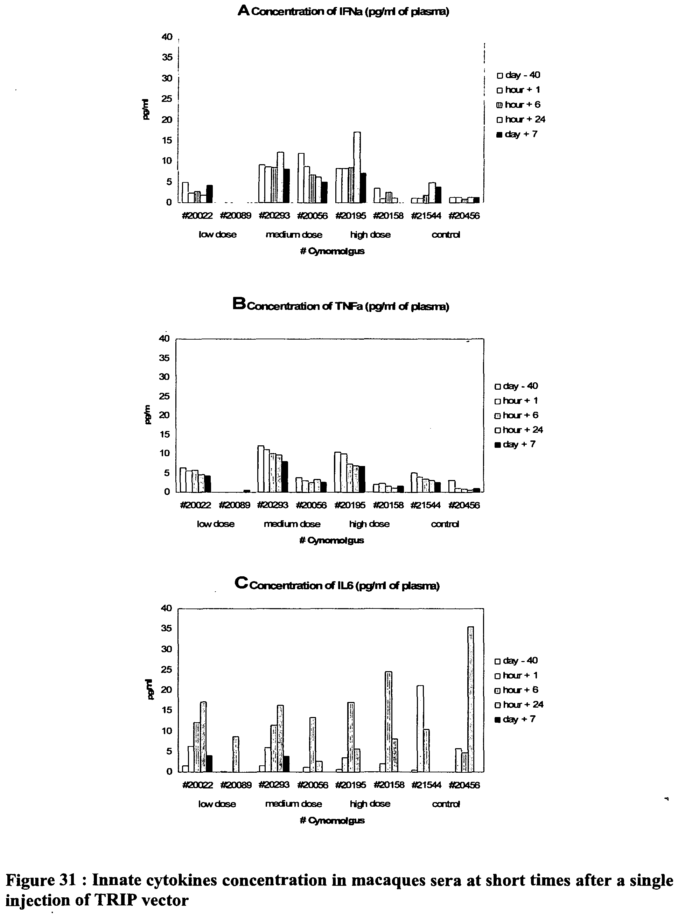

Figures 31 to 37 relates to the experimental results obtained in macaques.

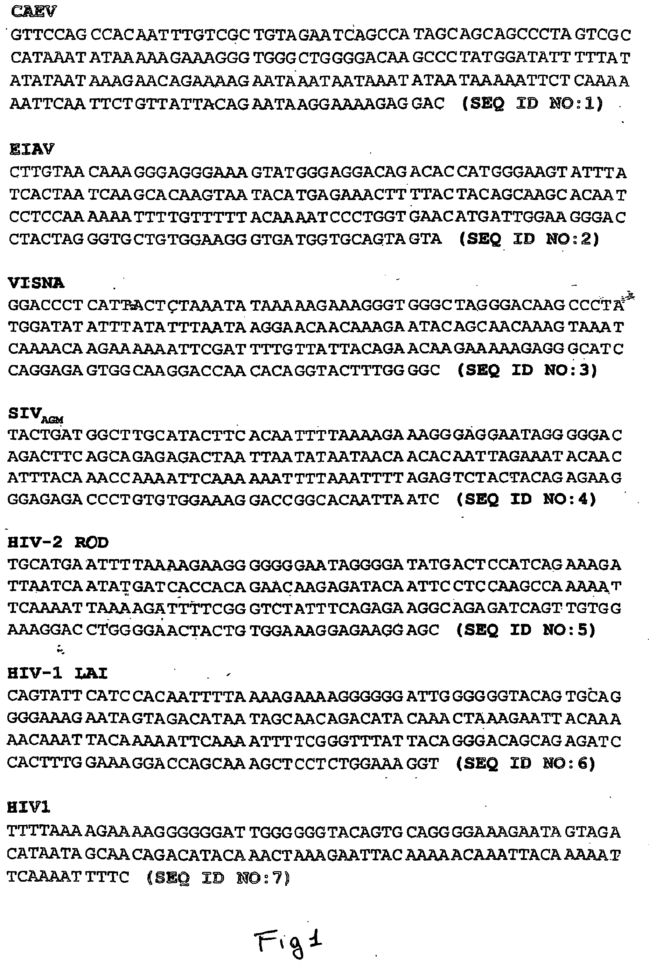

Figure 31: innate cytokines
IFN-a (figure 1A), TNF-a (figure 1B) and IL-6 (figure 1C)
White day -40 pre-immunization
Dots 1 hour post-prime
Striped 6 hours post-prime
Grey 24 hours post-prime
Black 7 days post-prime

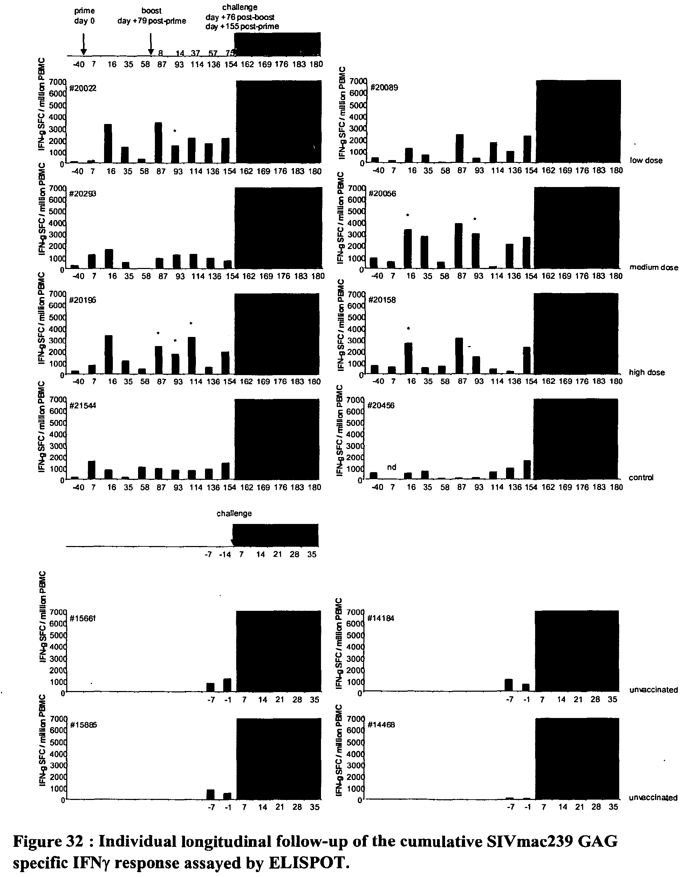

Figure 32: longitudinal follow-up of the gag-specific T cells responses by IFN-g ELISPOT

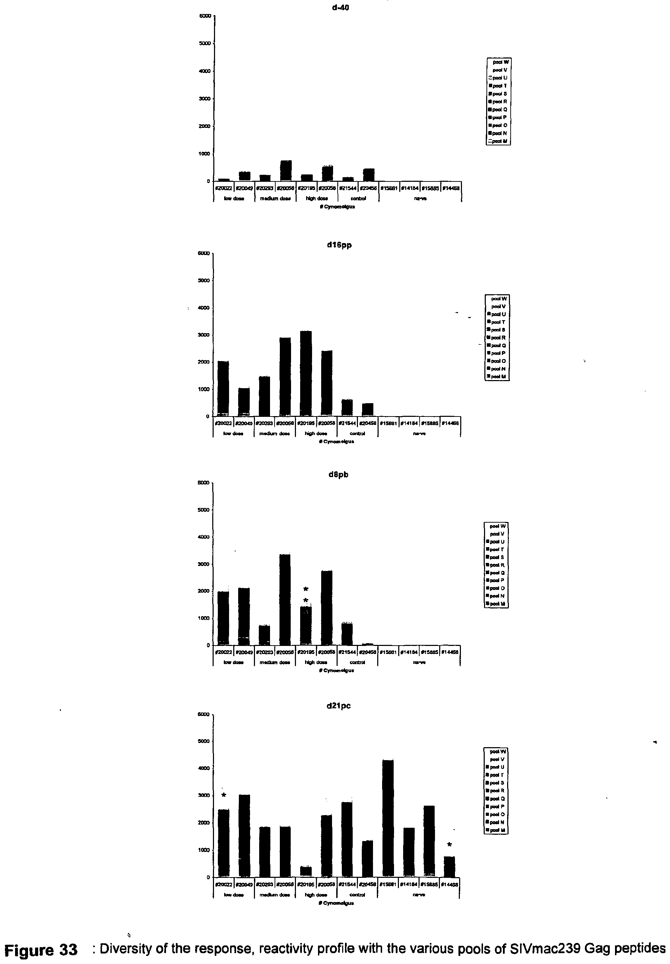

Figure 33 : Diversity of the response, reactivity profile with the various pools of SIVmac239 Gag peptides. We assessed the frequency of IFNγ SFC/million PBMC as the number of cells capable of secreting IFNγ after *ex vivo* restimulation with each individual pool of peptides encompassing the SIVmac239 GAG protein. Preimmunization (d-40), peak of primary response (d16 pp) and peak of secondary response (day 8 pb) and 21 days after challenge (21pc).

nd: not determined

*: saturation

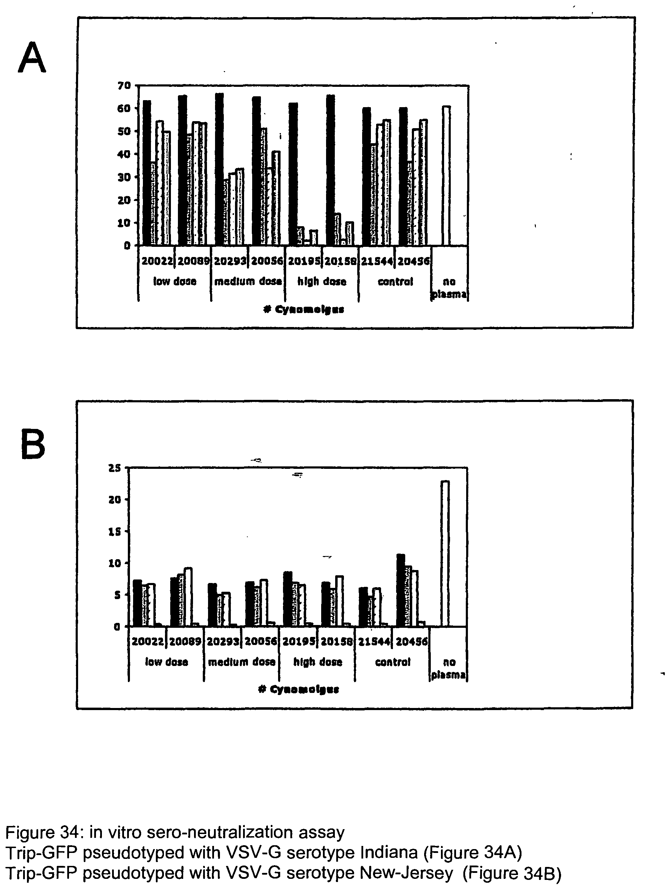

Figure 34: in vitro sero-neutralization assay
Trip-GFP pseudotyped with VSV-G serotype Indiana (Figure 34A)
Trip-GFP pseudotyped with VSV-G serotype New-Jersey (Figure 34B)
black day -40 pre-immunization
grey day 16 post-prime
dots day 58 post-prime
striped day 93 post-prime i.e. day 14 post-boost

white no plasma.

Macaques were immunized with TRIP vector pseudotyped with Indiana serotype VSV-G (prime) and then with TRIP vector pseudotyped with New-Jersey serotype VSV-G (boost). On the day of transduction, cells were cultured with TRIP-GFP (A: pseudotyped with VSV-G serotyped Indiana or B: New-Jersey serotype) with different dilutions of plasma from the macaques. Cells were mocked transduced with the same volume of complete medium. Seventy-two hours after transduction, efficiency of transduction was assessed by analysing the GFP fluorescence. We compared the fluorescence of cells incubated with plasma from vaccinated animals and mock-incubated.

We also took as comparison the level of fluorescence prior immunization (day-40).

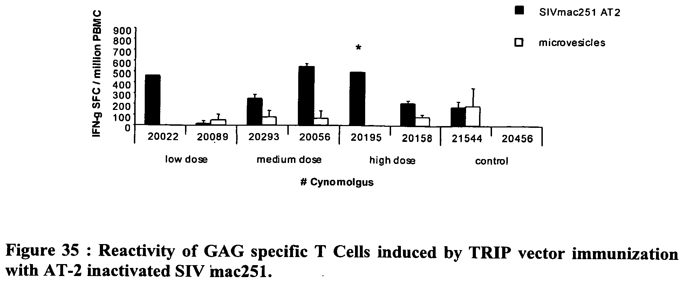

Figure 35: reactivity with AT-2 inactivated SIVmac251 16 days post-boost

We assessed the frequency of IFNγ SFC/million PBMC as the number of cells capable of secreting IFNγ after *ex vivo* restimulation with SIV AT-2.

Mock stimulation was done using microvesicles, present in the supernatant of SIV-infected and non infected producer cells. *: saturation

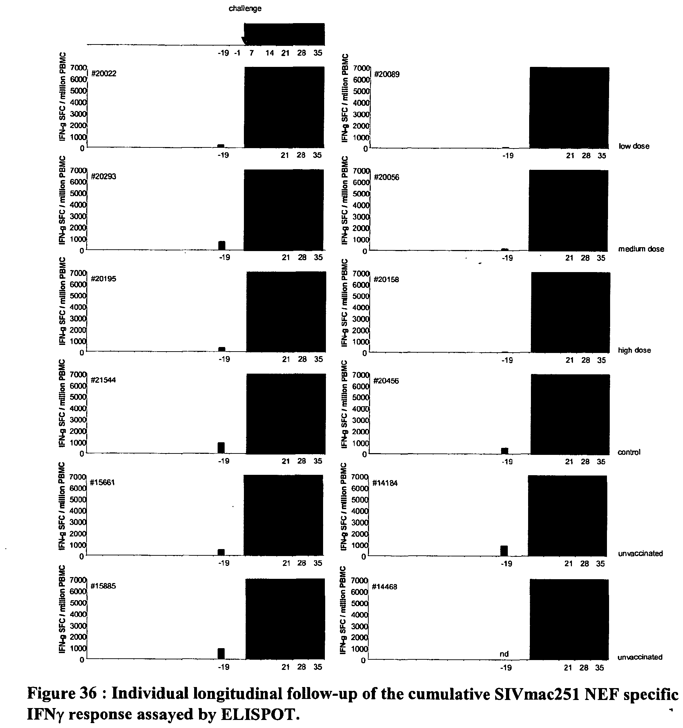

Figure 36: longitudinal follow-up of the nef-specific T cells responses by IFN-g ELISPOT

PBMC were restimulated *in vitro* with 8 pools of peptides (named from letter a to h) encompassing the whole NEF protein. The cumulative response was calculated as the sum of the individual specific responses obtained with each of the 8 peptides. Background was substracted before calculating the cumulative response. nd: not determined

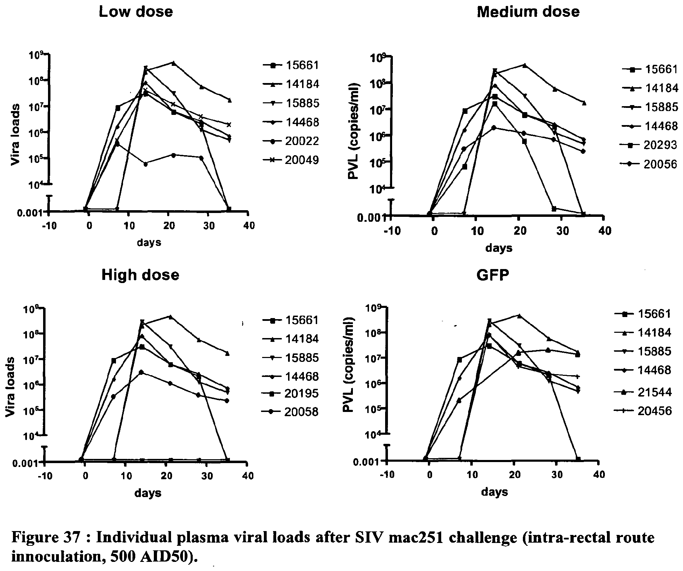

Figure 37: The plasma viral loads are quantified as the number of viral RNA copies per ml plasma


Plasma samples were tested 10 days prior to infection, the day of the challenge and at 7 days, 14 days, 21 days, 28 days and 35 days post- challenge.

Unvaccinated animals are represented in blue, vaccinated animals in red and GFP control in green.

Results are given as a comparison between unvaccinated and vaccinated animals according to the type and dose of TRIP vector received at the prime.

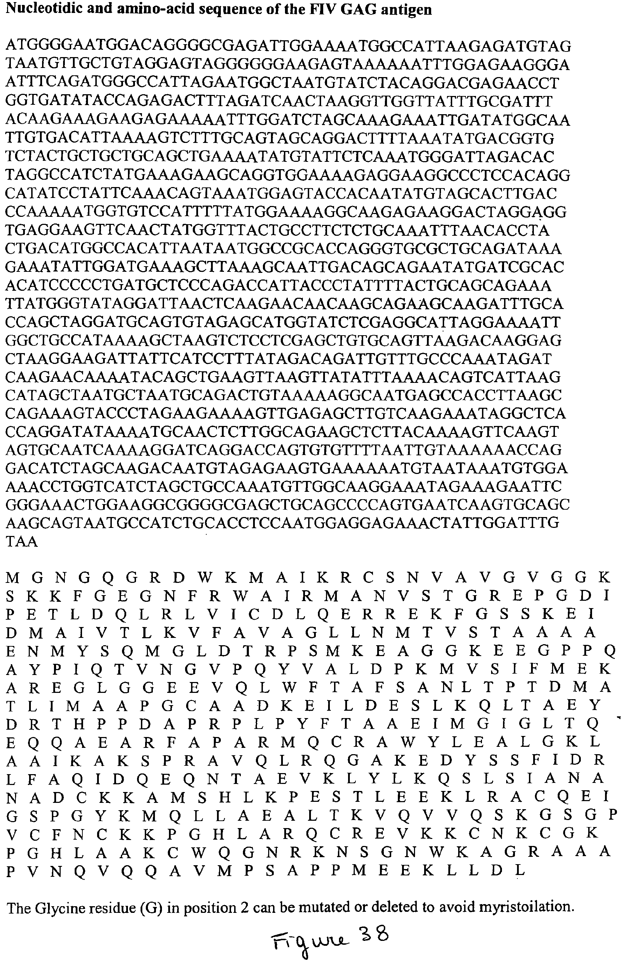

Figure 38: the amino acid sequence of the FIV GAG and its nucleic acid sequence.

# THE APPLICATION OF TRIP LENTIVIRAL VECTORS IN A VACCINATION STRATEGY AGAINST SIV INFECTION AS A MODEL FOR ILLUSTRATION OF VACCINATION AGAINST HIV INFECTION.

**I. Potential of the TRIP vector to induce anti-SIV specific T cells responses in mice models.**

To determine if lentivirus vectors harbouring an envelope protein originating from a VSV virus could be modified to allow boosting of immune responses, we developed a new vector strategy based on lentiviral vectors expressing the glycoprotein from different VSV serotypes expected not to be cross-reactive.

Isolates of *Vesicular stomatitis virus (VSV)* are enveloped, non segmented, negative-strand RNA viruses that belong to the genus *Vesiculovirus* in the *Rhabdoviridae* family. VSV infects domestic animals such as cattle, swine, and horses, causing vesicular lesions in the tongue, oral tissues, udders, and hooves. The VSV genome is delivered to the cytoplasm of host cells, where replication occurs, via receptor-mediated endocytosis of viral particles and subsequent pH-induced fusion of the viral envelope with the endosomal membrane. The VSV G protein, the sole viral surface glycoprotein, is required for attachment and fusion. There are two major serotypes of VSV, Indiana and New Jersey, which are distinguished by neutralizing antibodies against the G glycoprotein. In addition to their antigenic structures, the Indiana and New Jersey glycoproteins also differ in the number (511 and 517, respectively) and composition of amino acids (only 50% identity), in post-translational modifications, and in folding. Correspondingly, Indiana and New Jersey strains are not equally important regarding VSV pathogenesis. Outbreaks caused by New Jersey strains are more frequent and more severe than those caused by Indiana strains.

**Materials and Methods**

**Mice.** Female C57BL/6 mice (Breeding Janvier Labs, France) were bred at the Pasteur Institute facilities.

**Cell culture.** HeLa (human cervical adenocarcinoma) available at the ECACC) and human embryonic kidney 293T cells (available at the ATCC), used for lentiviral vector production, were grown in Dulbecco's modified Eagle medium (DMEM) Glutamax (GIBCO) supplemented with 10% heat-inactivated fetal calf serum (FCS) and antibiotics (penicillin-streptomycin).

**Vector construction and production.**

The vector plasmid pTRIP.ΔU3.CMV.SIVmac239gagΔmyr contain a non myristoylated form of SIVmac239 gag sequence under the control of the cytomegalovirus immediate early promoter (CMVie).

Vector particles were produced by transient calcium phosphate co-transfection of 293T cells with the vector plasmid, an encapsidation plasmid (p8.7) and a VSV-G envelope expression plasmid, Indiana serotype (pHCMV-G) (10) vs New Jersey serotype (pcDNA3.1(-) NJ-G) (derived from commercialized pcDNA3.1 plasmid available from Invitrogen). The protein sequence is disclosed on Figure 3.

The nucleotide sequence of the VSV-G of Indiana serotype is disclosed in Figure 6B. Cloning strategy encompassed the following steps:

A plasmid containing the gene from the glycoprotein from the New Jersey VSV serotype (pBS VSV-G NJ) has been used.

It was cloned into a pcDNA 3.1 (-) vector (Invitrogen) after XhoI/NotI digestion. The plasmid derived by this method was designated pcDNA3.1 (-) VSV-G NJ.

The WPRE sequence (Woodchuck hepatitis virus postregulatory element) (11) is a posttranscriptional regulatory element known to significantly increase gene expression. It was cloned into a TOPO® Cloning vector (Invitrogen).

The WPRE sequence was cloned into the pcDNA3.1 (-) VSV-G NJ after EcoRI digestion and dephosphorylation. The plasmid derived by this method was designated pcDNA3.1(-) VSV-G NJ WPRE.

WPRE Quantification of p24 antigen content of concentrated vector particles was done with a commercial HIV-1 p24 ELISA kit (Perkin-Elmer Life Sciences). For vector stock titration, 293T cells were transduced with different vector concentrations for 72h, and lysed. Lysats were treated with Rnase and proteinase K and then used for quantitative PCR (Lightcycler).

**In vitro transduction inhibition assay**. HeLa cells were plated at 10,000 cells per 96 wells-plates. A day later, cells were transduced with lentiviral vectors encoding eGFP (enhanced GFP) and pseudotyped with the glycoprotein from VSV Indiana or New Jersey serotype, after 30min preincubation with decomplemented mouse serum diluted at 1:6. Mice were either naive mice or mice immunized once with 0.25 $10^7$ transduction units (TU) of lentiviral vector coding for a non myristoylated form of SIVmac239 Gag and pseudotyped with the glycoprotein from VSV Indiana serotype and bled 14 days post-immunization. After 72h, transduction was assayed by flow cytometry. The percentage of transduction neutralization was calculated in comparison to transduction in the absence of serum.

**Mice immunization.** All animal experiments were conducted in accordance with the guidelines of the Office Laboratory of Animal Care at the Pasteur institute. Nine-weeks-old mice were intrapritoneally (i.p.) inoculated with 0.25 $10^7$ transduction units (TU) of pTRIP.ΔU3.CMV.SIVmac239gagΔmyr vector particles in 0.2 ml Dulbecco's PBS twice on day 0 and then on day 21. Mice were bled on day 14. Immune responses were analyzed on day 28.

For the prime, a lentiviral vector encoding a non myristoylated form of SIVmac239 Gag and pseudotyped with the glycoprotein from VSV Indiana serotype was administered, whereas for the boost, the same vector but pseudotyped with the glycoprotein from VSV New Jersey serotype was injected.

The comparison was done with the homologous prime/boost strategy using two subsequent injections of lentiviral vector pseudotyped with the glycoprotein from VSV Indiana serotype. As controls, the primary (day 7) and memory (day 28) responses were characterized after a single injection of lentiviral vector pseudotyped with the glycoprotein from VSV Indiana serotype. The primary (day 7) response of mice immunized only once with lentiviral vector pseudotyped with the glycoprotein from VSV New Jersey serotype was also assayed.

## IFN-γ Elispot assay

Nitrocellulose microplates (MAHA S4510, Millipore) were coated overnight with capture antibody (Mouse IFN-γ Elispot pair, BD Pharmingen) and blocked with complete medium composed of RPMI1640 Glutamax supplemented with 10% FCS, antibiotics, hepes, non essential amino-acids, b-mercaptoethanol and sodium pyruvate. Splenocytes from vector-immunized mice were added to the plates in triplicates at $0,25 \times 10^6$ cells/well and stimulated with SIVmac239 gag peptides (NIH AIDS Research and Reference Reagent Program), concanavalin A (1μg/ml). Forty hours later, spots were revealed with the biotine-conjugated antibody (Mouse IFN-γ Elispot pair, BD Pharmingen) followed by streptavidin-AP (Roche) and BCIP/NBT substrate solution (Promega). Spots were counted using a Bioreader 2000 (Biosys, Karben, Germany) and results were expressed as IFN-g spot-forming cells (SFC) per million splenocytes.

## In-vivo cytotoxicity assay

For target cell preparation, splenocytes from naïve mice were labelled with various concentrations (high, 5 μM; medium, 1 μM; low, 0.2 μM) of CFSE (carbosyfluorescein-diacetate succinimidyl ester, Vybrant CFDA-SE cell-tracer kit, Molecular Probes) Splenocytes were then pulsed with peptides at 5μg/ml. Each mouse received $10^7$ CFSE-labelled cells of a mix containing an equal number of cells from each fraction, through the retroorbital vein. After 15-18h, single-cell suspensions from spleens were analyzed by flow cytometry (Becton Dickingson, CellQuest software). The disappearance of peptide-pulsed cells was determined by comparing the ratio of pulsed (high/medium CFSE fluorescence intensity) to non-pulsed (low CFSE fluorescence intensity) populations in immunized versus naïve mice. The percentage of specific killing was established according to the following calculation:

$$[1-[(CFSE_{low}\text{naïve} \quad / \quad CFSE_{high/medium}\text{naive}) \quad / \quad (CFSE_{low}\text{immunized} \quad / \quad CFSE_{high/medium}\text{immunized})]]x100.$$

## Results (figures 28-29)

We first showed that mice immunized only once and with a low dose (0,25 10e7 TU/mouse, corresponding to 650 ng p24 for this batch) of lentiviral vector pseudotyped with the glycoprotein from VSV Indiana serotype do develop strong humoral response which neutralize the *in vitro* transduction of cells with a lentiviral vector pseudotyped with the same envelope (figure 28). On the contrary, there was only a low sero-neutralization of transduction by vector pseudotyped with the glycoprotein from VSV New Jersey serotype detectable.

A preliminary dose response experiment using the lentiviral vector encoding a non myristoylated form of SIVmac239 Gag and pseudotyped with the glycoprotein from VSV Indiana serotype allowed us to characterize the immune responses and identify peptides containing an immunodominant CD8 epitope (SIVmac239 gag : 309-323 (QTDAAVKNWMTQTLL)) as well as a subdominant CD8 epitope (SIVmac239 gag : 73-97 (ENLKSLYNTVCVIWC) (data not shown). A dose as low as 0,45 $10^7$ TU/mouse was sufficient to reach a plateau of 100% responding mice with a specific lysis of almost 100% for the immunodominant CD8 epitope-containing peptide. In contrast, even high doses (up to 23 $10^7$ TU/mouse) were non enough to stimulate an *in vivo* cytolytic activity of 100% in the case of the subdominant-CD8 epitope-containing peptide.

In parallel, a recently published paper using adenoviral vectors coding for the same antigen characterized a peptide containing a CD4 epitope (SIVmac239 gag :297-311 (YVDRFYKSLRAEQTD)).

Therefore, we chose to monitor immunity directed against these 3 peptides and to immunize mice with a sub-optimal dose of vector (0.25 $10^7$ TU/mouse) in order to be able to detect a boosting effect both in terms of number of responding mice and amplitude of the responses.

## II - Protective Response against SIVMAC in non-human primate model

### Introduction

### 1 HIV infection and AIDS

### 1.1 HIV and its impacts

### 1.1.1 Epidemiology

Since the first cases of acquired immunodeficiency syndrome (AIDS) were reported in 1981, the global spread of Human Immunodeficiency Virus (HIV) has reached pandemic proportions and represents now a global developmental and public health threat (Girard et al., 2006). Indeed, the number of people living with HIV throughout the world is nowadays around 39.5 million and is still growing exponentially, with 4.3 million people infected in the previous year and an estimated 14.000 people becoming infected every day (http//www.unaids.org).

### 1.1.2 HIV biology

The physiopathology of the infection is directly correlated with the characteristics of the HIV. This virus belongs to the family of *Retroviridae*, genus *lentivirus.* It is an enveloped virus of around 110 to 120 nm in diameter. The gp120 glycoprotein is responsible for the virus tropism; indeed it allows the fixation to the cellular receptor CD4 and co-receptors CCR5 or CXCR4, making thus CD4$^+$ lymphocytes its major target cells. Once virus attaches to CD4$^+$ lymphocytes, the viral cycle is divided in 2 major steps: early and late step. In the cytoplasm, viral RNA is reverse transcribed into double stranded DNA inside the viral capsid and actively imported to the nucleus where it can integrate in the cell genome (Arhel et al., 2007). Transcription of viral DNA and translation of viral mRNA allows the formation of new viral particles.

Most studies of AIDS pathogenesis are carried-out in non-human primates with an HIV simian equivalent: SIV. Indeed, SIV viral structure and biology are closely related to HIV ones.

### 1.1.3 Physiopathology of HIV infection

Disease progression is accurately defined by combined measurement of plasma HIV-1 RNA and CD4$^+$ lymphocytes. Natural HIV infection can be divided into 3 major phases: primo infection or acute infection, characterized by a peak in viral load (around $10^6$ copies RNA/ml of blood) and by a rapid but transient decrease in circulating T CD4$^+$ (Weber, 2001). Moreover, at this early stage of infection, HIV specific CD4$^+$ T cells are the major targets of the virus and are preferentially destroyed in the absence of any treatment (Rosenberg et al., 2000). However, this increase in viral load is generally well controlled by a specific immune response, principally cellular. Indeed, there is evidence for a temporal correlation between the appearance of HIV-specific CD8$^+$ T cells and the decline of primary viremia (Koup et al., 1994). As a consequence, T CD4$^+$ number gets back to a higher level (inferior to the one prior to infection) and viremia stabilizes (between $10^3$ and $10^6$ RNA copies/ml): the set-point (SP) is reached; its level often correlates with the evolution of the disease (Mellors, 1996). The infected individual then enters an asymptomatic period, which can last anything from months to years. This period is characterized by a slow and linear decrease in the number of circulating CD4$^+$, due to an equilibrium between the immune system and HIV replication. In absence of treatment, this asymptomatic phase is followed by AIDS. At this point, viremia progressively returns to a high level and an inflection in the CD4$^+$ T cells depletion slope is observed (CD4 count inferior to 200 cells/mm$^3$ of blood). Eventually, the immune system collapses and disease causing agents that are usually either completely controlled or easily cleared become potentially lethal.

### 2 Medical treatments

### 2.1 From Monotherapy to HAART

In order to slow-down the progression of the disease to AIDS, new medications were put on the market in 1986. They were called antiretroviral drugs, their goal was to prevent HIV replication and thus to postpone CD4$^+$ T cells depletion. The most famous of these drugs was certainly AZT (Zidovudine), an inhibitor of the virus Reverse Transcriptase (RT). However, this monotherapeutic approach was eventually found to be of limited effectiveness, as HIV is a virus that has the potential to quickly develop a resistance (through mutations) to any antiretroviral medication. In 1996, new inhibitors of RT were commercialized; they were chemically different from AZT-like inhibitors. Eventually, a new class of HIV medication appeared in 1995, protease inhibitors (PI). The combination that is nowadays the "standard" in anti-HIV therapy, called Highly Active Antiretroviral Therapy (HAART), consists of an association of 3 classes of antiretroviral medications, usually 2 different inhibitors of RT and one of PI. HAART allows a powerful long-lasting viral load decrease (Figure 5B), for most of the patients, virus copies in blood can even become undetectable (Gulick et al., 2000). As a consequence, CD4 count increases, the immune system recovers partially and can again push back opportunistic pathogens (Autran et al., 1997). For patients who have access to the treatment, HAART has allowed an impressive reduction of AIDS related morbidity (Palella et al., 1998).

### 2.2 HAART limits

Although HAART success is irrefutable, it presents some limits and questions can be raised concerning its long-term use. First of all, HAART treatment is really expensive and is still non accessible to developing countries. Then, the toxicity of these medications is relatively high, they often triggers major side effects (diabetes, lipodystrophia, diarrhoea, headaches...). Moreover, it has been shown that HIV was capable of developing resistances against HAART treatment. Mutations often appear in regions of HIV constrained by the treatment. HAART treatment also limits the production of HIV antigens, apparently to a threshold below what is needed to stimulate HIV-specific effector T cells or to expand HIV-specific naïve T cells. Immune memory to HIV still persists however, as indicated by the transient restoration of CD4 and CD8 immune responses to HIV when the immune system is re-exposed to the virus after treatment interruptions (Autran et al., 2004).

### 2.3 HIV vaccination

### 2.3.1 Prophylactic / therapeutic vaccine

Because the efficacy of drugs is still limited and because HAART should become a lifelong therapy, too expensive and difficult to administer in most Third World settings, other strategies have to be found to durably prevent the onset of AIDS. The development of an HIV vaccine may represent the only way to slow the pandemic. Two different strategies of vaccination are being tested. On the one hand, a prophylactic vaccine should be capable of inducing sterilizing immunity, and would prevent both infection and its complications. Such a vaccine should be able to operate at the time of virus entry and at the very early stage of infection, before the virus can disseminate to lymphoid organs. On the other hand, a therapeutic vaccine is designed for chronically infected patients under HAART treatment (Autran et al., 2004). It would consist of first treating patients with HAART to restore immune competence, and then immunize them to subsequently boost their rested immune responses to HIV before interrupting treatment. Eventually, if immune control of the virus could be enhanced, disease progression would be attenuated, allowing treatment interruptions, and consequently a limitation in the use of HAART, thus minimizing their toxicity and cost.

### 2.3.2 State of current AIDS vaccine research

Whatever strategy is chosen, vaccine development is facing huge scientific challenges, such as high genetic variability of the virus, lack of immune correlates of protection and limitations in the existing animal models. Until now, more than fifty vaccine candidates have been tested in phase I/II clinical trials (www.iavi.org) (See appendix 1 for a summary of anti HIV-1 on going trials). Multiple vaccination strategies have been tested so far (Tonks, 2007). At first, traditional live attenuated vaccines were tested because of their past success against small pox, polio or measles,. A live attenuated virus with a deletion in the Nef gene (SIV-Δnef) has been the most effective vaccine in the SIV/macaque model. However, its application is restricted since the vaccine virus persists at a low level indefinitely in vaccinated macaques and can be pathogenic to neonates. In addition SIV-Δnef can cause disease in adults several years after vaccination. Nevertheless these live attenuated vaccines provide a critical proof of principle for the feasibility of HIV vaccine development and allow the characterization of the nature of protective immunity (Koff et al., 2006). Another traditional vaccine strategy was to induce broad and long-lasting neutralizing antibodies to disable viral entry and prevent infection. To this end, subunit vaccines were developed. They were composed of HIV proteins or peptides, often recombinant. We can cite here the VaxGen trial, evaluated in phase II in the USA, with a vaccine based on a monomeric gp120 administered in alum. However, none of these subunits vaccine trials showed a statistically significant reduction of the HIV infection in the vaccinees. As vaccines eliciting humoral responses failed to give encouraging results, researchers have turned instead to the cell-mediated arm. Indeed, it was shown previously that CD8$^+$ cytotoxic effector T cells could clear infected cells displaying viral peptides on their class I MHC molecules. Moreover, CD8$^+$T cells are known to be important in controlling SIV and HIV infection because (i) the depletion of CD8$^+$ T cells during chronic SIV infection in monkeys increases the viral load (Jin et al., 1999), (ii) HIV-positive patients who are heterozygous at class I HLA loci have slower rates of disease progression (Carrington et al., 1999) and (iii) the virus accumulates mutations in CD8+ T cells epitopes (Goulder and Watkins, 2004). A vaccine stimulating T cell responses would not prevent infection in the traditional way but could at least suppress it long enough to prevent the onsets of AIDS. Among T cell vaccines are found the DNA vaccines, currently in phase I trials, using isolated HIV genes encoded by plasmids, but which face problems of immunogenicity. The most commonly used strategy to elicit T cells responses is the one of recombinant vectors. It consists of using viral vectors (derived from pox, vaccinia or adenovirus) to transport isolated HIV genes into human cells.

Finally, it is also worth mentioning the technique of dendritic cell-based vaccination, whose results against SIV challenges were very encouraging. It consists of immunizing macaques with autologous dendritic cells (DC) pulsed with chemically inactivated SIV (inactivation with aldrithiol-2, AT-2). The inactivated virus is not capable of reverse transcription but the viral particles conserve their structure intact and most of all fusion capacity. This technique was even tested with success in chronically infected and non-treated humans, with autologous DC pulsed with inactivated autologous HIV (Andrieu and Lu, 2007). Despite its efficiency, this technique is rather expensive and time-consuming.

### 2.3.3 Problems encountered by prior vaccine strategies

Although many types of vaccines have been and are still being tested, none of them has been completely successful until now. Indeed, no long-term effect on viral load has ever been observed with DNA vaccines, even if CTL specific responses were stimulated. Vaccines eliciting a humoral response suffer from the huge variability of the virus and even if antibodies were generated, they were never versatile enough to cope with HIV genetic diversity. Even passive immunization of HIV-infected individuals with neutralizing monoclonal antibodies failed, underlining the limits of humoral immunity in controlling HIV-1 infection (Trkola et al., 2005). Pox vectors succeeded in eliciting specific CD4$^+$ and CD8$^+$ T cells responses, but did not allow a better control of viral load after many weeks of HAART interruption. Consequently, other vaccination strategies need to be tested. We propose here to test a new HIV-1 vaccine strategy, based on the use of a Lentiviral Vectors (LV) derived from HIV-1 as candidate vaccine.

### 3 Lentiviral Vectors as candidates for HIV vaccination

### 3.1 Technology of Lentiviral Vectors

LV were described for the first time 20 years ago (Poznansky et al., 1991). As a recombinant vector, a LV is capable of integrating a transgene (until 8-10 kb) into the DNA of the host cell. The unique particularity of HIV-1 derived vectors and of all LV is their ability to transduce non-dividing cells. Indeed, LV like lentiviruses, are able to integrate independently of the cell mitosis. This capacity derives from an active nuclear-import of the viral DNA (or vector DNA) through the nuclear membrane of the host cell. One explanation for this active nuclear import is the formation of an unique triple-stranded DNA, called DNA Flap or Triplex via two cis-active sequences in the pol sequence: cPPT (central Polypurine Tract) and CTS (Central Termination Sequence) discovered in the laboratory (Zennou et al., 2000).

Our vaccination project uses an HIV-1 derived LV commonly named TRIP (because it contains the central DNA Flap/Triplex structure). This vector, belonging to the third generation of LV, has been optimized in term of design, production, transduction efficiency and bio-safety parameters (Delenda, 2004).

One major interest for using HIV-1 as a gene transfer vector is that retroviruses, contrary to RNA positive or DNA viruses are not directly infectious. Indeed a RNA positive genome needs reverse transcription and many accessory proteins to begin viral replication and pathogenesis *in vivo.* However, in order to be used as a gene transfer vector, HIV-1 genome has been reduced to the minimal viral sequences necessary for transgene expression and packaging (Figure 2). The cis-acting sequences necessary for a transgenic expression cassette are the following ones:

1. The LTR sequence (Long-Terminal Repeat) is required for reverse transcription, viral DNA integration and transcription. This 3'LTR has been deleted in the U3 region, without perturbing the functions necessary for gene transfer, for two major reasons: first, to avoid trans-activation of a host gene, once the DNA integrated in the genome and secondly to allow self-inactivation of the viral cis-sequences after retrotranscription. Thus, in target cells only sequences from the internal promotor will be transcribed (transgene) (Figure 2A).

2. The ψ region is necessary for viral RNA encapsidation.

3. The RRE sequence (REV Responsive Element) allows export of viral messenger RNA from the nucleus to the cytosol after binding of the *Rev* protein.4. The DNA flap sequence (cPPT/CTS, normally contained in *Pol*) facilitates nuclear import.

5. The WPRE cis-active sequence (Woodchuck hepatitis B virus Post-Responsive Element) is also added to optimize stability of mRNA (Zufferey et al., 1999). WPRE is not translated.

The gene of interest (i.e. encoding the antigen) is inserted in the transfer vector plasmid under the control of a strong and often ubiquitous promoter.

In order to generate viral particles (RNA, capsid and envelope), certain HIV-1 helper packaging proteins have to be brought concomitantly within producer cells. They are encoded by two additional plasmids called the packaging or encapsidation plasmid and the envelope expression plasmid. The packaging plasmid encodes only the viral proteins essential for viral particle synthesis. Accessory genes whose presence in the plasmid could raise safety concerns were removed. Viral proteins brought in *trans* are respectively:

4. *Gag* proteins for building of the matrix (MA, p17), the capsid (CA, p24) and nucleocapsid (NC, p6).

5. *Pol* encoded enzymes: integrase, protease and reverse transcriptase.

6. *Tat* and *Rev* coding regulatory proteins, *Tat* is necessary for the initiation of LTR-mediated transcription.

In order to avoid any packaging of these generated mRNA in the viral particles, the ψ region was removed. An heterologous promoter was chosen to avoid recombination issues.

The envelope expression plasmid does not encode the HIV-1 natural *env* proteins (gp120, gp41). Indeed, these proteins are too labile to allow an efficient production and concentration by ultracentrifugation of vector particles. Moreover, the *env* proteins of HIV-1 have a limited tropism (CD4, CCR5, CXCR4). To counter these issues, LV production uses a process called pseudotyping. It consists in generating viral particles with an heterologous envelope glycoprotein. Among the first and still most widely used glycoproteins for pseudotyping LV is the Vesicular Stomatitis Virus Glycoprotein G (VSV-G) from the Indiana serotype. LV pseudotyped with VSV-G offer significant advantages in that VSV-G interacts with an ubiquitous cellular receptor on cells, endowing the vector with a broad host cells range. Moreover, VSV-G confers high vector particle stability allowing downstream processing of viral particles: principally concentration by ultracentrifugation.

**3.2 Why are Lentiviral Vectors promising candidates for vaccination against HIV-1?**

**3.2.1 Transduction of DC**

LV were initially used in gene therapy and their unique capacities as gene-transfer system are today undeniable.

First and contrary to adenovirus and vaccinia virus-derived vectors, there is no pre-existing immunity in humans against lentiviral viruses. Since their emergence, LV have been tested with success *in vitro* in a large variety of cells and tissues of therapeutic importance, including liver, brain and dendritic cells (DC) in the context of gene therapy protocols.

DC are a heterogeneous group of Antigen Presenting Cells (APC) which plays a crucial role in innate immunity as well as in initiating adaptive immune responses. DC act as sentinels of the immune system by continuously capturing antigens in peripheral tissues. Once activated by microbial products or inflammatory signals, they undergo maturation, migrate to draining lymphoid tissues where they subsequently process and present the captured antigens in the context of MHC I and II to CD8+ and CD4+ T cells. Interestingly, among the cell types that could be efficiently transduced by LV were found the mitotically hypoactive human CD34+- and monocyte-derived DC as well as mouse bone marrow derived DC. *In vitro,* transduction by LV did not affect their viability. Eventually, stable transduction of DC allows an endogenous presentation of the antigen during the whole lifespan of the cells. Thus, it makes LV good candidate vaccines.

### 3.2.2 History of the use of LV for vaccination purposes

Besides efficient expression of a transgenic protein, DC transduced *in vitro* with LV were shown to efficiently process and present peptides derived from the protein. Indeed, both human and murine lentivirally transduced DC were capable of restimulating specific T cell lines or clones *in vitro.* More importantly, several groups reported *in vitro* priming of naïve T cells against relevant antigens when using human DC.

Many groups then evaluated the use of lentivirally transduced DC as immunotherapeutic agents *in vivo,* principally in mouse models but also more recently in a primate model. It has consisted in immunizing animals with *ex vivo* lentivirally transduced DC, and in analyzing the resulting CD8+ T cells responses *in vitro.* When possible the capacity of protection was also tested *in vivo* in the context of a challenge. The majority of these studies used tumor antigens as models and tested the capacity of induced CTL responses to eliminate tumor cells. Very few research teams have proved the pertinence of *ex vivo* lentivirally transduced DC against viral infections. Zarei et al. for example demonstrated the capacity of protection against a LCMV challenge in mice immunized with DC transduced with LV encoding the virus glycoprotein (Zarei et al., 2004).

However, this technique seemed to be difficult to apply in a human vaccination protocol, consequently LV were rapidly tested *via* direct *in vivo* administration. Many groups have demonstrated the efficacy of *in vivo* injection of LV in mice in order to elicit a transgene-specific immune response. Once again, tumor antigens were principally used. For example, it was shown by the lab that direct *in vivo* inoculation of melanoma poly-epitope encoding lentiviruses in HLA-A*0201 transgenic mice could elicit vigorous CTL responses against most of the melanoma epitopes encoded (Firat et al., 1999). It has even been demonstrated that injection of LV was superior to the *ex vivo* transduced DC injection, both in terms of amplitude and longevity of the CTL response (Esslinger et al., 2003). Furthermore, a functional CD8+ T cells memory response could be generated after direct *in vivo* immunization with the TRIP vector even in the absence of CD4+ T cells, undeniable advantage towards HIV vaccination (Iglesias et al., 2007). Many research teams are now investigating the intricate mechanisms that could contribute to the high potential of LV as vaccination tools. The sustained antigenic expression, particularly in DC, as well as the activation of innate immunity might play a critical role (Breckpot et al., 2003).

### 4 Vaccinal trial in Cynomolgus macaques

### 1. Previous work in the laboratory, early days of the project

In the laboratory, immunogenicity studies have demonstrated the potential of anti-SIV specific T cells responses in inbred mice immunized with TRIP vector encoding a non-myristoylated form of SIVmac239 Gag (above). These murine models allowed to underline the potential of TRIP vectors as candidates for vaccination against HIV. However, they did not permit to test the capacity of protection of TRIP vector immunizations in the context of a viral challenge.

### 2. The macaque model

For this purpose, a non-human primate model was chosen for protective efficacy studies, more particularly the Cynomolgus macaque. The human/HIV-1 model was translated to the macaque/SIVmac non human primate model. Macaques are highly susceptible to SIVmac infection and progressively develop an immunodeficiency syndrome, which mimics human AIDS. Interestingly, plasma viral loads during primary and chronic infection parallel those observed in humans, as in HIV-1 infected people long-term non-progressors as well as rapid progressors can be observed. As in humans infected with HIV-1 the cellular immune responses to SIVmac during primary and chronic infection differ significantly and evidence of immune escape is readily documented. As in HIV-1 infected individuals, gut-associated lymphoid tissues is the major site of viral replication and CD4+ T cell depletion.

Nowadays, AIDS vaccine /challenge data are essentially generated in 3 main macaque species: mainly rhesus macaques of Indian origin, but also rhesus macaques of Chinese origin and Cynomolgus macaques. Each species model presents advantages and drawbacks for studying responses to viral infection. Cynomolgus macaques were chosen for our trial because they are more readily available in Europe than rhesus macaques. Reinman *et al.* showed that the pathogenicity of SIV was attenuated in Cynomolgus macaques compared to Indian rhesus (lower plasma viremia, preservation of CD4[+] T cells number, increased survival time). This attenuated pathogenicity was associated with earlier and stronger IFN-γ ELISPOT responses to GAG and ENV than in rhesus species. These observations support thus a role of early T cells immune responses. Finally, despite lower plasma viral load, viremia after challenge can be significantly used as experimental endpoint in Cynomolgus macaques, assuming that the dose of virus used for the challenge is high enough and that the naïve group is big enough to limit the statistical significance of spontaneous controllers. Interestingly, Cynomolgus macaques display viral loads more similar to those seen in the human infection. (Reimann et al., 2005).

**3. Choice of the antigene**

In the context of a vaccinal trial in non-human primates, the question of the choice of the antigen has to be raised. The GAG SIVmac239 non myristyllated protein was chosen as antigen. Previous results and observations, as well as data concerning natural HIV-1 infection and viral structure could justify the choice of this protein as potentially efficient antigen. First of all,. the important variability in HIV-1 strains constrained us to choose a protein well conserved among the different HIV-1/SIV strains. Only GAG, POL and NEF could fulfil this criteria. However, it has been shown that CTL recognise principally epitopes located on *gag* and *nef* (Addo et al., 2003). More recently, it was demonstrated that of the HIV-1 proteins targeted, only GAG specific responses were associated with lowering viremia and that independently of the particular HLA-type (Kiepiela et al., 2007). In addition, the more diversified the GAG specific responses were, the lower was the plasma viremia. Moreover, as it composes the viral matrix, GAG is the first protein to be processed and presented by MHC class I (Sacha et al., 2007), because entry/capture is sufficient and that there is no need of virus replication. GAG is also the most represented among HIV-1 proteins (1000-1500 CA) (Briggs et al., 2004). All these data justified the choice of this protein as relevant antigen for our first vaccinal trial. In addition, this trial was designed to give the proof of concept of the efficiency of TRIP vectors as vaccination tools. To this end, a simple antigen was voluntarily chosen in order to highlight the protective role played by the vector itself (gene transfer efficacy). Moreover, having the simple GAG protein as antigen allows to make comparisons with previous vaccine studies.

**4. Vaccination protocol**

A prime-boost strategy was chosen in order to strengthen primary responses. A second injection is supposed to increase the number of responders, the frequency and avidity of antigen specific T cells and the intensity of T cells responses. It should also improve the diversity of responses as well as T cells functions such as killing or migration to the periphery.

For the prime, 3 groups of 2 macaques were immunized with the LV vector TRIP-SIVmac239 Gag pseudotyped with an Indiana serotype VSV-G, at 3 different doses. Two animals received a TRIP-GFP vector pseudotyped with Indiana serotype VSV-G as irrelevant vector. For the boost, 3 months after the prime, all immunized animals received a similar dose of TRIP-SIVmac239 Gag or TRIP-GFP pseudotyped with an Indiana non cross-reactive serotype VSV-G.

In order to test the capacities of protection triggered by this TRIP vector based vaccine, two months after the boost the 8 animals were challenged intra-rectally with 500 Animal Infectious Dose 50 (AID50) of SIVmac251. The inoculation route and the very high dose of virus for the challenge were justified by the size of the cohort, indeed by increasing the infectious dose, we hoped to limit the number of spontaneous controllers in the naïve animals arm of the study composed only of 4 macaques.

A longitudinal follow-up of the cellular immune response after prime, boost and challenge by IFN-γ □ELISPOT on PBMC has been performed.

**MATERIALS AND METHODS**

**1 Materials**

**1.1 Antigens**

The SIVmac239 GAGΔmyr protein was chosen as antigen. It is a 511 amino-acid protein. The protein myristylation domain was deleted to permit manipulations in biosafety levels L1, labs, and to promote class I presentation by APC. The complete sequence of the GAG polyprotein from SIV mac239 can be found via the protein ID: *AAA47632.* The GFP protein was chosen as irrelevant antigen.

### 1.2 Plasmids

All plasmids used for transfections were produced in strain JM109 E.coli K12 bacteria (F' *traD36 proA+B+ lacI^q Δ(lacZ)M15/ Δ(lac-proAB) glnV44 e14- gyrA96 recA1 relA1 endA1 thi hsdR17)*, grown in LB medium supplemented with ampicillin and extracted with the Maxi-prep Nucleobound® kit from Macherey-Nagel (Hoerdt, France).

Three plasmid constructs were used to generate the particles of TRIP-ΔU3-CMV-Gag Δmyr-WPRE (named here **TRIP-SIVmac239 Gag,** Figure 25 A) or TRIP-AU3-CMV-eGFP- WPRE (named here **TRIP-GFP,** Figure 25 B). A vector plasmid, containing HIV-1 cis-active genes (LTR, ΔU3 in 3', encapsidation signal ψ, RRE and DNA Flap i.e., cPPT/CTS), and the transgene encoding either the SIVmac239 GAG Δmyr protein or the GFP, under control of heterologous transcriptional regulator elements: Cytomegalovirus promoter. The WPRE (Woodchuck hepatitis virus postregulatory element) (Donella J.E. et al, 1998) sequence was added to increase transgene expression.

- A packaging plasmid, containing the HIV-1 genes *gag, pol, tat* and *rev,* necessary for building of viral particles in the production cell line, which can be designed as p. 8.7.1 in Zufferey et al, 1998.
- An envelope plasmid, encoding the Glycoprotein G from Vesicular Stomatitis Virus (VSV-G) serotype Indiana (ph CMV VSV-G) (Yee J. et al, 1994) or Indiana non cross reactive serotype such as serotype New-Jersey (pcDNA3.1(-)NJ-G WPRE). pcDNA 3.1(-)NJG is derived from pcDNA3.1 plasmid available from Invitrogen.

Packaging and envelope plasmids have heterologous transcriptional elements (CMV promoter, and polyadenylation signal). All plasmids contain the ampicillin resistance gene to ease growth selection in bacteria.

### 1.3 Cell culture

The human embryonic kidney cell line (human 293T) was used for TRIP vector production. For inhibition of transduction assays, the P4 cell line, a HeLa derived cell line, was used.

These cells were grown in complete medium composed of Dulbecco's modified Eagle's Medium containing glutamine (DMEM, GlutaMAX-I Supplement, GIBCO), supplemented with 10% heat-inactivated Fetal Calf Serum (FCS) (PAA Laboratories GmbH, Pasching, Austria) and penicillin, streptomycin (100 Units/ml of penicillin G (sodium salt) and 100U/ml of streptomycin sulphate, GIBCO, Invitrogen). Macaques primary cells were cultured in RPMI GlutaMAX-I complete medium (10% FCS and antibiotics, similar concentrations as in DMEM).

### 1.4 Non-human primates

Twelve adult Cynomolgus macaques *(Macaca fascicularis),* males from the Indian Ocean Island of Mauritius were included in the vaccination trial. They were negative for SIV Herpes Virus B, filovirus, STLV-1, SRV-1, SRV-2, measles, hepatitis B-HbsAg, and hepatitis B-HBcAb before inclusion in this study. Immunizations, challenge and blood collection were handled, in accordance to the EC guidelines for experiments using non human primates.

### 1.5 SIV virus for challenge

The SIVmac251 strain (complete proviral genome and flanking sequence: accession number: *M19499)* was used for challenge.

### 1.6 SIVmac239 GAG and SIVmac251 NEF peptides sets

PBMC *in vitro* restimulation in ELISPOT were carried out with either a SIV mac239 GAG or SIVmac251 NEF peptide sets containing 125 peptides or 64 peptides respectively (NIH AIDS Research and Reference Reagent Program). Peptides were 15 amino acids in length, with 11 amino acids overlaps between sequential peptides. GAG peptides were dispatched into 11 pools containing 5 to 12 consecutive and overlapping peptides, named in order from letter M to W and recovering the SIVmac239 GAG protein (Figure 26). NEF peptides were divided into 12 pools of 8 peptides recovering the NEF SIV mac251 protein and named in order from letter a to h. Most of the peptides were more than 80% pure. They were delivered lyophilized at 1 mg each. At reception, they were resuspended at 2mg/ml in 5% DMSO for GAG peptides and at 1mg/ml in pure DMSO for NEF peptides, based on percentage of peptide content and HPLC purity.

## 2 Methods

### 2.1 Vectors production

**[0280]** Vector particles were produced by transcient calcium phosphate transfection of 293T cells ($CaCl_2$ 0,125mM, 1X HEPES-buffered saline pH 7.10, 70 mM NaCl, 0,75mM $Na_2HPO_4$ $2H_2O$, 25mM HEPES). Ten $\mu$g of vector plasmid encoding either GAGΔmyr or GFP was required with 5 to 10 $\mu$g of the plasmid encoding the VSV-G glycoprotein envelope, and 10 $\mu$g of the packaging plasmid as described previously by Zennou *et* al 2000 (Zennou et al., 2000) Cells were seeded in 10cm$^2$ polystyrene-treated tissue culture Petri dishes (Falcon) at 6.10$^6$ in complete medium 24h before transfection, and medium was changed prior to transfection. Cells were at least 80% confluent. Twenty-four hours after transfection, complete medium without FCS was added to the cells at a smaller volume to concentrate the particles. Forty-eight hours post-transfection, supernatants were collected from Petri-dishes, centrifuged to pellet floating cells (2500 rpm, 5 min) and treated 15 min at 37°C with DNAse I (Roche Boehringer, 20 U) and $MgCl_2$ (Sigma, 1mM) in order to eliminate residual plasmids DNA. Vectors were collected after ultracentrifugation of the supernatant (22 000 rpm; 1 hour) and resuspended in cold PBS. Vectors were conserved at -80°C in aliquots of small volume.

### 2.2 Measurement of p24 GAG antigen production

**[0281]** Vectors HIV-1 p24 GAG antigen contents were determined by Enzymed-Linked-Immunosorbent Assay (Perkin-Elmer Life Sciences, Paris, France). p24 concentrations were given in ng/ml of vector.

### 2.3 Vector titration

**[0282]** Titration was performed by transduction of 293T cells (seeded 24h prior to transduction at 5.10$^5$ cells/well in 6 well-Petri dishes) with 3 different volumes of vector. Cells were also transduced with the same amount of vector previously heat inactivated at 70°C. Seventy-two hours after transduction, cells were lysed with a lysing-buffer 1X (Tris 20 mM pH=8.8 ; NP40 0,1%;Tween 0,1% final) containing RNAse, Dnase-free (Roche Boehringer, 50$\mu$g/ml final). Cellular proteins were degraded by addition of Proteinase K (Proteinase K stabilised 100$\mu$g/ml final, Eurobio).

**[0283]** Vector titers were assessed by performing a real-time PCR on cells lysates, using the Light Cycler Instrument (Roche Diagnostics, Meylan France). Total HIV-1 DNA copy number was determined by detection of a viral DNA sequence, localized in the LTR U5 region (primers AASM reverse and M667 forward). Two hybridization probes were used for each PCR run, one probe labelled with Fluorescein (FL) as 3' end donor and the other labelled with the LightCycler Red 640 (FC) as 5' acceptor. Normalization to cell number was done by detecting the CD3 sequence (house keeping gene), with primers CD3 in 3'and CD3 in 5'and probes FL and FC (Table 1). For PCR, 5$\mu$L of lysate were tested in duplicates for each condition, in a 15 $\mu$L PCR-mix (Jumpstart™ taq readmix for Q-PCR, Sigma 1X, $MgCl_2$ 1,9 mM, 1.5 U of Taq polymerase (Invitrogen),1.5 $\mu$M forward and reverse primers and 0,2 $\mu$M fluorogenic hybridization probes). Copy number was determined in reference to a standard curve prepared by amplification of 10$^2$ to 10$^8$ of cloned plasmid diluted in mouse cells lysate (3T3) with matching sequences (U5R and CD3) (Figure 27).

Table 1: Sequences of primers and probes and Q-PCR program used for vector titration

| PCR | Oligos | Sequence 5' → 3' | |
|---|---|---|---|
| U5R forward primer | M667 | GGCTAACTAGGGAACCCACTG | |
| U5R reverse primer | AASM | GCTAGAGATTTTCCACACTGACTAA | |
| U5R 3' end donor probe | LTR FL | CACAACAGACGGGCACACACTACTTGA-FL | |
| U5R 5' end donor probe | LTR LC | LC-CACTCAAGGCAAGCTTTATTGAGGC | |
| CD3 forward primer | CD3 in 5' | GGCTATCATTCTTCTTCAAGGTA | |
| CD3 reverse primer | CD3 in 3' | CCTCTCTTCAGCCATTTAAGTA | |
| CD3 3' end donor probe | CD3 FL | GGCTGAAGGTTAGGGATACCAATATTCCTGTCTC-FL | |
| CD3 5'end donor probe | CD3 LC | LC-CTAGTGATGGGCTCTTCCCTTGAGCCCTTC | |
| **Step and number of cycles** | | **Temperature** | **duration** |
| 1 cycle | **1: Denaturation** | 95°C | 3 min |
| 40 cycles | **2: Denaturation** | 95°C | 5 sec |
| | **3: Annealing** | 57°C | 10 sec |
| | **4: Elongation** | 72°C | 8 sec |

**2.4 Macaques immunization**

Macaques were divided into four groups of 2 animals (table 2) and were sub-cutaneously injected in 2 points with TRIP-SIVmac239 Gag pseudotyped with the VSV-G envelope serotype Indiana, at 3 different doses (high dose 2,5.10$^8$ Transduction Unit (TU), 6863 ng p24; medium dose 1.10$^8$ TU, 2745 ng p24 or low dose 2.5 10$^7$ TU, 686 ng p24) or with TRIP-GFP at the same p24 dose than the high dose of TRIP-SIVmac239 Gag (6863 ng p24).

For the second immunization performed 87 days post prime, animals were injected sub-cutaneously in 4 points with a vector pseudotyped with an Indiana non cross-reactive VSV-G glycoprotein serotype (VSV-G serotype New-Jersey). Macaques received either 1.10$^8$ TU of TRIP-SIVmac239 Gag, 60185 ng p24 when primed with the GAGΔmyr antigen, or 60185 ng p24 of TRIP-GFP vector when primed with the GFP antigen.

**Table 2: Repartition of Cynomolgus macaques used in TRIP vaccination trial**

| Cynomolgus macaque tatoo number | Vector received at the prime | Category |
|---|---|---|
| **20022** | **TRIP-SIVmac239 Gag** 2.5 10$^7$ TU | **LOW DOSE** |
| **20089** | **TRIP-SIVmac239 Gag** 2.5 10$^7$ TU | |
| **20293** | **TRIP-SIVmac239 Gag** 1 10$^8$ TU | **MEDIUM DOSE** |
| **20056** | **TRIP-SIVmac239 Gag** 1 10$^8$ TU | |
| **20195** | **TRIP-SIVmac239 Gag** 2.5 10$^8$ TU | **HIGH DOSE** |
| **20158** | **TRIP-SIVmac239 Gag** 2.5 10$^8$ TU | |
| **21544** | **TRIP-GFP** 6862 ng p24 | CONTROL |
| **20456** | **TRIP-GFP** 6862 ng p24 | |
| **15661** | **None** | **UNVACCINATED** |
| **14184** | **None** | |
| **15885** | **None** | |
| **14468** | **None** | |

The animals are ranged according to the tattoo number and the nature/dose of the TRIP vector received at the prime immunization.

**2.5 SIV mac251 challenge**

Immunized and naïve macaques (12 macaques in total) were challenged intra-rectally 57 days post-boost (ie 136-days post prime) with a single dose of 500 AID50 in 1ml (Animal Infectious dose sufficient to infect 50% of the animals) of pathogenic SIVmac 251 (stock from A.M. AUBERTIN, Université Louis Pasteur, Strasbourg, France distributed by ANRS- or equivalent stock available from NIH). Animals were anaesthetized with 10 to 20 mg/kg of Ketamine (Imalgène, Rhône-Mérieux) and the whole procedure was done according to the EU regulations and guidelines of Animal Care and Use. After inoculation macaques were housed separately with precautions bound to a Level 3 bio security animal house.

**2.6 IFN-γ ELISPOT**

Animals were anaesthetized with 10 to 20 mg/Kg of Ketamine (Imalgène, Rhone-Mérieux) for blood collection. 8 ml of blood were collected for each macaque in Cell Preparation Tubes with Sodium Citrate (BD Vacutainer™ CPT™) for PBMC and citrate-plasma collection and 3 ml in serum separator tube (Vacuette®) for serum collection. After centrifugation (10 min, 2500 rpm for Vacuette® tubes and 30 min, 3000 rpm, no brake, for CPT™), and red blood cells lysis with 3 to 5 ml 1X lysis buffer (IOtest ® 10X lysis buffer, Beckman-Coulter), PBMC were pelleted by a 10 min 1600 rpm centrifugation, and then numerated in a Kova's chamber Hycor®, and distributed to 96-well ELISPOT plates in triplicates at $2.10^5$ cells/ well if enough cells were available.

96-well plates with Immobilon®-P (Polyvinylidene Fluoride, PVFD) membrane (MultiScreen HTS Assay System, MSIP; Millipore), were prewetted (ethanol 35%) and coated overnight at 4°C with capture antibody (mouse IgG1 anti-human-monkey-IFN-γ monoclonal antibody GZ-4 purified; Mabtech, 10μg/ml final in PBS; 50μL per well). Plates were washed 4 times in Dulbecco's PBS 1X and blocked with complete RPMI.

Cells were restimulated either by addition of one pool of peptides (2ug/ml of each peptide), AT-2 inactivated SIVmac251 (5μg/ml of total viral proteins), (or PMA-iono (0,1 μM PMA and 1 μM ionomycin) as positive control (4000 cells/well), or mocked stimulated with DMSO/ RPMI.

After 40 hours, spots were revealed with a biotin-conjugated antibody (mouse IgG1 anti-human-monkey interferon-y monoclonal antibody 7-B6-1 purified; Mabtech; 1μg/ml final in PBS 0,5% FCS;100 μL per well 2 h at 37°C), followed by streptavidin-AP (1h, 1/5000 in PBS 0,5% FCS , 100μL per well, 1h, 37°C) and BCIP/NBT substrate solution (Ready to use mixture, 60μL per well; 15 min, RT, in the dark). Spots were numerated using a Bioreader 4000 (Biosys, Karben, Germany). Results were expressed as IFN-γ Spot-Forming-Cells (SFC) per million PBMC. The IFN-γ SFC/million PBMC resulting from a 5% DMSO/RPMI stimulation were subtracted from the results as a background signal.

## 2.7 ELISA

Quantification of innate cytokines (IL6; TNF-α and IFN-α□ was performed via ELISA using commercial kits (Monkey IL-6 and TNF-a ELISA kit from U-Cytech Bioscience (Utrech, Netherlands), human IFN-α kit from PBL Biomedical Laboratories (New Jersey, United States)). Plasma were tested for each animal 40 days before prime injection, 1 hour, 6 hours, 24 hours and 7 days post prime injection.

## 2.8 *In vitro* seroneutralization assays

P4 cells were seeded at $1.10^5$/ well in 96-well plates in complete medium 24 h prior to transduction. On the day of transduction, cells were cultured with TRIP-GFP (pseudotyped with an Indiana serotype VSV-G or with an Indiana non cross-reactive VSV-G such as New-Jersey VSV-G) preincubated with different dilutions of plasma. Cells were mocked transduced with the same volume of complete medium. Seventy-two hours after transduction, efficiency of transduction was assessed by analysing the GFP fluorescence by flowcytometry using a FACScalibur (BD).

## 2.9 Viral load determination

Briefly, viral RNA was isolated from citrate-plasma (200μL in total) with the High Pure Viral RNA Kit from Roche. Elution was carried out in 50μL elution buffer (Nuclease-free, sterile, double distilled water). The number of SIV-RNA isolated from plasma was determined in a quantitative single-step RT-PCR using the Platinium qRT-PCR from Invitrogen Reactions were performed in duplicates in the Mastercycler ep realplex (Eppendorf) in 96-well plates from ABgene (AB1100) in a final volume of 25μL (10μL RNA extract and 15 μL Mix). The Taqman quantification method was chosen, with an internal probe (500nM final) containing the Fam and Tamra fluorophores respectively in 5' and 3'. The primers (450nM final) were respectively at position 389 and 456 of SIVmac 251 GAG mRNA genome (Table 3).

The quantity of viral RNA copies initially presents was assessed by extrapolation of threshold fluorescence values onto an internal standard curve prepared from serial dilutions in dH$_2$O of a virus stock SIVmac251 previously titered by the technique of "branched DNA". As positive control for PCR, the TRIP-SIVmac239 Gag vector plasmid was used ($10^4$ copies/μL).

**Table 3: Sequences of primers and probes and Taqman RT-PCR program used for plasma viral load determination.**

| Name | Sequence 5' → 3' | size |
|---|---|---|
| Primer Forward: SIVmac389F | GCAGAGGAGGAAATTACCCAGTAC | 24 bp |
| Primer Reverse: SIVmac456R | CAATTTTACCCAGGCATTTAATGTT | 25 bp |

(continued)

| Name | Sequence 5' → 3' | | size |
|------|------------------|---|------|
| Taqman probe: SIVmac TM | Fam-TGTCCACCTGCCATTAAGCCCGA-Tamra | | 23 bp |

| Step and number of cycles | | Temperature | duration |
|---|---|---|---|
| 1 cycle | 1: Reverse transcription (1Cycle) | 46°C | 30 min |
| | 2: Enzyme activation | 95°C | 4 min |
| 50 cycles | 3: Step one, PCR denaturation | 95°C | 15 s |
| | 3: Step two, PCR annealing and elongation | 60°C | 1 min |
| 1 cycle | 4: Cooling | 20°C | Hold |

## RESULTS

### 3 Cellular immunity is induced after a single injection of TRIP vector

**3.1 A sub-cutaneous injection of TRIP-SIVmac239 Gag induces strong T cells responses in every animal without inducing deleterious inflammation**

The first objective of this project was to evaluate the capacity of TRIP-SIVmac239 Gag to induce T cells responses, after *in vivo* sub-cutaneous (SC) injections in non human primates. To assess specific T cells responses, we measured the frequency of PBMC capable of secreting INF-γ after *in vitro* restimulation with 11 pools of peptides encompassing the whole SIVmac239 GAG protein. The cumulative response was calculated by summming the individual responses directed against the 11 pools.

We first observed that a SC injection of TRIP vector did not result in systemic inflammation as shown by the absence of significant increase in the level of innate/proinflammatory cytokines in the plasma, namely IL-6, TNF-α, IFN-α (Figure 31). These data did not exclude the induction of local inflammation at the site of injection, which was likely required for the induction of adaptive immunity in addition to the expression of the antigen.

A single injection was sufficient to induce strong T cells responses in every Cynomolgus macaque, regardless of the dose received (Figure 32). Responses were detected as soon as 7 days post-injection for some of the macaques (20293 and 21544 showed frequency of IFN-γ SFC / million PBMC superior to 1000) and were maximal 16 days post-prime, reaching a frequency of IFN-γ SFC / million PBMC as high as 3000 for macaque 20022 for instance. Some animals (20056 and 20158) even mounted saturated responses. Actually, for one pool of peptides (pool T), the frequency of specific T cells was so high that spots were too concentrated and overlapped, making impossible to enumerate them. In those cases, number of IFN-γ SFC was underestimated to 1400/million PBMC, before substracting the background (noted *). This threshold was determined by performing serial dilutions of PBMC stimulated *in vitro* with PMA-Iono. Actually, the ELISPOT reader could not count over 280 spots/well. At day 58 post-prime the responses turned back to the pre-immunization level (day-40).

**3.2 No significant correlation is observed between the dose of vector injected and the intensity of IFN-γ responses**

Another objective in this trial was to assess the optimal dose of TRIP vector to inject. On purpose, 3 doses were tested (either high dose $2.5.10^8$ TU; or medium dose $1.10^8$ TU or low dose 2, $5.10^7$ TU). As a comparison, previous studies performed in the lab with the same TRIP vector but in mouse had shown that $1.10^7$ TU was the dose reaching the plateau of the response directed against the immunodominant epitope (in terms of frequency of responding animals and intensity of response).

No obvious dose-response effect was observed. Indeed, macaques that mounted the best IFN-γ responses were macaques 20022, 20056, 20195 and 20158, respectively injected with low, medium and high dose of vector. This result could be related to the MHC of the macaques, whose sequences are going to be sequenced in the near future. However, the highest dose tested might be preferable since it appears that 2 macaques out of 2 (20195 and 20158) mounted strong responses, in contrast to the low and medium groups which are more heterogeneous. Larger cohorts should be studied in a next trial for statistical significance.

### 3.3 T cells responses are GAG SIV specific

In order to distinguish the SIV GAG specific responses resulting from the expression of the transgene from the one resulting from the exogenous presentation on MHC class II molecules and from the cross-presentation on MHC class I molecules of peptides from the HIV-1 derived proteins present in the TRIP vector particles (since SIV and HIV-1 GAG share around 60% sequence similarity), we also included 2 control animals (macaques 21544 and 20456) immunized with a TRIP vector encoding an irrelevant antigen, TRIP-GFP, at the same p24 dose than the high dose relevant group corresponding to 6862 ng p24/ animal. Compared to animals immunized with TRIP-SIVmac239 Gag, the frequency of SIV GAG specific T cells was low in the control group (Figure 32). As expected, the presentation of GAG HIV-1 proteins from the vector particles and the cross-reactivity between HIV-1 and SIV GAG-specific T cells were not by themselves responsible for the induction of anti-SIV GAG cellular immunity but the latter is rather induced by the presentation of the protein encoded by the transferred gene.

### 3.4 T cells responses are broad

By restimulating the PBMC *in vitro* with different pools of peptides recovering the SIV GAG protein, we also aimed to study the diversity of the response and to identify regions targeted by T cells. For all immunized animals, independently of the vector dose received, a single injection of TRIP-SIVmac239 Gag induced broad responses directed against various pools of peptides, up to 3 pools for which there were more than 375 IFN-$\gamma$ SFC/million PBMC (value we considered reflecting a good response) for macaques 20022, 20056 and 20195) (Figure 16). At day 16 post-injection, IFN-$\gamma$ responses were preferentially directed against pools T (6 macaques out of 6) and U (4 macaques out of 6), within the C-ter region of the protein. Some of the macaques also developed a strong immunity against pool V and pools N and P within the N-ter region of SIV GAG (macaque 20022 for pool V, macaques 6 for pool P and macaques 20293 and 20195 for pool N). These differences between animals are likely linked to MHC diversity.

### 4 Envelope exchange to allow an efficient boost effect

### 4.1 There is no pre-immunity to Indiana serotype VSV-G in Cynomolgus macaques

In order to ensure that there was no pre-existing immunity to the VSV-G serotype Indiana and to test whether a neutralizing humoral response had been mounted, thus forbidding a second injection with the TRIP vector pseudotyped with Indiana serotype VSV-G, we measured the presence of neutralizing anti-VSV-G antibodies in the plasma before and after injection. It was also an indirect way to confirm that animals had been correctly injected.
Plasma were pre-incubated with TRIP-GFP pseudotyped with the VSV-G Indiana serotype and then used to transduce *in vitro* 293-T cells. The presence of neutralizing antibodies in the plasma was expected to inhibit transduction, in comparison to transduction in the absence of plasma. No difference was observed between transduction rates with pre-immune plasma and without plasma, showing there was no pre-existing immunity to VSV-G serotype Indiana, what would have certainly limited *in vivo* the transduction of the vector and thus, its immunogenicity.(Figure 34).

### 4.2 There is a need to switch to another envelope for an efficient boost

As anticipated, inhibition of transduction was seen when TRIP-GFP pseudotyped with the Indiana serotype VSV-G was pre-incubated with plasma from injected animals. We noted that the intensity of inhibition of transduction, a consequence of the intensity of anti-VSV-G humoral immunity, did correlate with the dose of vector injected, in contrast to the anti-SIV GAG cellular immunity. At the high dose of vector injection, almost full neutralization of transduction was observed, likely precluding a boost with an homologous vaccine, at least for the high dose group.
Control macaques injected with TRIP-GFP pseudotyped with VSV-G Indiana at the same p24 dose than the high dose relevant group also did develop neutralizing anti-VSV-G antibody responses, however at a level comparable to the one observed for the low and medium relevant groups. It suggested that the 2 batches of TRIP-SIVmac239 Gag and TRIP-GFP not only differed in transduction unit and p24 concentrations, but also in VSV-G density at the surface of the vector particles. TRIP-GFP expressing probably less VSV-G/p24 compared to TRIP-SIVmac239 Gag. Previous unpublished experiments performed with mice and reported above had shown that a prime-boost regimen using 2 distinct TRIP-SIVmac239 Gag pseudotyped with VSV-G from 2 non cross-reactive serotypes was more efficient than an homologous prime-boost (data not shown). This strategy was tested in macaques. We first confirmed that, like in our murine system, anti- Indiana serotype VSV-G antibodies induced by the prime did not cross-react with the VSV-G from the second serotype, by pre-incubating plasma from primed macaques with TRIP-GFP pseudotyped with the second serotype VSV-G (Figure 34). A minor pre-existing immunity against the second serotype was otherwise detected. In any case, this decrease is negligible comparing to seroneutralization observed in sera after the first injection. Thus, it does not preclude a second injection with an Indiana non cross-reactive serotype VSV-G. The choice of these 2 different VSV-G serotypes did seem to be judicious.

Based on the data from the prime, we decided to inject a medium dose of TRIP-SIVmac239 Gag pseudotyped with the second VSV-G serotype to each macaque. corresponding to $1.2.10^8$ TU per animal. Control macaques were also boosted with TRIP-GFP pseudotyped with the second VSV-G serotype at equivalent p24 dose (60185 ng p24/animal). Every macaque did develop humoral immunity toward the second serotype, as shown by our in *vivo* seroneutralization assay (Figure 34).

**5 A second injection with TRIP vector pseudotyped with an Indiana non cross-reactive VSV-G serotype stimulates earlier and stronger T cells responses**

Responses after the first and the second injection were compared and contrasted in terms of kinetic, intensity and diversity (Figures 32, 33). Three weeks before the boost, the frequency of SIV GAG-specific T cells was back to low levels. The second injection did restimulate GAG-specific T cells responses, as early as 8 days post-boost, faster than after the prime (peak at day 16). The intensity of responses was also stronger, even saturated for macaques 20022, 20195 and 20056 against pools T, U or V. At day 58 post-prime the responses turned back to the pre-immunization level (day-40). The C-ter extremity of the SIV GAG protein was still preferentially targeted by T cells (Figure 16). Remarkably, 8 days after the boost, macaque 20195 had increased the breadth of its SIV GAG specific response, with 5 pools of peptides out of 11 (T, U and V plus N and W) restimulating more than 375 IFN- SFC-$\gamma$ SFC/million PBMC.

Such benefits of a second injection were however not obvious for macaque 20293, which stayed a "low responder" as it was already after the first administration of vector.

As expected, no enhancement of the anti-SIV GAG cellular responses was observed for macaques 21544 and 20456 from the control group (Figure 32).

In addition, 16 days post-boost, the reactivity of SIV GAG-specific T cells against AT-2 inactivated SIVmac251 was analyzed (Figure 35). When coculturing PBMC with AT-2 inactivated SIV, viral proteins including GAG are naturally processed and presented both on MHC class II and I molecules to CD4$^+$ and CD8$^+$ T cells (Frank et al., 2003). GAG specific T cells responses monitored with AT-2 inactivated SIV mirrored the cumulative responses obtained with pools of peptides encompassing the SIV GAG protein, with macaques 20022, 20195 and 20056 responding the best and macaque 20195 being the only animal to show a saturated IFN-$\gamma$ response.

**6 TRIP vector-based immunizations confers strong protection against a massive viral challenge**

The major goal of this study was to test the protective efficacy of our TRIP vector-based immunization strategy. Macaques were therefore challenged with the SIVmac251strain through the intra-rectal route, 76 days post-boost (i.e. 155 days post-prime). A dose of 500 AID50 was chosen instead of the "classical" 10-20 AID50 dose used for viral challenge. Indeed, with this "classical"dose 50% of Cynomolgus macaques are spontaneous controllers. Cellular immunity against SIV GAG as well as against SIV NEF, for which all animals were naïve, were followed, in addition to plasma viral load (PVL) and CD4 count. These analyses are still in progress. Indeed, macaques were challenged in June but the longitudinal immunological and virological follow-up is going to last at least 12 months. During this period, we will compare the peak of viral replication (days 0 to 35), the post-acute viremia (days 35 to 80) and the long-term Set-Point SP (days 80-300) for immunized and naïve animals. Presently, only data until day 35 post-challenge were available and could be shown.

**6.1 Challenge triggers an increase in the frequency of GAG-specific T cells**

Viral challenge boosted SIV GAG specific IFN-$\gamma$ responses (figure 32). The frequency of GAG specific T cells had never been so high (up to 6000 SFC/ million PBMC for macaque 20056) and peaked at 2 to 3 weeks after viral inoculation. Even macaques 20293 and 20089, which were until challenge "low responders" developed strong responses. By day 35 post-challenge, GAG-specific cellular immunity tended to decrease in all animals. However, this was not true for macaque 20195. Surprisingly, this animal was one of the "best responder" post-immunizations, but its GAG-specific responses mesured in the blood were very low after challenge.

As expected, control and unvaccinated animals also mounted anti-SIV GAG responses. However, they were not as strong as in the vaccinees, except for non immunized macaque 15661 (up to 5000 SFC/million PBMC 3 weeks after challenge). Actually, anti-GAG responses intensity in the control and naïve arm after challenge were comparable to the one induced in immunized animals by one injection of TRIP-SIVmac239 Gag. Because of defective ELISPOT plates, we could not characterize the GAG-specific response in naïve animals 2 weeks post-challenge, to assess whether their responses were not only lower but also delayed compared to TRIP vector-immunized animals.

NEF-specific responses were also analyzed, but only at later time points (still during the acute phase of infection) when sufficient number of PBMC was available (figure 36). Every macaque developed NEF-specific immunity, indicating they had been correctly inoculated with challenge virus. However, no correlation between anti-GAG and anti-NEF responses could be shown.

## 6.2 Reactivity profiles of GAG-specific responses after challenge differ from profiles observed after vaccination

After SIV infection of immunized, control and naïve animals, the reactivity profile of GAG-specific responses was slightly different from the one elicited by TRIP-SIVmac239 Gag injections. Responses were broader and not targeted almost exclusively against the C-ter region of GAG (Pools T, U, and V), as reported after prime and boost. Some N-ter regions appeared immunogenic (Pools M, N and P) (Figure 34).

## 6.3 At the peak of primo-infection plasma viral loads inversely correlates with GAG-specific T cells responses intensity

Our trial was designed to use PVL as one of read-outs of protection. On purpose, the dose of virus inoculated was high (500 AID50), to avoid as much as possible spontaneous controllers within the small naïve group (figure 37). In the plasma of naïve animals, viral RNA was detected as early as 7 days post-challenge (macaques 15661 and 14468) and 14 days post-challenge (macaques 14184 and 15885). The peak of PVL was high, between $9.10^7$ and $5.10^8$ copies/ml around days 14 to 21 post-inoculation. On day 35 post-challenge, 3 naïve animals out of 4 (14184, 15885 and 14468) still showed a high PVL between $5.10^5$ and $2.10^7$ copies/ml. However, despite an elevated peak of PVL (up to $1,5.10^7$ copies/ml), macaque 15661 showed a PVL below the detection level as soon as 35 weeks post-infection and could consequently be considered as a spontaneous controller.

In immunized animals, PVL at the peak (14 days post-challenge) and 35 days post-challenge were lower than those of naïve animals 14184, 15885 and 14468 and control animals 21544 and 20456. One exception is the macaque 20089, which was a "bad responder" to immunizations, and behaved much like naïve 14184, 15885 and 14468 and control animals. Actually, an inverse correlation between the intensity of the GAG-specific T cells responses and plasma viremia at the peak was observed. Indeed, macaque 20293, which was also a "bad responder" after immunizations, had a peak at $2.10^7$ copies/ml and had surprisingly no detectable viral RNA from day 35 post-inoculation. Almost 2 log10 reduction of PVL was observed at the peak for animals 20158 and 20056 (respectively "medium" and "good responders" after immunisation). Their plasma viremia was a bit lower than the one of naïve 14184, 15885 and 14468 and control animals 35 days post-challenge. However SP had neither been reached nor stabilized, thus no conclusion could be drawn yet. Macaque 20022 (one of the "best responder") had $4.10^5$ copies/ml (ie 3 log10) reduction at the peak and no detectable viral RNA by day 35 post-challenge. Macaque 20195, which was a "good responder" after TRIP vector immunizations, but which had surprisingly no more responses detectable in its blood after challenge, had viremia always under the threshold of detection (considering the cut-off at $5.10^3$ copies/ml). However its anti-NEF response suggested it was not a case of full sterilizing immunity.

Control animals immunized with TRIP-GFP had a course of infection comparable to naïve animals 14184, 15885 and 14468 ($1.10^7$ and $1.10^8$ copies/ml) 14 days post-challenge and still high viral load 35 days post-inoculation ($2.10^6$ and $1,5.10^7$ copies/ml). It indicated that protection was specifically conferred by T cells directed against SIV GAG. Neither potential cross-reactive T cells activated by HIV-1 GAG present in the vector particles, nor systemic polyclonal immune activation resulting from the vector injections were involved in suppression of PVL.

Further experiments are still on-going to improve sensitivity of PVL measurements. It should also be mentioned that PVL were done with citrate-plasma collected only once a week, with a 1:10 dilution of plasma in citrate and consequently with a risk of RT-PCR inhibition. PVL measurements are going to be repeated with EDTA-plasma, collected twice a week and with a detection level as low as 50 copies/ml.

## DISCUSSION

So far, every attempt to develop effective T cells-based prophylactic vaccines against HIV-1/SIV infection and AIDS has failed. Only vaccines based on live attenuated viruses such as SIV-nef, which cannot be used in humans for safety reasons, were capable of protecting Indian rhesus macaques against pathogenic SIV strains infection. With live attenuated virus based vaccines, 95 % animals were protected, whereas in other vaccine strategies, including DNA alone, DNA and vector, poxvirus vectors, other vectors, vector combinations, whole-inactivated SIV or proteins and/or peptides, there was only 7% of protected animals. Protection was characterized as suppression of viral load of the peak of primo-infection of more than 3 log10 and protection against disease progression for at least 6 months (Koff et al., 2006). According to this stringent definition, and assuming that it can be translated to the Cynomolgus macaques model we used, the innovative prime/boost vaccination strategy based on lentiviral vector derived from HIV-1 we described here is very powerful, especially if we keep in mind that this first antigen design was not optimized (see below) and that challenge was performed with a huge virus dose. For the first time, we showed that 2 consecutive sub-cutaneous injections of the TRIP lentiviral vector encoding SIVmac239 gag pseudotyped with 2 non cross-reactive serotype of VSV-G conferred strong protections against massive mucosal challenge with SIVmac251. A high dose of challenge virus was voluntarily inoculated (500AID50) in order to limit the number of spontaneous controllers in the small cohort of naïve animals. Vaccinated macaques mounted strong and broad GAG-specific IFN-γ primary and secondary responses after immunizations and potent anamnestic GAG-specific responses after challenge. An inverse correlation between the intensity of the GAG-specific T cells responses and PVL at the peak of viremia was observed. At the peak of primo-infection, 5 out of 6 vaccinated macaques showed suppression of viremia of more than 1log10 compared to unvaccinated animals (all vaccinated animals except macaque 20089), two of more than 3 log10 (20195 and 20022), two of more than 2log10 (20056 and 20058) and of 1 log10 (20293). Three macaques out of 6 vaccinated animals show early control of viral replication at day 35 (20195, 20293, 20022).

More sensitive PVL measurements for animal 20195 only, which had undetectable viremia at day 35 post-challenge showed that it was actually viremic, but with very low PVL (maximum $3 \cdot 10^4$ copies/ml 14 days post-challenge). After challenge, it also mounted responses against non vaccine antigen NEF. Its humoral immunity to SIV proteins, including ENV, will also be assessed by Western Blot in the near future. Long-term longitudinal follow-up of the macaque cohort is to be performed. Although PVL at the peak of replication strongly predicts progression to AIDS (Saag, 1997; Wei et al., 1995), prognosis is more accurately defined by combining measurement of peak and SP viral loads as well as CD4 counts (Mellors, 1996). Therefore, CD4 counts have been performed to add to the significance to our results.

How come that our TRIP LV-based vaccine was so efficient? The high intensity and diversity of the GAG-specific responses induced by our prime/boost vaccine certainly exerted strong pressure on the challenge virus, containing its early replication at the site of inoculation. More especially, mutations within the structural protein GAG decrease viral fitness and require additional compensatory mutations to restore a replication-competent strain.

Further functional studies should be carried out to characterize in more details the quality of SIV GAG specific CD8[+] T cells induced by our TRIP-based vaccine. In particular it would be interesting to test their capacity to simultaneously secrete cytokines (IL-2, MIP-1b, TNF-a), to degranulate (CD107a, granzyme B, perforin expression) and to home mucosal sites. Indeed, it has been reported recently that HIV-1 long term non progessors were capable of maintaining highly polyfunctional HIV-1 specific CD8[+] T cells (Betts et al., 2006). Actually, the low responses measured in the blood of animal 20195 might reflect an effective mucosal homing of its effector T cells.

In addition, although TRIP-GAG vector was designed to induce CTL responses, it would also be interesting to evaluate CD4 priming.

The GAG antigen seems to be well chosen, however it could be interesting to broaden immune responses by fusioning gag with other sequences encoding non functional HIV/SIV proteins such as nef, tat or rev or with clusters of epitopes or polyepitopes.

It also proposed to use so-called codon-optimized genes to allow a better translation in mammalian cells. It would be useful both for the vector plasmid (to improve antigen expression by transduced cells) and for the envelope expression plasmid (to improve density of VSV-G on vector particles, particularly for the production of TRIP vectors pseudotyped with the second envelope, Indiana non cross reactive).

It is also proposed to add other small sequences to the vector plasmid such as an ubiquitination sequence to improve antigen turn-over by proteasome, thus allowing a better targeting to MHC class I. Addition of the sequence from the invariant chain of MHC class II molecules could also enhance antigen presentation to CD4[+] T cells. Eventually, other rigorous pre-clinical trials will have to be performed to ensure a complete inocuity of the TRIP vector as a vaccination tool against HIV-1. Indeed, when working with lentiviral vectors, the major safety concern is certainly to limit the risks of insertional mutagenesis. That is to say to avoid any deregulation in the expression of cellular genes (particularly proto-oncogenes and tumor suppressor genes) due to enhancer activity in the integrated vector. This was unfortunately illustrated by 3 leukemia cases reported in the SCID-X1 gene therapy trial, due to the integration of the Moloney-derived retroviral vector (MoMLV) at close proximity of the LMO2 proto-oncogene in the hematopoietic stem cells (Hacein-Bey-Abina et al., 2003). To this end, the TRIP vector used in our study was deleted in the U3 region of the viral LTR, what suppressed enhancer sequences and viral promoter (Zufferey et al., 1998). Consequently in these vectors, only the internal promoter and enhancer could be responsible for the activation of cellular genes. For a human vaccine application, the internal promoter should not contain any "risky" enhancer activity. Thus, the human CMV promoter will have to be replaced in a human trial to avoid activation of cellular genes after integration. The ubiquitous promoters EF1-α (elongation factor) as well as human PGK (Phosphoglycerate Kinase), currently used in the laboratory are potentially good substitution candidates.

Eventually, to solve this problem of insertional mutagenesis linked to pseudo-random integration of the transgene in the genome, it is proposed to use integration deficient TRIP vectors (TRIP-NI). These vectors carry a defective integrase (mutation in catalytic domain), which blocks integration step. It was shown that TRIP-NI vectors were still capable of transducing non dividing cells and of allowing efficient transgene expression (Vargas et al., 2004).

Anyway, a recent study of the team of Pr Naldini, showed that transduction of hematopoietic stem cells from prone tumor mice (Cdkn2a+) with U3 deleted lentiviral vectors did not provoke any tumorgenesis acceleration (Montini et al., 2006). Despite a high-gene transfer efficacy, no evidence of genotoxicity was found for U3 deleted lentiviral vector. This was not true for retroviral vectors derived from oncogenic retroviruses such as MoMLV.These data provide a major scientific rationale for advancing U3 deleted lentiviral vectors and particularly the TRIP vector to clinical experimentation.

Use of VSV-G envelope protein of different serotypes for pseudotyping lentiviral vector particles and generation of VSV-G Indiana cytoplasmic tail - ectodomain from other serotypes recombinant proteins.

The glycoprotein of the vesicular stomatisis virus (VSV-G) is a transmembrane protein that functions as the surface coat of the wild type viral particles. It is also a common coat protein for engineered lentiviral vectors.

Presently, nine virus species are definitively classified in the VSV gender, and nineteen rhabdoviruses are provisionally classified in this gender (see the description above), all showing various degrees of cross-neutralisation. When sequenced, the protein G genes indicate sequence similarities. The VSV-G protein presents a N-terminal ectodomain, a transmembrane region and a C-terminal cytoplasmic tail. It is exported to the cell surface *via* the transGolgi network (endoplasmic reticulum and Golgi apparatus).

Commonly, the VSV-G$_{Indiana}$ serotype is used as coat protein for lentiviral vectors. A codon optimized gene has been generated, and cloned between the BamH1 and EcoR1 sites of the pThV vector, generating the pThV-VSV.G (IND-CO) (Figure 6). The codon optimization for the expression of VSV-G proteins in human cells can stimulate gene transfer efficiency of a 100 fold factor, as shown in the case of the New Jersey serotype (figure 20).

When further VSV-G serotypes are required to design a suitable combination for use in the vaccine assay including at least one a boost injection, other VSV-G serotypes have been tested for particles coating. The first one used was the VSV-G$_{NewJersey}$ serotype. A codon optimized gene has been synthesized, and cloned between the BamH1 and EcoR1 sites of the pThV-plasmid, generating the pThV-VSV.G (NJ CO) vector (Figure 7).

Presently, only five other VSV-G genes have been sequenced (Chandipura, Cocal, Piry, Isfahan and spring viremia of carp virus, Figure 3), among those which can be used for the invention. Codon optimized genes may be generated starting from these genes, and cloned between the BamH1 and EcroR1 sites of the pThV plasmid, to generate the following vectors: pThV-VSV.G (CHANDI-CO), pThV-VSV.G (COCAL-CO), pThV-VSV.G (PIRY-CO), pThV-VSV.G (ISFA-CO) and pThV-VSV.G (SVCV-CO), (Figures 8-12).

It has been reported that VSV-G$_{Indiana}$ uses the tyrosine-based di-acidic motif (-YTDIE-) found in its cytoplasmic tail for export from the trans-Golgi network, reflecting a need for both efficient endoplasmic reticulum export and concentration of VSV-G into specialized post-trans-Golgi network secretory-lysosome type transport containers to facilitate formation of viral coats at the cell surface (Nishimura N et al 2002). Furthermore, it has been shown that mutations in this motif YxDxE motif dramatically reduced the exportation level of the VSV-G protein (Nishimura N. et al 2002).

Surprisingly, among all the VSV-G sequences, only VSV-G$_{Indiana}$, VSV-G$_{Cocal}$ and VSV-G$_{Isfahan}$ present this YxDxE motif, when the other serotypes present less efficient sequences (xxDxE, Yxxxx or xxxxx). In order to optimize the expression and exportation of our envelope proteins, fusions polynucleotides have been performed between the VSV-G$_{Indiana}$ intracellular region (transmembrane and cytoplasmic tail) and other VSV-G ectodomains. In a first time, a VSV-G$_{NewJersey/Indiana}$ chimeric protein was generated. To do so, the VSV-G$_{Indiana}$ region encompassing the transmembrane and cytoplasmic tail (oligonuleotides 1 and 2) and the VSV-G$_{NewJersey}$ ectodomain (oligonucleotides 3 and 4, the oligo 4 harboring a floating tail corresponding to the 28 first amino acids of the VSV-G$_{Indiana}$ transmembrane domain) were amplified by PCR. The chimeric protein was generated by a overlapping PCR, using oligos 2 and 3, cloned into pTOPO and then between the EcoR1 and BamH1 sites of the pThV plasmid, leading to the vector pThV-VSV.G(NJ/IND)CO (Figure).

The same operation is performed to generate fusion between the VSV-GIndiana intracellular domains and all the others VSV-G serotypes, leading to the constructions of pThV-VSV.G(CHANDI/IND)CO, pThV-VSV.G(COCAL/IND)CO, pThV-VSV.G(PIRY/IND)CO, pThV-VSV.G(ISFA/IND)CO and pThV-VSV.G(SVCV/IND)CO (Figure 14-19)

Bibliography

Addo, M. M., Yu, X. G., Rathod, A., Cohen, D., Eldridge, R. L., Strick, D., Johnston, M. N., Corcoran, C., Wurcel, A. G., Fitzpatrick, C. A., et al. (2003). Comprehensive epitope analysis of human immunodeficiency virus type 1 (HIV-1)-specific T-cell responses directed against the entire expressed HIV-1 genome demonstrate broadly directed responses, but no correlation to viral load. J Virol 77, 2081-2092.

Andrieu, J. M., and Lu, W. (2007). A dendritic cell-based vaccine for treating HIV infection: background and preliminary results. J Intern Med 261, 123-131.

Arhel, N. J., Souquere-Besse, S., Munier, S., Souque, P., Guadagnini, S., Rutherford, S., Prevost, M. C., Allen, T. D., and Charneau, P. (2007). HIV-1 DNA Flap formation promotes uncoating of the pre-integration complex at the nuclear pore. Embo J 26, 3025-3037.

Autran, B., Carcelain, G., Combadiere, B., and Debre, P. (2004). Therapeutic vaccines for chronic infections. Science 305, 205-208.

Autran, B., Carcelain, G., Li, T. S., Blanc, C., Mathez, D., Tubiana, R., Katlama, C., Debre, P., and Leibowitch, J. (1997). Positive effects of combined antiretroviral therapy on CD4+ T cell homeostasis and function in advanced HIV disease. Science 277, 112-116.

Andreas Bergthaler, Nicolas U. Gerber, Doron Merkler, Edit Horvath, Juan Carlos de la Torre, Daniel D. Pinschewer, 3- PloS Pathogens Vol. 2, No. 6, e51, Envelope Exchange for the Generation of Live-Attenuated Arenavirus Vaccines

Betts, M. R., Nason, M. C., West, S. M., De Rosa, S. C., Migueles, S. A., Abraham, J., Lederman, M. M., Benito, J. M., Goepfert, P. A., Connors, M., et al. (2006). HIV nonprogressors preferentially maintain highly functional HIV-specific CD8+ T cells. Blood 107, 4781-4789.

Breckpot, K., Dullaers, M., Bonehill, A., van Meirvenne, S., Heirman, C., de Greef, C., van der Bruggen, P., and Thielemans, K. (2003). Lentivirally transduced dendritic cells as a tool for cancer immunotherapy. J Gene Med 5, 654-667.

Brenchley, J. M., Price, D. A., Schacker, T. W., Asher, T. E., Silvestri, G., Rao, S., Kazzaz, Z., Bornstein, E., Lambotte, O., Altmann, D., et al. (2006). Microbial translocation is a cause of systemic immune activation in chronic HIV infection. Nat Med 12, 1365-1371.

Briggs, J. A., Simon, M. N., Gross, I., Krausslich, H. G., Fuller, S. D., Vogt, V. M., and Johnson, M. C. (2004). The stoichiometry of Gag protein in HIV-1. Nat Struct Mol Biol 11, 672-675.

Carrington, M., Nelson, G. W., Martin, M. P., Kissner, T., Vlahov, D., Goedert, J. J., Kaslow, R., Buchbinder, S., Hoots, K., and O'Brien, S. J. (1999). HLA and HIV-1: heterozygote advantage and B*35-Cw*04 disadvantage. Science 283, 1748-1752.

Cronin J. et al, Curr Gene Ther. 2005, August; 5(4) : 387-398 Altering the Tropism of Lentiviral Vectors through Pseudotyping

Day, C. L., Kaufmann, D. E., Kiepiela, P., Brown, J. A., Moodley, E. S., Reddy, S., Mackey, E. W., Miller, J. D., Leslie, A. J., DePierres, C., et al. (2006). PD-1 expression on HIV-specific T cells is associated with T-cell exhaustion and disease progression. Nature 443, 350-354.

Delenda, C. (2004). Lentiviral vectors: optimization of packaging, transduction and gene expression. J Gene Med 6 Suppl 1, S125-138.

Despres P. et al. Infect Dis. 2005; 191: 207-214

Donello J.E. et al, J. Virol. 1998, June; 72(6): 5085-92

Dullaers, M., and Thielemans, K. (2006). From pathogen to medicine: HIV-1-derived lentiviral vectors as vehicles for dendritic cell based cancer immunotherapy. J Gene Med 8, 3-17.

Esslinger, C., Chapatte, L., Finke, D., Miconnet, I., Guillaume, P., Levy, F., and MacDonald, H. R. (2003). In vivo administration of a lentiviral vaccine targets DCs and induces efficient CD8(+) T cell responses. J Clin Invest 111, 1673-1681.

Firat, H., Garcia-Pons, F., Tourdot, S., Pascolo, S., Scardino, A., Garcia, Z., Michel, M. L., Jack, R. W., Jung, G., Kosmatopoulos, K., et al. (1999). H-2 class I knockout, HLA-A2.1-transgenic mice: a versatile animal model for preclinical evaluation of antitumor immunotherapeutic strategies. Eur J Immunol 29, 3112-3121.

Firat H. et al. The Journal of Gene Medicine 2002; 4: 38-45

Frank, I., Santos, J. J., Mehlhop, E., Villamide-Herrera, L., Santisteban, C., Gettie, A., Ignatius, R., Lifson, J. D., and Pope, M. (2003). Presentation of exogenous whole inactivated simian immunodeficiency virus by mature dendritic cells induces CD4+ and CD8+ T-cell responses. J Acquir Immune Defic Syndr 34, 7-19.

Fredericksen B.L. et al .J. Virol. (1995) 69: 1435-1443

Gauduin, M. C., Yu, Y., Barabasz, A., Carville, A., Piatak, M., Lifson, J. D., Desrosiers, R. C., and Johnson, R. P. (2006). Induction of a virus-specific effector-memory CD4+ T cell response by attenuated SIV infection. J Exp Med 203, 2661-2672.

Girard, M. P., Osmanov, S. K., and Kieny, M. P. (2006). A review of vaccine research and development: the human immunodeficiency virus (HIV). Vaccine 24, 4062-4081.

Goulder, P. J., and Watkins, D. I. (2004). HIV and SIV CTL escape: implications for vaccine design. Nat Rev Immunol 4, 630-640.

Gulick, R. M., Mellors, J. W., Havlir, D., Eron, J. J., Meibohm, A., Condra, J. H., Valentine, F. T., McMahon, D., Gonzalez, C., Jonas, L., et al. (2000). 3-year suppression of HIV viremia with indinavir, zidovudine, and lamivudine. Ann Intern Med 133, 35-39.

Hacein-Bey-Abina, S., Von Kalle, C., Schmidt, M., McCormack, M. P., Wulffraat, N., Leboulch, P., Lim, A., Osborne, C. S., Pawliuk, R., Morillon, E., et al. (2003). LMO2-associated clonal T cell proliferation in two patients after gene therapy for SCID-X1. Science 302, 415-419.

Iglesias, M. C., Mollier, K., Beignon, A. S., Souque, P., Adotevi, O., Lemonnier, F., and Charneau, P. (2007). Lentiviral vectors encoding HIV-1 polyepitopes induce broad CTL responses in vivo. Mol Ther 15, 1203-1210.

Jin, X., Bauer, D. E., Tuttleton, S. E., Lewin, S., Gettie, A., Blanchard, J., Irwin, C. E., Safrit, J. T., Mittler, J., Weinberger, L., et al. (1999). Dramatic rise in plasma viremia after CD8(+) T cell depletion in simian immunodeficiency virus-infected macaques. J Exp Med 189, 991-998.

Kiepiela, P., Ngumbela, K., Thobakgale, C., Ramduth, D., Honeyborne, I., Moodley, E., Reddy, S., de Pierres, C., Mncube, Z., Mkhwanazi, N., et al. (2007). CD8+ T-cell responses to different HIV proteins have discordant associations with viral load. Nat Med 13, 46-53.

Koff, W. C., Johnson, P. R., Watkins, D. I., Burton, D. R., Lifson, J. D., Hasenkrug, K. J., McDermott, A. B., Schultz, A., Zamb, T. J., Boyle, R., and Desrosiers, R. C. (2006). HIV vaccine design: insights from live attenuated SIV vaccines. Nat Immunol 7, 19-23.

Koup, R. A., Safrit, J. T., Cao, Y., Andrews, C. A., McLeod, G., Borkowsky, W., Farthing, C., and Ho, D. D. (1994). Temporal association of cellular immune responses with the initial control of viremia in primary human immunodeficiency virus type 1 syndrome. J Virol 68, 4650-4655.

Gallione, C.J. and Rose, J.K. - J. Virol. 46(1), 162-169.

Iglesias MC, et al, A single immunization with a minute dose of a lentiviral vector-based vaccine is highly effective protective humoral immunity against West Nile virus. J. Gene Med. 2006 Mar; 8(3):265-274.

Iglesias MC et al, Polyepitopes Induce Broad CTL Responses In Vivo. Mol Ther. 2007 Jun, 15(6) : 1203-10.

Isidoro Martinez and Gail W. Wertz, The Journal of Virology, Mar. 2005, p. 3578-3585 Vol. 79, No. 6, Biological Differences between Vesicular Stomatitis Virus Indiana and New Jersey Serotype Glycoproteins: Identification of Amino acid Residues Modulating pH-Dependent Infectivity

Mellors, J. W. (1996). Closing in on human immunodeficiency virus-1. Nat Med 2, 274-275.

Montini, E., Cesana, D., Schmidt, M., Sanvito, F., Ponzoni, M., Bartholomae, C., Sergi Sergi, L., Benedicenti, F., Ambrosi, A., Di Serio, C., et al. (2006). Hematopoietic stem cell gene transfer in a tumor-prone mouse model uncovers low genotoxicity of lentiviral vector integration. Nat Biotechnol 24, 687-696.

Palella, F. J., Jr., Delaney, K. M., Moorman, A. C., Loveless, M. O., Fuhrer, J., Satten, G. A., Aschman, D. J., and Holmberg, S. D. (1998). Declining morbidity and mortality among patients with advanced human immunodeficiency virus infection. HIV Outpatient Study Investigators. N Engl J Med 338, 853-860.

Poznansky, M., Lever, A., Bergeron, L., Haseltine, W., and Sodroski, J. (1991). Gene transfer into human lymphocytes by a defective human immunodeficiency virus type 1 vector. J Virol 65, 532-536.

Reimann, K. A., Parker, R. A., Seaman, M. S., Beaudry, K., Beddall, M., Peterson, L., Williams, K. C., Veazey, R. S., Montefiori, D. C., Mascola, J. R., et al. (2005). Pathogenicity of simian-human immunodeficiency virus SHIV-89.6P and SIVmac is attenuated in cynomolgus macaques and associated with early T-lymphocyte responses. J Virol 79, 8878-8885.

Nina F. Rose, Anjeanette Roberts, Linda Buonocore, and John K. Rose, The Journal of Virology, Dec. 2000, p. 10903-10910 Vol. 74, No. 23, Glycoprotein Exchange Vectors based on Vesicular Stomatitis Virus Allow Effective Boosting and Generation of Neutralizing Antibodies to a Primary Isolate of Human Immunodeficiency Virus Type 1

Nina F. Rose, Preston A. Marx, Amara Luckay, Douglas F. Nixon, Walter J. Moretto, Sean M. Donahoe, David Montefiori, Anjeanette Roberts, Linda Buonocore, and John K. Rose, Cell, Vol. 106, 539-549, September 7, 2001, An Effective AIDS Vaccine Based on Live Attenuated Vesicular Stomatitis Virus Recombinants

Nishimura N. et al (PNAS (2002) 99: 6755-6760

Rosenberg, E. S., Altfeld, M., Poon, S. H., Phillips, M. N., Wilkes, B. M., Eldridge, R. L., Robbins, G. K., D'Aquila, R. T., Goulder, P. J., and Walker, B. D. (2000). Immune control of HIV-1 after early treatment of acute infection. Nature 407, 523-526.

Saag, M. S. (1997). Use of virologic markers in clinical practice. J Acquir Immune Defic Syndr Hum Retrovirol 16 Suppl 1, S3-13.

Sacha, J. B., Chung, C., Rakasz, E. G., Spencer, S. P., Jonas, A. K., Bean, A. T., Lee, W., Burwitz, B. J., Stephany, J. J., Loffredo, J. T., et al. (2007). Gag-specific CD8+ T lymphocytes recognize infected cells before AIDS-virus integration and viral protein expression. J Immunol 178, 2746-2754.

Steven AC. And Spear PG, Viral Glycoproteins and an Evolutionary Conundrum. Science 14 July 2006: Vol. 313. no. 5784, pp. 177-178.

Tonks, A. (2007). Quest for the AIDS vaccine. Bmj 334, 1346-1348.

Trkola, A., Kuster, H., Rusert, P., Joos, B., Fischer, M., Leemann, C., Manrique, A., Huber, M., Rehr, M., Oxenius, A., et al. (2005). Delay of HIV-1 rebound after cessation of antiretroviral therapy through passive transfer of human neutralizing antibodies. Nat Med 11, 615-622.

VandenDriessche T. et al. Blood, 1 August 2002- vol. 100, n° 3, p. 813-822

Vargas, J., Jr., Gusella, G. L., Najfeld, V., Klotman, M. E., and Cara, A. (2004). Novel integrase-defective lentiviral episomal vectors for gene transfer. Hum Gene Ther 15, 361-372.

Weber, J. (2001). The pathogenesis of HIV-1 infection. Br Med Bull 58, 61-72.

Wei, X., Ghosh, S. K., Taylor, M. E., Johnson, V. A., Emini, E. A., Deutsch, P., Lifson, J. D., Bonhoeffer, S., Nowak, M. A., Hahn, B. H., and et al. (1995). Viral dynamics in human immunodeficiency virus type 1 infection. Nature 373, 117-122.

Wiseman, R. W., Wojcechowskyj, J. A., Greene, J. M., Blasky, A. J., Gopon, T., Soma, T., Friedrich, T. C., O'Connor, S. L., and O'Connor, D. H. (2007). Simian immunodeficiency virus SIVmac239 infection of major histocompatibility complex-identical cynomolgus macaques from Mauritius. J Virol 81, 349-361.

Yee J. et al, 1994, Proc. Natl. Acad. Sci. USA 91, 9564-9568.

Zarei, S., Abraham, S., Arrighi, J. F., Haller, O., Calzascia, T., Walker, P. R., Kundig, T. M., Hauser, C., and Piguet, V. (2004). Lentiviral transduction of dendritic cells confers protective antiviral immunity in vivo. J Virol 78, 7843-7845.

Zennou, V., Petit, C., Guetard, D., Nerhbass, U., Montagnier, L., and Charneau, P. (2000). HIV-1 genome nuclear import is mediated by a central DNA flap. Cell 101, 173-185.

Zufferey, R., Donello, J. E., Trono, D., and Hope, T. J. (1999). Woodchuck hepatitis virus posttranscriptional regulatory element enhances expression of transgenes delivered by retroviral vectors. J Virol 73, 2886-2892.

Zufferey, R., Dull, T., Mandel, R. J., Bukovsky, A., Quiroz, D., Naldini, L., and Trono, D. (1998). Self-inactivating lentivirus vector for safe and efficient in vivo gene delivery. J Virol 72, 9873-9880.

**Claims**

1. A lentiviral vector comprising a recombinant polynucleotide encoding one or several polypeptides comprising at least one antigen for use in treatment in a prime-boost regimen by eliciting an immune response in a host against said polypeptides , wherein said antigen is a chimeric HIV-1 derived antigen which is a fusion protein comprising or consisting in the combination of the GAG derived antigen having a sequence chosen among the following:

MASVLSGGELDRWEKIRLRPGGKKKYKLKHIVWASRELERFAVNPGLLETSEGC
RQILGQLQPSLQTGSEELRSLYNTVATLYCVHQRIEVKDTKEALDKIEEEQNKSKK
KAQQAAADTNHSSQVSQNYPIVQNLQGQMVHQAISPRTLNAWVKVVEEKAFSP
EVIPMFSALSEGATPQDLNTMLNTVGGHQAAMQMLKETINEEAAEWDRLHPVHA
GPIAPGQMREPRGSDIAGTTSTLQEQIGWMTNNPPIPVGEIYKRWIILGLNKIVRM
YSPTSILDIRQGPKEPFRDYVDRFYKTLRAEQASQEVKNWMTETLLVQNANPDC
KTILKALGPAATLEEMMTACQGVGGPGHKARVLAEAMSQVTNSATIMMQRGNF
RNQRKTVKCFNCGKEGHIAKNCRAPRKKGCWKCGKEGHQMKDCTERQANFLG
KIWPSHKGRPGNFLQSRPEPTAPPEESFRFGEETTTPSQKQEPIDKELYPLASLR
SLFGND, or

MGARASVLSGGELDRWEKIRLRPGGKKKYKLKHIVWASRELERFAVNPGLLETS
EGCRQILGQLQPSLQTGSEELRSLYNTVATLYCVHQRIEIKDTKEALDKIEEEQNK
SKKKAQQAAADTGHSSQVSQNYPIVQNIQGQMVHQAISPRTLNAWVKVVEEKAF
SPEVIPMFSALSEGATPQDLNTMLNTVGGHQAAMQMLKETINEEAAEWDRVHPV
HAGPIAPGQMREPRGSDIAGTTSTLQEQIGWMTNNPPIPVGEIYKRWIILGLNKIV
RMYSPTSILDIRQGPKEPFRDYVDRFYKTLRAEQASQEVKNWMTETLLVQNANP
DCKTILKALGPAATLEEMMTACQGVGGPGHKARVLAEAMSQVTNSATIMMQRG
NFRNQRKIVKCFNCGKEGHIARNCRAPRKKGCWKCGKEGHQMKDCTERQANF
LGKIWPSYKGRPGNFLQSRPEPTAPPFLQSRPEPTAPPEESFRSGVETTTPSQK
QEPIDKELYPLTSLRSLFGNDPSSQ

with an antigen derived from NEF, POL, TAT or REV of a HIV-1 virus strain or with a combination of such antigens.

**2.** A lentiviral vector comprising a recombinant polynucleotide for use according to claim 1, wherein the POL derived antigen comprises or has the following amino acid sequence:

KKSVTVLDVGDAYFSVPLDKDFRKYTAFTIPSINNETPGIRYQYNVLPQGWKGSP
AIFQSSMTKILEPFRKQNPDIVIYQYMDDLYVGSDLEIGQHRTEILKEPVHGVY.

**3.** A lentiviral vector comprising a recombinant polynucleotide for use according to claim 1 or 2, wherein the NEF derived antigen comprises or has the flowing amino acid sequence:

VGFPVRPQVPLRPMTYKAAVDLSHFLKEKGGLEGLIYSQKRQDILDLWVYHTQG
YFPDWQNYTPGPGIRYPLTFGWCFKLVPVDPEKEVLVWKFDSRLAFHHMAREL
HPEYYAPVKQTLNFDLLKLAGDVESNPGP.

**4.** A lentiviral vector comprising a recombinant polynucleotide for use according to claim 3, which has one of the following structures: 5' *gag pol nef 3',* or 5' *pol nef gag* 3' or 5' *pol gag nef 3',* or 5' *nef gag pol* 3' or 5' *nef pol gag* 3' or 5' *gag nef pol* 3'.

**5.** A lentiviral vector comprising a recombinant polynucleotide for use according to any of claims 1 to 4, wherein said recombinant polynucleotide is under the control of regulatory sequences for transcription and expression including a promoter selected from the following: EF1α promoter, CGA promoter, CD11c promoter or house keeping gene promoters such as PGK promoter, ubiquitin promoter, actin promoter, histone promoter, alpha-tubulin promoter, beta-tubulin promoter, superoxide dismutase 1 (SOD-1) promoter, dihydrofolate reductase (DHFR) promoter, hypoxanthine phosphorybosyltransferase (HPRT) promoter, adenosine deaminase promoter, thymidylate synthetase promoter, dihydrofolate reductase P1 promoter, glucose-6-phosphate dehydrogenase promoter or nucleolin promoter, or promoters of genes selected from EF1α, human PGK, PPI (preproinsulin), thiodextrin, HLA DR invariant chain (P33), HLA DR alpha chain, Ferritin L chain or Ferritin H chain, Beta2 microglobulin, Chymosin beta 4, Chimosin beta 10, Cystatin Ribosomal protein L41.

**6.** A lentiviral vector comprising a recombinant polynucleotide for use according to any of claims 1 to 5, wherein the envelope protein is under the control of regulatory sequences for transcription and expression including a promoter of genes selected from EF1α, human PGK, PPI (preproinsulin), thiodextrin, HLA DR invariant chain (P33), HLA DR alpha chain, Ferritin L chain, Ferritin H chain, Beta 2 microglobulin, Chymosin beta 4, Chymosin beta 10, or Cystatin Ribosomal protein L41.

7. A lentiviral vector comprising a recombinant polynucleotide for use according to any of claims 1 to 6, wherein a (i) lentiviral vector pseudotyped with a first viral envelope protein(s) is administered separately in time from a (ii) lentiviral vector pseudotyped with a second viral envelope protein(s), and, if any vector from a (iii), lentiviral vectors pseudotyped with a third viral envelope protein(s), each of said lentiviral vectors (i) and (ii) and if any (iii) being administered either for priming or for boosting the immune response.

8. A lentiviral vector comprising a recombinant polynucleotide for use according to any of claims 1 to 7, wherein the GAG-derived antigen is a GAG delta myr antigen, which is not myristylated.

9. A lentiviral vector comprising a recombinant polynucleotide for use according to any one of claims 1 to 8, wherein the coding sequences, especially the coding sequence of the antigen, are codon optimized to improve translation in mammalian cells, especially in human cells.

**Patentansprüche**

1. Lentiviraler Vektor, umfassend ein rekombinantes Polynukleotid, das für ein oder mehrere Polypeptide kodiert, umfassend wenigstens ein Antigen für die Verwendung bei einer Behandlung mit einem Prime / Boost-Regimen durch Auslösen einer Immunantwort bei einem Wirt gegen die genannten Polypeptide, wobei das genannte Antigen ein chimerisches, HIV-1 abgeleitetes Antigen ist, das ein Fusionsprotein ist, das eine Kombination des GAG-abgeleiteten Antigens umfasst oder daraus besteht, das eine Sequenz aufweist, die aus Folgendem ausgewählt ist:

MASVLSGGELDRWEKIRLRPGGKKKYKLKHIVWASRELERFAVNPGLLETSEGC
RQILGQLQPSLQTGSEELRSLYNTVATLYCVHQRIEVKDTKEALDKIEEEQNKSKK
KAQQAAADTNHSSQVSQNYPIVQNLQGQMVHQAISPRTLNAWVKVVEEKAFSP
EVIPMFSALSEGATPQDLNTMLNTVGGHQAAMQMLKETINEEAAEWDRLHPVHA
GPIAPGQMREPRGSDIAGTTSTLQEQIGWMTNNPPIPVGEIYKRWIILGLNKIVRM
YSPTSILDIRQGPKEPFRDYVDRFYKTLRAEQASQEVKNWMTETLLVQNANPDC
KTILKALGPAATLEEMMTACQGVGGPGHKARVLAEAMSQVTNSATIMMQRGNF
RNQRKTVKCFNCGKEGHIAKNCRAPRKKGCWKCGKEGHQMKDCTERQANFLG
KIWPSHKGRPGNFLQSRPEPTAPPEESFRFGEETTTPSQKQEPIDKELYPLASLR
SLFGND, oder

MGARASVLSGGELDRWEKIRLRPGGKKKYKLKHIVWASRELERFAVNPGLLETS
EGCRQILGQLQPSLQTGSEELRSLYNTVATLYCVHQRIEIKDTKEALDKIEEEQNK
SKKKAQQAAADTGHSSQVSQNYPIVQNIQGQMVHQAISPRTLNAWVKVVEEKAF
SPEVIPMFSALSEGATPQDLNTMLNTVGGHQAAMQMLKETINEEAAEWDRVHPV
HAGPIAPGQMREPRGSDIAGTTSTLQEQIGWMTNNPPIPVGEIYKRWIILGLNKIV
RMYSPTSILDIRQGPKEPFRDYVDRFYKTLRAEQASQEVKNWMTETLLVQNANP
DCKTILKALGPAATLEEMMTACQGVGGPGHKARVLAEAMSQVTNSATIMMQRG
NFRNQRKIVKCFNCGKEGHIARNCRAPRKKGCWKCGKEGHQMKDCTERQANF
LGKIWPSYKGRPGNFLQSRPEPTAPPFLQSRPEPTAPPEESFRSGVETTTPSQK
QEPIDKELYPLTSLRSLFGNDPSSQ

mit einem Antigen, das abgeleitet ist aus NEF, POL, TAT oder REV oder einem HIV-1 Virusstrang oder mit einer Kombination von solchen Antigenen.

2. Lentiviraler Vektor, umfassend ein rekombinantes Polynukleotid für die Verwendung gemäß Anspruch 1, wobei das POL-abgeleitete Antigen die folgende Aminosäuresequenz umfasst oder aufweist:

KKSVTVLDVGDAYFSVPLDKDFRKYTAFTIPSINNETPGIRYQYNVLPQGWKGSP

AIFQSSMTKILEPFRKQNPDIVIYQYMDDLYVGSDLEIGQHRTEILKEPVHGVY.

3. Lentiviraler Vektor, umfassend ein rekombinantes Polynukleotid für die Verwendung gemäß Anspruch 1 oder 2, wobei das NEF-abgeleitete Antigen die folgende Aminosäuresequenz umfasst oder aufweist:

VGFPVRPQVPLRPMTYKAAVDLSHFLKEKGGLEGLIYSQKRQDILDLWVYHTQG

YFPDWQNYTPGPGIRYPLTFGWCFKLVPVDPEKEVLVWKFDSRLAFHHMAREL

HPEYYAPVKQTLNFDLLKLAGDVESNPGP.

4. Lentiviraler Vektor, umfassend ein rekombinantes Polynukleotid für die Verwendung gemäß Anspruch 3, das eine der folgenden Strukturen aufweist: 5' *gag pol nef* 3', oder 5' *pol nef gag* 3' oder 5' *pol gag nef 3',* oder 5' *nef gag pol* 3' oder 5' *nef pol gag* 3' oder 5' *gag nef pol* 3'.

5. Lentiviraler Vektor, umfassend ein rekombinantes Polynukleotid für die Verwendung gemäß irgendeinem der Ansprüche 1 bis 4, wobei das genannte rekombinante Polynukleotid unter der Kontrolle von regulierenden Sequenzen für die Transkription und Expression gemäß einem Promotor ist, der aus Folgendem ausgewählt ist: EF1$\alpha$-Promotor, CGA-Promotor, CD11c-Promotor oder Housekeeping-Genpromotor, wie z. B. PGK-Promotor, Ubiquitin-Promotor, Actin-Promotor, Histon-Promotor, Alpha-Tubulinpromotor, Beta-Tubulinpromotor, Superoxid-Dismutase 1 (SOD-1) Promotor, Dihydrofolat-Reduktase (DHFR) Promotor, Hypoxanthin-Phosphorybosyltransferase (HPRT) Promotor, Adenosin-Deaminasepromotor, Thymidylate-Synthetasepromotor, Dishydrofolat-Reduktase P1 Promotor, Glukose-6-Phosphat-Dehydrogenasepromotor oder Nukleolin-Promotor oder Promotor der Gene, die aus EF1$\alpha$, menschlichem PGK, PPI (Präproinsulin), Thiodextrin, HLA DR invarianter Kette (P33), HLA DR Alpha-Kette, Ferritin-L-Kette oder Ferritin-H-Kette, Beta2-Mikroglobulin, Chymosin-Beta 4, Chimosin-Beta 10, Cystatin-Ribosomalprotein L41 ausgewählt ist.

6. Lentiviraler Virus, umfassend ein rekombinantes Polynukleotid für die Verwendung gemäß irgendeinem der Ansprüche 1 bis 5, wobei das Hüllprotein unter der Kontrolle von regulierenden Sequenzen für die Transkription und Expression unter Einschluss eines Promotors von Genen ist, der ausgewählt ist aus EF1$\alpha$, menschlichem PGK, PPI (Präproinsulin), Thiodextrin, HLA DR invarianter Kette (P33), HLA DR Alpha-Kette, Ferritin-L-Kette, Ferritin-H-Kette, Beta2-Mikroglobulin, Chymosin-Beta 4, Chimosin-Beta 10 oder Cystatin-Ribosomalprotein L41

7. Lentiviraler Virus, umfassend ein rekombinantes Polynukleotid für die Verwendung gemäß irgendeinem der Ansprüche 1 bis 6, wobei ein (i) lentiviraler Vektor, der mit einem ersten viralen Hüllprotein pseudotypisiert ist, zeitlich getrennt von einem (ii) lentiviralen Vektor, der pseudotypisiert mit einem zweiten viralen Hüllprotein verabreicht wird, und, wenn irgendein Vektor von einem (iii) lentiviralen Vektor, der mit einem dritten viralen Hüllprotein pseudotypisiert wird, wobei jeder der genannten lentiviralen Vektor(en) (i) und (ii) wenn irgendein (iii) entweder für ein Initiieren oder zum Boosten der Immunantwort verabreicht wird.

8. Lentiviraler Vektor, umfassend ein rekombinantes Polynukleotid für die Verwendung gemäß irgendeinem der Ansprüche 1 bis 7, wobei das GAG-abgeleitete Antigen ein GAG-Delta-myrAntigen ist, das nicht myristoyliert ist.

9. Lentiviraler Vektor, umfassend ein rekombinantes Polynukleotid für die Verwendung gemäß irgendeinem der Ansprüche 1 bis 8, wobei die kodierenden Sequenzen, insbesondere die kodierende Sequenz des Antigens, kodonoptimiert sind, um die Translation in Säugetierzellen, insbesondere in menschlichen Zellen, zu verbessern.

**Revendications**

1. Vecteur lentiviral comprenant un polynucléotide recombinant codant pour un ou plusieurs polypeptides comprenant au moins un antigène pour son utilisation dans le traitement dans un régime d'initiation-amplification (prime-boost) par déclenchement d'une réponse immunitaire contre lesdits polypeptides chez un hôte, dans lequel ledit antigène est un antigène chimérique dérivé du VIH-1 qui est une protéine de fusion comprenant la, ou constituée de la, combinaison de l'antigène dérivé de GAG ayant une séquence sélectionnée parmi les suivantes :

MASVLSGGELDRWEKIRLRPGGKKKYKLKHIVWASRELERFAVNPGLLETSEGC
RQILGQLQPSLQTGSEELRSLYNTVATLYCVHQRIEVKDTKEALDKIEEEQNKSKK
KAQQAAADTNHSSQVSQNYPIVQNLQGQMVHQAISPRTLNAWVKVVEEKAFSP
EVIPMFSALSEGATPQDLNTMLNTVGGHQAAMQMLKETINEEAAEWDRLHPVHA
GPIAPGQMREPRGSDIAGTTSTLQEQIGWMTNNPPIPVGEIYKRWIILGLNKIVRM
YSPTSILDIRQGPKEPFRDYVDRFYKTLRAEQASQEVKNWMTETLLVQNANPDC
KTILKALGPAATLEEMMTACQGVGGPGHKARVLAEAMSQVTNSATIMMQRGNF
RNQRKTVKCFNCGKEGHIAKNCRAPRKKGCWKCGKEGHQMKDCTERQANFLG
KIWPSHKGRPGNFLQSRPEPTAPPEESFRFGEETTTPSQKQEPIDKELYPLASLR
SLFGND, ou

MGARASVLSGGELDRWEKIRLRPGGKKKYKLKHIVWASRELERFAVNPGLLETS
EGCRQILGQLQPSLQTGSEELRSLYNTVATLYCVHQRIEIKDTKEALDKIEEEQNK
SKKKAQQAAADTGHSSQVSQNYPIVQNIQGQMVHQAISPRTLNAWVKVVEEKAF
SPEVIPMFSALSEGATPQDLNTMLNTVGGHQAAMQMLKETINEEAAEWDRVHPV
HAGPIAPGQMREPRGSDIAGTTSTLQEQIGWMTNNPPIPVGEIYKRWIILGLNKIV
RMYSPTSILDIRQGPKEPFRDYVDRFYKTLRAEQASQEVKNWMTETLLVQNANP
DCKTILKALGPAATLEEMMTACQGVGGPGHKARVLAEAMSQVTNSATIMMQRG
NFRNQRKIVKCFNCGKEGHIARNCRAPRKKGCWKCGKEGHQMKDCTERQANF
LGKIWPSYKGRPGNFLQSRPEPTAPPFLQSRPEPTAPPEESFRSGVETTTPSQK
QEPIDKELYPLTSLRSLFGNDPSSQ

avec un antigène dérivé de NEF, POL, TAT ou REV d'une souche du virus VIH-1 ou avec une combinaison de ces antigènes.

**2.** Vecteur lentiviral comprenant un polynucléotide recombinant pour son utilisation selon la revendication 1, dans lequel l'antigène dérivé de POL comprend ou a la séquence d'acides aminés suivante :

KKSVTVLDVGDAYFSVPLDKDFRKYTAFTIPSINNETPGIRYQYNVLPQGWKGSP
AIFQSSMTKILEPFRKQNPDIVIYQYMDDLYVGSDLEIGQHRTEILKEPVHGVY.

**3.** Vecteur lentiviral comprenant un polynucléotide recombinant pour son utilisation selon la revendication 1 ou 2, dans lequel l'antigène dérivé de NEF comprend ou a la séquence d'acides aminés suivante :

VGFPVRPQVPLRPMTYKAAVDLSHFLKEKGGLEGLIYSQKRQDILDLWVYHTQG
YFPDWQNYTPGPGIRYPLTFGWCFKLVPVDPEKEVLVWKFDSRLAFHHMAREL
HPEYYAPVKQTLNFDLLKLAGDVESNPGP.

**4.** Vecteur lentiviral comprenant un polynucléotide recombinant pour son utilisation selon la revendication 3, qui a l'une des structures suivantes : 5' *gag pol nef* 3', ou 5' *pol nef gag* 3' ou 5' *pol gag nef* 3', ou 5' *nef gag pol* 3' ou 5' *nef pol gag* 3' ou 5' *gag nef pol* 3'.

**5.** Vecteur lentiviral comprenant un polynucléotide recombinant pour son utilisation selon l'une quelconque des revendications 1 à 4, dans lequel ledit polynucléotide recombinant est sous le contrôle des séquences de régulation pour la transcription et l'expression comprenant un promoteur sélectionné parmi les suivants : promoteur EF1α, promoteur CGA, promoteur CD11c ou promoteurs des gènes domestiques tels que le promoteur PGK, le promoteur de l'ubiquitine, le promoteur de l'actine, le promoteur de l'histone, de promoteur de l'alpha-tubuline, le promoteur de la bêta-tubuline, le promoteur de la superoxyde dismutase 1 (SOD-1), le promoteur de la dihydrofolate réductase (DHFR), le promoteur de l'hypoxanthine phosphorybosyltransférase (HPRT), le promoteur de l'adénosine désaminase, le promoteur de la thymidylate synthétase, le promoteur de la dihydrofolate réductase P1, le promoteur de la glucose-6-phosphate déshydrogénase ou le promoteur de la nucléoline, ou des promoteurs de gènes sélectionnés parmi EF1α, PGK humain, PPI (préproinsuline), thiodextrine, chaîne invariante de HLA DR (P33), chaîne alpha de HLA DR, chaîne L de la ferritine ou chaîne H de la ferritine, microglobuline bêta 2, chymosine bêta 4, chymosine bêta 10, ou protéine ribosomique cystatine L41.

6. Vecteur lentiviral comprenant un polynucléotide recombinant pour son utilisation selon l'une quelconque des revendications 1 à 5, dans lequel la protéine d'enveloppe est sous le contrôle de séquences de régulation pour la transcription et l'expression comprenant un promoteur de gènes sélectionnés parmi EF1α, PGK humain, PPI (préproinsuline), thiodextrine, chaîne invariante de HLA DR (P33), chaîne alpha de HLA DR, chaîne L de la ferritine ou chaîne H de la ferritine, microglobuline bêta 2, chymosine bêta 4, chymosine bêta 10, ou protéine ribosomique cystatine L41.

7. Vecteur lentiviral comprenant un polynucléotide recombinant pour son utilisation selon l'une quelconque des revendications 1 à 6, dans lequel (i) un vecteur lentiviral pseudotypé avec une (des) première(s) protéine(s) d'enveloppe virale est administré séparément dans le temps d'un (ii) vecteur lentiviral pseudotypé avec une (des) deuxième(s) protéine(s) d'enveloppe virale, et, le cas échéant, d'un vecteur de (iii), des vecteurs lentiviraux pseudotypé avec une (des) troisième(s) protéine (s) d'enveloppe virale, chacun desdits vecteurs lentiviraux (i) et (ii) et le cas échéant (iii) étant administré soit pour l'initiation soit pour l'amplification de la réponse immunitaire.

8. Vecteur lentiviral comprenant un polynucléotide recombinant pour son utilisation selon l'une quelconque des revendications 1 à 7, dans lequel l'antigène dérivé de GAG est un antigène GAG delta myr qui n'est pas myristylé.

9. Vecteur lentiviral comprenant un polynucléotide recombinant pour son utilisation selon l'une quelconque des revendications 1 à 8, dans lequel les codons des séquences codantes, notamment de la séquence codante de l'antigène, sont optimisés pour améliorer la traduction dans les cellules de mammifère, notamment dans les cellules humaines.

**CAEV**

GTTCCAG CCAC AAT TTGTCGC TGTA GAA TCAGCCA TAGC AGC AGCCCTA GTCG C
CATAAAT ATAA AAA GAAAGGG TGGG CTG GGGACAA GCCC TATGGATATT TTTA T
ATATAAT AAAG AAC AGAAAAG AATA AAT AATAAAT ATAA TAAAAATTCT CAAA A
AATTCAA TTCT GTT ATTACAG AATA AGG AAAAGAG GAC (SEQ ID NO:1)

**EIAV**

CTTGTAA CAAA GGG AGGGAAA GTAT GGG AGGACAG ACAC CATGGGAAGT ATTT A
TCACTAA TCAA GCA CAAGTAA TACA TGA GAAACTT TTAC TACAGCAAGC ACAA T
CCTCCAA AAAA TTT TGTTTTT ACAA AAT CCCTGGT GAAC ATGATTGGAA GGGA C
CTACTAG GGTG CTG TGGAAGG GTGA TGG TGCAGTA GTA (SEQ ID NO:2)

**VISNA**

GGACCCT CATRACT CTAAATA TAAA AAG AAAGGGT GGGC TAGGGACAAG CCCTA
TGGATAT ATTT ATA TTTAATA AGGA ACA ACAAAGA ATAC AGCAACAAAG TAAA T
CAAAACA AGAA AAA ATTCGAT TTTG TTA TTACAGA ACAA GAAAAGAGG GCAT C
CAGGAGA GTGG CAA GGACCAA CACA GGTACTTTGG GGC (SEQ ID NO:3)

**SIV_{AGM}**

TACTGAT GGCT TGCATACTTC ACAA TTT TAAAAGA AAGG GAGGAATAGG GGGA C
AGACTTC AGCA GAGAGACTAA TTAA TAT AATAACA ACAC AATTAGAAAT ACAA C
ATTTACA AACC AAAATTCAAA AAAT TTT AAATTTT AGAG TCTACTACAG AGAA G
GGAGAGA CCCT GTG TGGAAAG GACC GGCACAATTA ATC (SEQ ID NO:4)

**HIV-2 ROD**

TGCATGA ATTT TAAAAGAAGG GGGG GAA TAGGGGA TATG ACTCCATCAG AAAG A
TTAATCA ATAT GAT CACCACA GAAC AAG AGATACA ATTC CTCCAAGCCA AAAA T
TCAAAAT TAAA AGA TTTTCGG GTCT ATT TCAGAGA AGGC AGAGATCAGT TGTG G
AAAGGAC CTGG GGA ACTACTG TGGA AAG GAGAAGG AGC (SEQ ID NO:5)

**HIV-1 LAI**

CAGTATT CATC CACAATTTTA AAAG AAA AGGGGGG ATTG GGGGGTACAG TGCA G
GGGAAAG AATA GTA GACATAA TAGC AAC AGACATA CAAA CTAAAGAATT ACAA A
AACAAAT TACA AAA ATTCAAA ATTT TCG GGTTTAT TACA GGGACAGCAG AGAT C
CACTTTG GAAA GGA CCAGCAA AGCT CCTCTGGAAA GGT (SEQ ID NO:6)

**HIV1**

TTTTAAA AGAA AAG GGGGGAT TGGG GGG TACAGTG CAGG GGAAAGAATA GTAG A
CATAATA GCAA CAG ACATACA AACT AAA GAATTAC AAAA ACAAATTACA AAAA T
TCAAAAT TTTC (SEQ ID NO:7)

Fig 1

48

Fig 2 (A)

Fig 2(B)

## Restriction map of pTRIP ΔU3 CMV GFP

Narl (639) NotI (1145)
EcoNI (1164)

Avall (1932)
EcoRI ((≈2000)

BamHI (≈2800)

Xho I (≈3400)

LTR

GAG

RRE

SD

SA

Ψ

TRIPLEX
cPPT -CTS

CMV

EGFP

R US

ΔU3

Figure 2c

## Restriction map of pTRIP ΔU3 EFIα GFP

Narl (639)  NotI (1145)
EcoNI (1164)

AvaII (1932)
EcoRI ((≈2000)

BamHI (≈2400)

Xho I (≈3200)

RRE

LTR

GAG

SA

SD

Ψ

CPPT CTS

EFIα

EGFP

R  U5

TRIPLEX

ε PPT_CTS

ΔU3

Figure 2 D

```
CHP     MTSSVTISVVLLISFITPLYSYLSIAFPENTKLDWKPVTKNTRYCPMGGEWFLEPGLQEESFLSSTPIGATPSKSD
COCAL    MNFLLLTFIVLPLCSHAKFSIVFPQSQKGNWKNVPSSYHYCPSSSDQNWHNDLLGITMKVKMPKTHKAIQAD
IND      MKCLLYLAFLFIGVNCKFTIVFPHNQKGNWKNVPSNYHYCPSSSDLNWHNDLVGTALQVKMPKSHKAIQAD
NJ       MLSYLILAIVVSPILGKIEIVFPQHTTGDWKRVPHEYNYCPTSADKNSHGTQTGIPIELTMPKGLTTHQVD
ISFA    MTSVLFMVGVLLGAFGSTHCSIQIVFPSETKLVWKPVLKGTRYCPQSAELNLEPDLKTMAFDSKVPIGITPSNSD
PIRY         MTDTVLGKFQIVFPDQNELEWTPVVGDSRHCPQSSEMQFDGSRSQTILTGKAPVGITPSKSD
SVCV     MSIISYIAFLLLIDSTLGIPIFVPSGQNISWQPVIQPFDYQCPIHGNLPNTMGLSATKLTIKSPSVFSTDKVS


CHP     GFLCHAAKWVTTCDFRWYGPKYITHSIHNIKPTRSDCDTALASYKSGTLVSLGFPPESCGYASVTDSEFLVIMITP
COCAL   GWMCHAAKWITTCDFRWYGPKYITHSIHSIQPTSEQCKESIKQTKQGTWMSPGFPPQNCGYATVTDSVAVVVQATP
IND     GWMCHASKWVTTCDFRWYGPKYITHSIRSFTPSVEQCKESIEQTKQGTWLNPGFPPQSCGYATVTDAEAAIVQVTP
NJ      GFMCHSALWMTTCDFRWYGPKYITHSIHNEEPTDYQCLEAIKAYKDGVSFNPGFPPQSCGYGTVTDAEAHIITVTP
ISFA    GYLCHAAKWVTTCDFRWYGPKYITHSVHSLRPTVSDCKAAVEAYNAGTLMYPGFPPESCGYASITDSEFYVMLVTP
PIRY    GFICHAAKWVTTCDFRWYGPKYITHSIHHLRPTTSDCETALQRYKDGSLINLGFPPESCGYATVTDSEAMLVQVTP
SVCV    GWICHAAEWKTTCDYRWYGPQYITHSIHPISPTIDECKRIISRIASGTDEDLGFPPQSCGWASVTTVSNTNYKVVP


CHP     HHVGVDDYRGHWVDPLFVGGECDQSYCDTIHNSSVWIPADQTKKNICGQSFTPLTVTVAYDKTK--EIAAGGIVFK
COCAL   HHVLVDEYTGEWIDSQFPNGKCETEECETVHNSTVWYSDYKV-TGLCDATLVDTEITFFSEDGKKESIGKPNTGYR
IND     HHVLVDEYTGEWVDSQFINGKCSNYICPTVHNSTTWHSDYKV-KGLCDSNLISMDITFFSEDGELSSLGKKGTGFR
NJ      HSVKVDEYTGEWIDPHFIGGRCKGQICETVHNSTKWFTSSDG-ESVCSQLFTLVGGTFFSDSEEITSMGLPETGIR
ISFA    HPVGVDDYRGHWVDPLFPTSECNSNFCETVHNATMWIPKDLKTHDVCSQDFQTIRVSVMYPQTK--PTKGADLTLK
PIRY    HHVGVDDYRGHWIDPLFPGGECSTNFCDTVHNSSVWIPKSQK-TDICAQSFKNIKMTASYPSEG--ALVSDRFAFH
SVCV    HSVHLEPYGGHWIDHDFNGGECREKVCEMKGNHSIWITDETV-QHECEKHIEEVEGIMYGNAPR-GDAIYINNFII


CHP     SKYHSHMEGARTCRLSYCGRNGIKFPNGEWVSLMLKLRSKRNLYFPCLKMCPTGIRGEIYPSIRWAQVLTSEIQRI
COCAL   SNYFAYEKGDKVCKMNYCKHAGVRLPSGVWFEFVDQDVYAAAK----LPECPVGATISAPTQTSVDVSLILDVERI
IND     SNYFAYETGDKACKMQYCKHWGVRLPSGVWFEMADKDLFAAAR----FPECPEGSSISAPSQTSVDVSLIQDVERI
NJ      SNYFPYISTEGICKMPFCRKPGYKLKNDLWFQITDPDLDKTVRDLPHIKDCDLSSSIITPGEHATDISLISDVERI
ISFA    SKFHAHMKGDRVCKMKFCNKNGLRLGNGEWIEVGDEVMLDNSKLLSLFPDCLVGSVVKSTLLSEGVQTALWETDRL
PIRY    SAYHPNMPGSTVCIMDFCEQKGLRFTNGEWMGLNVEQSIREKKISAIFPNCVAGTEIRATLESEGARTLTWETQRM
SVCV    DKHHRVYRFGGSCRMKFCNKDGIKFTRGDWVEKTAGTLTNIYEN---IPECADGTLVSGHRPGLDLIDTVFNLENV


CHP     LDYSLCQNTWDKVERKEPLSPLDLSYLASKSPGKGLAYTVINGTLSFAHTRYVRMWIDGPVLKEPKGKRESPSGIS
COCAL   LDYSLCQETWSKIRSKQPVSPVDLSYLAPKNPGTGPAFTIINGTLKYFETRYIRIDIDNPIISKMVGKISG-SQTE
IND     LDYSLCQETWSKIRAGLPISPVDLSYLAPKNPGTGPVFTIINGTLKYFETRYIRVDIAAPILSRMVGMISG-TTTE
NJ      LDYSLCQNTWSKIEAGEPITPVDLSYLGPKNPGVGPVFTIINGSLHYFTSKYLRVELENPVIPRMEGRVAG-TRIV
ISFA    LDYSLCQNTWEKIDRKEPLSAVDLSYLAPRSPGKGMAYIVANGSLMSAPARYIRVWIDSPILKEIKGKKESASGID
PIRY    LDYSLCQNTWDKVSRKEPLSPLDLSYLSPRAPGKGMAYTVINGTLHSAHAKYIRTWIDYGEMKEIKGGRGEYSKAP
SVCV    VEYTLCEGTKRKINKQEKLTSVDLSYLAPRIGGFGSVFRVRNGTLERGSTTYIRIEVEGPVVDSLNGIDPR-TNAS
-

CHP     SDIWTQWFKYGDMEIGPNGLLKTAGGYKFPWHLIGMGIVDNELHELSEANPLDHPQLPHAQSIADDS---EEIFFG
COCAL   RELWTEWFPYEGVEIGPNGILKTPTGYKFPLFMIGHGMLDSDLHKTSQAEVFEHPHLAEAPKQLPEE---ETLFFG
IND     RVLWDDWAPYEDVEIGPNGVLRTSSGYKFPLYMIGHGMLDSDLHLSSKAQVFEHPHIQDAASQLPDD---ETLFFG
NJ      RQLWDQWFPFGEAEIGPNGVLKTKQGYKFPLHIIGTGEVDSDIKMERVVKHWEHPHIEEAAQTFLKKDDTGEVLYYG
ISFA    TVLWEQWLPFNGMELGPNGLIKTKSGYKFPLYLLGMGIVDQDLQELSSVNPVDHPHVPIAQAFVSEG---EEVFFG
PIRY    ELLWSQWFDFGPFKIGPNGLLHTGKTFKFPLYLIGAGIIDEDLHELDEAAPIDHPQMPDAKSVLPED---EEIFFG
SVCV    RVFWDDWELDGNIYQGFNGVYKGKDGKIHIPLNMIESGIIDDELQHAFQADIIPHPHYDDDEIREDD-----IFFD


CHP     DTGVSKNPVELVTGWFTSWKESLAAGSCPDLRCPPLFPGIVYYLQKAQME-------ERGER[SDSFE]MRIFKPNNM
COCAL   DTGISKNPVELIEGWFSSWKSTVVTFFFAIGVFILLYVVARIVIAVRYRYQGS----NNKRI[YNDIE]MSRFRK---
IND     DTGLSKNPIEFVEGWFSSWKSSIASFFFIIGLIIGLFLVLRVGIYLCIKLKHT----KKRQI[YTDIE]MNRLGK---
NJ      DTGVSKNPVELVEGWFSGWRSSIMGVLAVIIGFVILIFLIRLIGVLSSLFRPK----RRPIY[KSDVE]MAHFR----
ISFA    DTGVSKNPIELISGWFSDWKETAAALGFAAISVILIIGLMRLLPLLCRRRK------QKKVI[YKDVE]LNSFDPRQA
PIRY    DTGVSKNPIELIQGWFSNWRESVMAIVGIVLLIVVTFLAIKTVRVLNCLWRPRKKRIVRQEV[DVESR]LNHFEMRGF
SVCV    NTGENGNPVDAVVEWVSGWGTSLKFFGMTLVALILIFLLIRCCVACTYLMK------KSKRP[ATESH]EMRSLV
                                Transmembrane Domain


CHP     RARV--
COCAL   ------
IND     ----
NJ      ----
ISFA    FHR---
PIRY    PEYVKR
```

# FIGURE 3

TGGAAGGGCTAATTCACTCCCAACGAAGACAAGATATCCTTGATCTGTGG
ATCTACCACACACAAGGCTACTTCCCTGATTAGCAGAACTACACACCAGG
GCCAGGGATCAGATATCCACTGACCTTTGGATGGTGCTACAAGCTAGTAC
CAGTTGAGCCAGAGAAGTTAGAAGAAGCCAACAAAGGAGAGAACACCAGC
TTGTTACAACCTGTGAGCCTGCATGGGATGGATGACCCGGAGAGAGAAGT
GTTAGAGTGGAGGTTTGACAGCCGCCTAGCATTTCATCACGGTGGCCCGA
GAGCTGCATCCGGAGTACTTCAAGAACTGCTGATATCGAGCTTGCTACAA
GGGACTTTCCGCTGGGGGACTTTCCAGGGAGGCGTGGCCTGGGCGGGACT
GGGGAGTGGCGAGCCCTCAGATCCTGCATATAAGCAGCTGCTTTTTGCCT
GTACTGGGTCTCTCTGGTTAGACCAGATCTGAGCCTGGGAGCTCTCTGGC
TAACTAGGGAACCCACTGCTTAAGCCTCAATAAAGCTTGCCTTGAGTGCT
TCAAGTAGTGTGTGCCCGTCTGTTGTGTGACTCTGGTAACTAGAGATCCC
TCAGACCCTTTTAGTCAGTGTGGAAAATCTCTAGCAGTGGCGCCCGAACA
GGGACTTGAAAGCGAAAGGGAAACCAGAGGAGCTCTCTCGACGCAGGACT
CGGCTTGCTGAAGCGCGGAATTCCGCGCCACGGCAAGAGGCGAGGGGCGG
CGACTGGTGAGTACGCCAAAAATTTTGACTAGCGGAGGCTAGAAGGAGAG
AGATGGGTGCGAGAGCGTCAGTATTAAGCGGGGGAGAATTAGATCGCGAT
GGGAAAAAATTCGGTTAAGGCCAGGGGGAAAGAAAAAATATAAATTAAAA
CATATAGTATGGGCAAGCAGGGAGCTAGAACGATTCGCAGTTAATCCTGG
CCTGTTAGAAACATCAGAAGGCTGTAGACAAATACTGGGACAGCTACAAC
CATCCCTTCAGACAGGATCAGAAGAACTTAGATCATTATATAATACAGTA
GCAACCCTCTATTGTGTGCATCAAAGGATAGAGATAAAAGACACCAAGGA
AGCTTTAGACAAGATAGAGGAAGAGCAAAACAAAAGTAAGACCACCGCAC
AGCAAGCGGCCGCTGATCTTCAGACCTGGAGGAGGAGATATGAGGGACAA
TTGGAGAAGTGAATTATATAAATATAAAGTAGTAAAAATTGAACCATTAG
GAGTAGCACCCACCAAGGCAAAGAGAAGAGTGGTGCAGAGAGAAAAAAGA
GCAGTGGGAATAGGAGCTTTGTTCCTTGGGTTCTTGGGAGCAGCAGGAAG
CACTATGGGCGCAGCGTCAATGACGCTGACGGTACAGGCCAGACAATTAT
TGTCTGGTATAGTGCAGCAGCAGAACAATTTGCTGAGGGCTATTGAGGCG
CAACAGCATCTGTTGCAACTCACAGTCTGGGGCATCAAGCAGCTCCAGGC
AAGAATCCTGGCTGTGGAAAGATACCTAAAGGATCAACAGCTCCTGGGGA
TTTGGGGTTGCTCTGGAAAACTCATTTGCACCACTGCTGTGCCTTGGAAT
GCTAGTTGGAGTAATAAATCTCTGGAACAGATTTGGAATCACACGACCTG
GATGGAGTGGGACAGAGAAATTAACAATTACACAAGCTTAATACACTCCT
TAATTGAAGAATCGCAAAACCAGCAAGAAAGAATGAACAAGAATTATTG
GAATTAGATAAATGGGCAAGTTTGTGGAATTGGTTTAACATAACAAATTG
GCTGTGGTATATAAAATTATTCATAATGATAGTAGGAGGCTTGGTAGGTT
TAAGAATAGTTTTTGCTGTACTTTCTATAGTGAATAGAGTTAGGCAGGGA
TATTCACCATTATCGTTTCAGACCCACCTCCCAACCCCGAGGGGACCCGA
CAGGCCCGAAGGAATAGAAGAAGAAGGTGGAGAGAGAGACAGAGACAGAT
CCATTCGATTAGTGAACGGATCTCGACGGTATCGCCGAATTCACAAATGG
CAGTATTCATCCACAATTTTaaaagaaaagggggggATTGGGGGGTACAGT
GCAGGGGAAAGAATAGTAGACATAATAGCAACAGACATACAAACTAAAGA
ATTACAAAAACAAATTACaaaaattcaaaattttCGGGTTTATTACAGGG
ACAGCAGAGATCCACTTTGGGGCGATAAGCTTGGGAGTTCCGCGTTACAT
AACTTACGGTAAATGGCCCGCCTGGCTGACCGCCCAACGACCCCCGCCCA

## FIGURE 4A

```
TTGACGTCAATAATGACGTATGTTCCCATAGTAACGCCAATAGGGACTTT
CCATTGACGTCAATGGGTGGAGTATTTACGGTAAACTGCCCACTTGGCAG
TACATCAAGTGTATCATATGCCAAGTACGCCCCCTATTGACGTCAATGAC
GGTAAATGGCCCGCCTGGCATTATGCCCAGTACATGACCTTATGGGACTT
TCCTACTTGGCAGTACATCTACGTATTAGTCATCGCTATTACCATGGTGA
TGCGGTTTTGGCAGTACATCAATGGGCGTGGATAGCGGTTTGACTCACGG
GGATTTCCAAGTCTCCACCCCATTGACGTCAATGGGAGTTTGTTTTGGCA
CCAAAATCAACGGGACTTTCCAAAATGTCGTAACAACTCCGCCCCATTGA
CGCAAATGGGCGGTAGGCGTGTACGGTGGGAGGTCTATATAAGCAGAGCT
CGTTTAGTGAACCGTCAGATCGCCTGGAGACGCCATCCACGCTGTTTTGA
CCTCCATAGAAGACACCGACTCTAGAgga**CGTACGATGAGAGTTGTGTTT**
**GTCGTGCTATTGCTTTTGGTGGCCCCAGCTTACAGCTTCAACTGCCTTGG**
**AATGAGCAACAGAGACTTCTTGGAAGGAGTGTCTGGAGCAACATGGGTGG**
**ATTTGGTTCTCGAAGGCGACAGCTGCGTGACTATCATGTCTAAGGACAAG**
**CCTACCATCGATGTGAAGATGATGAATATGGAGGCGGTCAACCTGGCAGA**
**GGTCCGCAGTTATTGCTATTTGGCTACCGTCAGCGATCTCTCCACCAAAG**
**CTGCGTGCCCGACCATGGGAGAAGCTCACAATGACAAACGTGCTGACCCA**
**GCTTTTGTGTGCAGACAAGGAGTGGTGGACAGGGGCTGGGGCAACGGCTG**
**CGGATTATTTGGCAAAGGAAGCATTGACACATGCGCCAAATTTGCCTGCT**
**CTACCAAGGCAATAGGAAGAACCATCTTGAAAGAGAATATCAAGTACGAA**
**GTGGCCATTTTTGTCCATGGACCAACTACTGTGGAGTCGCACGGAAACTA**
**CTCCACACAGGTTGGAGCCACTCAGGCAGGGAGATTCAGCATCACTCCTG**
**CGGCGCCTTCATACACACTAAAGCTTGGAGAATATGGAGAGGTGACAGTG**
**GACTGTGAACCACGGTCAGGGATTGACACCAATGCATACTACGTGATGAC**
**TGTTGGAACAAAGACGTTCTTGGTCCATCGTGAGTGGTTCATGGACCTCA**
**ACCTCCCTTGGAGCAGTGCTGGAAGTACTGTGTGGAGGAACAGAGAGACG**
**TTAATGGAGTTTGAGGAACCACACGCCACGAAGCAGTCTGTGATAGCATT**
**GGGCTCACAAGAGGGAGCTCTGCATCAAGCTTTGGCTGGAGCCATTCCTG**
**TGGAATTTTCAAGCAACACTGTCAAGTTGACGTCGGGTCATTTGAAGTGT**
**AGAGTGAAGATGGAAAAATTGCAGTTGAAGGGAACAACCTATGGCGTCTG**
**TTCAAAGGCTTTCAAGTTTCTTGGGACTCCCGCAGACACAGGTCACGGCA**
**CTGTGGTGTTGGAATTGCAGTACACTGGCACGGATGGACCTTGCAAAGTT**
**CCTATCTCGTCAGTGGCTTCATTGAACGACCTAACGCCAGTGGGCAGATT**
**GGTCACTGTCAACCCTTTTGTTTCAGTGGCCACGGCCAACGCTAAGGTCC**
**TGATTGAATTGGAACCACCCTTTGGAGACTCATACATAGTGGTGGGCAGA**
**GGAGAACAACAGATCAATCACCATTGGCACAAGTCTGGAAGCAGCATTGG**
**CAAAGCCTTTACAACCACCCTCAAAGGAGCGCAGAGACTAGCCGCTCTAG**
**GAGACACAGCTTGGGACTTTGGATCAGTTGGAGGGGTGTTCACCTCAGTT**
**GGGAAGGCTG**tctaatgcgcgcGGTACCTTTAAGACCAATGACTTACAAG
GCAGCTGTAGATCTTAGCCACTTTTTAAAAGAAAAGGGGGGACTGGAAGG
GCTAATTCACTCCCAACGAAGACAAGatcgtcgagAGATGCTGCATATAA
GCAGCTGCTTTTTGCTTGTACTGGGTCTCTCTGGTTAGACCAGATCTGAG
CCTGGGAGCTCTCTGGCTAACTAGGGAACCCACTGCTTAAGCCTCAATAA
AGCTTGCCTTGAGTGCTTCAAGTAGTGTGTGCCCGTCTGTTGTGTGACTC
TGGTAACTAGAGATCCCTCAGACCCTTTTAGTCAGTGTGGAAAATCTCTA
GCAGT
```

**FIGURE 4B**

```
TGGAAGGGCTAATTCACTCCCAACGAAGACAAGATATCCTTGATCTGTGG
ATCTACCACACACAAGGCTACTTCCCTGATTAGCAGAACTACACACCAGG
GCCAGGGATCAGATATCCACTGACCTTTGGATGGTGCTACAAGCTAGTAC
CAGTTGAGCCAGAGAAGTTAGAAGAAGCCAACAAAGGAGAGAACACCAGC
TTGTTACAACCTGTGAGCCTGCATGGGATGGATGACCCGGAGAGAGAAGT
GTTAGAGTGGAGGTTTGACAGCCGCCTAGCATTTCATCACGGTGGCCCGA
GAGCTGCATCCGGAGTACTTCAAGAACTGCTGATATCGAGCTTGCTACAA
GGGACTTTCCGCTGGGGGACTTTCCAGGGAGGCGTGGCCTGGGCGGGACT
GGGGAGTGGCGAGCCCTCAGATCCTGCATATAAGCAGCTGCTTTTTGCCT
GTACTGGGTCTCTCTGGTTAGACCAGATCTGAGCCTGGGAGCTCTCTGGC
TAACTAGGGAACCCACTGCTTAAGCCTCAATAAAGCTTGCCTTGAGTGCT
TCAAGTAGTGTGTGCCCGTCTGTTGTGTGACTCTGGTAACTAGAGATCCC
TCAGACCCTTTTAGTCAGTGTGGAAAATCTCTAGCAGTGGCGCCCGAACA
GGGACTTGAAAGCGAAAGGGAAACCAGAGGAGCTCTCTCGACGCAGGACT
CGGCTTGCTGAAGCGCGCACGGCAAGAGGCGAGGGGCGGCGACTGGTGAG
TACGCCAAAAATTTTGACTAGCGGAGGCTAGAAGGAGAGAGATGGGTGCG
AGAGCGTCAGTATTAAGCGGGGGAGAATTAGATCGCGATGGGAAAAAATT
CGGTTAAGGCCAGGGGGAAAGAAAAAATATAAATTAAAACATATAGTATG
GGCAAGCAGGGAGCTAGAACGATTCGCAGTTAATCCTGGCCTGTTAGAAA
CATCAGAAGGCTGTAGACAAATACTGGGACAGCTACAACCATCCCTTCAG
ACAGGATCAGAAGAACTTAGATCATTATATAATACAGTAGCAACCCTCTA
TTGTGTGCATCAAAGGATAGAGATAAAAGACACCAAGGAAGCTTTAGACA
AGATAGAGGAAGAGCAAAACAAAAGTAAGACCACCGCACAGCAAGCGGCC
GCTGATCTTCAGACCTGGAGGAGGAGATATGAGGGACAATTGGAGAAGTG
AATTATATAAATATAAAGTAGTAAAAATTGAACCATTAGGAGTAGCACCC
ACCAAGGCAAAGAGAAGAGTGGTGCAGAGAGAAAAAAGAGCAGTGGGAAT
AGGAGCTTTGTTCCTTGGGTTCTTGGGAGCAGCAGGAAGCACTATGGGCG
CAGCGTCAATGACGCTGACGGTACAGGCCAGACAATTATTGTCTGGTATA
GTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGG
CTGTGGAAAGATACCTAAAGGATCAACAGCTCCTGGGGATTTGGGGTTGC
TCTGGAAAACTCATTTGCACCACTGCTGTGCCTTGGAATGCTAGTTGGAG
TAATAAATCTCTGGAACAGATTTGGAATCACACGACCTGGATGGAGTGGG
ACAGAGAAATTAACAATTACACAAGCTTAATACACTCCTTAATTGAAGAA
TCGCAAAACCAGCAAGAAAGAATGAACAAGAATTATTGGAATTAGATAA
ATGGGCAAGTTTGTGGAATTGGTTTAACATAACAAATTGGCTGTGGTATA
TAAAATTATTCATAATGATAGTAGGAGGCTTGGTAGGTTTAAGAATAGTT
TTTGCTGTACTTTCTATAGTGAATAGAGTTAGGCAGGGATATTCACCATT
ATCGTTTCAGACCCACCTCCCAACCCCGAGGGGACCCGACAGGCCCGAAG
GAATAGAAGAAGAAGGTGGAGAGAGAGACAGAGACAGATCCATTCGATTA
GTGAACGGATCTCGACGGTATCGCCGAATTCACAAATGGCAGTATTCATC
CACAATTTTaaaagaaaaggggggATTGGGGGGTACAGTGCAGGGGAAAG
AATAGTAGACATAATAGCAACAGACATACAAACTAAAGAATTACAAAAAC
AAATTACaaaaattcaaaattttCGGGTTTATTACAGGGACAGCAGAGAT
CCACTTTGGGGCGATAAGCTTGGGAGTTCCGCGTTACATAACTTACGGTA
AATGGCCCGCCTGGCTGACCGCCCAACGACCCCCGCCCATTGACGTCAAT
```

**FIGURE 5A**

AATGACGTATGTTCCCATAGTAACGCCAATAGGGACTTTCCATTGACGTC
AATGGGTGGAGTATTTACGGTAAACTGCCCACTTGGCAGTACATCAAGTG
TATCATATGCCAAGTACGCCCCCTATTGACGTCAATGACGGTAAATGGCC
CGCCTGGCATTATGCCCAGTACATGACCTTATGGGACTTTCCTACTTGGC
AGTACATCTACGTATTAGTCATCGCTATTACCATGGTGATGCGGTTTTGG
CAGTACATCAATGGGCGTGGATAGCGGTTTGACTCACGGGGATTTCCAAG
TCTCCACCCCATTGACGTCAATGGGAGTTTGTTTTGGCACCAAAATCAAC
GGGACTTTCCAAAATGTCGTAACAACTCCGCCCCATTGACGCAAATGGGC
GGTAGGCGTGTACGGTGGGAGGTCTATATAAGCAGAGCTCGTTTAGTGAA
CCGTCAGATCGCCTGGAGACGCCATCCACGCTGTTTTGACCTCCATAGAA
GACACCGACTCTAGAggatccccaccggtcgccaccatggtgagcaaggg
cgaggagctgttcaccggggtggtgcccatcctggtcgagctggacggcg
acgtaaacggccacaagttcagcgtgtccggcgagggcgagggcgatgcc
acctacggcaagctgaccctgaagttcatctgcaccaccggcaagctgcc
cgtgccctggcccaccctcgtgaccaccctgacctacggcgtgcagtgct
tcagccgctaccccgaccacatgaagcagcacgacttcttcaagtccgcc
atgcccgaaggctacgtccaggagcgcaccatcttcttcaaggacgacgg
caactacaagacccgcgccgaggtgaagttcgagggcgacaccctggtga
accgcatcgagctgaagggcatcgacttcaaggaggacggcaacatcctg
gggcacaagctggagtacaactacaacagccacaacgtctatatcatggc
cgacaagcagaagaacggcatcaaggtgaacttcaagatccgccacaaca
tcgaggacggcagcgtgcagctcgccgaccactaccagcagaacacccc
atcggcgacggccccgtgctgctgcccgacaaccactacctgagcaccca
gtccgccctgagcaaagaccccaacgagaagcgcgatcacatggtcctgc
tggagttcgtgaccgccgccgggatcactctcggcatggacgagctgtac
aagtaaagcggccggactctagctcgagACCTAGAAAAACATGGAGCAAT
CACAAGTAGCAATACAGCAGCTACCAATGCTGATTGTGCCTGGCTAGAAG
CACAAGAGGAGGAGGAGGTGGGTTTTCCAGTCACACCTCAGGTACCTTTA
AGACCAATGACTTACAAGGCAGCTGTAGATCTTAGCCACTTTTTAAAAGA
AAAGGGGGGACTGGAAGGGCTAATTCACTCCCAACGAAGACAAGatcgtc
gagAGATGCTGCATATAAGCAGCTGCTTTTGCTTGTACTGGGTCTCTCT
GGTTAGACCAGATCTGAGCCTGGGAGCTCTCTGGCTAACTAGGGAACCCA
CTGCTTAAGCCTCAATAAAGCTTGCCTTGAGTGCTTCAAGTAGTGTGTGC
CCGTCTGTTGTGTGACTCTGGTAACTAGAGATCCCTCAGACCCTTTTAGT
CAGTGTGGAAAATCTCTAGCAGT

**FIGURE 5B**

## Figure 6 : VSV-G Indiana gene, codon optimized

<u>6A - VSV-G Indiana (GenBank # M11048)</u>
protein sequence. Underline : transmembrane domain

```
MKCLLYLAFLFIGVNCKFTIVFPHNQKGNWKNVPSNYHYCPSSSDLNWHNDLVGTALQVKMPKSHKAIQ
ADGWMCHASKWVTTCDFRWYGPKYITHSIRSFTPSVEQCKESIEQTKQGTWLNPGFPPQSCGYATVTDA
EAAIVQVTPHHVLVDEYTGEWVDSQFINGKCSNYICPTVHNSTTWHSDYKVKGLCDSNLISMDITFFSE
DGELSSLGKKGTGFRSNYFAYETGDKACKMQYCKHWGVRLPSGVWFEMADKDLFAAARFPECPEGSSIS
APSQTSVDVSLIQDVERILDYSLCQETWSKIRAGLPISPVDLSYLAPKNPGTGPVFTIINGTLKYFETR
YIRVDIAAPILSRMVGMISGTTTERVLWDDWAPYEDVEIGPNGVLRTSSGYKFPLYMIGHGMLDSDLHL
SSKAQVFEHPHIQDAASQLPDDETLFFGDTGLSKNPIEFVEGWFSSWKS S̲I̲A̲S̲F̲F̲F̲I̲I̲G̲L̲I̲I̲G̲L̲F̲L̲V̲L̲R̲
V̲G̲I̲Y̲L̲C̲I̲K̲L̲K̲H̲T̲K̲K̲RQIYTDIEMNRLGK
```

## 6 B - Optimized nucleotidic sequence

```
        M   K   C   L   L   Y   L   A   F   L   F   I   G   V   N   C   K   F   T   I   V   F   P   H   N   Q   K   G   N   W   K   N   V   P ·
  1  ATGAAATGCC TGCTCTATCT GGCCTTCCTC TTTATCGGCG TGAACTGTAA GTTCACGATC GTGTTTCCCC ACAATCAGAA GGGAAACTGG AAGAACGTCC
     TACTTTACGG ACGAGATAGA CCGGAAGGAG AAATAGCCGC ACTTGACATT CAAGTGCTAG CACAAAGGGG TGTTAGTCTT CCCTTTGACC TTCTTGCAGG

       · S   N   Y   H   Y   C   P   S   S   S   D   L   N   W   H   N   D   L   I   G   T   A   I   Q   V   K   M   P   K   S   H   K   A ·
101  CGAGCAACTA CCACTACTGC CCTAGCTCAA GCGACCTGAA CTGGCACAAC GACCTGATCG GCACCGCTAT CCAGGTGAAG ATGCCAAAGA GCCACAAGGC
     GCTCGTTGAT GGTGATGACG GGATCGAGTT CGCTGGACTT GACCGTGTTG CTGGACTAGC CGTGGCGATA GGTCCACTTC TACGGTTTCT CGGTGTTCCG

       · I   Q   A   D   G   W   M   C   H   A   S   K   W   V   T   T   C   D   F   R   W   Y   G   P   K   Y   I   T   Q   S   I   R   S
201  CATCCAAGCC GACGGCTGGA TGTGTCACGC CAGCAAATGG GTGACGACGT GCGATTTTCG CTGGTATGGC CCCAAGTACA TCACCCAATC AATCCGCTCA
     GTAGGTTCGG CTGCCGACCT ACACAGTGCG GTCGTTTACC CACTGCTGCA CGCTAAAAGC GACCATACCG GGGTTCATGT AGTGGGTTAG TTAGGCGAGT

         F   T   P   S   V   E   Q   C   K   E   S   I   E   Q   T   K   Q   G   T   W   L   N   P   G   F   P   P   Q   S   C   G   Y   A   T ·
301  TTTACACCCA GCGTGGAGCA ATGTAAGGAG AGCATCGAGC AGACCAAGCA GGGGACCTGG CTCAACCCCG GCTTCCCACC GCAAAGCTGC GGATACGCCA
     AAATGTGGGT CGCACCTCGT TACATTCCTC TCGTAGCTCG TCTGGTTCGT CCCCTGGACC GAGTTGGGGC CGAAGGGTGG CGTTTCGACG CCTATGCGGT

       ·  V   T   D   A   E   A   V   I   V   Q   V   T   P   H   H   V   L   V   D   E   Y   T   G   E   W   V   D   S   Q   F   I   N   G ·
401  CCGTGACCGA CGCTGAGGCC GTCATCGTGC AGGTGACCCC GCACCACGTG CTGGTGGACG AGTACACCGG CGAGTGGGTG GATTCACAGT TTATCAACGG
     GGCACTGGCT GCGACTCCGG CAGTAGCACG TCCACTGGGG CGTGGTGCAC GACCACCTGC TCATGTGGCC GCTCACCCAC CTAAGTGTCA AATAGTTGCC

       · K   C   S   N   Y   I   C   P   T   V   H   N   S   T   T   W   H   S   D   Y   K   V   K   G   L   C   D   S   N   L   I   S   M
501  AAAGTGTAGC AATTACATCT GCCCCACCGT GCACAACAGC ACCACCTGGC ACTCAGACTA TAAGGTGAAG GGCCTCTGCG ACAGCAATCT GATCTCAATG
     TTTCACATCG TTAATGTAGA CGGGGTGGCA CGTGTTGTCG TGGTGGACCG TGAGTCTGAT ATTCCACTTC CCGGAGACGC TGTCGTTAGA CTAGAGTTAC

         D   I   T   F   F   S   E   D   G   E   L   S   S   L   G   K   E   G   T   G   F   R   S   N   Y   F   A   Y   E   T   G   G   K   A ·
601  GACATCACCT TCTTTAGCGA AGACGGCGAA CTCTCAAGCC TCGGGAAGGA AGGCACCGGG TTCCGCAGCA ATTACTTTGC TTACGAAACC GGCGGCAAGG
     CTGTAGTGGA AGAAATCGCT TCTGCCGCTT GAGAGTTCGG AGCCCTTCCT TCCGTGGCCC AAGGCGTCGT TAATGAAACG AATGCTTTGG CCGCCGTTCC

       · C   K   M   Q   Y   C   K   H   W   G   V   R   L   P   S   G   V   W   F   E   M   A   D   K   D   L   F   A   A   A   R   F   P ·
701  CCTGCAAGAT GCAATACTGC AAGCACTGGG GCGTGCGCCT GCCAAGCGGC GTGTGGTTTG AGATGGCTGA TAAGGACCTG TTCGCCGCTG CCCGCTTCCC
     GGACGTTCTA CGTTATGACG TTCGTGACCC CGCACGCGGA CGGTTCGCCG CACACCAAAC TCTACCGACT ATTCCTGGAC AAGCGGCGAC GGGCGAAGGG

       · E   C   P   E   G   S   S   I   S   A   P   S   Q   T   S   V   D   V   S   L   I   Q   D   V   E   R   I   L   D   Y   S   L   C
801  GGAATGCCCC GAGGGGAGCA GCATCAGCGC CCCCAGCCAG ACATCAGTGG ACGTGAGCCT GATCCAGGAT GTGGAACGCA TCCTGGACTA CAGCCTGTGT
     CCTTACGGGG CTCCCCTCGT CGTAGTCGCG GGGGTCGGTC TGTAGTCACC TGCACTCGGA CTAGGTCCTA CACCTTGCGT AGGACCTGAT GTCGGACACA

         Q   E   T   W   S   K   I   R   A   G   L   P   I   S   P   V   D   L   S   Y   L   A   P   K   N   P   G   T   G   P   A   F   T   I ·
901  CAGGAAACGT GGAGCAAGAT CCGCGCCGGA CTGCCTATCA GCCCCGTGGA TCTCAGCTAC CTGGCCCCAA AGAACCCAGG CACCGGACCC GCCTTTACAA
     GTCCTTTGCA CCTCGTTCTA GGCGCGGCCT GACGGATAGT CGGGGCACCT AGAGTCGATG GACCGGGGTT TCTTGGGTCC GTGGCCTGGG CGGAAATGTT

       ·  I   N   G   T   L   K   Y   F   E   T   R   Y   I   R   V   D   I   A   A   P   I   L   S   R   M   V   G   M   I   S   G   T   T ·
1001 TCATCAACGG CACCCTGAAG TACTTTGAAA CACGCTACAT CCGCGTCGAC ATCGCCGCTC CCATCCTCTC ACGCATGGTG GGCATGATCT CAGGGACGAC
     AGTAGTTGCC GTGGGACTTC ATGAAACTTT GTGCGATGTA GGCGCAGCTG TAGCGGCGAG GGTAGGAGAG TGCGTACCAC CCGTACTAGA GTCCCTGCTG

       · T   E   R   E   L   W   D   D   W   A   P   Y   E   D   V   E   I   G   P   N   G   V   L   R   T   S   S   G   Y   K   F   P   L
1101 CACGGAGCGC GAGCTGTGGG ATGACTGGGC CCCGTATGAA GATGTGGAGA TCGGACCTAA CGGCGTGCTG CGCACATCAA GCGGGTACAA GTTCCCGCTG
     GTGCCTCGCG CTCGACACCC TACTGACCCG GGGCATACTT CTACACCTCT AGCCTGGATT GCCGCACGAC GCGTGTAGTT CGCCCATGTT CAAGGGCGAC

         Y   M   I   G   H   G   M   L   D   S   D   L   H   L   S   S   K   A   Q   V   F   E   H   P   H   I   Q   D   A   A   S   Q   L   P ·
1201 TACATGATCG GCCACGGCAT GCTGGACAGC GACCTGCACC TCAGCTCAAA GGCCCAGGTC TTTGAGCACC CACACATCCA GGACGCTGCC AGCCAGCTCC
     ATGTACTAGC CGGTGCCGTA CGACCTGTCG CTGGACGTGG AGTCGAGTTT CCGGGTCCAG AAACTCGTGG GTGTGTAGGT CCTGCGACGG TCGGTCGAGG

       · D   D   E   S   L   F   F   G   D   T   G   L   S   K   N   P   I   E   L   V   E   G   W   F   S   S   W   K   S   S   I   A   S ·
1301 CCGACGACGA AAGCCTGTTC TTTGGAGATA CAGGGCTCAG CAAGAACCCC ATCGAGCTGG TCGAGGGCTG GTTCTCAAGC TGGAAGAGCA GCATCGCTTC
     GGCTGCTGCT TTCGGACAAG AAACCTCTAT GTCCCGAGTC GTTCTTGGGG TAGCTCGACC AGCTCCCGAC CAAGAGTTCG ACCTTCTCGT CGTAGCGAAG

       · F   F   F   I   I   G   L   I   I   G   L   F   L   V   L   R   V   G   I   H   L   C   I   K   L   K   H   T   K   K   R   Q   I
1401 ATTTTTTTTC ATCATCGGCC TCATCATCGG GCTGTTTCTG GTGCTGCGCG TCGGCATCCA CCTGTGCATC AAGCTGAAGC ACACCAAGAA GCGCCAGATC
     TAAAAAAAAG TAGTAGCCGG AGTAGTAGCC CGACAAAGAC CACGACGCGC AGCCGTAGGT GGACACGTAG TTCGACTTCG TGTGGTTCTT CGCGGTCTAG

         Y   T   D   I   E   M   N   R   L   G   K   *
1501 TATACCGACA TCGAGATGAA TCGCCTGGGG AAGTAA
     ATATGGCTGT AGCTCTACTT AGCGGACCCC TTCATT
```

Plasmid

Figure 6C

## Figure 7 : VSV-G NewJersey gene and construct

### A - VSV-G New Jersey protein (GenBank # AF170624). Underlined : transmembrane domain

MLSYLILAIVVSPILGKIEIVFPQHTTGDWKRVPHEYNYCPTSADKNSHGTQTGIPIELTMPKGLTTHQ
VDGFMCHSALWMTTCDFRWYGPKYITHSIHNEEPTDYQCLEAIKAYKDGVSFNPGFPPQSCGYGTVTDA
EAHIITVTPHSVKVDEYTGEWIDPHFIGGRCKGQICETVHNSTKWFTSSDGESVCSQLFTLVGGTFFSD
SEEITSMGLPETGIRSNYFPYISTEGICKMPFCRKPGYKLKNDLWFQITDPDLDKTVRDLPHIKDCDLS
SSIITPGEHATDISLISDVERILDYSLCQNTWSKIEAGEPITPVDLSYLGPKNPGVGPVFTIINGSLHY
FTSKYLRVELENPVIPRMEGRVAGTRIVRQLWDQWFPFGEAEIGPNGVLKTKQGYKFPLHIIGTGEVDS
DIKMERVVKHWEHPHIEAAQTFLKKDDTGEVLYYGDTGVSKNPVELVEGWFSGWR<u>SSIMGVLAVIIGFV
ILIFLI</u>RLIGVLSSLFRPKRRPIYKSDVEMAHFR

### B - Optimized nucleotidic sequence

```
       M   L   S   Y   L   I   F   A   L   A   V   S   P   I   L   G   K   I   E   I   V   F   P   Q   H   T   T   G   D   W   K   R   V   P ·
  1    ATGCTGTCAT ATCTGATCTT TGCCCTGGCT GTGAGCCCAA TCCTCGGCAAA GATCGAAATC GTGTTCCCAC AACACACCAC AGGGGACTGG AAGCGCGTGC
       TACGACAGTA TAGACTAGAA ACGGGACCGA CACTCGGGTT AGGAGCCTTT CTAGCTTTAG CACAAGGGTG TTGTGTGGTG TCCCCTGACC TTCGCGCACG

     · H   E   Y   N   Y   C   P   T   S   A   D   K   N   S   H   G   T   Q   T   G   I   P   V   E   L   T   M   P   K   G   L   T   T ·
 101   CCCACGAGTA CAACTACTGC CCGACCTCAG CCGACAAGAA TAGCCACGGC ACGCAGACCG GCATCCCTGT GGAGCTGACC ATGCCCAAGG GGCTCACAAC
       GGGTGCTCAT GTTGATGACG GGCTGGAGTC GGCTGTTCTT ATCGGTGCCG TGCGTCTGGC CGTAGGGACA CCTCGACTGG TACGGGTTCC CCGAGTGTTG

     · H   Q   V   E   G   F   M   C   H   S   A   L   W   M   T   T   C   D   F   R   W   Y   G   P   K   Y   I   T   H   S   I   H   N
 201   GCACCAAGTC GAAGGCTTCA TGTGCCACAG CGCTCTCTGG ATGACAACCT GCGATTTTCG CTGGTATGGC CCCAAGTACA TCACGCACAG CATCCACAAT
       CGTGGTTCAG CTTCCGAAGT ACACGGTGTC GCGAGAGACC TACTGTTGGA CGCTAAAAGC GACCATACCG GGGTTCATGT AGTGCGTGTC GTAGGTGTTA

       E   E   P   T   D   Y   Q   C   L   E   A   I   K   S   Y   K   D   G   V   S   F   N   P   G   F   P   P   Q   S   C   G   Y   G   T ·
 301   GAGGAACCAA CCGACTACCA GTGCCTCGAA GCCATCAAGT CATACAAGGA TGGGGTGAGC TTCAACCCCG GCTTCCCGCC CAATCATGT GGCTACGGCA
       CTCCTTGGTT GGCTGATGGT CACGGAGCTT CGGTAGTTCA GTATGTTCCT ACCCCACTCG AAGTTGGGGC CGAAGGGCGG GGTTAGTACA CCGATGCCGT

     · V   T   D   A   E   A   H   I   V   T   V   T   P   H   S   V   K   V   D   E   Y   T   G   E   W   I   D   P   H   F   I   G   G ·
 401   CCGTGACCGA CGCCGAGGCC CACATCGTGA CCGTGACACC CCACTCAGTC AAGGTGGACG AGTACACAGG CGAATGGATC GACCCCCACT TCATCGGGGG
       GGCACTGGCT GCGGCTCCGG GTGTAGCACT GGCACTGTGG GGTGAGTCAG TTCCACCTGC TCATGTGTCC GCTTACCTAG CTGGGGGTGA AGTAGCCCCC

     · R   C   K   G   Q   I   C   E   T   V   H   N   S   T   K   W   F   T   S   S   D   G   E   S   V   C   S   Q   L   F   T   L   V
 501   CCGCTGTAAG GGCCAAATCT GCGAGACCGT GCACAACAGC ACCAAGTGGT TTACGTCATC AGACGGCGAA AGCGTGTGCA GCCAACTGTT TACGCTCGTG
       GGCGACATTC CCGGTTTAGA CGCTCTGGCA CGTGTTGTCG TGGTTCACCA AATGCAGTAG TCTGCCGCTT TCGCACACGT CGGTTGACAA ATGCGAGCAC

       G   G   I   F   F   S   D   S   E   E   I   T   S   M   G   L   P   E   T   G   I   R   S   N   Y   F   P   Y   I   S   T   E   G   I ·
 601   GGCGGCATCT TCTTTAGCGA CAGCGAGGAG ATCACCAGCA TGGGCCTCCC GGAGACAGGA ATCCGCAGCA ACTACTTTCC GTACATCAGC TGGCTCCCTT
       CCGCCGTAGA AGAAATCGCT GTCGCTCCTC TAGTGGTCGT ACCCGGAGGG CCTCTGTCCT TAGGCGTCGT TGATGAAAGG CATGTAGTCG TGGCTCCCTT

     · C   K   M   P   F   C   R   K   Q   G   Y   K   L   K   N   D   L   W   F   Q   I   M   D   P   D   L   D   K   T   V   R   D   L ·
 701   TCTGTAAGAT GCCTTTTTGC CGCAAGCAGG GATATAAGCT GAAGAATGAC CTGTGGTTCC AGATCATGGA CCCGGACCTG GACAAGACCG TCCGCGATCT
       AGACATTCTA CGGAAAAACG GCGTTCGTCC CTATATTCGA CTTCTTACTG GACACCAAGG TCTAGTACCT GGGCCTGGAC CTGTTCTGGC AGGCGCTAGA

     · P   H   I   K   D   C   D   L   S   S   S   I   I   T   P   G   E   H   A   T   D   I   S   L   I   S   D   V   E   R   I   L   D
 801   GCCCCACATC AAGGACTGTG ATCTGTCATC AAGCATCACC ACCCCCGGAG AACACGCCAC GGACATCAGC CTCATCAGCG ATGTGGAGCG CATCCTCGAC
       CGGGGGTGTAG TTCCTGACAC TAGACAGTAG TTCGTAGTAG TGGGGGCCTC TTGTGCGGTG CCTGTAGTCG GAGTAGTCGC TACACCTCGC GTAGGAGCTG

       Y   A   L   C   Q   N   T   W   S   K   I   E   S   G   E   P   I   T   P   V   D   L   S   Y   L   G   P   K   N   P   G   V   G   P ·
 901   TACGCTCTCT GCCAGAACAC ATGGAGCAAG ATCGAAAGCG GCGAACCCAT CACCCCAGTG GACCTGAGCT ATCTCGGCCC AAAGAACCCC GGCGTGGGGC
       ATGCGAGAGA CGGTCTTGTG TACCTCGTTC TAGCTTTCGC CGCTTGGGTA GTGGGGTCAC CTGGACTCGA TAGAGCCGGG TTTCTTGGGG CCGCACCCCG

     · V   F   T   I   I   N   G   S   L   H   Y   F   T   S   K   Y   L   R   V   E   L   E   S   P   V   I   P   R   M   E   G   K   V ·
1001   CCGTGTTCAC CATCATCAAC GGGAGCCTGC ACTACTTTAC AAGCAAGTAT CTGCGCGTGG AGCTCGAAAG CCCAGTCATC CCCCGACATG AGGGGAAGGT
       GGCACAAGTG GTAGTAGTTG CCCTCGGACG TGATGAAATG TTCGTTCATA GACGCGCACC TCGAGCTTTC GGGTCAGTAG GGGGCGTACC TCCCCTTCCA

     · A   G   T   R   I   V   R   Q   L   W   D   Q   W   F   P   F   G   E   V   E   I   G   P   N   G   V   L   K   T   K   Q   G   Y
1101   GGCCGGGACC CGCATCGTGC GCCAGCTGTG GGACCAGTGG TTCCCTTTTG GCGAGGTGGA AATCGGCCCC AACGGCGTGC TGAAGACCAA GCAAGGATAT
       CCGGCCCTGG GCGTAGCACG CGGTCGACAC CCTGGTCACC AAGGGAAAAC CGCTCCACCT TTAGCGGGG TTGCCGCACG ACTTCTGGTT CGTTCCTATA

       K · F   P   L   H   I   I   G   T   G   E   V   D   S   D   I   K   M   E   R   V   V   K   H   W   E   H   P   H   I   E   A   A   Q ·
1201   AAGTTCCCGC TGCACATCAT CGGGACGGGC GAAGTGGACA GCGATATCAA GATGGAGCGC GTGGTCAAGC ACTGGGAGCA CCCACACATC GAGGCTGCTC
       TTCAAGGGCG ACGTGTAGTA GCCCTGCCCG CTTCACCTGT CGCTATAGTT CTACCTCGCG CACCAGTTCG TGACCCTCGT GGGTGTGTAG CTCCGACGAG

     · T   F   L   K   K   D   D   T   G   E   V   L   Y   Y   G   D   T   G   V   S   K   N   P   V   E   L   V   E   G   W   F   S   G ·
1301   AGACCTTTCT CAAGAAGGAC GATACCGGCG AAGTCCTGTA TTACGGGGAT ACGGGAGTGA GCAAGAACCC TGTGGAGCTG GTGGAAGGCT GGTTCAGCGG
       TCTGGAAAGA GTTCTTCCTG CTATGGCCGC TTCAGGACAT AATGCCCCTA TGCCCTCACT CGTTCTTGGG ACACCTCGAC CACCTTCCGA CCAAGTCGCC

     · W   R   S   S   L   M   G   V   L   A   V   I   I   G   F   V   I   L   M   F   L   I   K   L   I   G   V   L   S   S   L   F   R
1401   ATGGCGCTCA AGCCTGATGG GCGTGCTGCA CGTCATCATC GGATTTGTGA TCCTGATGTT CCTCATCAAG CTGATCGGCG TGCTGTCAAG CCTGTTCCGC
       TACCGCGAGT TCGGACTACC CGCACGACCG GCAGTAGTAG CCTAAACACT AGGACTACAA GGACTAGTTC GACTAGCCGC ACGACAGTTC GGACAAGGCG

       P   K   R   R   P   I   Y   K   S   D   V   E   M   A   H   F   R   *
1501   CCTAAGCGCC GCCCAATCTA CAAGAGCGAC GTCGAGATGG CCCACTTTCG CTAA
       GGATTCGCGG CGGGTTAGAT GTTCTCGCTG CAGCTCTACC GGGTGAAAGC GATT
```

Plasmid

EcoRI (1)

NotI (28)

XhoI (34)

**WPRE/ΔEATG**

XhoI (644)

XbaI (650)

**BGH pA**

**VSV/G NJ CO**

**pThV-VSV.G (NJ-CO)**

5162 bp

**KanR**

NcoI (1644)

BamHI (3591)

KpnI (3583)

HindIII (3573)

NheI (3557)

**CMV**

NcoI (3272)

NdeI (3146)

**pUC ORI**

**Figure 7C**

## Figure 8 : VSV-G Chandipura gene and construct

### A - VSV-G Chandipura protein (GenBank # J04350) Underlined : transmembrane domain

MTSSVTISVVLLISFITPLYSYLSIAFPENTKLDWKPVTKNTRYCPMGGEWFLEPGLQEESFLSSTPIG
ATPSKSDGFLCHAAKWVTTCDFRWYGPKYITHSIHNIKPTRSDCDTALASYKSGTLVSLGFPPESCGYA
SVTDSEFLVIMITPHHVGVDDYRGHWVDPLFVGGECDQSYCDTIHNSSVWIPADQTKKNICGQSFTPLT
VTVAYDKTKEIAAGGIVFKSKYHSHMEGARTCRLSYCGRNGIKFPNGEWVSLMLKLRSKRNLYFPCLKM
CPTGIRGEIYPSIRWAQVLTSEIQRILDYSLCQNTWDKVERKEPLSPLDLSYLASKSPGKGLAYTVING
TLSFAHTRYVRMWIDGPVLKEPKGKRESPSGISSDIWTQWFKYGDMEIGPNGLLKTAGGYKFPWHLIGM
GIVDNELHELSEANPLDHPQLPHAQSIADDSEEIFFGDTGVSKNPVELVTGWFTSWKESLAAGSCPDLR
CPPLFPGIVYYLQKAQMEERGERSDSFEMRIFKPNNMRARV

### B - Optimized nucleotidic sequence

```
        BamHI
                    M   T   S   S   V   T   I   S   V   V   L   L   I .S   F   I   T   P   L   Y   S   Y   L   S   I ·
   GGCGCGCCGG ATCCTGATCA GCCACCATGA CCAGCAGCGT GACCATCAGC GTGGTGCTGC TGATCAGCTT CATCACCCCC CTGTACAGCT ACCTGAGCAT
   CCGCGCGGCC TAGGACTAGT CGGTGGTACT GGTCGTCGCA CTGGTAGTCG CACCACGACG ACTAGTCGAA GTAGTGGGGG GACATGTCGA TGGACTCGTA

    · A   F   P   E   N   T   K   L   D   W   K   P   V   T   K   N   T   R   Y   C   P   M   G   E   W   F   L   E   P   G   L   Q
   TGCCTTCCCC GAGAACACCA AGCTGGACTG GAAGCCCGTG ACCAAGAACA CCCGGTACTG CCCCATGGGC GGCGAGTGGT TTCTGGAACC CGGCCTGCAG
   ACGGAAGGGG CTCTTGTGGT CGACCTGAC CTTCGGGCAC TGGTTCTTGT GGGCCATGAC GGGGTACCCG CCGCTCACCA AAGACCTTGG GCCGGACGTC

    E   E   S   F   L   S   S   T   P   I   G   A   T   P   S   K   S   D   G   F   L   C   H   A   A   K   W   V   T   T   C   D   F   R ·
   GAAGAGAGCT TCCTGAGCAG CACCCCCATC GGCGCCACCC CCAGCAAGAG CGACGGCTTC CTGTGCCACG CCGCCAAGTG GGTGACCACC TGCGACTTCC
   CTTCTCTCGA AGGACTCGTC GTGGGGGTAG CCGCGGTGGG GGTCGTTCTC GCTGCCGAAG GACACGGTGC GGCGGTTCAC CCACTGGTGG ACGCTGAAGG

    · W   Y   G   P   K   Y   I   T   H   S   I   H   N   I   K   P   T   R   S   D   C   D   T   A   L   A   S   Y   K   S   G   T   L ·
   GGTGGTACGG CCCCAAGTAC ATCACCCACA GCATCCACAA CATCAAGCCC ACCAGAAGCG ACTGCGACAC AGCCCTGGCC TCTTACAAGA GCGGCACCCT
   CCACCATGCC GGGGTTCATG TAGTGGGTGT CGTAGGTGTT GTAGTTCGGG TGGTCTTCGC TGACGCTGTG TCGGGACCGG AGAATGTTCT CGCCGTGGGA

    · V   S   L   G   F   P   P   E   S   C   G   Y   A   S   V   T   D   S   E   F   L   V   I   M   I   T   P   H   H   V   G   V   D
   GGTGTCCCTG GGCTTCCCTC CCGAGAGCTG CGGCTACGCC AGCGTGACCG ACAGCGAGTT CCTGGTGATT ATGATTACCC CCCACCACGT GGGCGTGGAC
   CCACAGGGAC CCGAAGGGAG GGCTCTCGAC GCCGATGCGG TCGCACTGGC TGTCGCTCAA GGACCACTAA TACTAATGGG GGGTGGTGCA CCCGCACCTG

    D   Y   R   G   H   W   V   D   P   L   F   V   G   G   E   C   D   Q   S   Y   C   D   T   I   H   N   S   S   V   W   I   P   A   D ·
   GACTACCGGG CCACTGGGT GGACCCTCTG TTCGTGGGAG GGGAATGCGA CCAGAGCTAC TGCGATACCA TCCACAACTC CAGCGTGTGG ATTCCCGCCG
   CTGATGGCCC CGGTGACCCA CCTGGGAGAC AAGCACCCTC CCCTTACGCT GGTCTCGATG ACGCTATGGT AGGTGTTGAG GTCGCACACC TAAGGGCGGC

    · Q   T   K   K   N   I   C   G   Q   S   F   T   P   L   T   V   T   V   A   Y   D   K   T   K   E   I   A   A   G   G   I   V   F ·
   ACCAGACCAA GAAGAACATC TGCGGCCAGA GCTTCACCCC TCTGACCGTG ACCGTGGCCT ACGACAAGAC CAAAGAGATT GCCGCCGGAG GGATCGTGTT
   TGGTCTGGTT CTTCTTGTAG ACGCCGGTCT CGAAGTGGGG AGACTGGCAC TGGCACCGGA TGCTGTTCTG GTTTCTCTAA CGGCGGCCTC CCTAGCACAA

    · K   S   K   Y   H   S   H   M   E   G   A   R   T   C   R   L   S   Y   C   G   R   N   G   I   K   F   P   N   G   E   W   V   S
   CAAGAGCAAG TACCACAGCC ACATGGAAGG CGCCAGGACC TGCAGACTGT CCTACTGCGG CCGGAACGGC ATCAAGTTCC CCAACGGCGA GTGGGTGTCC
   GTTCTCGTTC ATGGTGTCGG TGTACCTTCC GCGGTCCTGG ACGTCTGACA GGATGACGCC GGCCTTGCCG TAGTTCAAGG GGTTGCCGCT CACCCACAGG

    L   M   L   K   L   R   S   K   R   N   L   Y   F   P   C   L   K   M   C   P   T   G   I   R   G   E   I   Y   P   S   I   R   W   A ·
   CTGATGCTGA AGCTGCGGAG CAAGCGGAAC CTGTACTTCC CCTGCCTGAA GATGTGCCCC ACCGGCATCC GGGGCGAGAT CTACCCCAGC ATCAGATGGG
   GACTACGACT TCGACGCCTC GTTCGCCTTG GACATGAAGG GGACGGACTT CTACACGGGG TGGCCGTAGG CCCCGCTCTA GATGGGGTCG TAGTCTACCC

    · Q   V   L   T   S   E   I   Q   R   I   L   D   Y   S   L   C   Q   N   T   W   D   K   V   E   R   K   E   P   L   S   P   L   D ·
   CCCAGGTGCT GACCAGCGAG ATCCAGAGAA TCCTGGACTA CAGCCTGTGC CAGAACACCT GGGACAAGGT GGAGCGGAAA GAGCCCCTGA GCCCCCTGGA
   GGGTCCACGA CTGGTCGCTC TAGGTCTCTT AGGACCTGAT GTCGGACACG GTCTTGTGGA CCCTGTTCCA CCTCGCCTTT CTCGGGGACT CGGGGGACCT

    · L   S   Y   L   A   S   K   S   P   G   K   G   L   A   Y   T   V   I   N   G   T   L   S   F   A   H   T   R   Y   V   R   M   W
   CCTGAGCTAC CTGGCCAGCA AGTCCCCCGG CAAGGGCCTG GCCTACACCG TGATCAACGG CACCCTGAGC TTCGCCCACA CCAGATACGT GCGGATGTGG
   GGACTCGATG GACCGGTCGT TCAGGGGGCC GTTCCCGGAC CGGATGTGGC ACTAGTTGCC GTGGGACTCG AAGCGGGTGT GGTCTATGCA CGCCTACACC

    I   D   G   P   V   L   K   E   P   K   G   K   R   E   S   P   S   G   I   S   S   D   I   W   T   Q   W   F   K   Y   G   D   M   E ·
   ATCGACGGCC CCGTGCTGAA AGAGCCCAAG GGCAAGAGAG AGAGCCCCAG CGGCATCAGC AGCGACATCT GGACCCAGTG GTTCAAGTAC GGCGACATGG
   TAGCTGCCGG GGCACGACTT TCTCGGGTTC CCGTTCTCTC TCTCGGGGTC GCCGTAGTCG TCGCTGTAGA CCTGGGTCAC CAAGTTCATG CCGCTGTACC

    · I   G   P   N   G   L   L   K   T   A   G   G   Y   K   F   P   W   H   L   I   G   M   G   I   V   D   N   E   L   H   E   L   S ·
   AAATCGGCCC CAACGGCCTG CTGAAAACAG CCGGCGGATA CAAGTTTCCT TGGCACCTGA TCGGCATGGG CATCGTGGAC AACGAGCTGC ACGAGCTGTC
   TTTAGCCGGG GTTGCCGGAC GACTTTTGTC GGCCGCCTAT GTTCAAAGGA ACCGTGGACT AGCCGTACCC GTAGCACCTG TTGCTCGACG TGCTCGACAG

    · E   A   N   P   L   D   H   P   Q   L   P   H   A   Q   S   I   A   D   D   S   E   E   I   F   F   G   D   T   G   V   S   K   N
   CGAGGCCAAC CCCCTGGATC ACCCCCAGCT GCCCCACGCC CAGAGCATTG CCGACGACAG CGAGGAAATC TTCTTCGGCG ACACCGGCGT GAGCAAGAAC
   GCTCCGGTTG GGGGACCTAG TGGGGGTCGA CGGGGTGCGG GTCTCGTAAC GGCTGCTGTC GCTCCTTTAG AAGAAGCCGC TGTGGCCGCA CTCGTTCTTG

    P   V   E   L   V   T   G   W   F   T   S   W   K   E   S   L   A   A   G   S   C   P   D   L   R   C   P   P   L   F   P   G   I   V ·
   CCCGTGGAAC TGGTGACAGG CTGGTTCACC AGCTGGAAAG AGAGCCTGGC CGCCGGATCT TGCCCCGACC TGCGGTGCCC CCCTCTGTTC CCCGGCATCG
   GGGCACCTTG ACCACTGTCC GACCAAGTGG TCGACCTTTC TCTCGGACCG GCGGCCTAGA ACGGGGCTGG ACGCCACGGG GGGAGACAAG GGGCCGTAGC

    · Y   Y   L   Q   K   A   Q   M   E   E   R   G   E   R   S   D   S   F   E   M   R   I   F   K   P   N   N   M   R   A   R   V   * ·
   TGTACTACCT GCAGAAAGCC CAGATGGAAG AGCGGGGCGA GCGGAGCGAC AGCTTCGAGA TGCGGATCTT CAAGCCCAAC AACATGCGGG CCAGAGTGTG
   ACATGATGGA CGTCTTTCGG GTCTACCTTC TCGCCCCGCT CGCCTCGCTG TCGAAGCTCT ACGCCTAGAA GTTCGGGTTG TTGTACGCCC GGTCTCACAC
              EcoRI
              ~~~~~~~
    · *
   ATGAGAATTC TTAATTAA
   TACTCTTAAG AATTAATT
```

Plasmid

*Eco*RI (1)
*Not*I (28)
*Xho*I (34)
**WPRE/EATG**
*Xho*I (644)
*Xba*I (650)
**BGH pA**

*Bgl*II (4459)
**VSV.G Chandipura CO**
*Bst*EII (3863)
*Nco*I (3745)
*Bam*HI (3591)
*Kpn*I (3583)
*Hind*III (3573)
*Nhe*I (3557)
**CMV**
*Nco*I (3272)
*Nde*I (3146)

**pThV-VSV.G (CHANDI-CO)**
5186 bp

**KanR**
*Nco*I (1644)

**pUC ORI**

Figure 8C

## Figure 9 : VSV-G Cocal gene and construct

### A - VSV-G Cocal (GenBank # AF045556) Underlined : transmembrane domain

MNFLLLTFIVLPLCSHAKFSIVFPQSQKGNWKNVPSSYHYCPSSSDQNWHNDLLGITMKVKMPKTHKAI
QADGWMCHAAKWITTCDFRWYGPKYITHSIHSIQPTSEQCKESIKQTKQGTWMSPGFPPQNCGYATVTD
SVAVVVQATPHHVLVDEYTGEWIDSQFPNGKCETEECETVHNSTVWYSDYKVTGLCDATLVDTEITFFS
EDGKKESIGKPNTGYRSNYFAYEKGDKVCKMNYCKHAGVRLPSGVWFEFVDQDVYAAAKLPECPVGATI
SAPTQTSVDVSLILDVERILDYSLCQETWSKIRSKQPVSPVDLSYLAPKNPGTPAFTIINGTLKYFET
RYIRIDIDNPIISKMVGKISGSQTERELWTEWFPYEGVEIGPNGILKTPTGYKFPLFMIGHGMLDSDLH
KTSQAEVFEHPHLAEAPKQLPEEETLFFGDTGISKNPVELIEGWFSSWK<u>STVVTFFFAIGVFILLYVVA</u>
RIVIAVRYRYQGSNNKRIYNDIEMSRFRK

### B - Optimized nucleotidic sequence

```
                    BamHI
                                M  N   F  L  L    L  T  F   I  V  L  P   L C S   H  A  K   F  S  I  V   F  P  Q ·
     GGCGCGCCGG ATCCTGATCA GCCACCATGA ACTTTCTGCT GCTGACATTC ATCGTGCTGC CTCTGTGCAG CCACGCCAAG TTCAGCATCG TGTTCCCCCA
     CCGCGCGGCC TAGGACTAGT CGGTGGTACT TGAAAGACGA CGACTGTAAG TAGCACGACG GAGACACGTC GGTGCGGTTC AAGTCGTAGC ACAAGGGGGT

              · S  Q  K   G  N  W  K   N  V  P    S  S  Y   H  Y  C   P  S  S  S    D  Q  N   W  H  N  D    L  L  G   I  T  M
     GAGCCAGAAG GGCAACTGGA AGAACGTGCC CAGCAGCTAC CACTACTGCC CCAGCAGCAG CGACCAGAAC TGGCACAACG ACCTGCTGGG CATCACCATG
     CTCGGTCTTC CCGTTGACCT TCTTGCACGG GTCGTCGATG GTGATGACGG GGTCGTCGTC GCTGGTCTTG ACCGTGTTGC TGGACGACCC GTAGTGGTAC

              K  V  K  M   P  K  T   H  K  A   I  Q  A  D    G  W  M    C  H  A    A  K  W   I  T  T  C   D  F  R    W  Y  G  P ·
     AAGGTGAAAA TGCCCAAGAC CCACAAGGCC ATTCAGGCTG ACGGCTGGAT GTGCCACGCC GCCAAGTGGA TCACCACCTG CGACTTCCGG TGGTACGGCC
     TTCCACTTTT ACGGGTTCTG GGTGTTCCGG TAAGTCCGAC TGCCGACCTA CACGGTGCGG CGGTTCACCT AGTGGTGGAC GCTGAAGGCC ACCATGCCGG

              · K  Y  I   T  H  S   I  H  S  I    Q  P  T   S  E  Q    C  K  E  S    I  K  Q   T  K  Q    G  T  W  M   S  P  G ·
     CCAAGTACAT CACCCACAGC ATCCACTCCA TCCAGCCCAC CTCCGAGCAG TGCAAAGAGA GCATCAAGCA GACCAAGCAG GGCACCTGGA TGAGCCCCGG
     GGTTCATGTA GTGGGTGTCG TAGGTGAGGT AGGTCGGGTG GAGGCTCGTC ACGTTTCTCT CGTAGTTCGT CTGGTTCGTC CCGTGGACCT ACTCGGGGCC

              · F  P  P   Q  N  C  G   Y  A  T    V  T  D   S  V  A  V    V  V  Q   A  T  P    H  H  V  L   V  D  E    Y  T  G
     CTTCCCACCC CAGAACTGCG GCTACGCCAC CGTGACCGAC AGCGTGGCCG TGGTGGTGCA GGCCACCCCC CACCACGTGC TGGTCGACGA GTACACCGGC
     GAAGGGTGGG GTCTTGACGC CGATGCGGTG GCACTGGCTG TCGCACCGGC ACCACCACGT CCGGTGGGGG GTGGTGCACG ACCAGCTGCT CATGTGGCCG

              E  W  I  D   S  Q  F   P  N  G   K  C  E  T    E  E  C    E  T  V   H  N  S  T    V  W  Y   S  D  Y    K  V  T  G ·
     GAGTGGATCG ACAGCCAGTT CCCCAACGGC AAGTGCGAGA CAGAGGAATG CGAGACAGTG CACAACAGCA CCGTGTGGTA CAGCGACTAC AAGGTGACCG
     CTCACCTAGC TGTCGGTCAA GGGGTTGCCG TTCACGCTCT GTCTCCTTAC GCTCTGTCAC GTGTTGTCGT GGCACACCAT GTCGCTGATG TTCCACTGGC

              · L  C  D   A  T  L   V  D  T  E    I  T  F   F  S  E    D  G  K  K    E  S  I   G  K  P    N  T  G  Y   R  S  N ·
     GCCTGTGCGA CGCCACCCTG GTGGACACCG AGATCACCTT TTTCAGCGAG GACGGCAAGA AAGAGTCCAT CGGCAAGCCC AACACCGGCT ACAGAAGCAA
     CGGACACGCT GCGGTGGGAC CACCTGTGGC TCTAGTGGAA AAAGTCGCTC CTGCCGTTCT TTCTCAGGTA GCCGTTCGGG TTGTGGCCGA TGTCTTCGTT

              · Y  F  A   Y  E  K  G   D  K  V    C  K  M   N  Y  C  K    H  A  G   V  R  L    P  S  G  V   W  F  E    F  V  D
     CTACTTCGCC TACGAGAAGG GCGACAAAGT GTGCAAGATG AACTACTGCA AGCATGCCGG AGTGAGGCTG CCTAGCGGCG TGTGGTTCGA GTTCGTGGAC
     GATGAAGCGG ATGCTCTTCC CGCTGTTTCA CACGTTCTAC TTGATGACGT TCGTACGGCC TCACTCCGAC GGATCGCCGC ACACCAAGCT CAAGCACCTG

              Q  D  V  Y   A  A  A   K  L  P   E  C  P  V    G  A  T    I  S  A   P  T  Q  T    S  V  D   V  S  L    I  L  D  V ·
     CAGGACGTGT ACGCCGCCGC CAAGCTGCCC GAGTGCCCCG TGGGCGCCAC CATCAGCGCC CCCACCCAGA CCAGCGTGGA CGTGAGCCTG ATCCTGGACG
     GTCCTGCACA TGCGGCGGCG GTTCGACGGG CTCACGGGGC ACCCGCGGTG GTAGTCGCGG GGGTGGGTCT GGTCGCACCT GCACTCGGAC TAGGACCTGC

              · E  R  I   L  D  Y   S  L  C  Q    E  T  W   S  K  I    R  S  K  Q    P  V  S   P  V  D    L  S  Y  L   A  P  K ·
     TGGAGAGAAT CCTGGACTAC TCTCTGTGTC AGGAAACCTG GTCCAAGATC AGATCCAAGC AGCCCGTGAG CCCTGTGGAC CTGAGCTACC TGGCCCCTAA
     ACCTCTCTTA GGACCTGATG AGAGACACAG TCCTTTGGAC CAGGTTCTAG TCTAGGTTCG TCGGGCACTC GGGACACCTG GACTCGATGG ACCGGGGATT

              · N  P  G   T  G  P  A   F  T  I    I  N  G   T  L  K  Y    F  E  T   R  Y  I    R  I  D  I   D  N  P    I  I  S
     GAACCCCGGC ACCGGCCCTG CCTTCACCAT CATCAACGGC ACCCTGAAGT ACTTCGAGAC ACGGTACATC CGACAACCC CATCATCAGC
     CTTGGGGCCG TGGCCGGGAC GGAAGTGGTA GTAGTTGCCG TGGGACTTCA TGAAGCTCTG TGCCATGTAG GCCTAGTGT GTAGTAGTCG

              K  M  V  G   K  I  S   G  S  Q   T  E  R  E    L  W  T    E  W  F   P  Y  E  G    V  E  I   G  P  N    G  I  L  K ·
     AAGATGGTGG GCAAGATCAG CGGCAGCCAG ACCGAGCGGG AGCTGTGGAC CGAGTGGTTC CCCTACGAGG GCGTGGAGAT CGGCCCCAAT GGCATCCTGA
     TTCTACCACC CGTTCTAGTC GCCGTCGGTC TGGCTCGCCC TCGACACCTG GCTCACCAAG GGGATGCTCC CGCACCTCTA GCCGGGGTTA CCGTAGGACT

              · T  P  T   G  Y  K   F  P  L  F    M  I  G   H  G  M    L  D  S  D    L  H  K   T  S  Q    A  E  V  F   E  H  P ·
     AAACCCCTAC CGGCTACAAG TTCCCCCTGT TCATGATCGG CCACGGCATG CTGGACAGCG ACCTGCACAA GACCTCCCAG GCCGAGGTGT TCGAGCACCC
     TTTGGGGATG GCCGATGTTC AAGGGGGACA AGTACTAGCC GGTGCCGTAC GACCTGTCGC TGGACGTGTT CTGGAGGGTC CGGCTCCACA AGCTCGTGGG

              · H  L  A   E  A  P  K   Q  L  P    E  E  E   T  L  F  F    G  D  T   G  I  S    K  N  P  V   E  L  I    E  G  W
     CCACCTGGCC GAGGCCCCCA AGCAGCTGCC CGAAGAGGAA ACCCTGTTCT TCGGCGACAC CGGCATCTCC AAGAACCCTG TGGAGCTGAT CGAGGGCTGG
     GGTGGACCGG CTCCGGGGGT TCGTCGACGG GCTTCTCCTT TGGGACAAGA AGCCGCTGTG GCCGTAGAGG TTCTTGGGAC ACCTCGACTA GCTCCCGACC

              F  S  S  W   K  S  T   V  V  T   F  F  F  A    I  G  V   F  I  L    L  Y  V  V   A  R  I    V  I  A    V  R  Y  R ·
     TTCAGCAGCT GGAAGAGCAC CGTGGTGACC TTTTTCTTCG CCATCGGCGT GTTCATCCTG CTGTACGTGG TGGCCCGGAT CGTGATCGCC GTGCGGTACA
     AAGTCGTCGA CCTTCTCGTG GCACCACTGG AAAAAGAAGC GGTAGCCGCA CAAGTAGGAC GACATGCACC ACCGGGCCTA GCACTAGCGG CACGCCATGT

                                                                            EcoRI
              · Y  Q  G   S  N  N   K  R  I  Y    N  D  I   E  M  S    R  F  R  K   *  *
     GATACCAGGC CAGCAACAAC AAGCGGATCT ACAACGACAT CGAGATGAGC CGGTTCCGGA AGTGATGAGA ATTCTTAATT AA
     CTATGGTCCG GTCGTTGTTG TTCGCCTAGA TGTTGCTGTA GCTCTACTCG GCCAAGGCCT TCACTACTCT TAAGAATTAA TT
```

**Figure 9C**

Plasmid

## Figure 10 : VSV-G Piry gene and construct

### A - VSV-G Piry (GenBank # D26175) Underlined : transmembrane domain

MTDTVLGKFQIVFPDQNELEWTPVVGDSRHCPQSSEMQFDGSRSQTILTGKAPVGITPSKSDGFICHAA
KWVTTCDFRWYGPKYITHSIHHLRPTTSDCETALQRYKDGSLINLGFPPESCGYATVTDSEAMLVQVTP
HHVGVDDYRGHWIDPLFPGGECSTNFCDTVHNSSVWIPKSQKTDICAQSFKNIKMTASYPSEGALVSDR
FAFHSAYHPNMPGSTVCIMDFCEQKGLRFTNGEWMGLNVEQSIREKKISAIFPNCVAGTEIRATLESEG
ARTLTWETQRMLDYSLCQNTWDKVSRKEPLSPLDLSYLSPRAPGKGMAYTVINGTLHSAHAKYIRTWID
YGEMKEIKGGRGEYSKAPELLWSQWFDFGPFKIGPNGLLHTGKTFKFPLYLIGAGIIDEDLHELDEAAP
IDHPQMPDAKSVLPEDEEIFFGDTGVSKNPIELIQGWFSNWRESVMAIVGIVLLIVVTFLAIKTVRVLN
CLWRPRKKRIVRQEVDVESRLNHFEMRGFPEYVKR

### B - Optimized nucleotidic sequence

```
                    BamHI
                              M   T   D   T   V   L   G   K   F   Q   I   V   F   P   D   Q   N   E   L   E   W   T   P   V   V ·
      GGCGCGCCGG ATCCTGATCA GCCACCATGA CCGATACAGT GCTGGGCAAG TTCCAGATCG TGTTCCCCGA CCAGAACGAG CTGGAATGGA CCCCCGTCGT
      CCGCGCGGCC TAGGACTAGT CGGTGGTACT GGCTATGTCA CGACCCGTTC AAGGTCTAGC ACAAGGGGCT GGTCTTGCTC GACCTTACCT GGGGGCAGCA
       · G   D   S   R   H   C   P   Q   S   S       E   M   Q   F   D   G   S   R   S   Q       T   I   L   T   G   K   A   P   V   G       I   T   P
      GGGCGACAGC CGGCATTGCC CTCAGTCCAG CGAGATGCAG TTCGACGGCA GCAGAAGCCA GACCATCCTG ACCGGCAAGG CCCCCGTGGG CATCACACCC
      CCCGCTGTCG GCCGTAACGG GAGTCAGGTC GCTCTACGTC AAGCTGCCGT CGTCTTCGGT CTGGTAGGAC TGGCCGTTCC GGGGGCACCC GTAGTGTGGG
       S   K   S   D       G   F   I       C   H   A       A   K   W   V       T   T   C       D   F   R       W   Y   G   P       K   Y   I       T   H   S       I   H   H   L ·
      AGCAAGAGCG ACGGCTTCAT CTGCCACGCC GCCAAGTGGG TGACCACCTG CGACTTCCGG TGGTACGGCC CCAAGTACAT CACCCACAGC ATCCACCACC
      TCGTTCTCGC TGCCGAAGTA GACGGTGCGG CGGTTCACCC ACTGGTGGAC GCTGAAGGCC ACCATGCCGG GGTTCATGTA GTGGGTGTCG TAGGTGGTGG
       · R   P   T       S   D       C   E   T   A       L   Q   R       Y   K   D       G   S   L   I       N   L   G       F   P   P       E   S   C   G       Y   A   T ·
      TGCGGCCCAC CACCTCCGAC TGCGAGACAG CCCTGCAGCG GTACAAGGAC GGCAGCCTGA TCAACCTGGG CTTCCCTCCC GAGAGCTGCG GCTACGCCAC
      ACGCCGGGTG GTGGAGGCTG ACGCTCTGTC GGGACGTCGC CATGTTCCTG CCGTCGGACT AGTTGGACCC GAAGGGAGGG CTCTCGACGC CGATGCGGTG
       · V   T   D       S   E   A   M       L   V   Q       V   T   P       H   H   V   G       V   D   D       Y   R   G       H   W   I   D       P   L   F       P   G   G
      CGTGACAGAC AGCGAGGCCA TGCTGGTGCA GGTGACCCCC CACCACGTGG GCGTGGACGA CTACCGGGGC CACTGGATCG ACCCCCTGTT CCCTGGCGGC
      GCACTGTCTG TCGCTCCGGT ACGACCACGT CCACTGGGGG GTGGTGCACC CGCACCTGCT GATGGCCCCG GTGACCTAGC TGGGGGACAA GGGACCGCCG
       E   C   S   T       N   F   C       D   T   V       H   N   S   S       V   W   I       P   K   S       Q   K   T   D       I   C   A       Q   S   F       K   N   I   K ·
      GAGTGCAGCA CCAATTTCTG CGATACCGTG CACAACAGCA GCGTGTGGAT TCCCAAGAGC CAGAAAACCG ACATCTGCGC CCAGAGCTTC AAGAACATCA
      CTCACGTCGT GGTTAAAGAC GCTATGGCAC GTGTTGTCGT CGCACACCTA AGGGTTCTCG GTCTTTTGGC TGTAGACGCG GGTCTCGAAG TTCTTGTAGT
       · M   T   A       S   Y   P       S   E   G   A       L   V   S       D   R   F       A   F   H   S       A   Y   H       P   N   M       P   G   S   T       V   C   I ·
      AGATGACCGC CAGCTACCCC AGCGAGGGAG CCCTGGTGTC CGACCGGTTC GCCTTCCACA GCGCCTACCA CCCCAACATG CCCGGCAGCA CCGTGTGCAT
      TCTACTGGCG GTCGATGGGG TCGCTCCCTC GGGACCACAG GCTGGCCAAG CGGAAGGTGT CGCGGATGGT GGGGTTGTAC GGGCCGTCGT GGCACACGTA
       · M   D   F       C   E   Q   K       G   L   R       F   T   N       G   E   W   M       G   L   N       V   E   Q       S   I   R   E       K   K   I       S   A   I
      CATGGATTTC TGCGAGCAGA AGGGCCTGCG GTTCACCAAC GGCGAGTGGA TGGGCCTGAA CGTGGAGCAG AGCATCCGGG AGAAGAAGAT CAGCGCCATC
      GTACCTAAAG ACGCTCGTCT TCCCGGACGC CAAGTGGTTG CCGCTCACCT ACCCGGACTT GCACCTCGTC TCGTAGGCCC TCTTCTTCTA GTCGCGGTAG
       F   P   N   C       V   A   G       T   E   I       R   A   T   L       E   S   E       G   A   R       T   L   T   W       E   T   Q       R   M   L       D   Y   S   L ·
      TTCCCCAACT GCGTGGCCGG CACCGAGATC CGGGCCACCC TGGAATCCGA GGGCGCCAGG ACCCTGACCT GGGAGACACA GCGGATGCTG GACTACAGCC
      AAGGGGTTGA CGCACCGGCC GTGGCTCTAG GCCCGGTGGG ACCTTAGGCT CCCGCGGTCC TGGGACTGGA CCCTCTGTGT CGCCTACGAC CTGATGTCGG
       · C   Q   N       T   W   D       K   V   S   R       K   E   P       L   S   P       L   D   L   S       Y   L   S       P   R   A       P   G   K   G       M   A   Y ·
      TGTGCCAGAA CACCTGGGAC AAGGTGTCCC GGAAAGAGCC TCTGTCCCCC CTGGACCTGA GCTACCTGAG CCCTAGAGCC CCTGGCAAGG GCATGGCCTA
      ACACGGTCTT GTGGACCCTG TTCCACAGGG CCTTTCTCGG AGACAGGGGG GACCTGGACT CGATGGACTC GGGATCTCGG GGACCGTTCC CGTACCGGAT
       · T   V   I       N   G   T   L       H   S   A       H   A   K       Y   I   R   T       W   I   D       Y   G   E       M   K   E   I       K   G   G       R   G   E
      CACCGTGATC AACGGCACCC TGCACAGCGC CCACGCCAAG TATATCCGGA CCTGGATCGA CTACGGCGAG ATGAAAGAGA TCAAGGGCGG CAGGGGCGAG
      GTGGCACTAG TTGCCGTGGG ACGTGTCGCG GGTGCGGTTC ATATAGGCCT GGACCTAGCT GATGCCGCTC TACTTTCTCT AGTTCCCGCC GTCCCCGCTC
       Y   S   K   A       P   E   L       L   W   S       Q   W   F   D       F   G   P       F   K   I       G   P   N   G       L   L   H       T   G   K       T   F   K   F ·
      TACAGCAAGG CCCCTGAGCT GCTGTGGAGC CAGTGGTTCG ACTTCGGCCC CTTCAAGATC GGCCCCAACG GCCTGCTGCA CACCGGCAAG ACCTTCAAGT
      ATGTCGTTCC GGGGACTCGA CGACACCTCG GTCACCAAGC TGAAGCCGGG GAAGTTCTAG CCGGGGTTGC CGGACGACGT GTGGCCGTTC TGGAAGTTCA
       · P   L   Y       L   I   G       A   G   I   I       D   E   D       L   H   E       L   D   E   A       A   P   I       D   H   P       Q   M   P   D       A   K   S ·
      TCCCTCTGTA TCTGATCGGA GCCGGCATCA TCGACGAGGA CCTGCACGAG CTGGACGAAG CCGCCCCTAT CGACCACCCC CAGATGCCCG ACGCCAAGAG
      AGGGAGACAT AGACTAGCCT CGGCCGTAGT AGCTGCTCCT GGACGTGCTC GACCTGCTTC GGCGGGGATA GCTGGTGGGG GTCTACGGGC TGCGGTTCTC
       · V   L   P       E   D   E   E       I   F   F       G   D   T       G   V   S   K       N   P   I       E   L   I       Q   G   W   F       S   N   W       R   E   S
      CGTGCTGCCC GAGGACGAGG AAATCTTCTT CGGCGACACC GGCGTGAGCA AGAACCCCAT CGAGCTGATC CAGGGCTGGT TCAGCAACTG GCGGGAGAGC
      GCACGACGGG CTCCTGCTCC TTTAGAAGAA GCCGCTGTGG CCGCACTCGT TCTTGGGGTA GCTCGACTAG GTCCCGACCA AGTCGTTGAC CGCCCTCTCG
       V   M   A   I       V   G   I       V   L   L       I   V   V   T       F   L   A       I   K   T       V   R   V   L       N   C   L       W   R   P       R   K   K   R ·
      GTGATGGCCA TCGTGGGCAT CGTGCTGCTG ATCGTGGTGA CCTTCCTGGC CATCAAGACC GTGCGGGTGC TGAACTGCCT GTGGCGGCCC AGGAAGAAAC
      CACTACCGGT AGCACCCGTA GCACGACGAC TAGCACCACT GGAAGGACCG GTAGTTCTGG CACGCCCACG ACTTGACGGA CACCGCCGGG TCCTTCTTTG
                                                                                                            EcoRI
       · I   V   R       Q   E   V       D   V   E   S       R   L   N       H   F   E       M   R   G   F       P   E   Y       V   K   R   *   *
      GGATCGTCCG GCAGGAAGTG GACGTCGAGA GCCGGCTGAA CCACTTCGAG ATGAGAGGCT TCCCCGAGTA CGTGAAGCGG TGATGAGAAT TCTTAATTAA
      CCTAGCAGGC CGTCCTTCAC CTGCAGCTCT CGGCCGACTT GGTGAAGCTC TACTCTCCGA AGGGGCTCAT GCACTTCGCC ACTACTCTTA AGAATTAATT
```

**Figure 10C**

Plasmid

## Figure 11 : VSV-G Isfahan gene and construct

A - VSV-G Isfahan (genBank # AJ810084) Underlined : transmembrane domain

MTSVLFMVGVLLGAFGSTHCSIQIVFPSETKLVWKPVLKGTRYCPQSAELNLEPDLKTMAFDSKVPIG
ITPSNSDGYLCHAAKWVTTCDFRWYGPKYITHSVHSLRPTVSDCKAAVEAYNAGTLMYPGFPPESCGY
ASITDSEFYVMLVTPHPVGVDDYRGHWVDPLFPTSECNSNFCETVHNATMWIPKDLKTHDVCSQDFQT
IRVSVMYPQTKPTKGADLTLKSKFHAHMKGDRVCKMKFCNKNGLRLGNGEWIEVGDEVMLDNSKLLSL
FPDCLVGSVVKSTLLSEGVQTALWETDRLLDYSLCQNTWEKIDRKEPLSAVDLSYLAPRSPGKGMAYI
VANGSLMSAPARYIRVWIDSPILKEIKGKKESASGIDTVLWEQWLPFNGMELGPNGLIKTKSGYKFPL
YLLGMGIVDQDLQELSSVNPVDHPHVPIAQAFVSEGEEVFFGDTG11VSKNPIELISGWFSDWKETAA
ALGFAAISVILIIGLMRLLPLLCRRRKQKKVIYKDVELNSFDPRQAFHR

## B - Optimized nucleotidic sequence

```
                    BamHI
                             M   T   D   T   V     L   G   K     F   Q   I   V     F   P   D     Q   N   E     L   E   W   T     P   V   V ·
     1     GGCGCGCCGG ATCCTGATCA GCCACCATGA CCGATACAGT GCTGGGCAAG TTCCAGATCG TGTTCCCCGA CCAGAACGAG CTGGAATGGA CCCCCGTCGT
           CCGCGCGGCC TAGGACTAGT CGGTGGTACT GGCTATGTCA CGACCCGTTC AAGGTCTAGC ACAAGGGGCT GGTCTTGCTC GACCTTACCT GGGGGCAGCA
           · G   D   S     R   H   C   P     Q   S   S     E   M   Q     F   D   G   S     R   S   Q     T   I   L     T   G   K     P   V   G     I   T   P
   101     GGGCGACAGC CGGCATTGCC CTCAGTCCAG CGAGATGCAG TTCGACGGCA GCAGAAGCCA GACCATCCTG ACCGGCAAGG CCCCCGTGGG CATCACACCC
           CCCGCTGTCG GCCGTAACGG GAGTCAGGTC GCTCTACGTC AAGCTGCCGT CGTCTTCGGT CTGGTAGGAC TGGCCGTTCC GGGGGCACCC GTAGTGTGGG

           S   K   S   D     G   F   I     C   H   A     A   K   W     V   T   T   C     D   F   R     W   Y   G   P     K   Y   I     T   H   S     I   H   H   L ·
   201     AGCAAGAGCG ACGGCTTCAT CTGCCACGCC GCCAAGTGGG TGACCACCTG CGACTTCCGG TGGTACGGCC CCAAGTACAT CACCCACAGC ATCCACCACC
           TCGTTCTCGC TGCCGAAGTA GACGGTGCGG CGGTTCACCC ACTGGTGGAC GCTGAAGGCC ACCATGCCGG GGTTCATGTA GTGGGTGTCG TAGGTGGTGG
           · R   P   T     T   S   D     C   E   T   A     L   Q   R     Y   K   D     G   S   L   I     N   L   G     F   P   P     E   S   C   G     Y   A   T ·
   301     TGCGGCCCAC CACCTCCGAC TGCGAGACAG CCCTGCAGCG GTACAAGGAC GGCAGCCTGA TCAACCTGGG CTTCCCTCCC GAGAGCTGCG GCTACGCCAC
           ACGCCGGGTG GTGGAGGCTG ACGCTCTGTC GGGACGTCGC CATGTTCCTG CCGTCGGACT AGTTGGACCC GAAGGGAGGG CTCTCGACGC CGATGCGGTG

           · V   T   D     S   E   A   M     L   V   Q     V   T   P     H   H   V   G     V   D   D     Y   R   G     H   W   I   D     P   L   F     P   G   G
   401     CGTGACAGAC AGCGAGGCCA TGCTGGTGCA GGTGACCCCC CACCACGTGG GCGTGGACGA CTACCGGGGC CACTGGATCG ACCCCCTGTT CCCTGGCGGC
           GCACTGTCTG TCGCTCCGGT ACGACCACGT CCACTGGGGG GTGGTGCACC CGCACCTGCT GATGGCCCCG GTGACCTAGC TGGGGGACAA GGGACCGCCG

           E   C   S   T     N   F   C     D   T   V     H   N   S   S     V   W   I     P   K   S     Q   K   T   D     I   C   A     Q   S   F     K   N   I   K ·
   501     GAGTGCAGCA CCAATTTCTG CGATACCGTG CACAACAGCA GCGTGTGGAT TCCCAAGAGC CAGAAAACCG ACATCTGCGC CCAGAGCTTC AAGAACATCA
           CTCACGTCGT GGTTAAAGAC GCTATGGCAC GTGTTGTCGT CGCACACCTA AGGGTTCTCG GTCTTTTGGC TGTAGACGCG GGTCTCGAAG TTCTTGTAGT
           · M   T   A     S   Y   P     S   E   G   A     L   V   S     D   R   F     A   F   H   S     A   Y   H     P   N   M     P   G   S   T     V   C   I ·
   601     AGATGACCGC CAGCTACCCC AGCGAGGGAG CCCTGGTGTC CGACCGGTTC GCCTTCCACA GCGCCTACCA CCCCAACATG CCCGGCAGCA CCGTGTGCAT
           TCTACTGGCG GTCGATGGGG TCGCTCCCTC GGGACCACAG GCTGGCCAAG CGGAAGGTGT CGCGGATGGT GGGGTTGTAC GGGCCGTCGT GGCACACGTA

           · M   D   F     C   E   Q   K     G   L   R     F   T   N     G   E   W   M     G   L   N     V   E   Q     S   I   R   E     K   K   I     S   A   I
   701     CATGGATTTC TGCGAGCAGA AGGGCCTGCG GTTCACCAAC GGCGAGTGGA TGGGCCTGAA CGTGGAGCAG AGCATCCGGG AGAAGAAGAT CAGCGCCATC
           GTACCTAAAG ACGCTCGTCT TCCCGGACGC CAAGTGGTTG CCGCTCACCT ACCCGGACTT GCACCTCGTC TCGTAGGCCC TCTTCTTCTA GTCGCGGTAG

           F   P   N   C     V   A   G     T   E   I     R   A   T   L     E   S   E     G   A   R     T   L   T   W     E   T   Q     R   M   L     D   Y   S   L ·
   801     TTCCCCAACT GCGTGGCCGG CACCGAGATC CGGGCCACCC TGGAATCCGA GGGCGCCAGG ACCCTGACCT GGGAGACACA GCGGATGCTG GACTACAGCC
           AAGGGGTTGA CGCACCGGCC GTGGCTCTAG GCCCGGTGGG ACCTTAGGCT CCCGCGGTCC TGGGACTGGA CCCTCTGTGT CGCCTACGAC CTGATGTCGG

           · C   Q   N     T   W   D     K   V   S   R     K   E   P     L   S   P     L   D   L   S     Y   L   S     P   R   A     P   G   K   G     M   A   Y ·
   901     TGTGCCAGAA CACCTGGGAC AAGGTGTCCC GGAAAGAGCC TCTGTCCCCC CTGGACCTGA GCTACCTGAG CCCTAGAGCC CCTGGCAAGG GCATGGCCTA
           ACACGGTCTT GTGGACCCTG TTCCACAGGG CCTTTCTCGG AGACAGGGGG GACCTGGACT CGATGGACTC GGGATCTCGG GGACCGTTCC CGTACCGGAT

           · T   V   I     N   G   T   L     H   S   A     H   A   K     Y   I   R   T     W   I   D     Y   G   E     M   K   E   I     K   G   G     R   G   E
  1001     CACCGTGATC AACGGCACCC TGCACAGCGC CCACGCCAAG TATATCCGGA CCTGGATCGA CTACGGCGAG ATGAAAGAGA TCAAGGGCGG CAGGGGCGAG
           GTGGCACTAG TTGCCGTGGG ACGTGTCGCG GGTGCGGTTC ATATAGGCCT GGACCTAGCT GATGCCGCTC TACTTTCTCT AGTTCCCGCC GTCCCGCTC

           Y   S   K   A     P   E   L     L   W   S     Q   W   F   D     F   G   P     F   K   I     G   P   N   G     L   L   H     T   G   K     T   F   K   F ·
  1101     TACAGCAAGG CCCCTGAGCT GCTGTGGAGC CAGTGGTTCG ACTTCGGCCC CTTCAAGATC GGCCCCAACG GCCTGCTGCA CACCGGCAAG ACCTTCAAGT
           ATGTCGTTCC GGGGACTCGA CGACACCTCG GTCACCAAGC TGAAGCCGGG GAAGTTCTAG CCGGGGTTGC CGGACGACGT GTGGCCGTTC TGGAAGTTCA

           · P   L   Y     L   I   G     A   G   I   I     D   E   D     L   H   E     L   D   E   A     A   P   I     D   H   P     Q   M   P   D     A   K   S ·
  1201     TCCCTCTGTA TCTGATCGGA GCCGGCATCA TCGACGAGGA CCTGCACGAG CTGGACGAAG CCGCCCCTAT CGACCACCCC CAGATGCCCG ACGCCAAGAG
           AGGGAGACAT AGACTAGCCT CGGCCGTAGT AGCTGCTCCT GGACGTGCTC GACCTGCTTC GGCGGGGATA GCTGGTGGGG GTCTACGGGC TGCGGTTCTC

           · V   L   P     E   D   E   E     C   E   T   A     L   Q   R     G   D   T     G   V   S   K     N   P   I     E   L   I     Q   G   W   F     S   N   W     R   E   S
  1301     CGTGCTGCCC GAGGACGAGG AAATCTTCTT CGGCGACACC GGCGTGAGCA AGAACCCCAT CGAGCTGATC CAGGGCTGGT TCAGCAACTG GCGGGAGAGC
           GCACGACGGG CTCCTGCTCC TTTAGAAGAA GCCGCTGTGG CCGCACTCGT TCTTGGGGTA GCTCGACTAG GTCCCGACCA AGTCGTTGAC CGCCCTCTCG

           V   M   A   I     V   G   I     V   L   L     I   V   V   T     F   L   A     I   K   T     V   R   V   L     N   C   L     W   R   P     R   K   K   R ·
  1401     GTGATGGCCA TCGTGGGCAT CGTGCTGCTG ATCGTGGTGA CCTTCCTGGC CATCAAGACC GTGCGGGTGC TGAACTGCCT GTGGCGGCCC AGGAAGAAAC
           CACTACCGGT AGCACCCGTA GCACGACGAC TAGCACCACT GGAAGGACCG GTAGTTCTGG CACGCCCACG ACTTGACGGA CACCGCCGGG TCCTTCTTTG

                                                                                                                            EcoRI
           · I   V   R     Q   E   V     D   V   E   S     R   L   N     H   F   E     M   R   G   F     P   E   Y     V   K   R     *   *
  1501     GGATCGTCCG GCAGGAAGTG GACGTCGAGA GCCGGCTGAA CCACTTCGAG ATGAGAGGCT TCCCCGAGTA CGTGAAGCGG TGATGAGAAT TCTTAATTAA
           CCTAGCAGGC CGTCCTTCAC CTGCAGCTCT CGGCCGACTT GGTGAAGCTC TACTCTCCGA AGGGGCTCAT GCACTTCGCC ACTACTCTTA AGAATTAATT
```

Plasmid

Figure 11C

# Figure 12 : VSV-G Spring viremia carp virus gene and construct

A - Protein G of Spring viremia of carp virus (GenBank #AAZ20272) Underlined : transmembrane domain

```
MSIISYIAFLLLIDSTLGIPIFVPSGQNISWQPVIQPFDYQCPIHGNLPNTMGLSATKLTIKSPSVFS
TDKVSGWICHAAEWKTTCDYRWYGPQYITHSIHPISPTIDECKRIISRIASGTDEDLGFPPQSCGWAS
VTTVSNTNYKVVPHSVHLEPYGGHWIDHDFNGGECREKVCEMKGNHSIWITDETVQHECEKHIEEVEG
IMYGNAPRGDAIYINNFIIDKHHRVYRFGGSCRMKFCNKDGIKFTRGDWVEKTAGTLTNIYENIPECA
DGTLVSGHRPGLDLIDTVFNLENVVEYTLCEGTKRKINKQEKLTSVDLSYLAPRIGGFGSVFRVRNGT
LERGSTTYIRIEVEGPVVDSLNGIDPRTNASRVFWDDWELDGNIYQGFNGVYKGKDGKIHIPLNMIES
GIIDDELQHAFQADIIPHPHYDDDEIREDDIFFDNTGENGNPVDAVVEWVSGWGTSLKFFGMTLVALI
LIFLLIRCCVACTYLMKKSKRPATESHEMRSLV
```

B - Optimized nucleotidic sequence

```
             BamHI
                              M   S   I   I   S   Y   I   A   F   L   L   L   I   D   S   T   L   G   I   P   I   F   V   P   S ·
1     GGCGCGCCGG ATCCTGATCA GCCACCATGA GCATCATCAG CTATATCGCC TTTCTGCTGC TGATCGACAG CACCCTGGGC ATCCCCATCT TCGTGCCCAG
      CCGCGCGGCC TAGGACTAGT CGGTGGTACT CGTAGTAGTC GATATAGCGG AAAGACGACG ACTAGCTGTC GTGGGACCCG TAGGGGTAGA AGCACGGGTC

     · G   Q   N   I   S   W   Q   P   V   I   Q   P   F   D   Y   Q   C   P   I   H   G   N   L   P   N   T   M   G   L   S   A   T   K
101   CGGCCAGAAC ATCAGCTGGC AGCCCGTGAT CCAGCCCTTC GACTACCAGT GCCCCATCCA CGGCAACCTG CCCAACACCA TGGGCCTGAG CGCCACCAAG
      GCCGGTCTTG TAGTCGACCG TCGGGCACTA GGTCGGGAAG CTGATGGTCA CGGGGTAGGT GCCGTTGGAC GGGTTGTGGT ACCCGGACTC GCGGTGGTTC

     L   T   I   K   S   P   S   V   F   S   T   D   K   V   S   G   W   I   C   H   A   A   E   W   K   T   T   C   D   Y   R   W   Y   G ·
201   CTGACCATCA AGAGCCCCAG CGTGTTCAGC ACCGACAAGG TGTCCGGCTG GATCTGCCAC GCCGCCGAGT GGAAAACCAC CTGCGACTAC CGGTGGTACG
      GACTGGTAGT TCTCGGGGTC GCACAAGTCG TGGCTGTTCC ACAGGCCGAC CTAGACGGTG CGGCGGCTCA CCTTTTGGTG GACGCTGATG GCCACCATGC

     · P   Q   Y   I   T   H   S   I   H   P   I   S   P   T   I   D   E   C   K   R   I   I   S   R   I   A   S   G   T   D   E   D   L ·
301   GCCCCAGTA CATCACCCAC AGCATCCACC CCATCAGCCC CACCATCGAC GAGTGCAAGC GGATCATCAG CCGGATCGCC AGCGGCACCG ACGAGGACCT
      CGGGGGTCAT GTAGTGGGTG TCGTAGGTGG GGTAGTCGGG GTGGTAGCTG CTCACGTTCG CCTAGTAGTC GGCCTAGCGG TCGCCGTGGC TGCTCCTGGA

     · G   F   P   P   Q   S   C   G   W   A   S   V   T   T   V   S   N   T   N   Y   K   V   V   P   H   S   V   H   L   E   P   Y   G
401   GGGCTTCCCA CCCCAGAGCT GCGGCTGGGC CAGCGTGACC ACCGTGAGCA ACACCAACTA CAAGGTGGTG CCCCACAGCG TGCACCTGGA ACCCTACGGC
      CCCGAAGGGT GGGGTCTCGA CGCCGACCCG GTCGCACTGG TGGCACTCGT TGTGGTTGAT GTTCCACCAC GGGGTGTCGC ACGTGGACCT TGGGATGCCG

     G   H   W   I   D   H   D   F   N   G   G   E   C   R   E   K   V   C   E   M   K   G   N   H   S   I   W   I   T   D   E   T   V   Q ·
501   GGCCACTGGA TCGACCACGA CTTCAACGGC GGCGAGTGCC GGGAGAAAGT GTGCGAGATG AAGGGCAACC ACAGCATCTG GATCACCGAC GAGACAGTGC
      CCGGTGACCT AGCTGGTGCT GAAGTTGCCG CCGCTCACGG CCCTCTTTCA CACGCTCTAC TTCCCGTTGG TGTCGTAGAC CTAGTGGCTG CTCTGTCACG

     · H   E   C   E   K   H   I   E   E   V   E   G   I   M   Y   G   N   A   P   R   G   D   A   I   Y   I   N   N   F   I   I   D   K ·
601   AGCACGAGTG CGAGAAGCAC ATCGAGGAAG TGGAGGGCAT CATGTACGGC AACGCCCCA GGGGCGACGC CATCTACATC AACAACTTCA TCATCGACAA
      TCGTGCTCAC GCTCTTCGTG TAGCTCCTTC ACCTCCCGTA GTACATGCCG TTGCGGGGGT CCCCGCTGCG GTAGATGTAG TTGTTGAAGT AGTAGCTGTT

     · H   H   R   V   Y   R   F   G   G   S   C   R   M   K   F   C   N   K   D   G   I   K   F   T   R   G   D   W   V   E   K   T   A
701   GCACCACCGG GTGTACCGGT TCGGCGGCTC CTGCCGGATG AAGTTCTGCA ACAAGGACGG CATCAAGTTC ACCAGAGGCG ACTGGGTGGA GAAAACCGCC
      CGTGGTGGCC CACATGGCCA AGCCGCCGAG GACGGCCTAC TTCAAGACGT TGTTCCTGCC GTAGTTCAAG TGGTCTCCGC TGACCCACCT CTTTTGGCGG

     G   T   L   T   N   I   Y   E   N   I   P   E   C   A   D   G   T   L   V   S   G   H   R   P   G   L   D   L   I   D   T   V   F   N ·
801   GGCACCCTGA CCAACATCTA CGAGAACATC CCCGAGTGCG CCGACGGCAC ACTGGTGTCC GGCCACAGAC CCGGCCTGGA CCTGATCGAC ACCGTGTTCA
      CCGTGGGACT GGTTGTAGAT GCTCTTGTAG GGGCTCACGC GGCTGCCGTG TGACCACAGG CCGGTGTCTG GGCCGGACCT GGACTAGCTG TGGCACAAGT

     · L   E   N   V   V   E   Y   T   L   C   E   G   T   K   R   K   I   N   K   Q   E   K   L   T   S   V   D   L   S   Y   L   A   P ·
901   ACCTGGAAAA CGTGGTGGAG TACACCCTGT GCGAGGGCAC CAAGCGGAAG ATCAACAAGC AGGAAAAGCT GACCAGCGTC GACCTGAGCT ACCTGGCCCC
      TGGACCTTTT GCACCACCTC ATGTGGGACA CGCTCCCGTG GTTCGCCTTC TAGTTGTTCG TCCTTTTCGA CTGGTCGCAG CTGGACTCGA TGGACCGGGG

     · R   I   G   G   F   G   S   V   F   R   V   R   N   G   T   L   E   R   G   S   T   T   Y   I   R   I   E   V   E   G   P   V   V
1001  CAGGATCGGC GGCTTCGGCA GCGTGTTCCG CGTGCGGAAT GGGACCCTGG AAAGAGGAAG CACAACATAC ATTCGGATCG AAGTGGAAGG CCCCGTGGTG
      GTCCTAGCCG CCGAAGCCGT CGCACAAGGC GCACGCCTTA CCCTGGGACC TTTCTCCTTC GTGTTGTATG TAAGCCTAGC TTCACCTTCC GGGGCACCAC

     D   S   L   N   G   I   D   P   R   T   N   A   S   R   V   F   W   D   D   W   E   L   D   G   N   I   Y   Q   G   F   N   G   V   Y ·
1101  GACAGCCTGA ACGGCATCGA CCCCCGGACC AACGCCAGCC GGGTGTTCTG GGACGACTGG GAGCTGGACG GCAACATCTA CCAGGGCTTC AATGGCGTGT
      CTGTCGGACT TGCCGTAGCT GGGGGCCTGG TTGCGGTCGG CCCACAAGAC CCTGCTGACC CTCGACCTGC CGTTGTAGAT GGTCCCGAAG TTACCGCACA

     · K   G   K   D   G   K   I   H   I   P   L   N   M   I   E   S   G   I   I   D   D   E   L   Q   H   A   F   Q   A   D   I   I   P ·
1201  ACAAGGGCAA GGATGGCAAG ATCCACATCC CCCTGAACAT GATCGAGAGC GGCATCATCG ACGACGAGCT GCAGCACGCC TTCCAGGCCG ACATCATCCC
      TGTTCCCGTT CCTACCGTTC TAGGTGTAGG GGGACTTGTA CTAGCTCTCG CCGTAGTAGC TGCTGCTCGA CGTCGTGCGG AAGGTCCGGC TGTAGTAGGG

     · H   P   H   Y   D   D   D   E   I   R   E   D   D   I   F   F   D   N   T   G   E   N   G   N   P   V   D   A   V   V   E   W   V
1301  CCACCCCAC TACGACGACG ACGAGATCCG GGAGGACGAC ATCTTCTTCG ACAACACCGG CGAGAACGGC AACCCCGTGG ACGCCGTGGT GGAATGGGTG
      GGTGGGGGTG ATGCTGCTGC TGCTCTAGGC CCTCCTGCTG TAGAAGAAGC TGTTGTGGCC GCTCTTGCCG TTGGGGCACC TGCGGCACCA CCTTACCCAC

     S   G   W   G   T   S   L   K   F   F   G   M   T   L   V   A   L   I   L   I   F   L   L   I   R   C   C   V   A   C   T   Y   L   M ·
1401  TCCGGATGGG GCACCAGCCT GAAGTTCTTC GGCATGACCC TGGTGGCCCT GATCCTGATC TTCCTGCTGA TCCGGTGCTG CGTGGCCTGC ACCTACCTGA
      AGGCCTACCC CGTGGTCGGA CTTCAAGAAG CCGTACTGGG ACCACCGGGA CTAGGACTAG AAGGACGACT AGGCCACGAC GCACCGGACG TGGATGGACT
                                                                                                                    EcoRI
     · K   K   S   K   R   P   A   T   E   S   H   E   M   R   S   L   V   *   *
1501  TGAAGAAGAG CAAGAGGCCC GCCACCGAGA GCCACGAGAT GCGGAGCCTG GTGTGATGAG AATTCTTAAT TAA
      ACTTCTTCTC GTTCTCCGGG CGGTGGCTCT CGGTGCTCTA CGCCTCGGAC CACACTACTC TTAAGAATTA ATT
```

Plasmid

Figure 12C

## Figure 13 : Fusion between the VSV-G$_{NewJersey}$ and the VSV-G$_{Indiana}$ proteins (Bold and underlined : transmembrane domain)

1-  Indiana PCR fragment

VSV-G$_{Indiana}$ sequence (optimized codons)

BamH1

```
5' CTC GGATCC TGATCAGCCACCATGAAATGCCTGCTCTATCTGGCCTTCCTCTTTATCGGCGTGAACTGTAAGTTCACGATC
GTGTTTCCCCACAATCAGAAGGGAAACTGGAAGAACGTCCCGAGCAACTACCACTACTGCCCTAGCTCAAGCGACCTGAACTG
GCACAACGACCTGATCGGCACCGCTATCCAGGTGAAGATGCCAAAGAGCCACAAGGCCATCCAAGCCGACGGCTGGATGTGTC
ACGCCAGCAAATGGGTGACGACGTGCGATTTTCGCTGGTATGGCCCCAAGTACATCACCCAATCAATCCGCTCATTTACACCC
AGCGTGGAGCAATGTAAGGAGAGCATCGAGCAGACCAAGCAGGGGACCTGGCTCAACCCCGGCTTCCCACCGCAAAGCTGCGG
ATACGCCACCGTGACCGACGCTGAGGCCGTCATCGTGCAGGTGACCCCGCACCACGTGCTGGTGGACGAGTACACCGGCGAGT
GGGTGGATTCACAGTTTATCAACGGAAAGTGTAGCAATTACATCTGCCCCACCGTGCACAACAGCACCACCTGGCACTCAGAC
TATAAGGTGAAGGGCCTCTGCGACAGCAATCTGATCTCAATGGACATCACCTTCTTTAGCGAAGACGGCGAACTCTCAAGCCT
CGGGAAGGAAGGCACCGGGTTCCGCAGCAATTACTTTGCTTACGAAACCGGCGGCAAGGCCTGCAAGATGCAATACTGCAAGC
ACTGGGGCGTGCGCCTGCCAAGCGGCGTGTGGTTTGAGATGGCTGATAAGGACCTGTTCGCCGCTGCCCGCTTCCCGGAATGC
CCCGAGGGGAGCAGCATCAGCGCCCCCAGCCAGACATCAGTGGACGTGAGCCTGATCCAGGATGTGGAACGCATCCTGGACTA
CAGCCTGTGTCAGGAAACGTGGAGCAAGATCCGCGCCGGACTGCCTATCAGCCCCGTGGATCTCAGCTACCTGGCCCCAAAGA
ACCCAGGCACCGGACCCGCCTTTACAATCATCAACGGCACCCTGAAGTACTTTGAAACACGCTACATCCGCGTCGACATCGCC
GCTCCCATCCTCTCACGCATGGTGGGCATGATCTCAGGGACGACCACGGAGCGCGAGCTGTGGGATGACTGGGCCCCGTATGA
AGATGTGGAGATCGGACCTAACGGCGTGCTGCGCACATCAAGCGGGTACAAGTTCCCGCTGTACATGATCGGCCACGGCATGC
TGGACAGCGACCTGCACCTCAGCTCAAAGGCCCAGGTCTTTGAGCACCCACACATCCAGGACGCTGCCAGCCAGCTCCCCGAC
GACGAAAGCCTGTTCTTTGGAGATACAGGGCTCAGCAAGAACCCCATCGAGCTGGTCGAGGGCTGGTTCTCAAGCTGGAAGAG
CAGCATCGCTTCATTTTTTTTTCATCATCGGCCTCATCATCGGGCTGTTTCTGGTGCTGCGCGTCGGCATCCACCTGTGCATCA
AGCTGAAGCACACCAAGAAGCGCCAGATCTATACCGACATCGAGATGAATCGCCTGGGGAAGTAA GAATTC TGCAGATATCCA
GCA-3'                                                              EcoR1
```

Oligonucleotides Indiana

- 1 (5'-AGCAGCATCGCTTCATTTTTTTTCATCATCGG-3')
- 2 (5'-GCTGGATATCTGCAGAATTCTTACTTCCCCAGGCG-3')

PCR fragment (160bp):

Indiana Transmembrane Domain

```
5' AGCAGCATCGCTTCATTTTTTTTCATCATCGGCCTCATCATCGGGCTGTTTCTGGTGCTGCGCGTCGGCATCCACCTGTGC
ATCAAGCTGAAGCACACCAAGAAGCGCCAGATCTATACCGACATCGAGATGAATCGCCTGGGGAAGTAA GAATTC TGCA3'
```

2.  New Jersey PCR fragment

VSV-G$_{NewJersey}$ sequence (optimized codons)

BamH1

```
5' TACCGAGCTC GGATCC TGATCAGCCACCATGCTGTCATATCTGATCTTTGCCCTGGCTGTGAGCCCAATCCTCGGAAAGAT
CGAAATCGTGTTCCCACAACACACCACAGGGGACTGGAAGCGCGTGCCCCACGAGTACAACTACTGCCCGACCTCAGCCGACA
AGAATAGCCACGGCACGCAGACCGGCATCCCTGTGGAGCTGACCATGCCCAAGGGGCTCACAACGCACCAAGTCGAAGGCTTC
ATGTGCCACAGCGCTCTCTGGATGACAACCTGCGATTTTCGCTGGTATGGCCCCAAGTACATCACGCACAGCATCCACAATGA
GGAACCAACCGACTACCAGTGCCTCGAAGCCATCAAGTCATACAAGGATGGGGTGAGCTTCAACCCCGGCTTCCCGCCCCAAT
CATGTGGCTACGGCACCGTGACCGACGCCGAGGCCCACATCGTGACCGTGACACCCCACTCAGTCAAGGTGGACGAGTACACA
GGCGAATGGATCGACCCCCACTTCATCGGGGGCCGCTGTAAGGGCCAAATCTGCGAGACCGTGCACAACAGCACCAAGTGGTT
TACGTCATCAGACGGCGAAAGCGTGTGCAGCCAACTGTTTACGCTCGTGGGCGGCATCTTCTTTAGCGACAGCGAGGAGATCA
CCAGCATGGGCCTCCCGGAGACAGGAATCCGCAGCAACTACTTTCCGTACATCAGCACCGAGGGAATCTGTAAGATGCCTTTT
TGCCGCAAGCAGGGATATAAGCTGAAGAATGACCTGTGGTTCCAGATCATGGACCCGGACCTGGACAAGACCGTCCGCGATCT
GCCCCACATCAAGGACTGTGATCTGTCATCAAGCATCATCACCCCCGGAGAACACGCCACGGACATCAGCCTCATCAGCGATG
TGGAGCGCATCCTCGACTACGCTCTCTGCCAGAACACATGGAGCAAGATCGAAAGCGGCGAACCCATCACCCCAGTGGACCTG
AGCTATCTCGGCCCAAAGAACCCCGGCGTGGGGCCCGTGTTCACCATCATCAACGGGAGCCTGCACTACTTTACAAGCAAGTA
TCTGCGCGTGGAGCTCGAAAGCCCAGTCATCCCCCGCATGGAGGGGAAGGTGGCCGGGACCCGCATCGTGCGCCAGCTGTGGG
ACCAGTGGTTCCCTTTTGGCGAGGTGGAAATCGGCCCCAACGGCGTGCTGAAGACCAAGCAAGGATATAAGTTCCCGCTGCAC
ATCATCGGGACGGGCGAAGTGGACAGCGATATCAAGATGGAGCGCGTGGTCAAGCACTGGGAGCACCCACACATCGAGGCTGC
TCAGACCTTTCTCAAGAAGGACGATACCGGCGAAGTCCTGTATTACGGGGATACGGGAGTGAGCAAGAACCCTGTGGAGCTGG
TGGAAGGCTGGTTCAGCGGATGGCGCTCAAGCCTGATGGGCGTGCTGGCCGTCATCATCGGATTTGTGATCCTGATGTTCCTC
ATCAAGCTGATCGGCGTGCTGTGTCAAGCCTGTTCCGCCCTAAGCGCCGCCCAATCTACAAGAGCGACGTCGAGATGGCCCACTT
TCGCTAA GAATTC TGCAGATAT-3'
   EcoR1
```

Oligonucleotides NewJersey :

- 3 (5'-CGAGCTCGGATCCTGATCAGCCACCATGCTGTC-3')
- 4 (5'-**GAAAAAAAATGAAGCGATGCTGCT**GCGCCATCCGCTGAACCAGCCTTCCAC-3').

The bold and underlined part of oligo 4 corresponds to 28 first Indiana transmembrane domain nucleotides)

PCR NewJersey (1446bp):

```
              BamH1
5' CGAGCTCGGATCCTGATCAGCCACCATGCTGTCATATCTGATCTTTGCCCTGGCTGTGAGCCCAATCCTCGGAAAGATCGA
AATCGTGTTCCCACAACACACCACAGGGGACTGGAAGCGCGTGCCCCACGAGTACAACTACTGCCCGACCTCAGCCGACAAGA
ATAGCCACGGCACGCAGACCGGCATCCCTGTGGAGCTGACCATGCCCAAGGGGCTCACAACGCACCAAGTCGAAGGCTTCATG
TGCCACAGCGCTCTCTGGATGACAACCTGCGATTTTCGCTGGTATGGCCCCAAGTACATCACGCACAGCATCCACAATGAGGA
ACCAACCGACTACCAGTGCCTCGAAGCCATCAAGTCATACAAGGATGGGGTGAGCTTCAACCCCGGCTTCCCGCCCCAATCAT
GTGGCTACGGCACCGTGACCGACGCCGAGGCCCACATCGTGACCGTGACACCCCACTCAGTCAAGGTGGACGAGTACACAGGC
GAATGGATCGACCCCCACTTCATCGGGGGCCGCTGTAAGGGCCAAATCTGCGAGACCGTGCACAACAGCACCAAGTGGTTTAC
GTCATCAGACGGCGAAAGCGTGTGCAGCCAACTGTTTACGCTCGTGGGCGGCATCTTCTTTAGCGACAGCGAGGAGATCACCA
GCATGGGCCTCCCGGAGACAGGAATCCGCAGCAACTACTTTCCGTACATCAGCACCGAGGGAATCTGTAAGATGCCTTTTTGC
CGCAAGCAGGGATATAAGCTGAAGAATGACCTGTGGTTCCAGATCATGGACCCGGACCTGGACAAGACCGTCCGCGATCTGCC
CCACATCAAGGACTGTGATCTGTCATCAAGCATCATCACCCCCGGAGAACACGCCACGGACATCAGCCTCATCAGCGATGTGG
AGCGCATCCTCGACTACGCTCTCTGCCAGAACACATGGAGCAAGATCGAAAGCGGCGAACCCATCACCCCAGTGGACCTGAGC
TATCTCGGCCCAAAGAACCCCGGCGTGGGGCCCGTGTTCACCATCATCAACGGGAGCCTGCACTACTTTACAAGCAAGTATCT
GCGCGTGGAGCTCGAAAGCCCAGTCATCCCCCGCATGGAGGGGAAGGTGGCCGGGACCCGCATCGTGCGCCAGCTGTGGGACC
AGTGGTTCCCTTTTGGCGAGGTGGAAATCGGCCCCAACGGCGTGCTGAAGACCAAGCAAGGATATAAGTTCCCGCTGCACATC
ATCGGGACGGGCGAAGTGGACAGCGATATCAAGATGGAGCGCGTGGTCAAGCACTGGGAGCACCCACACATCGAGGCTGCTCA
GACCTTTCTCAAGAAGGACGATACCGGCGAAGTCCTGTATTACGGGGATACGGGAGTGAGCAAGAACCCTGTGGAGCTGGTGG
AAGGCTGGTTCAGCGGATGGCGC**AGCAGCATCGCTTCATTTTTTTC**-3'
                    Indiana TransMembrane domain
```

## 3. Overlapping PCR (1620bp)

PCR with oligonucleotides 2 and 3

```
        OLIGO 3
        BamH1
5' GAGCTCGGATCCTGATCAGCCACCATGCTGTCATATCTGATCTTTGCCCTGGCTGTGAGCCCAATCCTCGGAAAGATCGAA
ATCGTGTTCCCACAACACACCACAGGGGACTGGAAGCGCGTGCCCCACGAGTACAACTACTGCCCGACCTCAGCCGACAAGAA
TAGCCACGGCACGCAGACCGGCATCCCTGTGGAGCTGACCATGCCCAAGGGGCTCACAACGCACCAAGTCGAAGGCTTCATGT
GCCACAGCGCTCTCTGGATGACAACCTGCGATTTTCGCTGGTATGGCCCCAAGTACATCACGCACAGCATCCACAATGAGGAA
CCAACCGACTACCAGTGCCTCGAAGCCATCAAGTCATACAAGGATGGGGTGAGCTTCAACCCCGGCTTCCCGCCCCAATCATG
TGGCTACGGCACCGTGACCGACGCCGAGGCCCACATCGTGACCGTGACACCCCACTCAGTCAAGGTGGACGAGTACACAGGCG
AATGGATCGACCCCCACTTCATCGGGGGCCGCTGTAAGGGCCAAATCTGCGAGACCGTGCACAACAGCACCAAGTGGTTTACG
TCATCAGACGGCGAAAGCGTGTGCAGCCAACTGTTTACGCTCGTGGGCGGCATCTTCTTTAGCGACAGCGAGGAGATCACCAG
CATGGGCCTCCCGGAGACAGGAATCCGCAGCAACTACTTTCCGTACATCAGCACCGAGGGAATCTGTAAGATGCCTTTTTGCC
GCAAGCAGGGATATAAGCTGAAGAATGACCTGTGGTTCCAGATCATGGACCCGGACCTGGACAAGACCGTCCGCGATCTGCCC
CACATCAAGGACTGTGATCTGTCATCAAGCATCATCACCCCCGGAGAACACGCCACGGACATCAGCCTCATCAGCGATGTGGA
GCGCATCCTCGACTACGCTCTCTGCCAGAACACATGGAGCAAGATCGAAAGCGGCGAACCCATCACCCCAGTGGACCTGAGCT
ATCTCGGCCCAAAGAACCCCGGCGTGGGGCCCGTGTTCACCATCATCAACGGGAGCCTGCACTACTTTACAAGCAAGTATCTG
CGCGTGGAGCTCGAAAGCCCAGTCATCCCCCGCATGGAGGGGAAGGTGGCCGGGACCCGCATCGTGCGCCAGCTGTGGGACCA
GTGGTTCCCTTTTGGCGAGGTGGAAATCGGCCCCAACGGCGTGCTGAAGACCAAGCAAGGATATAAGTTCCCGCTGCACATCA
TCGGGACGGGCGAAGTGGACAGCGATATCAAGATGGAGCGCGTGGTCAAGCACTGGGAGCACCCACACATCGAGGCTGCTCAG
ACCTTTCTCAAGAAGGACGATACCGGCGAAGTCCTGTATTACGGGGATACGGGAGTGAGCAAGAACCCTGTGGAGCTGGTGGA
AGGCTGGTTCAGCGGATGGCGC**AGCAGCATCGCTTCATTTTTTTTC**
                       **AGCAGCATCGCTTCATTTTTTTTC**ATCATCGGCCTCATCATCGGGCTGTTTCTGGTGCTG
CGCGTCGGCATCCACCTGTGCATCAAGCTGAAGCACACCAAGAAGCGCCAGATCTATACCGACATCGAGATGAAT CGCCTGGG
GAAGTAAGAATTGGTGCA-3'
        EcoR1
OLIGO 2
```

Figure 13 (suite)

# Figure 14A: Fusion protein of VSV-GChandipura/indiana

## Fusion potein

```
        M   T   S   S   V   T   I   S   V   V   L   L   I   S   F   I   T   P   L   Y   S   Y   L   S   I   A   F   P   E   N   T
3601    ATG ACCAGCAGCG TGACCATCAG CGTGGTGCTG CTGATCAGCT TCATCACCCC CCTGTACAGC TACCTGAGCA TTGCCTTCCC CGAGAACACC
        TAC TGGTCGTCGC ACTGGTAGTC GCACCACGAC GACTAGTCGA AGTAGTGGGG GGACATGTCG ATGGACTCGT AACGGAAGGG GCTCTTGTGG

        K   L   D   W   K   P   V   T   K   N   T   R   Y   C   P   M   G   G   E   W   F   L   E   P   G   L   Q   E   E   S   F   L   S   S ·
3701    AAGCTGGACT GGAAGCCCGT GACCAAGAAC ACCCGGTACT GCCCCATGGG CGGCGAGTGG TTTCTGGAAC CCGGCCTGCA GGAAGAGAGC TTCCTGAGCA
        TTCGACCTGA CCTTCGGGCA CTGGTTCTTG TGGGCCATGA CGGGGTACCC GCCGCTCACC AAAGACCTTG GGCCGGACGT CCTTCTCTCG AAGGACTCGT

        · T   P   I   G   A   T   P   S   K   S   D   G   F   L   C   H   A   A   K   W   V   T   T   C   D   F   R   W   Y   G   P   K   Y ·
3801    GCACCCCCAT CGGCGCCACC CCCAGCAAGA GCGACGGCTT CCTGTGCCAC GCCGCCAAGT GGGTGACCAC CTGCGACTTC CGGTGGTACG GCCCCAAGTA
        CGTGGGGGTA GCCGCGGTGG GGGTCGTTCT CGCTGCCGAA GGACACGGTG CGGCGGTTCA CCCACTGGTG GACGCTGAAG GCCACCATGC CGGGGTTCAT

        · I   T   H   S   I   H   N   I   K   P   T   R   S   D   C   D   T   A   L   A   S   Y   K   S   G   T   L   V   S   L   G   F   P
3901    CATCACCCAC AGCATCCACA ACATCAAGCC CACCAGAAGC GACTGCGACA CAGCCCTGGC CTCTTACAAG AGCGGCACCC TGGTGTCCCT GGGCTTCCCT
        GTAGTGGGTG TCGTAGGTGT TGTAGTTCGG GTGGTCTTCG CTGACGCTGT GTCGGGACCG GAGAATGTTC TCGCCGTGGG ACCACAGGGA CCCGAAGGGA

        P   E   S   C   G   Y   A   S   V   T   D   S   E   F   L   V   I   M   I   T   P   H   H   V   G   V   D   D   Y   R   G   H   W   V ·
4001    CCCGAGAGCT GCGGCTACGC CAGCGTGACC GACAGCGAGT TCCTGGTGAT TATGATTACC CCCCACCACG TGGGCGTGGA CGACTACCGG GGCCACTGGG
        GGGCTCTCGA CGCCGATGCG GTCGCACTGG CTGTCGCTCA AGGACCACTA ATACTAATGG GGGGTGGTGC ACCCGCACCT GCTGATGGCC CCGGTGACCC

        · D   P   L   F   V   G   G   E   C   D   Q   S   Y   C   D   T   I   H   N   S   V   W   I   P   A   D   Q   T   K   K   N   I ·
4101    TGGACCCTCT GTTCGTGGGA GGGGAATGCG ACCAGAGCTA CTGCGATACC ATCCACAACT CCAGCGTGTG GATTCCCGCC GACCAGACCA AGAAGAACAT
        ACCTGGGAGA CAAGCACCCT CCCCTTACGC TGGTCTCGAT GACGCTATGG TAGGTGTTGA GGTCGCACAC CTAAGGGCGG CTGGTCTGGT TCTTCTTGTA

        · C   G   Q   S   F   T   P   L   T   V   T   V   A   Y   D   K   T   K   E   I   A   A   G   G   I   V   F   K   S   K   Y   H   S
4201    CTGCGGCCAG AGCTTCACCC CTCTGACCGT GACCGTGGCC TACGACAAGA CCAAAGAGAT TGCCGCCGGA GGGATCGTGT TCAAGAGCAA GTACCACAGC
        GACGCCGGTC TCGAAGTGGG GAGACTGGCA CTGGCACCGG ATGCTGTTCT GGTTTCTCTA ACGGCGGCCT CCCTAGCACA AGTTCTCGTT CATGGTGTCG

        · H   M   E   G   A   R   T   C   R   L   S   Y   C   G   R   N ‾G ‗ I   K   F   P   N   G   E   W   V   S   L   M   L   K   L   R   S ·
4301    CACATGGAAG GCGCCAGGAC CTGCAGACTG TCCTACTGCG GCCGGAACGG CATCAAGTTC CCCAACGGCG AGTGGGTGTC CCTGATGCTG AAGCTGCGGA
        GTGTACCTTC CGCGGTCCTG GACGTCTGAC AGGATGACGC CGGCCTTGCC GTAGTTCAAG GGGTTGCCGC TCACCCACAG GGACTACGAC TTCGACGCCT

        · K   R   N   L   Y   F   P   C   L   K   M   C   P   T   G   I   R   G   E   I   Y   P   S   I   R   W   A   Q   V   L   T   S   E ·
4401    GCAAGCGGAA CCTGTACTTC CCCTGCCTGA AGATGTGCCC CACCGGCATC CGGGGCGAGA TCTACCCCAG CATCAGATGG GCCCAGGTGC TGACCAGCGA
        CGTTCGCCTT GGACATGAAG GGGACGGACT TCTACACGGG GTGGCCGTAG GCCCCGCTCT AGATGGGGTC GTAGTCTACC CGGGTCCACG ACTGGTCGCT

        · I   Q   R   I   L   D   Y   S   L   C   Q   N   T   W   D   K   V   E   R   K   E   P   L   S   P   L   D   L   S   Y   L   A   S
4501    GATCCAGAGA ATCCTGGACT ACAGCCTGTG CCAGAACACC TGGGACAAGG TGGAGCGGAA AGAGCCCCTG AGCCCCCTGG ACCTGAGCTA CCTGGCCAGC
        CTAGGTCTCT TAGGACCTGA TGTCGGACAC GGTCTTGTGG ACCCTGTTCC ACCTCGCCTT TCTCGGGGAC TCGGGGGACC TGGACTCGAT GGACCGGTCG
```

EP 2 047 861 B1

74

# FIGURE 14B

```
        K  S  P  G  K  G  L   A  Y  T   V  I  N  G   T  L  S   F  A  H   T  R  Y  V   R  M  W   I  D  G   P  V  L  K ·
        AAGTCCCCCG GCAAGGGCCT GGCCTACACC GTGATCAACG GCACCCTGAG CTTCGCCCAC ACCAGATACG TGCGGATGTG GATCGACGGC CCCGTGCTGA
        TTCAGGGGGC CGTTCCCGGA CCGGATGTGG CACTAGTTGC CGTGGGACTC GAAGCGGGTG TGGTCTATGC ACGCCTACAC CTAGCTGCCG GGGCACGACT

        ·  E  P  K   G  K  R   E  S  P  S   G  I  S   S  D  I   W  T  Q  W   F  K  Y   G  D  M   E  I  G  P   N  G  L ·
        AAGAGCCCAA GGGCAAGAGA GAGAGCCCCA GCGGCATCAG CAGCGACATC TGGACCCAGT GGTTCAAGTA CGGCGACATG GAAATCGGCC CCAACGGCCT
        TTCTCGGGTT CCCGTTCTCT CTCTCGGGGT CGCCGTAGTC GTCGCTGTAG ACCTGGGTCA CCAAGTTCAT GCCGCTGTAC CTTTAGCCGG GGTTGCCGGA

        ·  L  K  T   A  G  G  Y   K  F  P   W  H  L   I  G  M  G   I  V  D   N  E  L   H  E  L  S   E  A  N   ·P  L  D
        GCTGAAAACA GCCGGCGGAT ACAAGTTTCC TTGGCACCTG ATCGGCATGG GCATCGTGGA CAACGAGCTG CACGAGCTGT CCGAGGCCAA CCCCCTGGAT
        CGACTTTTGT CGGCCGCCTA TGTTCAAAGG AACCGTGGAC TAGCCGTACC CGTAGCACCT GTTGCTCGAC GTGCTCGACA GGCTCCGGTT GGGGGACCTA

        H  P  Q  L   P  H  A   Q  S  I   A  D  D  S   E  E  I   F  F  G   D  T  G  V   S  K  N   P  V  E   L  V  T  G ·
        CACCCCCAGC TGCCCCACGC CCAGAGCATT GCCGACGACA GCGAGGAAAT CTTCTTCGGC GACACCGGCG TGAGCAAGAA CCCCGTGGAA CTGGTGACAG
        GTGGGGGTCG ACGGGGTGCG GGTCTCGTAA CGGCTGCTGT CGCTCCTTTA GAAGAAGCCG CTGTGGCCGC ACTCGTTCTT GGGGCACCTT GACCACTGTC

        ·  W  F  T   S  W  K   S  S  I  A  S  F  E  F  I  I  G  L  I  I  S  L  F  L  V  L  R  V  G  I  H  L  C
        GCTGGTTCAC CAGCTGGAAA AGGAGCATCG CTTCATTTTT TTTCATCATC GGGCTCATCA TCGGCCTGTT TCTGGTGCTG CGCGTCGGCA TCCACCTGTG
        CGACCAAGTG GTCGACCTTT TCCTCGTAGC GAAGTAAAAA AAAGTAGTAG CCCGAGTAGT AGCCGGACAA AGACCACGAC GCGCAGCCGT AGGTGGACAC

        ·  I  K  L  K  I  T  K  K  R  Q  I  Y  T  D  I  E  M  N  R  L  G  K
        CATCAAGCTG AAGCACACCA AGAAGCGCCA GATCTATACC GACATCGAGA TGAATCGCCT GGGCAAGTAA
        GTAGTTCGAC TTCGTGTGGT TCTTCGCGGT CTAGATATGG CTGTAGCTCT ACTTAGCGGA CCCGTTCATT
```

FIGURE 14C

EP 2 047 861 B1

# Figure 15A: Fusion protein of VSV-G Cocal/indiana

## Fusion potein

```
         M  N  F  L  L    L  T  F   I  V  L   P  L  C  S   H  A  K   F  S  I   V  F  P  Q   S  Q  K   G  N  W
         ATG AACTTTCTGC TGCTGACATT CATCGTGCTG CCTCTGTGCA GCCACGCCAA GTTCAGCATC GTGTTCCCCC AGAGCCAGAA GGGCAACTGG
         TAC TTGAAAGACG ACGACTGTAA GTAGCACGAC GGAGACACGT CGGTGCGGTT CAAGTCGTAG CACAAGGGGG TCTCGGTCTT CCCGTTGACC

         K  N  V  P   S  S  Y   H  Y  C   P  S  S  S   D  Q  N   W  H  N   D  L  L  G   I  T  M   K  V  K   M  P  K  T ·
         AAGAACGTGC CCAGCAGCTA CCACTACTGC CCCAGCAGCA GCGACCAGAA CTGGCACAAC GACCTGCTGG GCATCACCAT GAAGGTGAAA ATGCCCAAGA
         TTCTTGCACG GGTCGTCGAT GGTGATGACG GGGTCGTCGT CGCTGGTCTT GACCGTGTTG CTGGACGACC CGTAGTGGTA CTTCCACTTT TACGGGTTCT

         · H  K  A   I  Q  A   D  G  W  M   C  H  A   A  K  W   I  T  T  C   D  F  R   W  Y  G   P  K  Y  I   T  H  S ·
         CCCACAAGGC CATTCAGGCT GACGGCTGGA TGTGCCACGC CGCCAAGTGG ATCACCACCT GCGACTTCCG GTGGTACGGC CCCAAGTACA TCACCCACAG
         GGGTGTTCCG GTAAGTCCGA CTGCCGACCT ACACGGTGCG GCGGTTCACC TAGTGGTGGA CGCTGAAGGC CACCATGCCG GGGTTCATGT AGTGGGTGTC

         · I  H  S   I  Q  P  T   S  E  Q   C  K  E   S  I  K  Q   T  K  Q   G  T  W   M  S  P  G   F  P  P   Q  N  C
         CATCCACTCC ATCCAGCCCA CCTCCGAGCA GTGCAAAGAG AGCATCAAGC AGACCAAGCA GGGCACCTGG ATGAGCCCCG GCTTCCCACC CCAGAACTGC
         GTAGGTGAGG TAGGTCGGGT GGAGGCTCGT CACGTTTCTC TCGTAGTTCG TCTGGTTCGT CCCGTGGACC TACTCGGGGC CGAAGGGTGG GGTCTTGACG

         G  Y  A  T   V  T  D   S  V  A   V  V  V  Q   A  T  P   H  H  V   L  V  D  E   Y  T  G   E  W  I   D  S  Q  F ·
         GGCTACGCCA CCGTGACCGA CAGCGTGGCC GTGGTGGTGC AGGCCACCCC CCACCACGTG CTGGTCGACG AGTACACCGG CGAGTGGATC GACAGCCAGT
         CCGATGCGGT GGCACTGGCT GTCGCACCGG CACCACCACG TCCGGTGGGG GGTGGTGCAC GACCAGCTGC TCATGTGGCC GCTCACCTAG CTGTCGGTCA

         · P  N  G   K  C  E   T  E  E  C   E  T  V   H  N  S   T  V  W  Y   S  D  Y   K  V  T ·G  L  C  D   A  T  L ·
         TCCCCAACGG CAAGTGCGAG ACAGAGGAAT GCGAGACAGT GCACAACAGC ACCGTGTGGT ACAGCGACTA CAAGGTGACC,GGCCTGTGCG ACGCCACCCT
         AGGGGTTGCC GTTCACGCTC TGTCTCCTTA CGCTCTGTCA CGTGTTGTCG TGGCACACCA TGTCGCTGAT GTTCCACTGG CCGGACACGC TGCGGTGGGA

         · V  D  T   E  I  T  F   F  S  E   D  G  K   K  E  S  I   G  K  P   N  T  G   Y  R  S  N   Y  F  A   Y  E  K
         GGTGGACACC GAGATCACCT TTTTCAGCGA GGACGGCAAG AAAGAGTCCA TCGGCAAGCC CAACACCGGC TACAGAAGCA ACTACTTCGC CTACGAGAAG
         CCACCTGTGG CTCTAGTGGA AAAAGTCGCT CCTGCCGTTC TTTCTCAGGT AGCCGTTCGG GTTGTGGCCG ATGTCTTCGT TGATGAAGCG GATGCTCTTC

         G  D  K  V   C  K  M   N  Y  C   K  H  A  G   V  R  L   P  S  G   V  W  F  E   F  V  D   Q  D  V   Y  A  A  A ·
         GGCGACAAAG TGTGCAAGAT GAACTACTGC AAGCATGCCG GAGTGAGGCT GCCTAGCGGC GTGTGGTTCG AGTTCGTGGA CCAGGACGTG TACGCCGCCG
         CCGCTGTTTC ACACGTTCTA CTTGATGACG TTCGTACGGC CTCACTCCGA CGGATGGCCG CACACCAAGC TCAAGCACCT GGTCCTGCAC ATGCGGCGGC

         · K  L  P   E  C  P   V  G  A  T   I  S  A   P  T  Q   T  S  V  D   V  S  L   I  L  D   V  E  R  I   L  D  Y ·
         CCAAGCTGCC CGAGTGCCCC GTGGGCGCCA CCATCAGCGC CCCCACCCAG ACCAGCGTGG ACGTGAGCCT GATCCTGGAC GTGGAGAGAA TCCTGGACTA
         GGTTCGACGG GCTCACGGGG CACCCGCGGT GGTAGTCGCG GGGGTGGGTC TGGTCGCACC TGCACTCGGA CTAGGACCTG CACCTCTCTT AGGACCTGAT

         · S  L  C   Q  E  T  W   S  K  I   R  S  K   Q  P  V  S   P  V  D   L  S  Y   L  A  P  K   N  P  G   T  G  P
         CTCTCTGTGT CAGGAAACCT GGTCCAAGAT CAGATCCAAG CAGCCCGTGA GCCCTGTGGA CCTGAGCTAC CTGGCCCCTA AGAACCCCGG CACCGGCCCT
         GAGAGACACA GTCCTTTGGA CCAGGTTCTA GTCTAGGTTC GTCGGGCACT CGGGACACCT GGACTCGATG GACCGGGGAT TCTTGGGGCC GTGGCCGGGA
```

EP 2 047 861 B1

## FIGURE 15B

```
       A  F  T  I     I  N  G     T  L  K     Y  F  E  T     R  Y  I     R  I  D     I  D  N  P     I  I  S     K  M  V     G  K  I  S  ·
       GCCTTCACCA TCATCAACGG CACCCTGAAG TACTTCGAGA CACGGTACAT CCGGATCGAC ATCGACAACC CCATCATCAG CAAGATGGTG GGCAAGATCA
       CGGAAGTGGT AGTAGTTGCC GTGGGACTTC ATGAAGCTCT GTGCCATGTA GGCCTAGCTG TAGCTGTTGG GGTAGTAGTC GTTCTACCAC CCGTTCTAGT

       ·  G  S  Q     T  E  R     E  L  W  T     E  W  F     P  Y  E     G  V. E  I     G  P  N     G  I  L     K  T  P  T     G  Y  K ·
       GCGGCAGCCA GACCGAGCGG GAGCTGTGGA CCGAGTGGTT CCCCTACGAG GGCGTGGAGA TCGGCCCCAA TGGCATCCTG AAAACCCCTA CCGGCTACAA
       CGCCGTCGGT CTGGCTCGCC CTCGACACCT GGCTCACCAA GGGGATGCTC CCGCACCTCT AGCCGGGGTT ACCGTAGGAC TTTTGGGGAT GGCCGATGTT

       · F  P  L     F  M  I  G     H  G  M     L  D  S     D  L  H  K     T  S  Q     A  E  V     F  E  H  P     H  L  A     E  A  P
       GTTCCCCCTG TTCATGATCG GCCACGGCAT GCTGGACAGC GACCTGCACA AGACCTCCCA GGCCGAGGTG TTCGAGCACC CCCACCTGGC CGAGGCCCCC
       CAAGGGGGAC AAGTACTAGC CGGTGCCGTA CGACCTGTCG CTGGACGTGT TCTGGAGGGT CCGGCTCCAC AAGCTCGTGG GGGTGGACCG GCTCCGGGGG

       K  Q  L  P     E  E  E     T  L  F     F  G  D  T     G  I  S     K  N  P     V  E  L  I     E  G  W     F  S  S     W  K  S  S ·
       AAGCAGCTGC CCGAAGAGGA AACCCTGTTC TTCGGCGACA CCGGCATCTC CAAGAACCCT GTGGAGCTGA TCGAGGGCTG GTTCAGCAGC TGGAAGAGCA
       TTCGTCGACG GGCTTCTCCT TTGGGACAAG AAGCCGCTGT GGCCGTAGAG GTTCTTGGGA CACCTCGACT AGCTCCCGAC CAAGTCGTCG ACCTTCTCGT

       · L  A  S     F  I  F     L  I  G  L     I  I  G     L  F  L     V  L  R  V     G  I  H     L  C     I  K  L  K  H  T  K  K
       GCATCGCTTC ATTTTTTTC ATCATCGGCC TCATCATCGG GCTGTTCCTG GTGCTGCGCG TGGGCATCCA CCTGTGCATC AAGCTCAAGC ACACCAAGAA
       CGTAGCGAAG TAAAAAAAG TAGTAGCCGG AGTAGTAGCC CGACAAGGAC CACGACGCGC ACCCGTAGGT GGACACGTAG TTCGAGTTCG TGTGGTTCTT

       · R  Q  I     Q  T  A     D  I  A  E  M     N  R  L  G  K ·
       GCGCCAGATC TATAGCCGCA TGGACATCAA TGCCCTGGGG AAGTAA
       CGCGGTCTAG ATATCGGCGT AGCTGTAGTT ACGGGACCCC TTCATT
```

**FIGURE 15C**

## Figure 16A

Fusion potein

```
        M   T   D   T   V   L   G   K   F   Q   I   V   F   P   D   Q   N   E   L   E   W   T   P   V   V   G   D   S   R   H   C
      ATG ACCGATACAG TGCTGGGCAA GTTCCAGATC GTGTTCCCCG ACCAGAACGA GCTGGAATGG ACCCCCGTCG TGGGCGACAG CCGGCATTGC
      TAC TGGCTATGTC ACGACCCGTT CAAGGTCTAG CACAAGGGGC TGGTCTTGCT CGACCTTACC TGGGGGCAGC ACCCGCTGTC GGCCGTAACG

        P   Q   S   S   E   M   Q   F   D   G   S   R   S   Q   T   I   L   T   G   K   A   P   V   G   I   T   P   S   K   S   D   G   F   I ·
      CCTCAGTCCA GCGAGATGCA GTTCGACGGC AGCAGAAGCC AGACCATCCT GACCGGCAAG GCCCCCGTGG GCATCACACC CAGCAAGAGC GACGGCTTCA
      GGAGTCAGGT CGCTCTACGT CAAGCTGCCG TCGTCTTCGG TCTGGTAGGA CTGGCCGTTC CGGGGGCACC CGTAGTGTGG GTCGTTCTCG CTGCCGAAGT

      · C   H   A   A   K   W   V   T   T   C   D   F   R   W   Y   G   P   K   Y   I   T   H   S   I   H   H   L   R   P   T   T   S   D ·
      TCTGCCACGC CGCCAAGTGG GTGACCACCT GCGACTTCCG GTGGTACGGC CCCAAGTACA TCACCCACAG CATCCACCAC CTGCGGCCCA CCACCTCCGA
      AGACGGTGCG GCGGTTCACC CACTGGTGGA CGCTGAAGGC CACCATGCCG GGGTTCATGT AGTGGGTGTC GTAGGTGGTG GACGCCGGGT GGTGGAGGCT

      · C   E   T   A   L   Q   R   Y   K   D   G   S   L   I   N   L   G   F   P   P   E   S   C   G   Y   A   T   V   T   D   S   E   A
      CTGCGAGACA GCCCTGCAGC GGTACAAGGA CGGCAGCCTG ATCAACCTGG GCTTCCCTCC CGAGAGCTGC GGCTACGCCA CCGTGACAGA CAGCGAGGCC
      GACGCTCTGT CGGGACGTCG CCATGTTCCT GCCGTCGGAC TAGTTGGACC CGAAGGGAGG GCTCTCGACG CCGATGCGGT GGCACTGTCT GTCGCTCCGG

        M   L   V   Q   V   T   P   H   H   V   G   V   D   D   Y   R   G   H   W   I   D   P   L   F   P   G   G   E   C   S   T   N   F   C ·
      ATGCTGGTGC AGGTGACCCC CCACCACGTG GGCGTGGACG ACTACCGGGG CCACTGGATC GACCCCCTGT TCCCTGGCGG CGAGTGCAGC ACCAATTTCT
      TACGACCACG TCCACTGGGG GGTGGTGCAC CCGCACCTGC TGATGGCCCC GGTGACCTAG CTGGGGGACA AGGGACCGCC GCTCACGTCG TGGTTAAAGA

      · D   T   V   H   N   S   S   V   W   I   P   K   S   Q   K   T   D   I   C   A   Q   S   F   K   N   I   K   M   T   A   S   Y   P ·
      GCGATACCGT GCACAACAGC AGCGTGTGGA TTCCCAAGAG CCAGAAAACC GACATCTGCG CCCAGAGCTT CAAGAACATC AAGATGACCG CCAGCTACCC
      CGCTATGGCA CGTGTTGTCG TCGCACACCT AAGGGTTCTC GGTCTTTTGG CTGTAGACGC GGGTCTCGAA GTTCTTGTAG TTCTACTGGC GGTCGATGGG

      · S   E   G   A   L   V   S   D   R   F   A   F   H   S   A   Y   H   P   N   M   P   G   S   T   V   C   I   M   D   F   C   E   Q
      CAGCGAGGGA GCCCTGGTGT CCGACCGGTT CGCCTTCCAC AGCGCCTACC ACCCCAACAT GCCCGGCAGC ACCGTGTGCA TCATGGATTT CTGCGAGCAG
      GTCGCTCCCT CGGGACCACA GGCTGGCCAA GCGGAAGGTG TCGCGGATGG TGGGGTTGTA CGGGCCGTCG TGGCACACGT AGTACCTAAA GACGCTCGTC

        K   G   L   R   F   T   N   G   E   W   M   G   L   N   V   E   Q   S   I   R   E   K   K   I   S   A   I   F   P   N   C   V   A   G ·
      AAGGGCCTGC GGTTCACCAA CGGCGAGTGG ATGGGCCTGA ACGTGGAGCA GAGCATCCGG GAGAAGAAGA TCAGCGCCAT CTTCCCCAAC TGCGTGGCCG
      TTCCCGGACG CCAAGTGGTT GCCGCTCACC TACCCGGACT TGCACCTCGT CTCGTAGGCC CTCTTCTTCT AGTCGCGGTA GAAGGGGTTG ACGCACCGGC

      · T   E   I   R   A   T   L   E   S   E   G   A   R   T   L   T   W   E   T   Q   R   M   L   D   Y   S   L   C   Q   N   T   W   D ·
      GCACCGAGAT CCGGGCCACC CTGGAATCCG AGGGCGCCAG GACCCTGACC TGGGAGACAC AGCGGATGCT GGACTACAGC CTGTGCCAGA ACACCTGGGA
      CGTGGCTCTA GGCCCGGTGG GACCTTAGGC TCCCGCGGTC CTGGGACTGG ACCCTCTGTG TCGCCTACGA CCTGATGTCG GACACGGTCT TGTGGACCCT
```

EP 2 047 861 B1

# FIGURE 16B

EP 2 047 861 B1

```
        ·K  V  S    R  K  E  P    L  S  P    L  D  L    S  Y  L  S    P  R̆ A      P  G  K    G  M  A  Y    T  V  I     N  G  T
        CAAGGTGTCC  CGGAAAGAGC  CTCTGTCCCC  CCTGGACCTG  AGCTACCTGA  GCCCTAGAGC  CCCTGGCAAG  GGCATGGCCT  ACACCGTGAT  CAACGGCACC
        GTTCCACAGG  GCCTTTCTCG  GAGACAGGGG  GGACCTGGAC  TCGATGGACT  CGGGATCTCG  GGGACCGTTC  CCGTACCGGA  TGTGGCACTA  GTTGCCGTGG

        L  H  S  A    H  A  K    Y  I  R    T  W  I  D    Y  G  E    M  K  E    I  K  G  G    R  G  E    Y  S  K    A  P  E  L ·
        CTGCACAGCG  CCCACGCCAA  GTATATCCGG  ACCTGGATCG  ACTACGGCGA  GATGAAAGAG  ATCAAGGGCG  GCAGGGGCGA  GTACAGCAAG  GCCCCTGAGC
        GACGTGTCGC  GGGTGCGGTT  CATATAGGCC  TGGACCTAGC  TGATGCCGCT  CTACTTTCTC  TAGTTCCCGC  CGTCCCCGCT  CATGTCGTTC  CGGGGACTCG

        ·L  W  S    Q  W  F    D  F  G  P    F  K  I    G  P  N    G  L  L  H    T  G  K    T  F  K    F  P  L  Y    L  I  G ·
        TGCTGTGGAG  CCAGTGGTTC  GACTTCGGCC  CCTTCAAGAT  CGGCCCCAAC  GGCCTGCTGC  ACACCGGCAA  GACCTTCAAG  TTCCCTCTGT  ATCTGATCGG
        ACGACACCTC  GGTCACCAAG  CTGAAGCCGG  GGAAGTTCTA  GCCGGGGTTG  CCGGACGACG  TGTGGCCGTT  CTGGAAGTTC  AAGGGAGACA  TAGACTAGCC

        ·A  G  I    I  D  E  D    L  H  E    L  D  E    A  A  P  I    D  H  P    Q  M  P    D  A  K  S    V  L  P    E  D  E
        AGCCGGCATC  ATCGACGAGG  ACCTGCACGA  GCTGGACGAA  GCCGCCCCTA  TCGACCACCC  CCAGATGCCC  GACGCCAAGA  GCGTGCTGCC  CGAGGACGAG
        TCGGCCGTAG  TAGCTGCTCC  TGGACGTGCT  CGACCTGCTT  CGGCGGGGAT  AGCTGGTGGG  GGTCTACGGG  CTGCGGTTCT  CGCACGACGG  GCTCCTGCTC

        E  I  F  F    G  D  T    G  V  S    K  N  P  I    E  L  I    Q  G  W    F  S  N  W    R  S̲ S̲ ̲L̲ ̲A̲ ̲S̲ ̲F̲ ̲F̲ ̲I̲ ·
        GAAATCTTCT  TCGGCGACAC  CGGCGTGAGC  AAGAACCCCA  TCGAGCTGAT  CCAGGGCTGG  TTCAGCAACT  GGCGA̲G̲C̲A̲G̲ ̲C̲A̲T̲C̲G̲C̲T̲T̲C̲A̲ ̲T̲T̲T̲T̲T̲T̲T̲G̲C̲
        CTTTAGAAGA  AGCCGCTGTG  GCCGCACTCG  TTCTTGGGGT  AGCTCGACTA  GGTCCCGACC  AAGTCGTTGA  CCGCC̲T̲C̲G̲T̲C̲ ̲G̲T̲A̲G̲C̲G̲A̲A̲G̲T̲ ̲A̲A̲A̲A̲A̲A̲A̲G̲T̲

        ̲L̲ ̲G̲ ̲I̲ ̲ ̲ ̲I̲ ̲I̲ ̲G̲ ̲ ̲ ̲L̲ ̲F̲ ̲L̲ ̲V̲ ̲ ̲ ̲L̲ ̲R̲ ̲V̲ ̲ ̲ ̲G̲ ̲I̲ ̲H̲ ̲ ̲ ̲L̲ ̲G̲ ̲L̲ ̲K̲ ̲ ̲ ̲L̲ ̲K̲ ̲H̲ ̲ ̲ ̲T̲ ̲K̲ ̲K̲ ̲ ̲ ̲R̲ ̲Q̲ ̲L̲ ̲ ̲ ̲Y̲ ̲I̲ ̲D̲ ̲I̲ ·
        ̲T̲C̲A̲T̲C̲G̲G̲C̲T̲ ̲C̲A̲T̲C̲A̲T̲G̲G̲G̲C̲ ̲C̲T̲G̲T̲T̲T̲C̲T̲C̲G̲ ̲T̲C̲C̲T̲G̲C̲G̲G̲C̲T̲ ̲C̲G̲G̲C̲A̲T̲C̲C̲A̲C̲ ̲C̲T̲G̲T̲C̲C̲A̲T̲C̲A̲ ̲A̲G̲C̲T̲G̲A̲A̲G̲C̲A̲ ̲C̲A̲C̲C̲A̲A̲G̲A̲A̲G̲ ̲C̲G̲G̲C̲A̲G̲T̲C̲T̲ ̲A̲T̲A̲G̲G̲C̲A̲G̲A̲T̲
        ̲A̲G̲T̲A̲G̲C̲C̲G̲A̲ ̲G̲T̲A̲G̲T̲A̲C̲C̲C̲ ̲G̲A̲C̲A̲A̲A̲G̲A̲C̲ ̲A̲G̲G̲A̲C̲G̲C̲C̲G̲A̲ ̲G̲C̲C̲G̲T̲A̲G̲G̲T̲G̲ ̲G̲A̲C̲A̲C̲G̲T̲A̲G̲T̲ ̲T̲C̲G̲A̲C̲T̲T̲C̲G̲T̲ ̲G̲T̲G̲G̲T̲T̲C̲T̲T̲C̲ ̲G̲C̲C̲G̲T̲C̲T̲G̲A̲ ̲T̲A̲T̲C̲C̲G̲T̲C̲T̲A̲

        ̲E̲ ̲M̲ ̲N̲ ̲ ̲ ̲R̲ ̲L̲ ̲G̲ ̲K̲ ̲*̲
        ̲G̲G̲A̲C̲A̲T̲G̲A̲A̲T̲ ̲C̲G̲C̲C̲T̲G̲G̲G̲G̲A̲ ̲A̲G̲T̲A̲A̲
        ̲G̲C̲T̲G̲T̲A̲C̲T̲T̲A̲ ̲G̲C̲G̲G̲A̲C̲C̲C̲C̲T̲ ̲T̲C̲A̲T̲T̲
```

*EcoRI* (1)

*BglII* (5086)      *Not*I (28)

*Bs*/EI (4627)

                                        *Xho*I (34)

**VSV.G Piry/Indiana CO**                    **WPRE/EATG**

*Bs*EII (4013)                          *Xho*I (644)

*Bs*EII (3821)                          *Xba*I (650)

*Bam*HI (3591)                          **BGH pA**

*Kpn*I (3583)      **pThV-VSV.G (PIRY/IND)CO**

*Hin*dIII (3573)              5126 bp

*Nhe*I (3557)

                                        **KanR**
**CMV**
                                        *Nco*I (1644)
*Nco*I (3272)

*Nde*I (3146)

                    **pUC ORI**

FIGURE 16C

# Figure 17A': Fusion protein of VSV-G Isfahan/indiana

## Fusion potein

```
        M   T   S   V   L   F   M   V   G   V   L   L   G   A   F   G   S   T   H   C   S   I   Q   I   V   F   P   S   E   T   K
3601   ATG ACATCCGTGC TGTTTATGGT GGGCGTGCTG CTCGGAGCTT TCGGATCCAC CCATTGCAGC ATCCAGATCG TGTTCCCCAG CGAGACAAAG
       TAC TGTAGGCACG ACAAAATACCA CCCGCACGAC GAGCCTCGAA AGCCTAGGTG GGTGACGTCG TAGGTCTAGC ACAAGGGGTC GCTCTGTTTC

        L   V   W   K   P   V   L   K   G   T   R   Y   C   P   Q   S   A   E   L   N   L   E   P   D   L   K   T   M   A   F   D   S   K   V .
3701   CTGGTGTGGA AGCCCGTGCT GAAGGGCACC CGGTACTGCC CCCAGAGCGC CGAGCTGAAC CTGGAACCCG ACCTGAAAAC CATGGCCTTC GACAGCAAGG
       GACCACACCT TCGGGCACGA CTTCCCGTGG GCCATGACGG GGGTCTCGCG GCTCGACTTG GACCTTGGGC TGGACTTTTG GTACCGGAAG CTGTCGTTCC

      . P   I   G   I   T   P   S   N   S   D   G   Y   L   C   H   A   A   K   W   V   T   T   C   D   F   R   W   Y   G   P   K   Y   I .
3801   TGCCCATCGG CATCACCCCC AGCAACAGCG ACGGCTACCT GTGCCACGCC GCCAAGTGGG TGACCACCTG CGACTTCCGG TGGTACGGCC CCAAGTACAT
       ACGGGTAGCC GTAGTGGGGG TCGTTGTCGC TGCCGATGGA CACGGTGCGG CGGTTCACCC ACTGGTGGAC GCTGAAGGCC ACCATGCCGG GGTTCATGTA

      . T   H   S   V   H   S   L   R   P   T   V   S   D   C   K   A   A   V   E   A   Y   N   A   G   T   L   M   Y   P   G   F   P   P
3901   CACCCACAGC GTGCACAGCC TGCGGCCCAC CGTGAGCGAC TGCAAGGCCG CCGTGGAAGC CTACAACGCT GGCACCCTGA TGTACCCCGG CTTCCCCCC
       GTGGGTGTCG CACGTGTCGG ACGCCGGGTG GCACTCGCTG ACGTTCCGGC GGCACCTTCG GATGTTGCGA CCGTGGGACT ACATGGGGCC GAAGGGGGGG

      . E   S   C   G   Y   A   S   I   T   D   S   E   F   Y   V   M   L   V   T   P   H   P   V   G   V   D   D   Y   R   G   H   W   V   D .
4001   GAGAGCTGCG GCTACGCCAG CATCACCGAC AGCGAGTTCT ACGTGATGCT GGTGACCCCC CACCCCGTGG GAGTGGACGA CTACCGCGGC CACTGGGTGG
       CTCTCGACGC CGATGCGGTC GTAGTGGCTG TCGCTCAAGA TGCACTACGA CCACTGGGGG GTGGGGCACC CTCACCTGCT GATGGCGCCG GTGACCCACC

      . P   L   F   P   T   S   E   C   N   S   N   F   C   E   T   V   H   N   A   T   M   W   I   P   K   D   L   K   T   H   D   V   C .
4101   ACCCTCTGTT CCCCACCTCC GAGTGCAACA GCAACTTCTG CGAGACAGTG CACAACGCCA CCATGTGGAT TCCCAAGGAT CTGAAAACCC ACGACGTGTG
       TGGGAGACAA GGGGTGGAGG CTCACGTTGT CGTTGAAGAC GCTCTGTCAC GTGTTGCGGT GGTACACCTA AGGGTTCCTA GACTTTTGGG TGCTGCACAC

      . S   Q   D   F   Q   T   I   R   V   S   V   M   Y   P   Q   T   K   G   A   D   L   T   L   K   S   K   F   H   A   H
4201   CAGCCAGGAC TTCCAGACCA TCAGAGTGAG CGTGATGTAC CCTCAGACCA AGGGCGCTGA CCTGACACTGA AGAGCAAGTT CCACGCCCAC
       GTCGGTCCTG AAGGTCTGGT AGTCTCACTC GCACTACATG GGAGTCTGGT TCCCGCGACT GGACTGTGACT TCTCGTTCAA GGTGCGGGTG

        M   K   G   D   R   V   C   K   M   K   F   C   N   K   N   G   L   R   L   G   N   G   E   W   I   E   V   G   D   E   V   M   L   D
4301   ATGAAGGGCG ACAGAGTGTG CAAGATGAAG TTCTGCAACA AGAACGGCCT GCGGCTGGGC AACGGCGAGT GGATCGAAGT GGGCGACGAG GTGATGCTGG
       TACTTCCCGC TGTCTCACAC GTTCTACTTC AAGACGTTGT TCTTGCCGGA CGCCGACCCG TTGCCGCTCA CCTAGCTTCA CCCGCTGCTC CACTACGACC

      . N   S   K   L   L   S   L   F   P   D   C   L   V   G   S   V   V   K   S   T   L   L   S   E   G   V   Q   T   A   L   W   E   T .
4401   ACAACAGCAA GCTGCTGTCC CTGTTCCCCG ACTGCCTGGT GGGCAGCGTG GTGAAGAGCA CCCTGCTGTC CGAGGGCGTG CAGACCGCCC TGTGGGAGAC
       TGTTGTCGTT CGACGACAGG GACAAGGGGC TGACGGACCA CCCGTCGCAC CACTTCTCGT GGGACGACAG GCTCCCGCAC GTCTGGCGGG ACACCCTCTG

      . D   R   L   L   D   Y   S   L   C   Q   N   T   W   E   K   I   D   R   K   E   P   L   S   A   V   D   L   S   Y   L   A   P   R
4501   AGACCGGCTG CTGGACTACA GCCTGTGCCA GAACACCTGG GAGAAGATCG ACAGAAAAGA ACCGGAAAGA GCCCCTGAGC GCCGTCGACC TGAGCTACCT GGCCCCTAGA
       TCTGGCCGAC GACCTGATGT CGGACACGGT CTTGTGGACC CTCTTCTAGC TGTCTTTTCT TGGCCTTTCT CGGGGACTCG CGGCAGCTGG ACTCGATGGA CCGGGGATCT
```

83

FIGURE 17B

```
          S   P   G   K   G   M   A   Y   I   V   A   N   G   S   L   M   S   A   P   A   R   Y   I   R   V   W   I   D   S   P   I   L   K   E ·
4601  AGCCCCGGCA AGGGCATGGC CTACATCGTG GCCAACGGCA GCCTGATGAG CGCCCCTGCC CGGTACATCA GAGTGTGGAT CGACAGCCCC ATCCTGAAAG
      TCGGGGCCGT TCCCGTACCG GATGTAGCAC CGGTTGCCGT CGGACTACTC GCGGGGACGG GCCATGTAGT CTCACACCTA GCTGTCGGGG TAGGACTTTC

          · I   K   G   K   K   E   S   A   S   G   I   D   T   V   L   W   E   Q   W   L   P   F   N   G   M   E   L   G   P   N   G   L   I ·
4701  AGATCAAGGG CAAGAAAGAG AGCGCCAGCG GCATCGACAC CGTGCTGTGG GAGCAGTGGC TGCCCTTCAA CGGCATGGAA CTGGGCCCCA ACGGCCTGAT
      TCTAGTTCCC GTTCTTTCTC TCGCGGTCGC CGTAGCTGTG GCACGACACC CTCGTCACCG ACGGGAAGTT GCCGTACCTT GACCCGGGGT TGCCGGACTA

          · K   T   K   S   G   Y   K   F   P   L   Y   L   L   G   M   G   I   V   D   Q   D   L   Q   E   L   S   S   V   N   P   V   D   H
4801  CAAGACCAAG AGCGGCTACA AGTTCCCCCT GTACCTGCTG GGCATGGGCA TCGTGGACCA GGACCTGCAG GAACTGAGCA GCGTCAACCC CGTGGACCAC
      GTTCTGGTTC TCGCCGATGT TCAAGGGGGA CATGGACGAC CCGTACCCGT AGCACCTGGT CCTGGACGTC CTTGACTCGT CGCAGTTGGG GCACCTGGTG

          P   H   V   P   I   A   Q   A   F   V   S   E   G   E   E   V   F   F   G   D   T   G   V   S   K   N   P   I   E   L   I   S   G   W ·
4901  CCCCACGTGC CTATCGCCCA GGCCTTCGTG AGCGAGGGCG AGGAAGTGTT CTTCGGCGAC ACCGGCGTGA GCAAGAACCC CATCGAGCTG ATCAGCGGCT
      GGGGTGCACG GATAGCGGGT CCGGAAGCAC TCGCTCCCGC TCCTTCACAA GAAGCCGCTG TGGCCGCACT CGTTCTTGGG GTAGCTCGAC TAGTCGCCGA

          · F   S   D   W   K   ▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓
5001  GGTTCAGCGA CTGGAAA▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓
      CCAAGTCGCT GACCTTT▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓

           ▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓
5101  ▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓
      ▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓
```

EP 2 047 861 B1

FIGURE 17C

## Figure 18A Fusion protein of VSV-G SVCV/indiana

## Fusion potein

```
              M   S I I S   Y I A   F L L   L I D S   T L G   I P I   F V P S   G Q N   I S W
     ATG AGCATCATCA GCTATATCGC CTTTCTGCTG CTGATCGACA GCACCCTGGG CATCCCCATC TTCGTGCCCA GCGGCCAGAA CATCAGCTGG
     TAC TCGTAGTAGT CGATATAGCG GAAAGACGAC GACTAGCTGT CGTGGGACCC GTAGGGGTAG AAGCACGGGT CGCCGGTCTT GTAGTCGACC ·

              Q P V I   Q P F   D Y Q   C P I H   G N L   P N T   M G L S   A T K   L T I   K S P S ·
     CAGCCCGTGA TCCAGCCCTT CGACTACCAG TGCCCCATCC ACGGCAACCT GCCCAACACC ATGGGCCTGA GCGCCACCAA GCTGACCATC AAGAGCCCCA
     GTCGGGCACT AGGTCGGGAA GCTGATGGTC ACGGGGTAGG TGCCGTTGGA CGGGTTGTGG TACCCGGACT CGCGGTGGTT CGACTGGTAG TTCTCGGGGT

              · V F S   T D K   V S G W   I C H   A A E   W K T T   C D Y   R W Y   G P Q Y   I T H ·
     GCGTGTTCAG CACCGACAAG GTGTCCGGCT GGATCTGCCA CGCCGCCGAG TGGAAAACCA CCTGCGACTA CCGGTGGTAC GGCCCCCAGT ACATCACCCA
     CGCACAAGTC GTGGCTGTTC CACAGGCCGA CCTAGACGGT GCGGCGGCTC ACCTTTTGGT GGACGCTGAT GGCCACCATG CCGGGGGTCA TGTAGTGGGT

              · S I H   P I S P   T I D   E C K   R I I S   R I A   S G T   D E D L   G F P   P Q S
     CAGCATCCAC CCCATCAGCC CCACCATCGA CGAGTGCAAG CGGATCATCA GCCGGATCGC CAGCGGCACC GACGAGGACC TGGGCTTCCC ACCCCAGAGC
     GTCGTAGGTG GGGTAGTCGG GGTGGTAGCT GCTCACGTTC GCCTAGTAGT CGGCCTAGCG GTCGCCGTGG CTGCTCCTGG ACCCGAAGGG TGGGGTCTCG

              C G W A   S V T   T V S   N T N Y   K V V   P H S   V H L E   P Y G   G H W   I D H D ·
     TGCGGCTGGG CCAGCGTGAC CACCGTGAGC AACACCAACT ACAAGGTGGT GCCCCACAGC GTGCACCTGG AACCCTACGG CGGCCACTGG ATCGACCACG
     ACGCCGACCC GGTCGCACTG GTGGCACTCG TTGTGGTTGA TGTTCCACCA CGGGGTGTCG CACGTGGACC TTGGGATGCC GCCGGTGACC TAGCTGGTGC

              · F N G   G E C   R E K V   C E M   K G N   H S I W   I T D   E T V   Q H E C   E K H ·
     ACTTCAACGG CGGCGAGTGC CGGGAGAAAG TGTGCGAGAT GAAGGGCAAC CACAGCATCT GGATCACCGA CGAGACAGTG CAGCACGAGT GCGAGAAGCA
     TGAAGTTGCC GCCGCTCACG GCCCTCTTTC ACACGCTCTA CTTCCCGTTG GTGTCGTAGA CCTAGTGGCT GCTCTGTCAC GTCGTGCTCA CGCTCTTCGT

              · I E E   V E G I   M Y G   N A P   R G D A   I Y I   N N F   I ' I D K   H H R   V Y R
     CATCGAGGAA GTGGAGGGCA TCATGTACGG CAACGCCCCC AGGGGCGACG CCATCTACAT CAACAACTTC ATCATCGACA AGCACCACCG GGTGTACCGG
     GTAGCTCCTT CACCTCCCGT AGTACATGCC GTTGCGGGGG TCCCCGCTGC GGTAGATGTA GTTGTTGAAG TAGTAGCTGT TCGTGGTGGC CCACATGGCC

              F G G S   C R M   K F C   N K D G   I K F   T R G   D W V E   K T A   G T L   T ·N I Y ·
     TTCGGCGGCT CCTGCCGGAT GAAGTTCTGC AACAAGGACG GCATCAAGTT CACCAGAGGC GACTGGGTGG AGAAAACCGC CGGCACCCTG ACCAACATCT
     AAGCCGCCGA GGACGGCCTA CTTCAAGACG TTGTTCCTGC CGTAGTTCAA GTGGTCTCCG CTGACCCACC TCTTTTGGCG GCCGTGGGAC TGGTTGTAGA

              · E N I   P E C   A D G T   L V S   G H R   P ·G L D   L I D   T V F   N L E N   V V E ·
     ACGAGAACAT CCCCGAGTGC GCCGACGGCA CACTGGTGTC CGGCCACAGA CCCGGCCTGG ACCTGATCGA CACCGTGTTC AACCTGGAAA ACGTGGTGGA
     TGCTCTTGTA GGGGCTCACG CGGCTGCCGT GTGACCACAG GCCGGTGTCT GGGCCGGACC TGGACTAGCT GTGGCACAAG TTGGACCTTT TGCACCACCT

              ·Y T L   C E G T   K R K   I N K   Q E K L   T S V   D L S   Y L A P   R I G   G F G
     GTACACCCTG TGCGAGGGCA CCAAGCGGAA GATCAACAAG CAGGAAAAGC TGACCAGCGT CGACCTGAGC TACCTGGCCC CCAGGATCGG CGGCTTCGGC
     CATGTGGGAC ACGCTCCCGT GGTTCGCCTT CTAGTTGTTC GTCCTTTTCG ACTGGTCGCA GCTGGACTCG ATGGACCGGG GGTCCTAGCC GCCGAAGCCG
```

EP 2 047 861 B1

## FIGURE 18B

```
       S  V  F  R   V  R  N   G  T  L   E  R  G  S   T  T  Y   I  R  I   E  V  E  G   P  V  V   D S  L   N  G  I  D ·
4601   AGCGTGTTCC GCGTGCGGAA TGGGACCCTG GAAAGAGGAA GCACAACATA CATTCGGATC GAAGTGGAAG GCCCCGTGGT GGACAGCCTG AACGGCATCG
       TCGCACAAGG CGCACGCCTT ACCCTGGGAC CTTTCTCCTT CGTGTTGTAT GTAAGCCTAG CTTCACCTTC CGGGGCACCA CCTGTCGGAC TTGCCGTAGC

       · P  R  T   N  A  S   R  V  F  W   D  D  W   E  L  D   G  N  I  Y   Q  G  F   N  G  V   Y  K  G  K   D  G  K ·
4701   ACCCCCGGAC CAACGCCAGC CGGGTGTTCT GGGACGACTG GGAGCTGGAC GGCAACATCT ACCAGGGCTT CAATGGCGTG TACAAGGGCA AGGATGGCAA
       TGGGGGCCTG GTTGCGGTCG GCCCACAAGA CCCTGCTGAC CCTCGACCTG CCGTTGTAGA TGGTCCCGAA GTTACCGCAC ATGTTCCCGT TCCTACCGTT

       · I  H  I   P  L  N  M   I  E  S   G  I  I   D  D  E  L   Q  H  A   F  Q  A   D  I  I  P   H  P  H   Y  D  D
4801   GATCCACATC CCCCTGAACA TGATCGAGAG CGGCATCATC GACGACGAGC TGCAGCACGC CTTCCAGGCC GACATCATCC CCCACCCCCA CTACGACGAC
       CTAGGTGTAG GGGGACTTGT ACTAGCTCTC GCCGTAGTAG CTGCTGCTCG ACGTCGTGCG GAAGGTCCGG CTGTAGTAGG GGGTGGGGGT GATGCTGCTG

       D  E  I  R   E  D  D   I  F  F   D  N  T  G   E  N  G   N  P  V   D  A  V  V   E  W  V   S  G  W   G  S S  I ·
4901   GACGAGATCC GGGAGGACGA CATCTTCTTC GACAACACCG GCGAGAACGG CAACCCCGTG GACGCCGTGG TGGAATGGGT GTCCGGATGG GGCAGCAGCA
       CTGCTCTAGG CCCTCCTGCT GTAGAAGAAG CTGTTGTGGC CGCTCTTGCC GTTGGGGCAC CTGCGGCACC ACCTTACCCA CAGGCCTACC CCGTCGTCGT

       · A  S  F   F  F  I   I  G  L   F  I  G  L   F  L  V   L  R  V  G   I  H  L   C  I  K   L  K  H  T   K  K ·
5001   TGCCTTCATT TTTTTTCATC ATCGGGCTCA TCATCGGGCT GTTTCTGGTG CTGCGCGTGG GCATCCACCT GTGCATCAAG CTGAAGCACA CCAAGAAGCC
       ACGGAAGTAA AAAAAAGTAG TAGCCCGAGT AGTAGCCCGA CAAAGACCAC GACGCGCACC CGTAGGTGGA CACGTAGTTC GACTTCGTGT GGTTCTTCGG

       · Q  I  Y   I  D  I  E  M  N  R   L  G  K ·
5101   CCACATCTAT ACCGACATCG AGATGAATCG CCTCGGCAAG TAT
       GGTGTAGATA TGGCTGTAGC TCTACTTAGC GGAGCCGTTC ATA
```

**FIGURE 18C**

## Figure 19A : Fusion protein of VSV-G NewJersey/indiana

Fusion potein

EP 2 047 861 B1

```
        M   L   S   Y   L   I   F   A   L   A   V   S   P   I   L   G   K   I   E   I   V   F   P   Q   H   T   T   G   D   W   K
       ATG CTGTCATATC TGATCTTTGC CCTGGCTGTG AGCCCAATCC TCGGAAAGAT CGAAATCGTG TTCCCACAAC ACACCACAGG GGACTGGAAG
       TAC GACAGTATAG ACTAGAAACG GGACCGACAC TCGGGTTAGG AGCCTTTCTA GCTTTAGCAC AAGGGTGTTG TGTGGTGTCC CCTGACCTTC

        R   V   P   H   E   Y   N   Y   C   P   T   S   A   D   K   N   S   H   G   T   Q   T   G   I   P   V   E   L   T   M   P   K   G   L .
       CGCGTGCCCC ACGAGTACAA CTACTGCCCG ACCTCAGCCG ACAAGAATAG CCACGGCACG CAGACCGGCA TCCCTGTGGA GCTGACCATG CCCAAGGGGC
       GCGCACGGGG TGCTCATGTT GATGACGGGC TGGAGTCGGC TGTTCTTATC GGTGCCGTGC GTCTGGCCGT AGGGACACCT CGACTGGTAC GGGTTCCCCG

        · T   T   H   Q   V   E   G   F   M   C   H   S   A   L   W   M   T   T   C   D   F   R   W   Y   G   P   K   Y   I   T   H   S   I .
       TCACAACGCA CCAAGTCGAA GGCTTCATGT GCCACAGCGC TCTCTGGATG ACAACCTGCG ATTTTCGCTG GTATGGCCCC AAGTACATCA CGCACAGCAT
       AGTGTTGCGT GGTTCAGCTT CCGAAGTACA CGGTGTCGCG AGAGACCTAC TGTTGGACGC TAAAAGCGAC CATACCGGGG TTCATGTAGT GCGTGTCGTA

        · H   N   E   E   P   T   D   Y   Q   C   L   E   A   I   K   S   Y   K   D   G   V   S   F   N   P   G   F   P   P   Q   S   C   G
       CCACAATGAG GAACCAACCG ACTACCAGTG CCTCGAAGCC ATCAAGTCAT ACAAGGATGG GGTGAGCTTC AACCCCGGCT TCCCGCCCCA ATCATGTGGC
       GGTGTTACTC CTTGGTTGGC TGATGGTCAC GGAGCTTCGG TAGTTCAGTA TGTTCCTACC CCACTCGAAG TTGGGGCCGA AGGGCGGGGT TAGTACACCG

        Y   G   T   V   T   D   A   E   A   H   I   V   T   V   T   P   H   S   V   K   V   D   E   Y   T   G   E   W   I   D   P   H   F   I .
       TACGGCACCG TGACCGACGC CGAGGCCCAC ATCGTGACCG TGACACCCCA CTCAGTCAAG GTGGACGAGT ACACAGGCGA ATGGATCGAC CCCCACTTCA
       ATGCCGTGGC ACTGGCTGCG GCTCCGGGTG TAGCACTGGC ACTGTGGGGT GAGTCAGTTC CACCTGCTCA TGTGTCCGCT TACCTAGCTG GGGGTGAAGT

        · G   G   R   C   K   G   Q   I   C   E   T   V   H   N   S   T   K   W   F   T   S   S   D   G   E   S   V   C   S   Q   L   F   T .
       TCGGGGGCCG CTGTAAGGGC CAAATCTGCG AGACCGTGCA CAACAGCACC AAGTGGTTTA CGTCATCAGA CGGCGAAAGC GTGTGCAGCC AACTGTTTAC
       AGCCCCCGGC GACATTCCCG GTTTAGACGC TCTGGCACGT GTTGTCGTGG TTCACCAAAT GCAGTAGTCT GCCGCTTTCG CACACGTCGG TTGACAAATG

        · L   V   G   G   I   F   F   S   D   S   E   E   I   T   S   M   G   L   P   E   T   G   I   R   S   N   Y   F   P   Y   I   S   T
       GCTCGTGGGC GGCATCTTCT TTAGCGACAG CGAGGAGATC ACCAGCATGG GCCTCCCGGA GACAGGAATC CGCAGCAACT ACTTTCCGTA CATCAGCACC
       CGAGCACCCG CCGTAGAAGA AATCGCTGTC GCTCCTCTAG TGGTCGTACC CGGAGGGCCT CTGTCCTTAG GCGTCGTTGA TGAAAGGCAT GTAGTCGTGG

        E   G   I   C   K   M   P   F   C   R   K   Q   G   Y   K   L   K   N   D   L   W   F   Q   I   M   D   P   D   L   D   K   T   V   R .
       GAGGGAATCT GTAAGATGCC TTTTTGCCGC AAGCAGGGAT ATAAGCTGAA GAATGACCTG TGGTTCCAGA TCATGGACCC GGACCTGGAC AAGACCGTCC
       CTCCCTTAGA CATTCTACGG AAAAACGGCG TTCGTCCCTA TATTCGACTT CTTACTGGAC ACCAAGGTCT AGTACCTGGG CCTGGACCTG TTCTGGCAGG

        · D   L   P   H   I   K   D   C   D   L   S   S   S   I   I   T   P   G   E   H   A   T   D   I   S   L   I   S   D   V   E   R   I .
       GCGATCTGCC CCACATCAAG GACTGTGATC TGTCATCAAG CATCATCACC CCCGGAGAAC ACGCCACGGA CATCAGCCTC ATCAGCGATG TGGAGCGCAT
       CGCTAGACGG GGTGTAGTTC CTGACACTAG ACAGTAGTTC GTAGTAGTGG GGGCCTCTTG TGCGGTGCCT GTAGTCGGAG TAGTCGCTAC ACCTCGCGTA

        · L   D   Y   A   L   C   Q   N   T   W   S   K   I   E   S   G   E   P   I   T   P   V   D   L   S   Y   L   G   P   K   N   P   G
       CCTCGACTAC GCTCTCTGCC AGAACACATG GAGCAAGATC GAAAGCGGCG AACCCATCAC CCCAGTGGAC CTGAGCTATC TCGGCCCAAA GAACCCCGGC
       GGAGCTGATG CGAGAGACGG TCTTGTGTAC CTCGTTCTAG CTTTCGCCGC TTGGGTAGTG GGGTCACCTG GACTCGATAG AGCCGGGTTT CTTGGGGCCG
```

## FIGURE 19B

```
        V  G  P  V   F  T  I    I  N  G   S  L  H  Y   F  T  S    K  Y  L   R  V  E  L   E  S  P   V  I  P   R  M  E  G .
        GTGGGGCCCG TGTTCACCAT CATCAACGGG AGCCTGCACT ACTTTACAAG CAAGTATCTG CGCGTGGAGC TCGAAAGCCC AGTCATCCCC CGCATGGAGG
        CACCCCGGGC ACAAGTGGTA GTAGTTGCCC TCGGACGTGA TGAAATGTTC GTTCATAGAC GCGCACCTCG AGCTTTCGGG TCAGTAGGGG GCGTACCTCC

        · K  V  A   G  T  R   I  V  R  Q   L  W  D   Q  W  F   P  F  G  E   V  E  I   G  P  N   G  V  L  K   T  K  Q ·
        GGAAGGTGGC CGGGACCCGC ATCGTGCGCC AGCTGTGGGA CCAGTGGTTC CCTTTTGGCG AGGTGGAAAT CGGCCCCAAC GGCGTGCTGA AGACCAAGCA
        CCTTCCACCG GCCCTGGGCG TAGCACGCGG TCGACACCCT GGTCACCAAG GGAAAACCGC TCCACCTTTA GCCGGGGTTG CCGCACGACT TCTGGTTCGT

        · G  Y  K   F  P  L  H   I  I  G   T  G  E   V  D  S  D   I  K  M   E  R  V   V  K  H  W   E  H  P   H  I  E
        AGGATATAAG TTCCCGCTGC ACATCATCGG GACGGGCGAA GTGGACAGCG ATATCAAGAT GGAGCGCGTG GTCAAGCACT GGGAGCACCC ACACATCGAG
        TCCTATATTC AAGGGCGACG TGTAGTAGCC CTGCCCGCTT CACCTGTCGC TATAGTTCTA CCTCGCGCAC CAGTTCGTGA CCCTCGTGGG TGTGTAGCTC

        A  A  Q  T   F  L  K   K  D  D   T  G  E  V   L  Y  Y   G  D  T   G  V  S  K   N  P  V   E  L  V   E  G  W  F ·
        GCTGCTCAGA CCTTTCTCAA GAAGGACGAT ACCGGCGAAG TCCTGTATTA CGGGGATACG GGAGTGAGCA AGAACCCTGT GGAGCTGGTG GAAGGCTGGT
        CGACGAGTCT GGAAAGAGTT CTTCCTGCTA TGGCCGCTTC AGGACATAAT GCCCCTATGC CCTCACTCGT TCTTGGGACA CCTCGACCAC CTTCCGACCA

        · S  G  W   R
        TCAGCGGATG GCGC▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓
        AGTCGCCTAC CGCG▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓

        ▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓
        ▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓▓
```

EP 2 047 861 B1

90

Figure 20: The human codon-optimization of the VSV-G gene (New Jersey serotype) stimulates gene transfer of a 100 X factor

Design of the HIV-1 human antigen for vaccination against AIDS:

A modified HIV-1 GAG may have the following protein sequence:

MASVLSGGELDRWEKIRLRPGGKKKYKLKHIVWASRELERFAVNPGLLETSEGCRQILGQLQPSLQT
GSEELRSLYNTVATLYCVHQRIEVKDTKEALDKIEEEQNKSKKKAQQAAADTNHSSQVSQNYPIVQNL
QGQMVHQAISPRTLNAWVKVVEEKAFSPEVIPMFSALSEGATPQDLNTMLNTVGGHQAAMQMLKETI
NEEAAEWDRLHPVHAGPIAPGQMREPRGSDIAGTTSTLQEQIGWMTNNPPIPVGEIYKRWIILGLNKIV
RMYSPTSILDIRQGPKEPFRDYVDRFYKTLRAEQASQEVKNWMTETLLVQNANPDCKTILKALGPAAT
LEEMMTACQGVGGPGHKARVLAEAMSQVTNSATIMMQRGNFRNQRKTVKCFNCGKEGHIAKNCRA
PRKKGCWKCGKEGHQMKDCTERQANFLGKIWPSHKGRPGNFLQSRPEPTAPPEESFRFGEETTTP
SQKQEPIDKELYPLASLRSLFGND

Segment from the POL polyprotein may be as follows:

KKSVTVLDVGDAYFSVPLDKDFRKYTAFTIPSINNETPGIRYQYNVLPQGWKGSPAIFQSSMTKILEPF
RKQNPDIVIYQYMDDLYVGSDLEIGQHRTEILKEPVHGVY

Segment from the Nef protein may be as follows:

VGFPVRPQVPLRPMTYKAAVDLSHFLKEKGGLEGLIYSQKRQDILDLWVYHTQGYFPDWQNYTPGP
GIRYPLTFGWCFKLVPVDPEKEVLVWKFDSRLAFHHMARELHPEYYAPVKQTLNFDLLKLAGDVESN
PGP

An alternative GAG sequence is provided hereafter, which is the HIV-1 GAG of LAI isolate (clade B):

```
M  G  A  R  A  S  V  L  S  G  G  E  L  D  R  W  E  K  I  R  L  R  P  G  G  K  K
K  Y  K  L  K  H  I  V  W  A  S  R  E  L  E  R  F  A  V  N  P  G  L  L  E  T  S
E  G  C  R  Q  I  L  G  Q  L  Q  P  S  L  Q  T  G  S  E  E  L  R  S  L  Y  N  T
V  A  T  L  Y  C  V  H  Q  R  I  E  I  K  D  T  K  E  A  L  D  K  I  E  E  E  Q
N  K  S  K  K  K  A  Q  Q  A  A  A  D  T  G  H  S  S  Q  V  S  Q  N  Y  P  I  V
Q  N  I  Q  G  Q  M  V  H  Q  A  I  S  P  R  T  L  N  A  W  V  K  V  V  E  E  K
A  F  S  P  E  V  I  P  M  F  S  A  L  S  E  G  A  T  P  Q  D  L  N  T  M  L  N
T  V  G  G  H  Q  A  A  M  Q  M  L  K  E  T  I  N  E  E  A  A  E  W  D  R  V
H  P  V  H  A  G  P  I  A  P  G  Q  M  R  E  P  R  G  S  D  I  A  G  T  T  S  T
L  Q  E  Q  I  G  W  M  T  N  N  P  P  I  P  V  G  E  I  Y  K  R  W  I  I  L  G
L  N  K  I  V  R  M  Y  S  P  T  S  I  L  D  I  R  Q  G  P  K  E  P  F  R  D  Y
V  D  R  F  Y  K  T  L  R  A  E  Q  A  S  Q  E  V  K  N  W  M  T  E  T  L  L
V  Q  N  A  N  P  D  C  K  T  I  L  K  A  L  G  P  A  A  T  L  E  E  M  M  T  A
C  Q  G  V  G  G  P  G  H  K  A  R  V  L  A  E  A  M  S  Q  V  T  N  S  A  T  I
M  M  Q  R  G  N  F  R  N  Q  R  K  I  V  K  C  F  N  C  G  K  E  G  H  I  A  R
N  C  R  A  P  R  K  K  G  C  W  K  C  G  K  E  G  H  Q  M  K  D  C  T  E  R
Q  A  N  F  L  G  K  I  W  P  S  Y  K  G  R  P  G  N  F  L  Q  S  R  P  E  P  T
A  P  P  F  L  Q  S  R  P  E  P  T  A  P  P  E  E  S  F  R  S  G  V  E  T  T  T  P
S  Q  K  Q  E  P  I  D  K  E  L  Y  P  L  T  S  L  R  S  L  F  G  N  D  P  S  S  Q
```

figure 21 (1)

**Nucleotidic encoding amino-acid sequence of the HIV-1 GAG isolate from the LAI isolate**
**(clade B)**

ATGGGTGCGAGAGCGTCAGTATTAAGCGGGGGAGAATTAGATCGATGGGA
AAAAATTCGGTTAAGGCCAGGGGGAAAGAAAAAATATAAATTAAAACATA
TAGTATGGGCAAGCAGGGAGCTAGAACGATTCGCAGTTAATCCTGGCCTG
TTAGAAACATCAGAAGGCTGTAGACAAATACTGGGACAGCTACAACCATC
CCTTCAGACAGGATCAGAAGAACTTAGATCATTATATAATACAGTAGCAA
CCCTCTATTGTGTGCATCAAAGGATAGAGATAAAAGACACCAAGGAAGCT
TTAGACAAGATAGAGGAAGAGCAAAACAAAAGTAAGAAAAAGCACAGCA
AGCAGCAGCTGACACAGGACACAGCAGCCAGGTCAGCCAAAATTACCCTA
TAGTGCAGAACATCCAGGGGCAAATGGTACATCAGGCCATATCACCTAGA
ACTTTAAATGCATGGGTAAAAGTAGTAGAAGAGAAGGCTTTCAGCCCAGA
AGTGATACCCATGTTTTCAGCATTATCAGAAGGAGCCACCCCACAAGATT
TAAACACCATGCTAAACACAGTGGGGGGACATCAAGCAGCCATGCAAATG
TTAAAAGAGACCATCAATGAGGAAGCTGCAGAATGGGATAGAGTGCATCC
AGTGCATGCAGGGCCTATTGCACCAGGCCAGATGAGAGÁACCAAGGGGAA
GTGACATAGCAGGAACTACTAGTACCCTTCAGGAACAAATAGGATGGATG
ACAAATAATCCACCTATCCCAGTAGGAGAAATTTATAAAAGATGGATAAT
CCTGGGATTAAATAAAATAGTAAGAATGTATAGCCCTACCAGCATTCTGG
ACATAAGACAAGGACCAAAAGAACCCTTTAGAGACTATGTAGACCGGTTC
TATAAAACTCTAAGAGCCGAGCAAGCTTCACAGGAGGTAAAAAATTGGAT
GACAGAAACCTTGTTGGTCCAAAATGCGAACCCAGATTGTAAGACTATTT
TAAAAGCATTGGGACCAGCAGCTACACTAGAAGAAATGATGACAGCATGT
CAGGGAGTGGGAGGACCCGGCCATAAGGCAAGAGTTTTGGCTGAAGCAAT
GAGCCAAGTAACAAATTCAGCTACCATAATGATGCAAAGAGGCAATTTTA
GGAACCAAAGAAAGATTGTTAAGTGTTTCAATTGTGGCAAAGAAGGGCAC
ATAGCCAGAAATTGCAGGGCCCCTAGGAAAAAGGGCTGTTGGAAATGTGG
AAAGGAAGGACACCAAATGAAAGATTGTACTGAGAGACAGGCTAATTTTT
TAGGGAAGATCTGGCCTTCCTACAAGGGAAGGCCAGGGAATTTTCTTCAG
AGCAGACCAGAGCCAACAGCCCCACCATTTCTTCAGAGCAGACCAGAGCC
AACAGCCCCACCAGAAGAGAGCTTCAGGTCTGGGGTAGAGACAACAACTC
CCTCTCAGAAGCAGGAGCCGATAGACAAGGAACTGTATCCTTTAACTTCC
CTCAGATCACTCTTTGGCAACGACCCCTCGTCACAATAA

Figure 21(2)

Ag1

Gag | Pol-Nef Fragment

Ag2

Gag | | Pol-Nef Fragment

Ag3

Pol-Nef Fragment | Gag

Ag4

Pol-Nef Fragment | | Gag

Fig 22: Design of the human HIV-1 antigen for vaccination against AIDS

Figure 23 : Principle of TRIP Lentiviral Vectors generation.

**Figure 24 : Principle of U3'deleted Lentiviral Vector**

A

B

**Figure 25 : Schematic representation of the 2 vector plasmids used for TRIP vectors production**

## FIG 25CA

pTRIPDeltaU3-CMV.SIVGag.WPRE
5322 bp

## FIG 25CB.1

```
tggaagggctaattcactcccaacgaagacaagatatccttgatctgtggatctaccacacacaaggc
tacttccctgattagcagaactacacaccagggccagggatcagatatccactgacctttggatggtg
ctacaagctagtaccagttgagccagagaagttagaagaagccaacaaaggagagaacaccagcttgt
tacaacctgtgagcctgcatgggatggatgacccggagagagaagtgttagagtggaggtttgacagc
cgcctagcatttcatcacggtggcccgagagctgcatccggagtacttcaagaactgctgatatcgag
cttgctacaagggactttccgctggggactttccagggaggcgtggcctgggcgggactggggagtg
gcgagccctcagatcctgcatataagcagctgcttttgcctgtactgggtctctctggttagaccag
atctgagcctgggagctctctggctaactagggaacccactgcttaagcctcaataaagcttgccttg
agtgcttcaagtagtgtgtgcccgtctgttgtgtgactctggtaactagagatccctcagaccctttt
agtcagtgtggaaaatctctagcagtggcgcccgaacagggacttgaaagcgaaagggaaaccagagg
agctctctcgacgcaggactcggcttgctgaagcgcgcacggcaagaggcgaggggcggcgactggtg
agtacgccaaaaattttgactagcggaggctagaaggagagagatgggtgcgagagcgtcagtattaa
gcggggggagaattagatcgcgatgggaaaaaattcggttaaggccagggggaaagaaaaaatataaat
taaaacatatagtatgggcaagcagggagctagaacgattcgcagttaatcctggcctgttagaaaca
tcagaaggctgtagacaaatactgggacagctacaaccatcccttcagacaggatcagaagaacttag
atcattatataatacagtagcaaccctctattgtgtgcatcaaaggatagagataaaagacaccaagg
aagctttagacaagatagaggaagagcaaaacaaaagtaagaccaccgcacagcaagcggccgctgat
cttcagacctggaggaggagatatgagggacaattggagaagtgaattatataaatataaagtagtaa
aaattgaaccattaggagtagcacccaccaaggcaaagagaagagtggtgcagagagaaaaaagagca
gtgggaataggagctttgttccttgggttcttgggagcagcaggaagcactatgggcgcagcgtcaat
gacgctgacggtacaggccagacaattattgtctggtatagtgcagcagcagaacaatttgctgaggg
ctattgaggcgcaacagcatctgttgcaactcacagtctggggcatcaagcagctccaggcaagaatc
ctggctgtggaaagatacctaaaggatcaacagctcctggggatttggggttgctctggaaaactcat
ttgcaccactgctgtgccttggaatgctagttggagtaataaatctctggaacagatttggaatcaca
cgacctggatggagtgggacagagaaattaacaattacacaagcttaatacactccttaattgaagaa
tcgcaaaaccagcaagaaaagaatgaacaagaattattggaattagataaatgggcaagtttgtggaa
ttggtttaacataacaaattggctgtggtatataaaattattcataatgatagtaggaggcttggtag
gtttaagaatagttttttgctgtactttctatagtgaatagagttaggcagggatattcaccattatcg
tttcagacccacctcccaaccccgaggggacccgacaggcccgaaggaatagaagaagaaggtggaga
```

## FIG 25CB.2

gagagacagagacagatccattcgattagtgaacggatctcgacggtatcgccgaattcacaaatggc
agtattcatccacaatttttaaaagaaaaggggggattggggggtacagtgcaggggaaagaatagtag
acataatagcaacagacatacaaactaaagaattacaaaaacaaattacaaaaattcaaaattttcgg
gtttattacagggacagcagagatccactttggggcgataagcttgggagttccgcgttacataactt
acggtaaatggcccgcctggctgaccgcccaacgaccccgcccattgacgtcaataatgacgtatgt
tcccatagtaacgccaatagggactttccattgacgtcaatgggtggagtatttacggtaaactgccc
acttggcagtacatcaagtgtatcatatgccaagtacgccccctattgacgtcaatgacggtaaatgg
cccgcctggcattatgcccagtacatgaccttatgggactttcctacttggcagtacatctacgtatt
agtcatcgctattaccatggtgatgcggttttggcagtacatcaatgggcgtggatagcggtttgact
cacggggatttccaagtctccaccccattgacgtcaatgggagtttgttttggcaccaaaatcaacgg
gactttccaaaatgtcgtaacaactccgccccattgacgcaaatgggcggtaggcgtgtacggtggga
ggtctatataagcagagctcgtttagtgaaccgtcagatcgcctggagacgccatccacgctgttttg
acctccatagaagacaccgactctagaggatctgccacc**atg**gtgagaaactccgtcttgtcagggaa
gaaagcagatgaattagaaaaaattaggctacgacccaacggaaagaaaaagtacatgttgaagcatg
tagtatgggcagcaaatgaattagatagatttggattagcagaaagcctgttggagaacaaagaagga
tgtcaaaaaatactttcggtcttagctccattagtgccaacaggctcagaaaatttaaaaagccttta
taatactgtctgcgtcatctggtgcattcacgcagaagagaaagtgaaacacactgaggaagcaaaac
agatagtgcagagacacctagtggtggaaacaggaacaacagaaactatgccaaaaacaagtagacca
acagcaccatctagcggcagaggaggaaattacccagtacaacaaataggtggtaactatgtccacct
gccattaagcccgagaacattaaatgcctgggtaaaattgatagaggaaaagaaatttggagcagaag
tagtgccaggatttcaggcactgtcagaaggttgcacccctatgacattaatcagatgttaaattgt
gtgggagaccatcaagcggctatgcagattatcagagatattataaacgaggaggctgcagattggga
cttgcagcacccacaaccagctccacaacaaggacaacttagggagccgtcaggatcagatattgcag
gaacaactagttcagtagatgaacaaatccagtggatgtacagacaacagaaccccataccagtaggc
aacatttacaggagatggatccaactgggggttgcaaaaatgtgtcagaatgtataacccaacaaacat
tctagatgtaaaacaagggccaaaagagccatttcagagctatgtagacaggttctacaaaagtttaa
gagcagaacagacagatgcagcagtaaagaattggatgactcaaacactgctgattcaaaatgctaac
ccagattgcaagctagtgctgaaggggctgggtgtgaatcccaccctagaagaaatgctgacggcttg
tcaaggagtagggggggccgggacagaaggctagattaatggcagaagccctgaaagaggccctcgcac
cagtgccaatcccttttgcagcagcccaacagagggggaccaagaaagccaattaagtgttggaattgt
gggaaagagggacactctgcaaggcaatgcagagccccaagaagacagggatgctggaaatgtggaaa
aatggaccatgttatggccaaatgcccagacagacaggcgggttttttaggccttggtccatggggaa
agaagccccgcaatttccccatggctcaagtgcatcaggggctgatgccaactgctcccccagaggac
ccagctgtggatctgctaaagaactacatgcagttgggcaagcagcagagagagaaaagcagagagaaag
cagagagaagccttacaaggaggtgacagaggatttgctgcacctcaattctctctttggaggagacc
agtagctcgagctcaagcttcgaattcccgataatcaacctctggattacaaaatttgtgaaagattg
actggtattcttaactatgttgctccttttacgctatgtggatacgctgctttaatgcctttgtatca
tgctattgcttcccgtatggctttcattttctcctccttgtataaatcctggttgctgtctctttatg
aggagttgtggcccgttgtcaggcaacgtggcgtggtgtgcactgtgtttgctgacgcaacccccact
ggttggggcattgccaccacctgtcagctcctttccgggactttcgctttccccctccctattgccac
ggcggaactcatcgccgcctgccttgcccgctgctggacaggggctcggctgttgggcactgacaatt
ccgtggtgttgtcggggaagctgacgtcctttccatggctgctcgcctgtgttgccacctggattctg
cgcgggacgtccttctgctacgtcccttcggccctcaatccagcggaccttccttccgcggcctgct
gccggctctgcggcctcttccgcgtcttcgccttcgccctcagacgagtcggatctcccttgggccg
cctccccgcgtcgacgcgtgaattcggtacctttaagaccaatgacttacaaggcagctgtagatctt
agccacttttaaaagaaaaggggggactggaagggctaattcactcccaacgaagacaagatcgtcg
agagatgctgcatataagcagctgcttttgcttgtactgggtctctctggttagaccagatctgagc
ctgggagctctctggctaactagggaacccactgcttaagcctcaataaagcttgccttgagtgcttc
aagtagtgtgtgcccgtctgttgtgtgactctggtaactagagatccctcagacccttttagtcagtg
tggaaaatctctagcagt

**FIG 25 DA**

**pTRIPDeltaU3-CMV-SIVGag CO-WPRE**
5335 bp

**FIG 25 DB.1**

```
tggaagggctaattcactcccaacgaagacaagatatccttgatctgtggatctaccacacacaaggc
tacttccctgattagcagaactacacaccagggccagggatcagatatccactgacctttggatggtg
ctacaagctagtaccagttgagccagagaagttagaagaagccaacaaaggagagaacaccagcttgt
tacaacctgtgagcctgcatgggatggatgacccggagagagaagtgttagagtggaggtttgacagc
cgcctagcatttcatcacggtggcccgagagctgcatccggagtacttcaagaactgctgatatcgag
cttgctacaagggactttccgctggggggactttccagggaggcgtggcctgggcgggactggggagtg
gcgagccctcagatcctgcatataagcagctgctttttgcctgtactgggtctctctggttagaccag
atctgagcctgggagctctctggctaactagggaacccactgcttaagcctcaataaagcttgccttg
agtgcttcaagtagtgtgtgcccgtctgttgtgtgactctggtaactagagatccctcagacccttttt
agtcagtgtggaaaatctctagcagtggcgcccgaacagggacttgaaagcgaaagggaaaccagagg
agctctctcgacgcaggactcggcttgctgaagcgcgcacggcaagaggcgaggggcggcgactggtg
agtacgccaaaaattttgactagcggaggctagaaggagagagatgggtgcgagagcgtcagtattaa
gcggggggagaattagatcgcgatgggaaaaaattcggttaaggccagggggaaagaaaaaatataaat
taaaacatatagtatgggcaagcagggagctagaacgattcgcagttaatcctggcctgttagaaaca
tcagaaggctgtagacaaatactgggacagctacaaccatcccttcagacaggatcagaagaacttag
atcattatataatacagtagcaaccctctattgtgtgcatcaaaggatagagataaaagacaccaagg
aagctttagacaagatagaggaagagcaaaacaaaagtaagaccaccgcacagcaagcggccgctgat
cttcagacctggaggaggagatatgagggacaattggagaagtgaattatataaatataaagtagtaa
aaattgaaccattaggagtagcacccaccaaggcaaagagaagagtggtgcagagagaaaaaagagca
gtgggaataggagctttgttccttgggttcttgggagcagcaggaagcactatgggcgcagcgtcaat
gacgctgacggtacaggccagacaattattgtctggtatagtgcagcagcagaacaatttgctgaggg
ctattgaggcgcaacagcatctgttgcaactcacagtctggggcatcaagcagctccaggcaagaatc
ctggctgtggaaagatacctaaaggatcaacagctcctggggatttggggttgctctggaaaactcat
ttgcaccactgctgtgccttggaatgctagttggagtaataaatctctggaacagatttggaatcaca
cgacctggatggagtgggacagagaaattaacaattacacaagcttaatacactccttaattgaagaa
tcgcaaaaccagcaagaaaagaatgaacaagaattattggaattagataaatgggcaagtttgtggaa
ttggtttaacataacaaattggctgtggtatataaaattattcataatgatagtaggaggcttggtag
gtttaagaatagtttttgctgtactttctatagtgaatagagttaggcagggatattcaccattatcg
tttcagacccacctcccaaccccgaggggacccgacaggcccgaaggaatagaagaagaaggtggaga
gagagacagagacagatccattcgattagtgaacggatctcgacggtatcgccgaattcacaaatggc
agtattcatccacaatttttaaaagaaaaggggggattggggggtacagtgcaggggaaagaatagtag
```

**FIG 25DB.2**

```
acataatagcaacagacatacaaactaaagaattacaaaaacaaattacaaaaattcaaaattttcgg
gtttattacagggacagcagagatccactttggggcgataagcttgggagttccgcgttacataactt
acggtaaatggcccgcctggctgaccgcccaacgaccccgcccattgacgtcaataatgacgtatgt
tcccatagtaacgccaataggactttccattgacgtcaatgggtggagtatttacggtaaactgccc
acttggcagtacatcaagtgtatcatatgccaagtacgccccctattgacgtcaatgacggtaaatgg
cccgcctggcattatgcccagtacatgaccttatgggactttcctacttggcagtacatctacgtatt
agtcatcgctattaccatggtgatgcggttttggcagtacatcaatgggcgtggatagcggtttgact
cacggggatttccaagtctccaccccattgacgtcaatgggagtttgttttggcaccaaaatcaacgg
gactttccaaaatgtcgtaacaactccgccccattgacgcaaatgggcggtaggcgtgtacggtggga
ggtctatataagcagagctcgtttagtgaaccgtcagatcgcctggagacgccatccacgctgttttg
acctccatagaagacaccgactctagaggatctcgatcggccacc**atg**ggcgtgcgcaacagcgtgct
gagcggcaagaaggccgacgagctggagaagatccgcctgcgccccaacggcaagaagaagtacatgc
tgaagcacgtggtgtgggccgctaacgagctggaccggttcggcctggccgagagcctgctggagaac
aaggagggctgccagaagatcctgagcgtgctggcccctctggtgcccaccggcagcgagaacctgaa
gagcctgtacaacaccgtgtgcgtgatctggtgcatccacgccgaggagaaggtgaagcacaccgagg
aggccaagcagatcgtgcagcgccacctggtggtggagaccggcaccaccgagaccatgcccaagacc
agcaggcccaccgcccctagcagcggcagaggcgggaactaccccgtgcagcagatcggcggcaacta
cgtgcacctgcccctgagccccaggaccctgaacgcctgggtgaagctgatcgaggagaagaagttcg
gcgctgaggtggtgcccggcttccaggccctgagcgagggctgcacccctacgacatcaaccagatg
ctgaactgcgtgggcgaccaccaggccgccatgcagatcatccgcgacatcatcaacgaggaagccgc
cgactgggacctgcagcaccccagcctgcccccagcagggccagctgcgcgagcccagcggctccg
acatcgccggcaccaccagcagcgtcgacgagcagatccagtggatgtaccgccagcagaacccatc
cccgtgggcaacatctaccgccgctggatccagctgggcctgcagaagtgcgtgcgcatgtacaaccc
caccaacatcctggacgtgaagcagggccccaaggagcccttccagagctacgtggaccgcttctaca
agagcctgagggccgagcagaccgatgccgccgtgaagaactggatgacccagaccctgctgatccag
aacgccaaccccgactgcaagctggtgctgaagggcctgggcgtgaaccccaccctggaggagatgct
gaccgcctgccagggcgtgggaggacctggccagaaggccaggctgatggccgaagccctgaaggagg
ccctggcccctgtgcccatccccttcgccgctgcccagcagagggggccctcgcaagcccatcaagtgt
tggaactgcggcaaggagggccacagcgccaggcagtgccgcgctccccgcaggcagggctgctggaa
gtgtgggaagatggaccacgtgatggccaagtgccccgaccgccaggccggcttcctgggcctgggcc
cctgggggaagaagccccgcaacttccctatggcgcaggtgcaccagggcctcatgcctaccgcccct
cccgaggaccctgccgtggacctgctgaagaactacatgcagctgggcaagcagcagcgcgagaagca
gcgcgagagccgcgagaagccctacaaggaggtgaccgaggacctgctgcacctgaacagcctgttcg
gcggagaccagtaatgaactcgagctcaagcttcgaattcccgataatcaacctctggattacaaaat
ttgtgaaagattgactggtattcttaactatgttgctccttttacgctatgtggatacgctgctttaa
tgcctttgtatcatgctattgcttcccgtatggctttcattttctcctccttgtataaatcctggttg
ctgtctctttatgaggagttgtggcccgttgtcaggcaacgtggcgtggtgtgcactgtgtttgctga
cgcaacccccactggttggggcattgccaccacctgtcagctcctttccgggactttcgctttcccc
tccctattgccacggcggaactcatcgccgcctgccttgcccgctgctggacaggggctcggctgttg
ggcactgacaattccgtggtgttgtcggggaagctgacgtcctttccatggctgctcgcctgtgttgc
cacctggattctgcgcgggacgtccttctgctacgtcccttcggccctcaatccagcggaccttcctt
cccgcggcctgctgccggctctgcggcctcttccgcgtcttcgccttcgccctcagacgagtcggatc
tccctttgggccgcctccccgcgtcgacgcgtgaattcggtacctttaagaccaatgacttacaaggc
agctgtagatcttagccactttttaaaagaaaaggggggactggaagggctaattcactcccaacgaa
gacaagatcgtcgagagatgctgcatataagcagctgcttttttgcttgtactgggtctctctggttag
accagatctgagcctgggagctctctggctaactagggaacccactgcttaagcctcaataaagcttg
ccttgagtgcttcaagtagtgtgtgcccgtctgttgtgtgactctggtaactagagatccctcagacc
cttttagtcagtgtggaaaatctctagcagt
```

# N ter

1

mgvmsvlsgkkadelekirlrpngkkkymlkhvvwaaneldrfglaesllenkegcqkilsvlaplvptgsenlkslyntvcviwcihaeekvkhteeakqivqrhlvvetgttetmpk

pool M (1-59)

pool N (49-107)

121

pool O (97-155)

tsrptapssgrggnypvqqiggnyvhlplsprtlnawvklieekkfgaevvpgfqalsegctpydinqmlncvgdhqaamqiirdiineeaadwdlqhpqpapqqgqlrepsgsdiagtt

pool P (145-203)

241

pool Q (193-251)

ssvdeqiqwmyrqqnpipvgniyrrwiqlglqkcvrmynptnildvkqgpkepfqsyvdrfykslraeqtdaavknwmtqtlliqnanpdcklvlkglgvnptleemltacqgvggpgqk

pool R (241-299)

pool S (289-347)

361

pool T (337-395)

arlmaealkealapvpipfaaaqqrgprkpikcwncgkeghsarqcrapr rqgcwkcgkmdhvmakcpdrqagflglgpwgkkpmfpmaqvhqglmptappedpavdllknymqlgkqq

pool U (385-443)

pool V (433-491)

481

rekqresrekpykevtedllhlnslfggdq  C ter

pool W (481-511)

Figure 26: Schematic representation of the SIVmac239 GAG protein divided in 15mer long peptides

| PCR | Oligos | Sequence 5' → 3' |
|---|---|---|
| U5R forward primer | M667 | GGCTAACTAGGGAACCCACTG |
| U5R reverse primer | AASM | GCTAGAGATTTTCCACACTGACTAA |
| U5R 3'end donor probe | LTR FL | CACAACAGACGGGCACACACTACTTGA-FL |
| U5R 5' end donor probe | LTR LC | LC-CACTCAAGGCAAGCTTTATTGAGGC |
| CD3 forward primer | CD3 in 5' | GGCTATCATTCTTCTTCAAGGTA |
| CD3 reverse primer | CD3 in 3' | CCTCTCTTCAGCCATTTAAGTA |
| CD3 3'end donor probe | CD3 FL | GGCTGAAGGTTAGGGATACCAATATTCCTGTCTC-FL |
| CD3 5'end donor probe | CD3 LC | LC-CTAGTGATGGGCTCTTCCCTTGAGCCCTTC |

| Step and number of cycles | | Temperature | duration |
|---|---|---|---|
| 1 cycle | 1: Denaturation | 95°C | 3 min |
| 40 cycles | 2: Denaturation | 95°C | 5 sec |
| | 3: Annealing | 57°C | 10 sec |
| | 4: Elongation | 72°C | 8 sec |

**Table 1: Sequences of primers and probes and Q-PCR program used for vector titration**

**Figure 27** : Scheme of the standardisation plasmid used for building standard curve in Q-PCR vector titration with localization of probes and primers annealing sites.

Figure 28

**Figure 29**

Figure 30

**A** Concentration of IFNa (pg/ml of plasma)

**B** Concentration of TNFa (pg/ml of plasma)

**C** Concentration of IL6 (pg/ml of plasma)

**Figure 31 : Innate cytokines concentration in macaques sera at short times after a single injection of TRIP vector**

**Figure 32 : Individual longitudinal follow-up of the cumulative SIVmac239 GAG specific IFNγ response assayed by ELISPOT.**

Figure 33 : Diversity of the response, reactivity profile with the various pools of SIVmac239 Gag peptides

Figure 34: in vitro sero-neutralization assay
Trip-GFP pseudotyped with VSV-G serotype Indiana (Figure 34A)
Trip-GFP pseudotyped with VSV-G serotype New-Jersey (Figure 34B)

Figure 35 : Reactivity of GAG specific T Cells induced by TRIP vector immunization with AT-2 inactivated SIV mac251.

**Figure 36 : Individual longitudinal follow-up of the cumulative SIVmac251 NEF specific IFNγ response assayed by ELISPOT.**

Figure 37 : Individual plasma viral loads after SIV mac251 challenge (intra-rectal route innoculation, 500 AID50).

**Nucleotidic and amino-acid sequence of the FIV GAG antigen**

```
ATGGGGAATGGACAGGGGCGAGATTGGAAAATGGCCATTAAGAGATGTAG
TAATGTTGCTGTAGGAGTAGGGGGGAAGAGTAAAAAATTTGGAGAAGGGA
ATTTCAGATGGGCCATTAGAATGGCTAATGTATCTACAGGACGAGAACCT
GGTGATATACCAGAGACTTTAGATCAACTAAGGTTGGTTATTTGCGATTT
ACAAGAAAGAAGAGAAAAATTTGGATCTAGCAAAGAAATTGATATGGCAA
TTGTGACATTAAAAGTCTTTGCAGTAGCAGGACTTTTAAATATGACGGTG
TCTACTGCTGCTGCAGCTGAAAATATGTATTCTCAAATGGGATTAGACAC
TAGGCCATCTATGAAAGAAGCAGGTGGAAAAGAGGAAGGCCCTCCACAGG
CATATCCTATTCAAACAGTAAATGGAGTACCACAATATGTAGCACTTGAC
CCAAAAATGGTGTCCATTTTTATGGAAAAGGCAAGAGAAGGACTAGGAGG
TGAGGAAGTTCAACTATGGTTTACTGCCTTCTCTGCAAATTTAACACCTA
CTGACATGGCCACATTAATAATGGCCGCACCAGGGTGCGCTGCAGATAAA
GAAATATTGGATGAAAGCTTAAAGCAATTGACAGCAGAATATGATCGCAC
ACATCCCCCTGATGCTCCCAGACCATTACCCTATTTTACTGCAGCAGAAA
TTATGGGTATAGGATTAACTCAAGAACAACAAGCAGAAGCAAGATTTGCA
CCAGCTAGGATGCAGTGTAGAGCATGGTATCTCGAGGCATTAGGAAAATT
GGCTGCCATAAAAGCTAAGTCTCCTCGAGCTGTGCAGTTAAGACAAGGAG
CTAAGGAAGATTATTCATCCTTTATAGACAGATTGTTTGCCCAAATAGAT
CAAGAACAAAATACAGCTGAAGTTAAGTTATATTTAAAACAGTCATTAAG
CATAGCTAATGCTAATGCAGACTGTAAAAAGGCAATGAGCCACCTTAAGC
CAGAAAGTACCCTAGAAGAAAGTTGAGAGCTTGTCAAGAAATAGGCTCA
CCAGGATATAAAATGCAACTCTTGGCAGAAGCTCTTACAAAAGTTCAAGT
AGTGCAATCAAAAGGATCAGGACCAGTGTGTTTTAATTGTAAAAAACCAG
GACATCTAGCAAGACAATGTAGAGAAGTGAAAAAATGTAATAAATGTGGA
AAACCTGGTCATCTAGCTGCCAAATGTTGGCAAGGAAATAGAAAGAATTC
GGGAAACTGGAAGGCGGGGCGAGCTGCAGCCCCAGTGAATCAAGTGCAGC
AAGCAGTAATGCCATCTGCACCTCCAATGGAGGAGAAACTATTGGATTTG
TAA
```

```
M  G  N  G  Q  G  R  D  W  K  M  A  I  K  R  C  S  N  V  A  V  G  V  G  G  K
S  K  K  F  G  E  G  N  F  R  W  A  I  R  M  A  N  V  S  T  G  R  E  P  G  D  I
P  E  T  L  D  Q  L  R  L  V  I  C  D  L  Q  E  R  R  E  K  F  G  S  S  K  E  I
D  M  A  I  V  T  L  K  V  F  A  V  A  G  L  L  N  M  T  V  S  T  A  A  A  A
E  N  M  Y  S  Q  M  G  L  D  T  R  P  S  M  K  E  A  G  G  K  E  E  G  P  P  Q
A  Y  P  I  Q  T  V  N  G  V  P  Q  Y  V  A  L  D  P  K  M  V  S  I  F  M  E  K
A  R  E  G  L  G  G  E  E  V  Q  L  W  F  T  A  F  S  A  N  L  T  P  T  D  M  A
T  L  I  M  A  A  P  G  C  A  A  D  K  E  I  L  D  E  S  L  K  Q  L  T  A  E  Y
D  R  T  H  P  P  D  A  P  R  P  L  P  Y  F  T  A  A  E  I  M  G  I  G  L  T  Q
E  Q  Q  A  E  A  R  F  A  P  A  R  M  Q  C  R  A  W  Y  L  E  A  L  G  K  L
A  A  I  K  A  K  S  P  R  A  V  Q  L  R  Q  G  A  K  E  D  Y  S  S  F  I  D  R
L  F  A  Q  I  D  Q  E  Q  N  T  A  E  V  K  L  Y  L  K  Q  S  L  S  I  A  N  A
N  A  D  C  K  K  A  M  S  H  L  K  P  E  S  T  L  E  E  K  L  R  A  C  Q  E  I
G  S  P  G  Y  K  M  Q  L  L  A  E  A  L  T  K  V  Q  V  V  Q  S  K  G  S  G  P
V  C  F  N  C  K  K  P  G  H  L  A  R  Q  C  R  E  V  K  K  C  N  K  C  G  K
P  G  H  L  A  A  K  C  W  Q  G  N  R  K  N  S  G  N  W  K  A  G  R  A  A  A
P  V  N  Q  V  Q  Q  A  V  M  P  S  A  P  P  M  E  E  K  L  L  D  L
```

The Glycine residue (G) in position 2 can be mutated or deleted to avoid myristoilation.

Figure 38

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 9955892 A
- WO 0127304 A
- WO 0127300 A
- WO 2006010834 A

## Non-patent literature cited in the description

- **FIELDS.** Virology. 1996, vol. 2, 1889-1893
- **ZENNOU V. et al.** *Cell,* 2000, vol. 101, 173-185
- **ZENOU et al.** *Cell,* 2000
- **ADDO, M. M. ; YU, X. G. ; RATHOD, A. ; COHEN, D. ; ELDRIDGE, R. L. ; STRICK, D. ; JOHNSTON, M. N. ; CORCORAN, C. ; WURCEL, A. G. ; FITZ-PATRICK, C. A. et al.** Comprehensive epitope analysis of human immunodeficiency virus type 1 (HIV-1)-specific T-cell responses directed against the entire expressed HIV-1 genome demonstrate broadly directed responses, but no correlation to viral load. *J Virol,* 2003, vol. 77, 2081-2092
- **ANDRIEU, J. M. ; LU, W.** A dendritic cell-based vaccine for treating HIV infection: background and preliminary results. *J Intern Med,* 2007, vol. 261, 123-131
- **ARHEL, N. J. ; SOUQUERE-BESSE, S. ; MUNIER, S. ; SOUQUE, P. ; GUADAGNINI, S. ; RUTHER-FORD, S. ; PREVOST, M. C. ; ALLEN, T. D. ; CHARNEAU, P.** HIV-1 DNA Flap formation promotes uncoating of the pre-integration complex at the nuclear pore. *Embo J,* 2007, vol. 26, 3025-3037
- **AUTRAN, B. ; CARCELAIN, G. ; COMBADIERE, B. ; DEBRE, P.** Therapeutic vaccines for chronic infections. *Science,* 2004, vol. 305, 205-208
- **AUTRAN, B. ; CARCELAIN, G. ; LI, T. S. ; BLANC, C. ; MATHEZ, D. ; TUBIANA, R. ; KATLAMA, C. ; DEBRE, P. ; LEIBOWITCH, J.** Positive effects of combined antiretroviral therapy on CD4+ T cell homeostasis and function in advanced HIV disease. *Science,* 1997, vol. 277, 112-116
- **ANDREAS BERGTHALER ; NICOLAS U. GERBER ; DORON MERKLER ; EDIT HORVATH ; JUAN CARLOS DE LA TORRE ; DANIEL D. PIN-SCHEWER.** Envelope Exchange for the Generation of Live-Attenuated Arenavirus Vaccines. *3- PloS Pathogens,* vol. 2 (6), e51
- **BETTS, M. R. ; NASON, M. C. ; WEST, S. M. ; DE ROSA, S. C. ; MIGUELES, S. A. ; ABRAHAM, J. ; LEDERMAN, M. M. ; BENITO, J. M. ; GOEPFERT, P. A. ; CONNORS, M. et al.** HIV nonprogressors preferentially maintain highly functional HIV-specific CD8+ T cells. *Blood,* 2006, vol. 107, 4781-4789
- **BRECKPOT, K. ; DULLAERS, M. ; BONEHILL, A. ; VAN MEIRVENNE, S. ; HEIRMAN, C. ; DE GREEF, C. ; VAN DER BRUGGEN, P. ; THIELEMANS, K.** Lentivirally transduced dendritic cells as a tool for cancer immunotherapy. *J Gene Med,* 2003, vol. 5, 654-667
- **BRENCHLEY, J. M. ; PRICE, D. A. ; SCHACKER, T. W. ; ASHER, T. E. ; SILVESTRI, G. ; RAO, S. ; KAZZAZ, Z. ; BORNSTEIN, E. ; LAMBOTTE, O. ; ALTMANN, D. et al.** Microbial translocation is a cause of systemic immune activation in chronic HIV infection. *Nat Med,* 2006, vol. 12, 1365-1371
- **BRIGGS, J. A. ; SIMON, M. N. ; GROSS, I. ; KRAUSSLICH, H. G. ; FULLER, S. D. ; VOGT, V. M. ; JOHNSON, M. C.** The stoichiometry of Gag protein in HIV-1. *Nat Struct Mol Biol,* 2004, vol. 11, 672-675
- **CARRINGTON, M. ; NELSON, G. W. ; MARTIN, M. P. ; KISSNER, T. ; VLAHOV, D. ; GOEDERT, J. J. ; KASLOW, R. ; BUCHBINDER, S. ; HOOTS, K. ; O'BRIEN, S. J.** HLA and HIV-1: heterozygote advantage and B*35-Cw*04 disadvantage. *Science,* 1999, vol. 283, 1748-1752
- **CRONIN J. et al.** Altering the Tropism of Lentiviral Vectors through Pseudotyping. *Curr Gene Ther.,* August 2005, vol. 5 (4), 387-398
- **DAY, C. L. ; KAUFMANN, D. E. ; KIEPIELA, P. ; BROWN, J. A. ; MOODLEY, E. S. ; REDDY, S. ; MACKEY, E. W. ; MILLER, J. D. ; LESLIE, A. J. ; DEPIERRES, C. et al.** PD-1 expression on HIV-specific T cells is associated with T-cell exhaustion and disease progression. *Nature,* 2006, vol. 443, 350-354
- **DELENDA, C.** Lentiviral vectors: optimization of packaging, transduction and gene expression. *J Gene Med,* 2004, vol. 6 (1), 125-138

- **DESPRES P. et al.** *Infect Dis.,* 2005, vol. 191, 207-214
- **DONELLO J.E. et al.** *J. Virol.,* June 1998, vol. 72 (6), 5085-92
- **DULLAERS, M. ; THIELEMANS, K.** From pathogen to medicine: HIV-1-derived lentiviral vectors as vehicles for dendritic cell based cancer immunotherapy. *J Gene Med,* 2006, vol. 8, 3-17
- **ESSLINGER, C. ; CHAPATTE, L. ; FINKE, D. ; MICONNET, I. ; GUILLAUME, P. ; LEVY, F. ; MACDONALD, H. R.** In vivo administration of a lentiviral vaccine targets DCs and induces efficient CD8(+) T cell responses. *J Clin Invest,* 2003, vol. 111, 1673-1681
- **FIRAT, H. ; GARCIA-PONS, F. ; TOURDOT, S. ; PASCOLO, S. ; SCARDINO, A. ; GARCIA, Z. ; MICHEL, M. L. ; JACK, R. W. ; JUNG, G. ; KOSMATOPOULOS, K. et al.** H-2 class I knockout, HLA-A2.1-transgenic mice: a versatile animal model for preclinical evaluation of antitumor immunotherapeutic strategies. *Eur J Immunol,* 1999, vol. 29, 3112-3121
- **FIRAT H. et al.** *The Journal of Gene Medicine,* 2002, vol. 4, 38-45
- **FRANK, I. ; SANTOS, J. J. ; MEHLHOP, E. ; VILLAMIDE-HERRERA, L. ; SANTISTEBAN, C. ; GETTIE, A. ; IGNATIUS, R. ; LIFSON, J. D. ; POPE, M.** Presentation of exogenous whole inactivated simian immunodeficiency virus by mature dendritic cells induces CD4+ and CD8+ T-cell responses. *J Acquir Immune Defic Syndr,* 2003, vol. 34, 7-19
- **FREDERICKSEN B.L. et al.** *J. Virol.,* 1995, vol. 69, 1435-1443
- **GAUDUIN, M. C. ; YU, Y. ; BARABASZ, A. ; CARVILLE, A. ; PIATAK, M. ; LIFSON, J. D. ; DESROSIERS, R. C. ; JOHNSON, R. P.** Induction of a virus-specific effector-memory CD4+ T cell response by attenuated SIV infection. *J Exp Med,* 2006, vol. 203, 2661-2672
- **GIRARD, M. P. ; OSMANOV, S. K. ; KIENY, M. P.** A review of vaccine research and development: the human immunodeficiency virus (HIV). *Vaccine,* 2006, vol. 24, 4062-4081
- **GOULDER, P. J. ; WATKINS, D. I.** HIV and SIV CTL escape: implications for vaccine design. *Nat Rev Immunol,* 2004, vol. 4, 630-640
- **GULICK, R. M. ; MELLORS, J. W. ; HAVLIR, D. ; ERON, J. J. ; MEIBOHM, A. ; CONDRA, J. H. ; VALENTINE, F. T. ; MCMAHON, D. ; GONZALEZ, C. ; JONAS, L. et al.** 3-year suppression of HIV viremia with indinavir, zidovudine, and lamivudine. *Ann Intern Med,* 2000, vol. 133, 35-39
- **HACEIN-BEY-ABINA, S. ; VON KALLE, C. ; SCHMIDT, M. ; MCCORMACK, M. P. ; WULF-FRAAT, N. ; LEBOULCH, P. ; LIM, A. ; OSBORNE, C. S. ; PAWLIUK, R. ; MORILLON, E. et al.** LMO2-associated clonal T cell proliferation in two patients after gene therapy for SCID-X1. *Science,* 2003, vol. 302, 415-419
- **IGLESIAS, M. C. ; MOLLIER, K. ; BEIGNON, A. S. ; SOUQUE, P. ; ADOTEVI, O. ; LEMONNIER, F. ; CHARNEAU, P.** Lentiviral vectors encoding HIV-1 polyepitopes induce broad CTL responses in vivo. *Mol Ther,* 2007, vol. 15, 1203-1210
- **JIN, X. ; BAUER, D. E. ; TUTTLETON, S. E. ; LEWIN, S. ; GETTIE, A. ; BLANCHARD, J. ; IRWIN, C. E. ; SAFRIT, J. T. ; MITTLER, J. ; WEINBERGER, L. et al.** Dramatic rise in plasma viremia after CD8(+) T cell depletion in simian immunodeficiency virus-infected macaques. *J Exp Med,* 1999, vol. 189, 991-998
- **KIEPIELA, P. ; NGUMBELA, K. ; THOBAKGALE, C. ; RAMDUTH, D. ; HONEYBORNE, I. ; MOODLEY, E. ; REDDY, S. ; DE PIERRES, C. ; MNCUBE, Z. ; MKHWANAZI, N. et al.** CD8+ T-cell responses to different HIV proteins have discordant associations with viral load. *Nat Med,* 2007, vol. 13, 46-53
- **KOFF, W. C. ; JOHNSON, P. R. ; WATKINS, D. I. ; BURTON, D. R. ; LIFSON, J. D. ; HASENKRUG, K. J. ; MCDERMOTT, A. B. ; SCHULTZ, A. ; ZAMB, T. J. ; BOYLE, R.** HIV vaccine design: insights from live attenuated SIV vaccines. *Nat Immunol,* 2006, vol. 7, 19-23
- **KOUP, R. A. ; SAFRIT, J. T. ; CAO, Y. ; ANDREWS, C. A. ; MCLEOD, G. ; BORKOWSKY, W. ; FARTHING, C. ; HO, D. D.** Temporal association of cellular immune responses with the initial control of viremia in primary human immunodeficiency virus type 1 syndrome. *J Virol,* 1994, vol. 68, 4650-4655
- **GALLIONE, C.J. ; ROSE, J.K.** *J. Virol.,* vol. 46 (1), 162-169
- **IGLESIAS MC et al.** A single immunization with a minute dose of a lentiviral vector-based vaccine is highly effective protective humoral immunity against West Nile virus. *J. Gene Med.,* March 2006, vol. 8 (3), 265-274
- **IGLESIAS MC et al.** Polyepitopes Induce Broad CTL Responses In Vivo. *Mol Ther.,* June 2007, vol. 15 (6), 1203-10
- **ISIDORO MARTINEZ ; GAIL W. WERTZ.** Biological Differences between Vesicular Stomatitis Virus Indiana and New Jersey Serotype Glycoproteins: Identification of Amino acid Residues Modulating pH-Dependent Infectivity. *The Journal of Virology,* March 2005, vol. 79 (6), 3578-3585
- **MELLORS, J. W.** Closing in on human immunodeficiency virus-1. *Nat Med,* 1996, vol. 2, 274-275

- **MONTINI, E. ; CESANA, D. ; SCHMIDT, M. ; SAN-VITO, F. ; PONZONI, M. ; BARTHOLOMAE, C. ; SERGI SERGI, L. ; BENEDICENTI, F. ; AMBROSI, A. ; DI SERIO, C. et al.** Hematopoietic stem cell gene transfer in a tumor-prone mouse model uncovers low genotoxicity of lentiviral vector integration. *Nat Biotechnol,* 2006, vol. 24, 687-696
- **PALELLA, F. J., JR. ; DELANEY, K. M. ; MOOR-MAN, A. C. ; LOVELESS, M. O. ; FUHRER, J. ; SATTEN, G. A. ; ASCHMAN, D. J. ; HOLMBERG, S. D.** Declining morbidity and mortality among patients with advanced human immunodeficiency virus infection. HIV Outpatient Study Investigators. *N Engl J Med,* 1998, vol. 338, 853-860
- **POZNANSKY, M. ; LEVER, A. ; BERGERON, L. ; HASELTINE, W. ; SODROSKI, J.** Gene transfer into human lymphocytes by a defective human immunodeficiency virus type 1 vector. *J Virol,* 1991, vol. 65, 532-536
- **REIMANN, K. A. ; PARKER, R. A. ; SEAMAN, M. S. ; BEAUDRY, K. ; BEDDALL, M. ; PETERSON, L. ; WILLIAMS, K. C. ; VEAZEY, R. S. ; MONTE-FIORI, D. C. ; MASCOLA, J. R. et al.** Pathogenicity of simian-human immunodeficiency virus SHIV-89.6P and SIVmac is attenuated in cynomolgus macaques and associated with early T-lymphocyte responses. *J Virol,* 2005, vol. 79, 8878-8885
- **NINA F. ROSE ; ANJEANETTE ROBERTS ; LINDA BUONOCORE ; JOHN K. ROSE.** Glycoprotein Exchange Vectors based on Vesicular Stomatitis Virus Allow Effective Boosting and Generation of Neutralizing Antibodies to a Primary Isolate of Human Immunodeficiency Virus Type 1. *The Journal of Virology,* December 2000, vol. 74 (23), 10903-10910
- **NINA F. ROSE ; PRESTON A. MARX ; AMARA LUCKAY ; DOUGLAS F. NIXON ; WALTER J. MORETTO ; SEAN M. DONAHOE ; DAVID MONTEFIORI ; ANJEANETTE ROBERTS ; LINDA BUONOCORE ; JOHN K. ROSE.** An Effective AIDS Vaccine Based on Live Attenuated Vesicular Stomatitis Virus Recombinants. *Cell,* 07 September 2001, vol. 106, 539-549
- **NISHIMURA N. et al.** *PNAS,* 2002, vol. 99, 6755-6760
- **ROSENBERG, E. S. ; ALTFELD, M. ; POON, S. H. ; PHILLIPS, M. N. ; WILKES, B. M. ; ELDRIDGE, R. L. ; ROBBINS, G. K. ; D'AQUILA, R. T. ; GOULDER, P. J. ; WALKER, B. D.** Immune control of HIV-1 after early treatment of acute infection. *Nature,* 2000, vol. 407, 523-526
- **SAAG, M. S.** Use of virologic markers in clinical practice. *J Acquir Immune Defic Syndr Hum Retrovirol,* 1997, vol. 16 (1), 3-13
- **SACHA, J. B. ; CHUNG, C. ; RAKASZ, E. G. ; SPENCER, S. P. ; JONAS, A. K. ; BEAN, A. T. ; LEE, W. ; BURWITZ, B. J. ; STEPHANY, J. J. ; LOF-FREDO, J. T. et al.** Gag-specific CD8+ T lymphocytes recognize infected cells before AIDS-virus integration and viral protein expression. *J Immunol,* 2007, vol. 178, 2746-2754
- **STEVEN AC. ; SPEAR PG.** Viral Glycoproteins and an Evolutionary Conundrum. *Science,* 14 July 2006, vol. 313 (5784), 177-178
- **TONKS, A.** Quest for the AIDS vaccine. *Bmj,* 2007, vol. 334, 1346-1348
- **TRKOLA, A. ; KUSTER, H. ; RUSERT, P. ; JOOS, B. ; FISCHER, M. ; LEEMANN, C. ; MANRIQUE, A. ; HUBER, M. ; REHR, M. ; OXENIUS, A. et al.** Delay of HIV-1 rebound after cessation of antiretroviral therapy through passive transfer of human neutralizing antibodies. *Nat Med,* 2005, vol. 11, 615-622
- **VANDENDRIESSCHE T. et al.** *Blood,* 01 August 2002, vol. 100 (3), 813-822
- **VARGAS, J., JR. ; GUSELLA, G. L. ; NAJFELD, V. ; KLOTMAN, M. E. ; CARA, A.** Novel integrase-defective lentiviral episomal vectors for gene transfer. *Hum Gene Ther,* 2004, vol. 15, 361-372
- **WEBER, J.** The pathogenesis of HIV-1 infection. *Br Med Bull,* 2001, vol. 58, 61-72
- **WEI, X. ; GHOSH, S. K. ; TAYLOR, M. E. ; JOHNSON, V. A. ; EMINI, E. A. ; DEUTSCH, P. ; LIFSON, J. D. ; BONHOEFFER, S. ; NOWAK, M. A. ; HAHN, B. H. et al.** Viral dynamics in human immunodeficiency virus type 1 infection. *Nature,* 1995, vol. 373, 117-122
- **WISEMAN, R. W. ; WOJCECHOWSKYJ, J. A. ; GREENE, J. M. ; BLASKY, A. J. ; GOPON, T. ; SOMA, T. ; FRIEDRICH, T. C. ; O'CONNOR, S. L. ; O'CONNOR, D. H.** Simian immunodeficiency virus SIVmac239 infection of major histocompatibility complex-identical cynomolgus macaques from Mauritius. *J Virol,* 2007, vol. 81, 349-361
- **YEE J. et al.** *Proc. Natl. Acad. Sci. USA,* 1994, vol. 91, 9564-9568
- **ZAREI, S. ; ABRAHAM, S. ; ARRIGHI, J. F. ; HALLER, O. ; CALZASCIA, T. ; WALKER, P. R. ; KUNDIG, T. M. ; HAUSER, C. ; PIGUET, V.** Lentiviral transduction of dendritic cells confers protective antiviral immunity in vivo. *J Virol,* 2004, vol. 78, 7843-7845
- **ZENNOU, V. ; PETIT, C. ; GUETARD, D. ; NERH-BASS, U. ; MONTAGNIER, L. ; CHARNEAU, P.** HIV-1 genome nuclear import is mediated by a central DNA flap. *Cell,* 2000, vol. 101, 173-185
- **ZUFFEREY, R. ; DONELLO, J. E. ; TRONO, D. ; HOPE, T. J.** Woodchuck hepatitis virus posttranscriptional regulatory element enhances expression of transgenes delivered by retroviral vectors. *J Virol,* 1999, vol. 73, 2886-2892

- **ZUFFEREY, R. ; DULL, T. ; MANDEL, R. J. ; BUK-OVSKY, A. ; QUIROZ, D. ; NALDINI, L. ; TRONO, D.** Self-inactivating lentivirus vector for safe and efficient in vivo gene delivery. *J Virol,* 1998, vol. 72, 9873-9880